# EUROPEAN PATENT APPLICATION

(11) **EP 3 943 488 A1**
(43) Date of publication of application: **26.01.2022**
(21) Application number: 20777468.8
(22) Date of filing: 20.03.2020
(51) Int. Cl.: C07D 277/82, C07D 471/04, A61K 31/428, A61K 31/437, A61P 31/00

(54) **SUBSTITUTED HETEROCYCLIC AMIDE COMPOUND AND PREPARATION METHOD THEREFOR AND PHARMACEUTICAL USE THEREOF**

(30) Priority: 22.03.2019 CN 201910221799
(71) Applicant: Genfleet Therapeutics (Shanghai) Inc., Pudong District Shanghai 201203 (CN); Zhejiang Genfleet Therapeutics Co., Ltd., Shaoxing Binhai New City, Zhejiang 312000 (CN)
(72) Inventor: ZHOU, Fusheng, Shanghai 201203 (CN); XU, Xiaoming, Shanghai 201203 (CN); ZHANG, Leitao, Shanghai 201203 (CN); LIU, Zhubo, Shanghai 201203 (CN); HU, Gang, Shanghai 201203 (CN); DING, Qian, Shanghai 201203 (CN); XIE, Fubo, Shanghai 201203 (CN); ZHENG, Biao, Shanghai 201203 (CN); LV, Qiang, Shanghai 201203 (CN); LAN, Jiong, Shanghai 201203 (CN)
(74) Representative: Abel & Imray LLP
(86) International application number: PCT/CN2020/080306
(87) International publication number: WO 2020/192562

(57) **Abstract**

Provided are a substituted heterocyclic amide compound having a selective inhibitory effect on RIPK1, and a pharmaceutically acceptable salt, a stereoisomer, a solvate or a prodrug thereof, and a pharmaceutical composition containing the compound, and the use of same in the preparation of a drug for treating a RIPK1-related diseases or conditions.

## Description

### Technical Field

The present invention relates to the technical field of medicine, in particular, to a substituted heterocyclic amide compound, and its use as a RIPK1 inhibitor as well as a pharmaceutical composition prepared therefrom.

### Background of the Invention

Receptor interacting protein 1 (RIP1) kinase is a serine/threonine protein kinase of the TKL family involved in innate immune signaling. RIP1 kinase is a protein containing RHIM domain with an N-terminal kinase domain and a C-terminal death domain ((2005) Trends Biochem.Sci.30,151-159). The RIP1 death domain mediates an interaction with another protein containing a death domain, including Fas and TNFR-1 ((1995) Cell81 513-523), TRAIL-R1 and TRAIL-R2 ((1997) Immunity 7,821-830) and TRADD ((1996) Immunity 4,387-396), while the RHIM domain is critical for binding to another protein containing a RHIM domain, such as, TRIF ((2004) Nat Immunol.5,503-507), DAI ((2009) EMBO Rep.10,916-922) and RIP3 ((1999) J.Biol.Chem.274,16871-16875), (1999) Curr.Biol.9,539-542). A variety of effects are achieved through these interactions.

The effect of RIP1 on cell signaling has been evaluated under virious conditions [including TLR3 ((2004) Nat Immunol.5,503-507), TLR4 ((2005) J.Biol.Chem.280,36560-36566), TRAIL (Cell Signal. 2015Feb; 27(2):306-14), FAS((2004) J.Biol.Chem.279,7925-7933)], but is best interpreted in a signaling mediated downstream the death receptor TNFR1 ((2003)Cell 114,181-190). TNFR adaption is achieved through TNF, resulting in oligomerization, which recruits various proteins, including linear K63 linked polyubiquitinated RIP1 ((2006) Mol.Cell22,245-257), TRAF2/5 ((2010) J.Mol.Biol.396,528-539), TRADD ((2008) Nat.Immunol.9,1037-1046) and cIAPs ((2008) Proc.Natl.Acad.Sci.USA.105,11778-11783), to the cytoplasmic tail of the receptor. A RIP1-dependent complex acting as a scaffold protein (i.e., non-kinase-dependent) is called complex I, which provides a platform for pro-survival signaling pathway by activating the NFκB and MAP kinase pathways ((2010) Sci.Signal.115, re4). Moreover, in a condition promoting the RIP1 deubiquitination, TNF binding to its receptor (inhibited by, such as, A20 and CYLD proteins or cIAP) will result in internalization of the receptor and formation of a complex II or DISC (death-inducing signaling complex) ((2011) Cell Death Dis.2,e230). Formation of DISC (including RIP1, TRADD, FADD and Caspase 8) results in activation of Caspase 8, and starts a pogrammed apoptosis cell death in a non-RIP1 kinase-dependent manner ((2012) FEBS J 278,877-887). Apoptosis is a static form of cell death to a great extent, which is involved in routine processes such as development and cell homeostasis.

In a condition under which DISC is formed and RIP3 is expressed, but apoptosis is inhibited (such as, deleting FADD/Caspase 8, inhibiting Caspase or infected by virus), it is possible that threre is a third RIP1 kinase dependency. At present, RIP3 can enter such a compex, and is phosphorylated through RIP1, and starts a Caspaseindependent programmed necrosis apoptosis through activation of MLKL and PGAM5 ((2012) Cell 148,213-227); ((2012) Cell 148,228-243); ((2012) Proc.Natl.Acad.Sci.USA.109, 5322-5327). In contrast to apoptosis, programmed necrosis (not to be confused with non-programmed passive necrosis) results in release of a risk-associated molecular pattern (DAMP) from the cell. These DAMP is capable of provding "risk signal" to surrounding cells and tissues, triggering a proinflammatory response including inflammasome activation, cytokine production, and cell recruitment ((2008 Nat.Rev.Immunol 8,279-289).

Abnormal regulation of programmed cell death mediated by RIP1 kinase has been demonstrated to be associated with various types of inflammation by using RIP3 gene knockout mice (in which the RIP1-mediated programmed necrosis is completely blocked) and Necrostin-1 (a tool inhibitor of RIP1 kinase activity with poor oral bioavailability). RIP3 knockout mice have been shown to be protective against inflammatory bowel diseases (including ulcerative colitis and Crohn's disease) ((2011) Nature 477,330-334), psoriasis ((2011) Immunity 35,572-582), retinal detachment induced photoreceptor necrosis ((2010) PNAS 107, 21695-21700), retinitis pigmentosa ((2012) Proc.Natl.Acad.Sci.,109:36,14598-14603), bombesin induced acute pancreatitis ((2009) Cell 137,1100-1111), and sepsis/systemic inflammatory response syndrome (SIRS) ((2011) Immunity 35,908-918). Necrostin-1 has been shown to be effective in alleviating ischemic brain injury ((2005) Nat.Chem.Biol.1,112-119), retinal ischemia/reperfusion injury ((2010) J.Neurosci.Res.88, 1569-1576), Huntington's disease ((2011) Cell Death Dis.2e115), renal ischemia reperfusion injury ((2012) Kidney Int.81,751-761), cisplatin-induced renal injury ((2012) Ren.Fail.34,373-377), and traumatic brain injury ((2012) Neurochem.Res.37,1849-1858). Other diseases or conditions regulated at least in part by RIP1-dependent apoptosis, necrosis, or cytokine production include malignancies of the blood and solid organs ((2013) Genes Dev.27:1640-1649), bacterial and viral infections ((2014) Cell Host&Microbe 15,23-35) (including but not limited to tuberculosis and influenza) ((2013) Cell 153,1-14))), and lysosomal storage disorders (especially, Gaucher disease, Nature Medicine Advance Online Publication, January 19, 2014, doi:10.1038/nm.3449).

Provided is an effective and selective small molecule RIP1 kinase activity inhibitor, which is capable of blocking RIP1-dependent cell necrosis, and thereby providing therapeutic effect for a disease or even associated with DAMP, cell death and/or inflammation.

### Summary of the Invention

The present invention provides a substituted heterocyclic amide compound, as a RIPK1 inhibitor, which is advantageous for its high activity, high selectivity and low toxic/side effect and the like.

In one respect, the present invention provides a benzothiazole-substituted heterocyclic amide compound, or a pharmaceutically acceptable salt, stereoisomer, solvate or prodrug thereof, the compound has a structure as represented by formula (I): 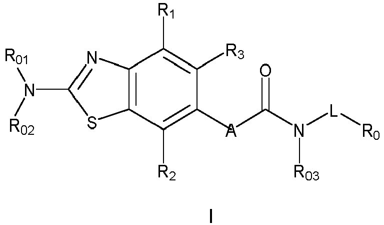 wherein,
R₀ is a substituted or unsubstituted C₆₋₁₄ aryl, or substituted or unsubstituted C₅₋₁₄ heteroaryl;
R₁, R₂, R₃ are each independently hydrogen, hydroxy, halo, nitro, substituted or unsubstituted C₁₋₁₀ alkyl, substituted or unsubstituted C₁₋₁₀ alkoxy, substituted or unsubstituted C₃₋₂₀ cycloalkyl, -(CH₂)ᵤ-C₃₋₆ monocyclic heterocyclyl, -(CH₂)ᵤ-phenyl, -(CH₂)ᵤ-5- or 6-membered monoheteroaryl, -(CH₂)ᵤ-C₃₋₈ monocyclic cycloalkyl, - SO₂C₁₋₆ alkyl, -(CH₂)ᵤ-NRₐ₀R_{b0}, -(CH₂)ᵤ-C(O)NRₐ₀R_{b0}, -C(O)C₁₋₆ alkyl, -C(O)OC₁₋₆ alkyl; wherein the phenyl, C₃₋₈ monocyclic cycloalkyl, C₃₋₆ monocyclic heterocyclyl, 5- or 6-membered monoheteroaryl is optionally substituted by 1, 2 or 3 substituent(s) selected from the group consisting of halo, cyano, C₁₋₃ alkyl, C₁₋₃ alkoxy and C₃₋₆ monocyclic cycloalkyl; u is 0, 1, 2, 3 or 4; Rₐ₀, R_{b0} are each independently hydrogen or C₁₋₃ alkyl, or Rₐ₀ and R_{b0} together with the nitrogen atom attached thereto form a C₃₋₈ monocyclic heterocyclyl, the C₃₋₈ monocyclic heterocyclyl is optionally substituted by 1, 2 or 3 substituent(s) selected from the group consisting of halo or C₁₋₃ alkyl;

A has a structure as represented by formula (a) or formula (b) 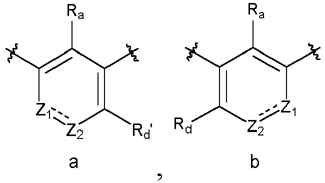 wherein Z₁, Z₂ and " 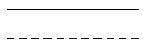 " are selected from the group consisting of:
(a1) Z₁ is CR_{b}, Z₂ is N, and " 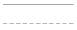 " is a double bond; wherein R_{b} is hydrogen, halo, substituted or unsubstituted C₁₋₁₀ alkyl, substituted or unsubstituted C₁₋₁₀ alkoxy or NRₐ₀R_{b0};
(b1) Z₁ is C(O), Z₂ is NR_{c}, and " 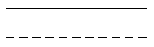 " is a single bond; wherein R_{c} is hydrogen or substituted or unsubstituted C₁₋₁₀ alkyl;
(c1) Z₁ is CR_{b}, Z₂ is CR_{c}, and " 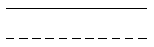 " is a single bond or a double bond; wherein R_{b}, R_{c} together with the ring attached thereto form a substituted or unsubstituted 9- or 10-membered biheteroaryl fused ring;
(d1) Z₁ is NR_{b}, Z₂ is CR_{c}, and " 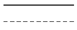 " is a single bond; wherein R_{b}, R_{c} together with the ring attached thereto form a substituted or unsubstituted 9- or 10-membered biheteroaryl fused ring;
(e1) Z₁ is CR_{b}, Z₂ is NR_{c}, and " 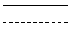 " is a single bond; wherein R_{b}, R_{c} together with the ring attached thereto form a substituted or unsubstituted 9- or 10-membered biheteroaryl fused ring;
   Rₐ, R_{d} are each independently hydrogen, halo, substituted or unsubstituted C₁₋₁₀ alkyl, substituted or unsubstituted C₁₋₁₀ alkoxy or NRₐ₀R_{b0};
   R₀₁, R₀₂ are each independently hydrogen, -C(O)C₁₋₆ alkyl or -C(O)C₃₋₈ monocyclic cycloalkyl;
   L is a bond, -(CR₁₁R₁₂)ₜ₁-(CR₂₁R₂₂)ₜ₂-(CR₃₁R₃₂)ₜ₃-(CR₄₁R₄₂)ₜ₄-(O)ₜ₅ or - (CR₁₁R₁₂)ₜ₁-(CR₂₁R₂₂)ₜ₂-(NR₃₁)ₜ₃-(CR₄₁R₄₂)ₜ₄-(O)ₜ₅; wherein t1, t2, t3, t4, t5 are each independently 0 or 1;
   R_{d}', R₀₃, R₁₁, R₁₂, R₂₁, R₂₂, R₃₁, R₃₂, R_{41,} R₄₂ are selected from the combination selected from the group consisting of:
(a2) R_{d}' are each independently hydrogen, halo, substituted or unsubstituted C₁₋₁₀ alkyl, substituted or unsubstituted C₁₋₁₀ alkoxy or NRₐ₀R_{b0};
   R₀₃ is hydrogen, or substituted or unsubstituted C₁₋₁₀ alkyl;
   R₁₁, R₁₂, R₄₁, R₄₂ are each independently hydrogen, halo, hydroxy, hydroxymethyl, hydroxyethyl or C₁₋₃ alkyl;
   R₂₁, R₂₂, R₃₁, R₃₂ are each independently hydrogen, halo, hydroxy, hydroxymethyl, hydroxyethyl or C₁₋₃ alkyl;
(b2) R_{d}' are each independently hydrogen, halo, substituted or unsubstituted C₁₋₁₀ alkyl, substituted or unsubstituted C₁₋₁₀ alkoxy or NRₐ₀R_{b0};
   R₀₃ is hydrogen, or substituted or unsubstituted C₁₋₁₀ alkyl;
   R₁₁, R₁₂, R₄₁, R₄₂ are each independently hydrogen, halo, hydroxy, hydroxymethyl, hydroxyethyl or C₁₋₃ alkyl;
   R₃₁, R₃₂ are each independently hydrogen, halo, hydroxy, hydroxymethyl, hydroxyethyl or C₁₋₃ alkyl;
   R₂₁, R₂₂ together with the carbon atom attached thereto form a substituted or unsubstituted C₃₋₈ monocyclic cycloalkyl, or substituted or unsubstituted C₃₋₆ monocyclic heterocyclyl;
(c2) R_{d}' are each independently hydrogen, halo, substituted or unsubstituted C₁₋₁₀ alkyl, substituted or unsubstituted C₁₋₁₀ alkoxy or NRₐ₀R_{b0};
   R₀₃ is hydrogen, or substituted or unsubstituted C₁₋₁₀ alkyl;
   R₁₁, R₁₂, R₄₁, R₄₂ are each independently hydrogen, halo, hydroxy, hydroxymethyl, hydroxyethyl or C₁₋₃ alkyl;
   R₂₁, R₂₂ are each independently hydrogen, halo, hydroxy, hydroxymethyl, hydroxyethyl or C₁₋₃ alkyl;
   R₃₁, R₃₂ together with the carbon atom attached thereto form a substituted or unsubstituted C₃₋₈ monocyclic cycloalkyl, or substituted or unsubstituted C₃₋₆ monocyclic heterocyclyl;
(d2) R_{d}' are each independently hydrogen, halo, substituted or unsubstituted C₁₋₁₀ alkyl, substituted or unsubstituted C₁₋₁₀ alkoxy or NRₐ₀R_{b0};
   R₀₃ is hydrogen, or substituted or unsubstituted C₁₋₁₀ alkyl;
   R₁₁, R₁₂, R₄₁, R₄₂ are each independently hydrogen, halo, hydroxy, hydroxymethyl, hydorxyethyl or C₁₋₃ alkyl;
   R₂₂, R₃₂ are each independently hydrogen, halo, hydroxy, hydroxymethyl, hydorxyethyl or C₁₋₃ alkyl;
   R₂₁, R₃₁ together with the carbon atom attached thereto form a substituted or unsubstituted C₃₋₈ monocyclic cycloalkyl, or substituted or unsubstituted C₃₋₆ monocyclic heterocyclyl;
(e2) R_{d}' are each independently hydrogen, halo, substituted or unsubstituted C₁₋₁₀ alkyl, substituted or unsubstituted C₁₋₁₀ alkoxy or NRₐ₀R_{b0};
   R₁₁, R₁₂, R₄₁, R₄₂ are each independently hydrogen, halo, hydroxy, hydroxymethyl, hydroxyethyl or C₁₋₃ alkyl;
   R₂₁, R₂₂, R₃₂ are each independently hydrogen, halo, hydroxy, hydroxymethyl, hydroxyethyl or C₁₋₃ alkyl;
   R₀₃ and R₃₁ are linked together to form a substituted or unsubstituted C₃₋₈ monocyclic heterocyclyl;
(f2) R_{d}' are each independently hydrogen, halo, substituted or unsubstituted C₁₋₁₀ alkyl, substituted or unsubstituted C₁₋₁₀ alkoxy or NRₐ₀R_{b0};
   R₁₁, R₁₂, R₄₁, R₄₂ are each independently hydrogen, halo, hydroxy, hydroxymethyl, hydroxyethyl or C₁₋₃ alkyl;
   R₂₂, R₃₁, R₃₂ are each independently hydrogen, halo, hydroxy, hydroxymethyl, hydroxyethyl or C₁₋₃ alkyl;
   R₀₃ and R₂₁ are linked together to form a substituted or unsubstituted C₃₋₈ monocyclic heterocyclyl;
(g2) R₁₁, R₁₂, R₄₁, R₄₂ are each independently hydrogen, halo, hydroxy, hydroxymethyl, hydroxyethyl or C₁₋₃ alkyl;
   R₂₁, R₂₂, R₃₁, R₃₂ are each independently hydrogen, halo, hydroxy, hydroxymethyl, hydroxyethyl or C₁₋₃ alkyl;
   R_{d}' and R₀₃ are linked together to form a substituted or unsubstituted 4- to 7-membered oxo-substituted heterocyclyl;
(h2) R_{d}' are each independently hydrogen, halo, substituted or unsubstituted C₁₋₁₀ alkyl, substituted or unsubstituted C₁₋₁₀ alkoxy or NRₐ₀R_{b0};
   R₀₃ is hydrogen or substituted or unsubstituted C₁₋₁₀ alkyl;
   R₁₂, R₄₁, R₄₂ are each independently hydrogen, halo, hydroxy, hydroxymethyl, hydroxyethyl or C₁₋₃ alkyl;
   R₂₁, R₂₂, R₃₂ are each independently hydrogen, halo, hydroxy, hydroxymethyl, hydroxyethyl or C₁₋₃ alkyl;
   R₁₁ and R₃₁ are linked together to form a substituted or unsubstituted C₃₋₈ monocyclic cycloalkyl, or substituted or unsubstituted C₃₋₆ monocyclic heterocyclyl;
in the above groups, the "substituted" means that 1, 2, 3 or 4 hydrogen atom(s) in the group are each independently substituted by a substituent selected from the group S consisting of: hydroxy, halo, nitro, oxo, C₁₋₆ alkyl, hydroxy-substituted C₁₋₆ alkyl, benzyl, -S-C₁₋₆ alkyl, -S-halo C₁₋₆ alkyl, -(CRₐ₁R_{b1})ᵤ-cyano, -(CRₐ₁R_{b1})ᵤ-C₁₋₆ alkoxy, - (CRₐ₁R_{b1})ᵤ-halo C₁₋₆ alkoxy, -(CRₐ₁R_{b1})ᵤ-halo C₁₋₆ alkyl, -(CRₐ₁R_{b1})ᵤ-C₃₋₆ monocyclic heterocyclyl, -(CRₐ₁R_{b1})ᵤ-C₃₋₈ monocyclic cycloalkyl, -(CRₐ₁R_{b1})ᵤ-phenyl, - (CRₐ₁R_{b1})ᵤ-5- or 6-membered monoheteroaryl, -(CRₐ₁R_{b1})ᵤ-O-(CRₐ₂R_{b2})ᵥ-halo C₁₋₆ alkyl, -(CRₐ₁R_{b1})ᵤ-O-(CRₐ₂R_{b2})ᵥ-C₃₋₈ monocyclic cycloalkyl, -(CRₐ₁R_{b1})ᵤ-O-(CRₐ₂R_{b2})ᵥ-C₃₋₆ monocyclic heterocyclyl, -(CRₐ₁R_{b1})ᵤ-O-(CRₐ₂R_{b2})ᵥ-phenyl, - (CRₐ₁R_{b1})ᵤ-O-(CRₐ₂R_{b2})ᵥ-5- or 6-membered monoheteroaryl, -(CRₐ₁R_{b1})ᵤ-S-(CRₐ₂R_{b2})ᵥ-phenyl, -(CRₐ₁R_{b1})ᵤ-SO₂-(CRₐ₂R_{b2})ᵥ-phenyl, -(CRₐ₁R_{b1})ᵤ-O-C(O)NRₐ₀R_{b0}, -(CRₐ₁R_{b1})ᵤ-O-(CRₐ₂R_{b2})ᵥ-C₁₋₆ alkoxy, -(CRₐ₁R_{b1})ᵤ-O-(CRₐ₂R_{b2})ᵥOH, -(CRₐ₁R_{b1})ᵤ-SO₂C₁₋₆alkyl, -(CRₐ₁R_{b1})ᵤ-SO₂NRₐ₀R_{b0}, -(CRₐ₁R_{b1})ᵤ-C(O)NRₐ₀R_{b0}, -(CRₐ₁R_{b1})ᵤ-NRₐ₀R_{b0}, -(CRₐ₁R_{b1})ᵤ-C(O)C₁₋₆ alkyl, -C(O)OC₁₋₆ alkyl, NRₐ₀C(O)-(CRₐ₁R_{b1})ᵤ-NRₐ₀R_{b0}, NRₐ₀C(O)-(CRₐ₁R_{b1})ᵤOH, NRₐ₀C(O)-halo C₁₋₆ alkyl; wherein the C₃₋₈ monocyclic cycloalkyl, C₃₋₆ monocyclic heterocyclyl, phenyl, 5- or 6-membered monoheteroaryl is optionally substituted by 1, 2 or 3 substituent(s) selected from the group consisting of hydroxy, hydroxymethyl, hydroxyethyl, halo, cyano, cyanomethyl, cyanoethyl, C₁₋₃ alkyl, C₁₋₃ alkoxy and C₃₋₆ monocyclic cycloalkyl;
u, v are each independently 0, 1, 2, 3 or 4;
   Rₐ₀, R_{b0} are each independently hydrogen or C₁₋₃ alkyl; or Rₐ₀, R_{b0} together with the nitrogen atom attached thereto form a C₃₋₈ monocyclic heterocyclyl, the C₃₋₈ monocyclic heterocyclyl is optionally substituted by 1, 2 or 3 substituent(s) selected from the group consisting of halo or C₁₋₃ alkyl;
   Rₐ₁, R_{b1}, Rₐ₂, R_{b2} are the same or different, and are each independently hydrogen, hydroxyl or C₁₋₃ alkyl.

In another respect, the present invention provides a pyridotriazole-substituted heterocyclic amide compoud or a pharmaceutically acceptable salt, stereoisomer, solvate or prodrug thereof, the compound has a structure as represented by formula (II): 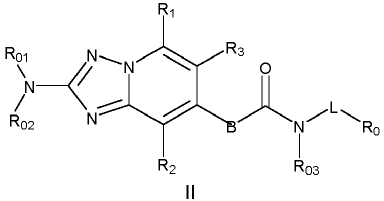 wherein,
R₀ is a substituted or unsubstituted C₆₋₁₄ aryl, or a substituted or unsubstituted C₅₋₁₄ heteroaryl;
R₁, R₂, R₃ are each independently hydrogen, hydroxy, halo, nitro, substituted or unsubstituted C₁₋₁₀ alkyl, substituted or unsubstituted C₁₋₁₀ alkoxy, substituted or unsubstituted C₃₋₂₀ cycloalkyl, -(CH₂)ᵤ-C₃₋₆ monocyclic heterocyclyl, -(CH₂)ᵤ-phenyl, -(CH₂)ᵤ-5- or 6-membered monoheteroaryl, -(CH₂)ᵤ-C₃₋₈ monocyclic cycloalkyl, - SO₂C₁₋₆ alkyl, -(CH₂)ᵤ-NRₐ₀R_{b0}, -(CH₂)ᵤ-C(O)NRₐ₀R_{b0}, -C(O)C₁₋₆ alkyl, -C(O)OC₁₋₆ alkyl; wherein the phenyl, C₃₋₈ monocyclic cycloalkyl, C₃₋₆ monocyclic heterocyclyl, 5- or 6-membered monoheteroaryl is optionally substituted by 1, 2 or 3 substituent(s) selected from the group consisting of halo, cyano, C₁₋₃ alkyl, C₁₋₃ alkoxy and C₃₋₆ monocyclic cycloalkyl; u is 0, 1, 2, 3 or 4; Rₐ₀, R_{b0} are each independently hydrogen or C₁₋₃ alkyl, or Rₐ₀ and R_{b0} together with the nitrogen atom attached thereto form a C₃₋₈ monocyclic heterocyclyl, the C₃₋₈ monocyclic heterocyclyl is optionally substituted by 1, 2 or 3 substituent(s) selected from the group consisting of halo or C₁₋₃ alkyl;
B has a structure as represented by formula (II-a) or formula (II-b) 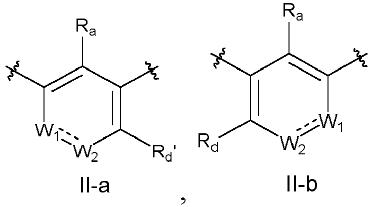 wherein W₁, W₂ and " 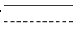 " are selected from the group consisting of:
   (a1) W₁ is C(O), W₂ is NR_{c}, and " 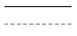 " is a single bond; wherein R_{c} is hydrogen, or substituted or unsubstituted C₁₋₁₀ alkyl;
   (b1) W₁ is CR_{b}, W₂ is CR_{c}, and " 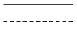 " is a single bond or a double bond; wherein R_{b}, R_{c} together with the ring attached thereto form a substituted or unsubstituted 9- or 10-membered biheteroaryl fused ring;
   (c1) W₁ is NR_{b}, W₂ is CR_{c}, and " 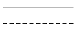 " is a single bond; wherein R_{b}, R_{c} together with the ring attached thereto form a substituted or unsubstituted 9- or 10-membered biheteroaryl fused ring;
   (d1) W₁ is CR_{b}, W₂ is NR_{c}, and " 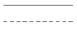 " is a single bond; wherein R_{b}, R_{c} together with the ring attached thereto form a substituted or unsubstituted 9- or 10-membered biheteroaryl fused ring;
Rₐ and R_{d} are each independently hydrogen, halo, substituted or unsubstituted C₁₋₁₀ alkyl, substituted or unsubstituted C₁₋₁₀ alkoxy or NRₐ₀R_{b0};
R₀₁ and R₀₂ are each independently hydrogen, -C(O)C₁₋₆ alkyl or -C(O)C₃₋₈ monocyclic cycloalkyl;
L is a bond, -(CR₁₁R₁₂)ₜ₁-(CR₂₁R₂₂)ₜ₂-(CR₃₁R₃₂)ₜ₃-(CR₄₁R₄₂)ₜ₄-(O)ₜ₅ or - (CR₁₁R₁₂)ₜ₁-(CR₂₁R₂₂)ₜ₂-(NR₃₁)ₜ₃-(CR₄₁R₄₂)ₜ₄-(O)ₜ₅; wherein t1, t2, t3, t4 and t5 are each independently 0 or 1;
R_{d}', R₀₃, R₁₁, R₁₂, R₂₁, R₂₂, R₃₁, R₃₂, R_{41,} R₄₂ are selected from the combination selected from the group consisting of:
   (a2) R_{d}' are each independently hydrogen, halo, substituted or unsubstituted C₁₋₁₀ alkyl, substituted or unsubstituted C₁₋₁₀ alkoxy or NRₐ₀R_{b0};
      R₀₃ is hydrogen, or substituted or unsubstituted C₁₋₁₀ alkyl;
      R₁₁, R₁₂, R₄₁, R₄₂ are each independently hydrogen, halo, hydroxy, hydroxymethyl, hydroxyethyl or C₁₋₃ alkyl;
      R₂₁, R₂₂, R₃₁, R₃₂ are each independently hydrogen, halo, hydroxy, hydroxymethyl, hydroxyethyl or C₁₋₃ alkyl;
   (b2) R_{d}' are each independently hydrogen, halo, substituted or unsubstituted C₁₋₁₀ alkyl, substituted or unsubstituted C₁₋₁₀ alkoxy or NRₐ₀R_{b0};
      R₀₃ is hydrogen, or substituted or unsubstituted C₁₋₁₀ alkyl;
      R₁₁, R₁₂, R₄₁, R₄₂ are each independently hydrogen, halo, hydroxy, hydroxymethyl, hydroxyethyl or C₁₋₃ alkyl;
      R₃₁, R₃₂ are each independently hydrogen, halo, hydroxy, hydroxymethyl, hydroxyethyl or C₁₋₃ alkyl;
      R₂₁, R₂₂ together with the carbon atom attached thereto form a substituted or unsubstituted C₃₋₈ monocyclic cycloalkyl, or substituted or unsubstituted C₃₋₆ monocyclic heterocyclyl;
   (c2) R_{d}' are each independently hydrogen, halo, substituted or unsubstituted C₁₋₁₀ alkyl, substituted or unsubstituted C₁₋₁₀ alkoxy or NRₐ₀R_{b0};
      R₀₃ is hydrogen, or substituted or unsubstituted C₁₋₁₀ alkyl;
      R₁₁, R₁₂, R₄₁, R₄₂ are each independently hydrogen, halo, hydroxy, hydroxymethyl, hydroxyethyl or C₁₋₃ alkyl;
      R₂₁, R₂₂ are each independently hydrogen, halo, hydroxy, hydroxymethyl, hydroxyethyl or C₁₋₃ alkyl;
      R₃₁, R₃₂ together with the carbon atom attached thereto form a substituted or unsubstituted C₃₋₈ monocyclic cycloalkyl, or substituted or unsubstituted C₃₋₆ monocyclic heterocyclyl;
   (d2) R_{d}' are each independently hydrogen, halo, substituted or unsubstituted C₁₋₁₀ alkyl, substituted or unsubstituted C₁₋₁₀ alkoxy or NRₐ₀R_{b0};
      R₀₃ is hydrogen, or substituted or unsubstituted C₁₋₁₀ alkyl;
      R₁₁, R₁₂, R₄₁, R₄₂ are each independently hydrogen, halo, hydroxy, hydroxymethyl, hydroxyethyl or C₁₋₃ alkyl;
      R₂₂, R₃₂ are each independently hydrogen, halo, hydroxy, hydroxymethyl, hydorxyethyl or C₁₋₃ alkyl;
      R₂₁, R₃₁ together with the carbon atom attached thereto form a substituted or unsubstituted C₃₋₈ monocyclic cycloalkyl, or substituted or unsubstituted C₃₋₆ monocyclic heterocyclyl;
   (e2) R_{d}' are each independently hydrogen, halo, substituted or unsubstituted C₁₋₁₀ alkyl, substituted or unsubstituted C₁₋₁₀ alkoxy or NRₐ₀R_{b0};
      R₁₁, R₁₂, R₄₁, R₄₂ are each independently hydrogen, halo, hydroxy, hydroxymethyl, hydroxyethyl or C₁₋₃ alkyl;
      R₂₁, R₂₂, R₃₂ are each independently hydrogen, halo, hydroxy, hydroxymethyl, hydroxyethyl or C₁₋₃ alkyl;
      R₀₃ and R₃₁ are linked together to form a substituted or unsubstituted C₃₋₈ monocyclic heterocyclyl;
   (f2) R_{d}' are each independently hydrogen, halo, substituted or unsubstituted C₁₋₁₀ alkyl, substituted or unsubstituted C₁₋₁₀ alkoxy or NRₐ₀R_{b0};
      R₁₁, R₁₂, R₄₁, R₄₂ are each independently hydrogen, halo, hydroxy, hydroxymethyl, hydroxyethyl or C₁₋₃ alkyl;
      R₂₂, R₃₁, R₃₂ are each independently hydrogen, halo, hydroxy, hydroxymethyl, hydroxyethyl or C₁₋₃ alkyl;
      R₀₃ and R₂₁ are linked together to form a substituted or unsubstituted C₃₋₈ monocyclic heterocyclyl;
   (g2) R₁₁, R₁₂, R₄₁, R₄₂ are each independently hydrogen, halo, hydroxy, hydroxymethyl, hydroxyethyl or C₁₋₃ alkyl;
      R₂₁, R₂₂, R₃₁, R₃₂ are each independently hydrogen, halo, hydroxy, hydroxymethyl, hydroxyethyl or C₁₋₃ alkyl;
      R_{d}' and R₀₃ are linked together to form a substituted or unsubstituted 4- to 7-membered oxo-substituted heterocyclyl;
   (h2) R_{d}' are each independently hydrogen, halo, substituted or unsubstituted C₁₋₁₀ alkyl, substituted or unsubstituted C₁₋₁₀ alkoxy or NRₐ₀R_{b0};
      R₀₃ is hydrogen, or substituted or unsubstituted C₁₋₁₀ alkyl;
      R₁₂, R₄₁, R₄₂ are each independently hydrogen, halo, hydroxy, hydroxymethyl, hydroxyethyl or C₁₋₃ alkyl;
      R₂₁, R₂₂, R₃₂ are each independently hydrogen, halo, hydroxy, hydroxymethyl, hydroxyethyl or C₁₋₃ alkyl;
      R₁₁ and R₃₁ are linked together to form a substituted or unsubstituted C₃₋₈ monocyclic cycloalkyl, or substituted or unsubstituted C₃₋₆ monocyclic heterocyclyl;
in the above groups, the "substituted" means that 1, 2, 3 or 4 hydrogen atom(s) in the group are substituted by a substituent each independently selected from the group S consisting of: hydroxy, halo, nitro, oxo, C₁₋₆ alkyl, hydroxy-substituted C₁₋₆ alkyl, benzyl, -S-C₁₋₆ alkyl, -S-halo C₁₋₆ alkyl, -(CRₐ₁R_{b1})ᵤ-cyano, -(CRₐ₁R_{b1})ᵤ-C₁₋₆ alkoxy, - (CRₐ₁R_{b1})ᵤ-halo C₁₋₆ alkoxy, -(CRₐ₁R_{b1})ᵤ-halo C₁₋₆ alkyl, -(CRₐ₁R_{b1})ᵤ-C₃₋₆ monocyclic heterocyclyl, -(CRₐ₁R_{b1})ᵤ-C₃₋₈ monocyclic cycloalkyl, -(CRₐ₁R_{b1})ᵤ-phenyl, - (CRₐ₁R_{b1})ᵤ-5- or 6-membered monoheteroaryl, -(CRₐ₁R_{b1})ᵤ-O-(CRₐ₂R_{b2})ᵥ-halo C₁₋₆ alkyl, -(CRₐ₁R_{b1})ᵤ-O-(CRₐ₂R_{b2})ᵥ-C₃₋₈ monocyclic cycloalkyl, -(CRₐ₁R_{b1})ᵤ-O-(CRₐ₂R_{b2})ᵥ-C₃₋₆ monocyclic heterocyclyl, -(CRₐ₁R_{b1})ᵤ-O-(CRₐ₂R_{b2})ᵥ-phenyl, - (CRₐ₁R_{b1})ᵤ-O-(CRₐ₂R_{b2})ᵥ-5- or 6-membered monoheteroaryl, -(CRₐ₁R_{b1})ᵤ-S-(CRₐ₂Rb₂)ᵥ-phenyl, -(CRₐ₁R_{b1})ᵤ-SO₂-(CRₐ₂R_{b2})ᵥ-phenyl, -(CRₐ₁R_{b1})ᵤ-O-C(O)NRₐ₀R_{b0}, -(CRₐ₁R_{b1})ᵤ-O-(CRₐ₂R_{b2})ᵥ-C₁₋₆ alkoxy, -(CRₐ₁R_{b1})ᵤ-O-(CRₐ₂R_{b2})ᵥOH, -(CRₐ₁R_{b1})ᵤ-SO₂C₁₋₆ alkyl, -(CRₐ₁R_{b1})ᵤ-SO₂NRₐ₀R_{b0}, -(CRₐ₁R_{b1})ᵤ-C(O)NRₐ₀R_{b0}, -(CRₐ₁R_{b1})ᵤ-NRₐ₀R_{b0}, -(CRₐ₁R_{b1})ᵤ-C(O)C₁₋₆ alkyl, -C(O)OC₁₋₆ alkyl, NRₐ₀C(O)-(CRₐ₁R_{b1})ᵤ-NRₐ₀R_{b0}, NRₐ₀C(O)-(CRₐ₁R_{b1})ᵤOH, NRₐ₀C(O)-halo C₁₋₆ alkyl; wherein the C₃₋₈ monocyclic cycloalkyl, C₃₋₆ monocyclic heterocyclyl, phenyl, 5-or 6-membered monoheteroaryl is optionally substituted by 1, 2 or 3 substituent(s) selected from the group consisting of hydroxy, hydroxymethyl, hydroxyethyl, halo, cyano, cyanomethyl, cyanoethyl, C₁₋₃ alkyl, C₁₋₃ alkoxy and C₃₋₆ monocyclic cycloalkyl;
u, v are each independently 0, 1, 2, 3 or 4;
Rₐ₀, R_{b0} are each independently hydrogen or C₁₋₃ alkyl; or Rₐ₀, R_{b0} together with the nitrogen atom attached thereto form a C₃₋₈ monocyclic heterocyclyl, the C₃₋₈ monocyclic heterocyclyl is optionally substituted by 1, 2 or 3 substituent(s) selected from the group consisting of halo or C₁₋₃ alkyl;
Rₐ₁, R_{b1}, Rₐ₂, R_{b2} are the same or different, and are each independently hydrogen, hydroxyl or C₁₋₃ alkyl.

In one embodiment of the present invention, R_{d}' and R₀₃ are linked together to form a 4- to 7-membered oxo-substituted heterocyclyl having a structure as represented by formula (h): 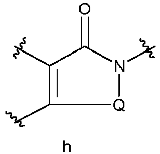 wherein Q is -(CR_{q1}R_{q2})ₛ₁, -N(R_{q3})-(CR_{q1}R_{q2})ₛ₂, -O-(CR_{q1}R_{q2})_{S2}, -(CR_{q1}R_{q2})-N(R_{q3})-(CR_{q3}R_{q4}), -(CR_{q1}R_{q2})-O-(CR_{q3}R_{q4}), -N=CR_{q5}-(CR_{q1}R_{q2})ₛ₃, -CR_{q5}=CR_{q6}-(CR_{q1}R_{q2})ₛ₃, -CR_{q5}=N-(CR_{q1}R_{q2})ₛ₃; wherein s1 is 0, 1, 2 or 3; s2 is 1 or 2; s3 is 0 or 1; R_{q1}, R_{q2}, R_{q3}, R_{q4}, R_{q5}, R_{q6} are each independently hydrogen or C₁₋₃ alkyl.

In one embodiment of the present invention, R_{d}' and R₀₃ are linked together to form a 4- to 7-membered oxo-substituted heterocyclyl having a structure selected from the group consisting of:

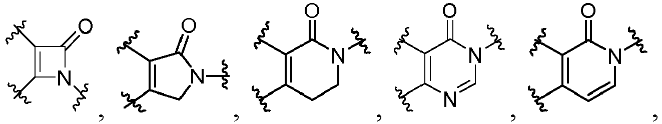

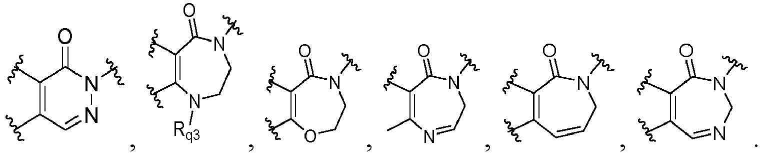

In one embodiment of the present invention, -N(R₀₃)-L- has a structure as represented by formula (c), formula (d), formula (e) or fomula (f): 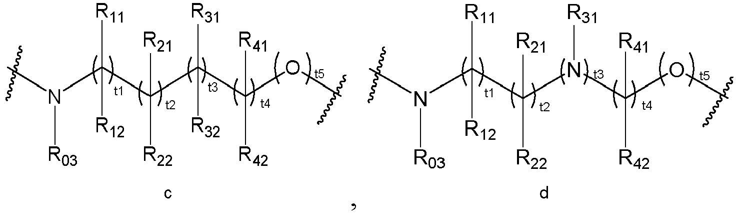 in the formula (c), formula (d), t1, t2, t3, t4, t5 are each independently 0 or 1;
R₀₃ is hydrogen, or a substituted or unsubstituted C₁₋₁₀ alkyl;
R₁₁, R₁₂, R₂₁, R₂₂, R₃₁, R₃₂, R₄₁, R₄₂ are selected from the combination selected from the group consisting of:
   (1) R₁₁, R₁₂, R₂₁, R₂₂, R₃₁, R₃₂, R₄₁, R₄₂ are each independently hydrogen, halo, hydroxy, hydroxymethyl, hydroxyethyl or C₁₋₃ alkyl;
   (2) R₁₁, R₁₂, R₃₁, R₃₂, R_{41,} R₄₂ are each independently hydrogen, halo, hydroxy, hydroxymethyl, hydroxyethyl or C₁₋₃ alkyl;
      R₂₁, R₂₂ together with the carbon atom attached thereto form a substituted or unsubstituted C₃₋₈ monocyclic cycloalkyl, or substituted or unsubstituted C₃₋₆ monocyclic heterocyclyl;
   (3) R₁₁, R₁₂, R₂₁, R₂₂, R_{41,} R₄₂ are each independently hydrogen, halo, hydroxy, hydroxymethyl, hydroxyethyl or C₁₋₃alkyl;
      R₃₁, R₃₂ together with the carbon atom attached thereto form a substituted or unsubstituted C₃₋₈ monocyclic cycloalkyl, or substituted or unsubstituted C₃₋₆ monocyclic heterocyclyl;
   (4) R₁₁, R₁₂, R₂₂, R₃₂, R_{41,} R₄₂ are each independently hydrogen, halo, hydroxy, hydroxymethyl, hydroxyethyl or C₁₋₃ alkyl;
      R₂₁, R₃₁ together with the carbon atom attached thereto form a substituted or unsubstituted C₃₋₈ monocyclic cycloalkyl, or substituted or unsubstituted C₃₋₆ monocyclic heterocyclyl;
   (5) R₁₂, R₂₁, R₂₂, R₃₂, R_{41,} R₄₂ are each independently hydrogen, halo, hydroxy, hydroxymethyl, hydroxyethyl or C₁₋₃ alkyl;
      R₁₁, R₃₁ are linked together to form a substituted or unsubstituted C₃₋₈ monocyclic cycloalkyl, or substituted or unsubstituted C₃₋₆ monocyclic heterocyclyl; 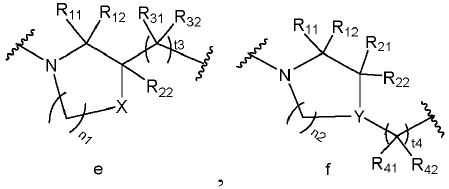 in the formula (e), formula (f), t3, t4 are each independently 0 or 1;
R₁₁, R₁₂, R₂₁, R₂₂, R₃₁, R₃₂, R₄₁, R₄₂ are each independently hydrogen, halo, hydroxy, hydroxymethyl, hydroxyethyl or C₁₋₃ alkyl;
X is NRₓ₁, O or CRₓ₂Rₓ₃;
Y is N or CR_{y};
Rₓ₁, Rₓ₂, Rₓ₃, R_{y} are each independently hydrogen or C₁₋₃ alkyl;
n1, n2 are each independently 1, 2 or 3.

In one embodiment of the present invention, the formula (c) has a structure selected from the the group consisting of:

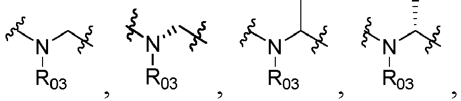

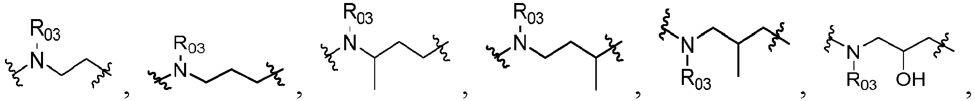

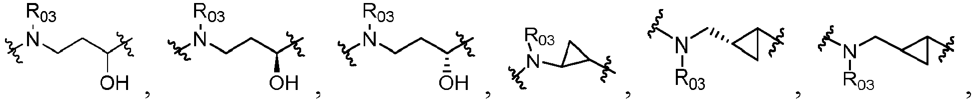

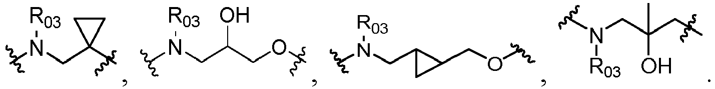

In one embodiment of the present invention, the formula (d) has a structure selected from the group consisting of:

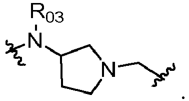

In one embodiment of the present invention, the formula (e) has a structure selected from the group consisting of:

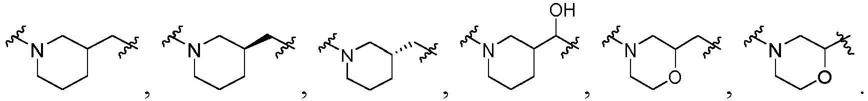

In one embodiment of the present invention, the formula (f) has a structure selected from the group consisting of:

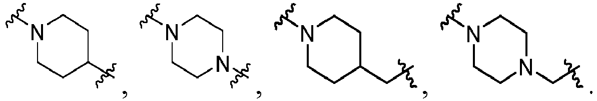

In one embodiment of the present invention, A has a structure as resresented by formula (a); -N(R₀₃)-L- has a structure as represented by formula (c) or formula (d). In one embodiment of the present invention, A has a structure as resresented by formula (a); -N(R₀₃)-L- has a structure as represented by formula (e) or formula (f). In one embodiment of the present invention, A has a structure as resresented by formula (b); -N(R₀₃)-L- has a structure as represented by formula (c) or formula (d). In one embodiment of the present invention, A has a structure as resresented by formula (b); -N(R₀₃)-L- has a structure as represented by formula (e) or formula (f).

In one embodiment of the present invention, the formula (a) has a structure selected from the group consisting of: 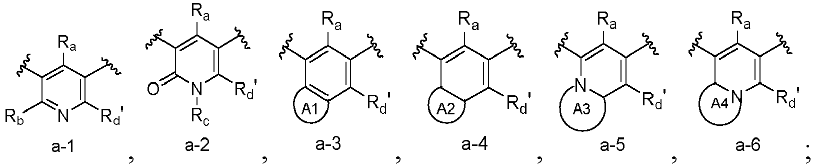
wherein the A1, A2 rings are each independently 5- or 6-membered monoheteroaryl selected from the group consisting of: thiophene, furan, thiazole, isothiazole, imidazole, oxazole, pyrrole, pyrazole, triazole, 1,2,3-triazole, 1,2,4-triazole, 1,2,5-triazole, 1,3,4-triazole, tetrazole, isoxazole, oxadiazole, 1,2,3-oxadiazole, 1,2,4-oxadiazole, 1,2,5-oxadiazole, 1,3,4-oxadiazole, thiadiazole, pyridine, pyridazine, pyrimidine or pyrazine; the 5- or 6-membered monoheteroaryl is unsubstituted or substituted by 1, 2 or 3 substituent(s) each independently selected from the group S;
the A3, A4 rings are each independently 5- or 6-membered monoheteroaryl selected from the group consisting of: imidazole, pyrrole, 2,3-dihydrothiazole, 2,3-dihydrooxazole, 2,3-dihydro-1H-imidazole, 2,5-dihydro-1H-imidazole, 1,2,4-triazole; the 5- or 6-membered monoheteroaryl is unsubstituted or substituted by 1, 2 or 3 substituent(s) each independently selected from the group S.

In one embodiment of the present invention, in the formula (a-1), Rₐ is hydrogen; R_{b}, R_{d}' are each independeantly hydrogen, halo, C₁₋₃ alkyl, C₁₋₃ alkoxy or NRₐ₀R_{b0}.

In one embodiment of the present invention, in the formula (a-1), Rₐ is hydrogen; R_{b}, R_{d}' are each independeantly hydrogen, fluoro, chloro, methyl, ethyl, *n*-propyl, *iso*propyl, methoxy, ethoxy, *n*-propoxy, iso-propoxy, NH₂, NHCH₃ or N(CH₃)₂.

In one embodiment of the present invention, the formula (b) has a structure selected from the group consisting of: 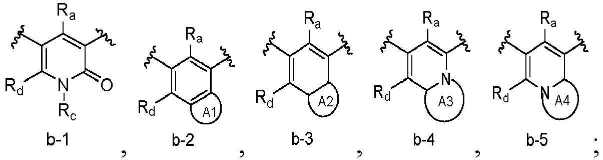
wherein the A1, A2 rings are each independently 5- or 6-membered monoheteroaryl selected from the group consisting of: thiophene, furan, thiazole, isothiazole, imidazole, oxazole, pyrrole, pyrazole, triazole, 1,2,3-triazole, 1,2,4-triazole, 1,2,5-triazole, 1,3,4-triazole, tetrazole, isoxazole, oxadiazole, 1,2,3-oxadiazole, 1,2,4-oxadiazole, 1,2,5-oxadiazole, 1,3,4-oxadiazole, thiadiazole, pyridine, pyridazine, pyrimidine or pyrazine; the 5- or 6-membered monoheteroaryl is unsubstituted or substituted by 1, 2 or 3 substituent(s) each independently selected from the group S;
the A3, A4 rings are each independently 5- or 6-membered monoheteroaryl selected from the group consisting of: imidazole, pyrrole, 2,3-dihydrothiazole, 2,3-dihydrooxazole, 2,3-dihydro-1H-imidazole, 2,5-dihydro-1H-imidazole, 1,2,4-triazole; the 5- or 6-membered monoheteroaryl is unsubstituted or substituted by 1, 2 or 3 substituent(s) each independently selected from the group S.

In one embodiment of the present invention, A has a structure selected from those as represented by formula (a-1), formula (a-2), formula (a-3), formula (a-4), formula (a-5), formula (a-6), formula (b-1), formula (b-2), formula (b-3), formula (b-4) or formula (b-5); -N(R₀₃)-L- has a structure selected from those as represented by formula (c), formula (d), formula (e), formula (f).

In one embodiment of the present invention, -N(R₀₃)-L- is selected from the group consisting of:

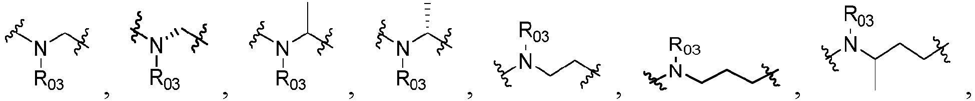

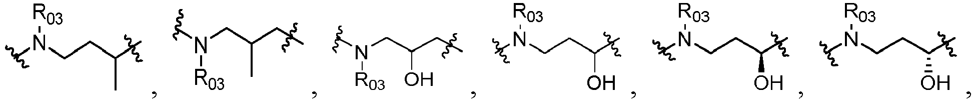

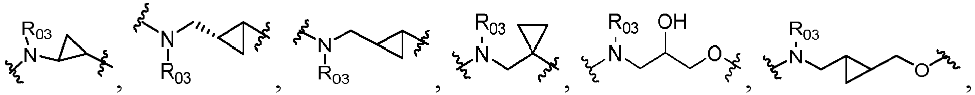

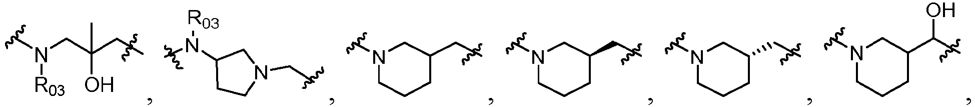

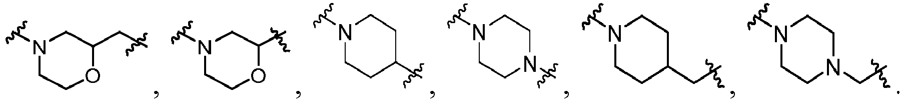

In one embodiment of the present invention, -A-C(O)-N(R₀₃)- has a structure as represented by formula (g): 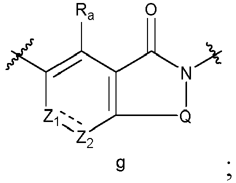
Q is -(CR_{q1}R_{q2})ₛ₁, -N(R_{q3})-(CR_{q1}R_{q2})ₛ₂, -O-(CR_{q1}R_{q2})ₛ₂, -(CR_{q1}R_{q2})-N(R_{q3})-(CR_{q3}R_{q4}), -(CR_{q1}R_{q2})-O-(CR_{q3}R_{q4}), -N=CR_{q5}-(CR_{q1}R_{q2})ₛ₃, -CR_{q5}=CR_{q6}-(CR_{q1}R_{q2})ₛ₃, - CR_{q5=}N-(CR_{q1}R_{q2})ₛ₃; wherein s1 is 0, 1, 2 or 3; s2 is 1 or 2; s3 is 0 or 1;
R_{q1}, R_{q2}, R_{q3}, R_{q4}, R_{q5}, R_{q6} are each independently hydrogen or C₁₋₃ alkyl;
Rₐ, Z₁, Z₂, " 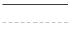 " are defined as in the specification;
L is -(CR₁₁R₁₂)ₜ₁-(CR₂₁R₂₂)ₜ₂-(CR₃₁R₃₂)ₜ₃-(CR₄₁R₄₂)ₜ₄-(O)ₜ₅ or -(CR₁₁R₁₂)ₜ₁-(CR₂₁R₂₂)ₜ₂-(NR₃₁)ₜ₃-(CR₄₁Rₜ₂)ₜ₄-(O)ₜ₅; wherein t1, t2, t3, t4, t5 are each independently 0 or 1;
R₁₁, R₁₂, R₂₁, R₂₂, R₃₁, R₃₂, R₄₁, R₄₂ are selected from the combination selected from the group consisting of:
   (1) R₁₁, R₁₂, R₂₁, R₂₂, R₃₁, R₃₂, R₄₁, R₄₂ are each independently hydrogen, halo, hydroxy, hydroxymethyl, hydroxyethyl or C₁₋₃ alkyl;
   (2) R₁₁, R₁₂, R₃₁, R₃₂, R₄₁, R₄₂ are each independently hydrogen, halo, hydroxy, hydroxymethyl, hydroxyethyl or C₁₋₃ alkyl;
      R₂₁, R₂₂ together with the carbon atom attached thereto form a substituted or unsubstituted C₃₋₈ monocyclic cycloalkyl, or substituted or unsubstituted C₃₋₆ monocyclic heterocyclyl;
   (3) R₁₁, R₁₂, R₂₁, R₂₂, R₄₁, R₄₂ are each independently hydrogen, halo, hydroxy, hydroxymethyl, hydroxyethyl or C₁₋₃ alkyl;
      R₃₁, R₃₂ together with the carbon atom attached thereto form a substituted or unsubstituted C₃₋₈ monocyclic cycloalkyl, or substituted or unsubstituted C₃₋₆ monocyclic heterocyclyl;
   (4) R₁₁, R₁₂, R₂₂, R₃₂, R₄₁, R₄₂ are each independently hydrogen, halo, hydroxy, hydroxymethyl, hydroxyethyl or C₁₋₃ alkyl;
      R₂₁, R₃₁ together with the carbon atom attached thereto form a substituted or unsubstituted C₃₋₈ monocyclic cycloalkyl, or substituted or unsubstituted C₃₋₆ monocyclic heterocyclyl;
   (5) R₁₂, R₂₁, R₂₂, R₃₂, R₄₁, R₄₂ are each independently hydrogen, halo, hydroxyl, hydroxymethyl, hydroxyethyl or C₁₋₃ alkyl;
      R₁₁, R₃₁ are linked together to form a substituted or unsubstituted C₃₋₈ monocyclic cycloalkyl, or substituted or unsubstituted C₃₋₆ monocyclic heterocyclyl.

In one embodiment of the present invention, in the formula (g), Z₁ is CH; Z₂ is N; " 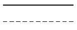 " is a double bond; Q is (CH₂)₂.

In one embodiment of the present invention, B has a structure as represented by formula (II-a); -N(R₀₃)-L- has a structure as represented by formula (c) or formula (d).

In one embodiment of the present invention, B has a structure as represented by formula (II-a); -N(R₀₃)-L- has a structure as represented by formula (e) or formula (f).

In one embodiment of the present invention, B has a structure as represented by formula (II-b); -N(R₀₃)-L- has a structure as represented by formula (c) or formula (d).

In one embodiment of the present invention, B has a structure as represented by formula (II-b); -N(R₀₃)-L- has a structure as represented by formula (e) or formula (f).

In one embodiment of the present invention, the formula (II-a) has a structure selected from the group consisting of: 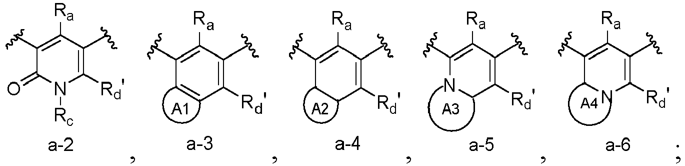
wherein the A1, A2 rings are each independently 5- or 6-membered monoheteroaryl selected from the group consisting of: thiophene, furan, thiazole, isothiazole, imidazole, oxazole, pyrrole, pyrazole, triazole, 1,2,3-triazole, 1,2,4-triazole, 1,2,5-triazole, 1,3,4-triazole, tetrazole, isoxazole, oxadiazole, 1,2,3-oxadiazole, 1,2,4-oxadiazole, 1,2,5-oxadiazole, 1,3,4-oxadiazole, thiadiazole, pyridine, pyridazine, pyrimidine or pyrazine; the 5- or 6-membered monoheteroaryl is unsubstituted or substituted by 1, 2 or 3 substituent(s) each independently selected from the group S;
the A3, A4 rings are each independently 5- or 6-membered monoheteroaryl selected from the group consisting of: imidazole, pyrrole, 2,3-dihydrothiazole, 2,3-dihydrooxazole, 2,3-dihydro-1H-imidazole, 2,5-dihydro-1H-imidazole, 1,2,4-triazole; the 5- or 6-membered monoheteroaryl is unsubstituted or substituted by 1, 2 or 3 substituent(s) each independently selected from the group S.

In one embodiment of the present invention, in the formula (a-2), Rₐ is hydrogen; R_{c}, R_{d}' are each independently hydrogen or C₁₋₃ alkyl.

In one embodiment of the present invention, in the formula (a-2), Rₐ is hydrogen; R_{c}, R_{d}' are each independently hydrogen or methyl.

In one embodiment of the present invention, the formula (II-b) has a structure selected from the group consisting of: 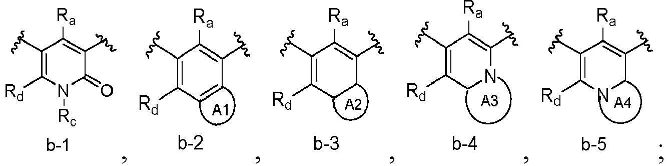
wherein the A1, A2 rings are each independently 5- or 6-membered monoheteroaryl selected from the group consisting of: thiophene, furan, thiazole, isothiazole, imidazole, oxazole, pyrrole, pyrazole, triazole, 1,2,3-triazole, 1,2,4-triazole, 1,2,5-triazole, 1,3,4-triazole, tetrazole, isoxazole, oxadiazole, 1,2,3-oxadiazole, 1,2,4-oxadiazole, 1,2,5-oxadiazole, 1,3,4-oxadiazole, thiadiazole, pyridine, pyridazine, pyrimidine or pyrazine; the 5- or 6-membered monoheteroaryl is unsubstituted or substituted by 1, 2 or 3 substituent(s) each independently selected from the group S;
the A3, A4 rings are each independently 5- or 6-membered monoheteroaryl selected from the group consisting of: imidazole, pyrrole, 2,3-dihydrothiazole, 2,3-dihydrooxazole, 2,3-dihydro-1H-imidazole, 2,5-dihydro-1H-imidazole, 1,2,4-triazole; the 5- or 6-membered monoheteroaryl is unsubstituted or substituted by 1, 2 or 3 substituent(s) each independently selected from the group S.

In one embodiment of the present invention, in the formula (b-1), Rₐ is hydrogen; R_{c}, R_{d} are each independently hydrogen or C₁₋₃ alkyl.

In one embodiment of the present invention, in the formula (b-1), Rₐ is hydrogen; R_{c}, R_{d} are each independently hydrogen or methyl.

In one embodiment of the present invention, B has a structure as represented by formula (a-2), formula (a-3), formula (a-4), formula (a-5), formula (a-6), formula (b-1), formula (b-2), formula (b-3), formula (b-4), formula (b-5); -N(R₀₃)-L- has a structure as represented by formula (c), formula (d), formula (e), formula (f).

In one embodiment of the present invention, -B-C(O)-N(R₀₃)- has a structure as represented by formula (II-g): 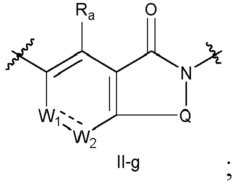
Q is -(CR_{q1}R_{q2})ₛ₁, -N(R_{q3})-(CR_{q1}R_{q2})ₛ₂, -O-(CR_{q1}R_{q2})ₛ₂, -(CR_{q1}R_{q2})-N(R_{q3})-(CR_{q3}R_{q4}), -(CR_{q1}R_{q2})-O-(CR_{q3}R_{q4}), -N=CR_{q5}-(CR_{q1}R_{q2})ₛ₃, -CR_{q5}=CR_{q6}-(CR_{q1}R_{q2})ₛ₃, - CR_{q5=}N-(CR_{q1}R_{q2})ₛ₃; wherein s1 is 0, 1, 2 or 3; s2 is 1 or 2; s3 is 0 or 1;
R_{q1}, R_{q2}, R_{q3}, R_{q4}, R_{q5}, R_{q6} are each independently hydrogen or C₁₋₃alkyl;
Rₐ, W₁, W₂, " 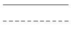 " are defined as in the specification;
L is -(CR₁₁R₁₂)ₜ₁-(CR₂₁R₂₂)ₜ₂-(CR₃₁R₃₂)ₜ₃-(CR₄₁R₄₂)ₜ₄-(O)ₜ₅ or -(CR₁₁R₁₂)ₜ₁-(CR₂₁R₂₂)ₜ₂-(NR₃₁)ₜ₃-(CR₄₁R₄₂)ₜ₄-(O)ₜ₅; wherein t1, t2, t3, t4, t5 are each independently 0 or 1;
R₁₁, R₁₂, R₂₁, R₂₂, R₃₁, R₃₂, R₄₁, R₄₂ are selected from the combination selected from the group consisting of:
   (1) R₁₁, R₁₂, R₂₁, R₂₂, R₃₁, R₃₂, R_{41,} R₄₂ are each independently hydrogen, halo, hydroxy, hydroxymethyl, hydroxyethyl or C₁₋₃ alkyl;
   (2) R₁₁, R₁₂, R₃₁, R₃₂, R₄₁, R₄₂ are each independently hydrogen, halo, hydroxy, hydroxymethyl, hydroxyethyl or C₁₋₃alkyl;
      R₂₁, R₂₂ together with the carbon atom attached thereto form a substituted or unsubstituted C₃₋₈ monocyclic cycloalkyl, or substituted or unsubstituted C₃₋₆ monocyclic heterocyclyl;
   (3) R₁₁, R₁₂, R₂₁, R₂₂, R₄₁, R₄₂ are each independently hydrogen, halo, hydroxy, hydroxymethyl, hydroxyethyl or C₁₋₃ alkyl;
      R₃₁, R₃₂ together with the carbon atom attached thereto form a substituted or unsubstituted C₃₋₈ monocyclic cycloalkyl, or substituted or unsubstituted C₃₋₆ monocyclic heterocyclyl;
   (4) R₁₁, R₁₂, R₂₂, R₃₂, R₄₁, R₄₂ are each independently hydrogen, halo, hydroxy, hydroxymethyl, hydroxyethyl or C₁₋₃ alkyl;
      R₂₁, R₃₁ together with the carbon atom attached thereto form a substituted or unsubstituted C₃₋₈ monocyclic cycloalkyl, or substituted or unsubstituted C₃₋₆ monocyclic heterocyclyl;
   (5) R₁₂, R₂₁, R₂₂, R₃₂, R₄₁, R₄₂ are each independently hydrogen, halo, hydroxy, hydroxymethyl, hydroxyethyl or C₁₋₃ alkyl;
      R₁₁, R₃₁ are linked together to form a substituted or unsubstituted C₃₋₈ monocyclic cycloalkyl, or substituted or unsubstituted C₃₋₆ monocyclic heterocyclyl.

In one embodiment of the present invention, L is selected from the group consisting of:

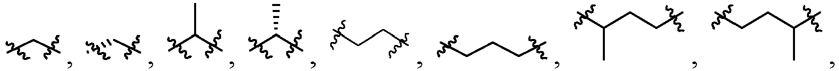

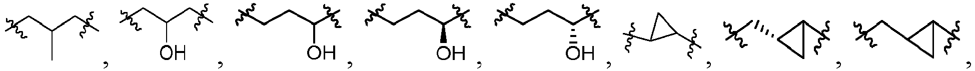

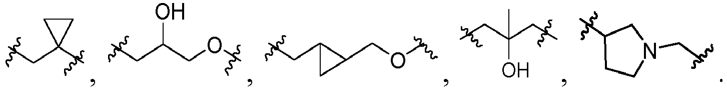

In one embodiment of the present invention, the A1 ring has a structure selected from the group consisting of: 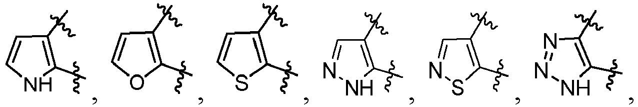 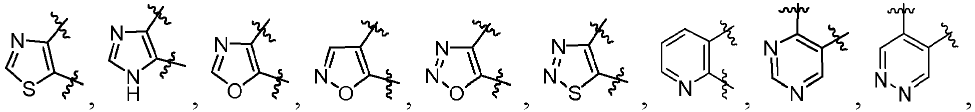 wherein " 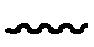 " represents a pair of adjacent carbon atoms shared as fused to phenyl; the above structure is unsubstituted or substituted by 1, 2 or 3 substituent(s) each independently selected from the group S.

In one embodiment of the present invention, the A2 ring has a structure selected from the group consisting of: 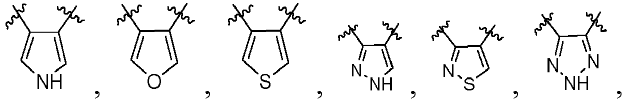 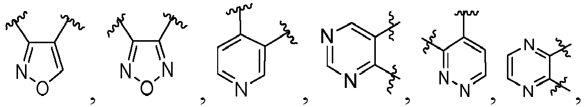 wherein " 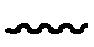 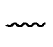 " represents a pair of adjacent carbon atoms shared as fused to phenyl; the above structure is unsubstituted or substituted by 1, 2 or 3 substituent(s) each independently selected from the group S.

In one embodiment of the present invention, the A3 ring has a structure selected from the group consisting of: 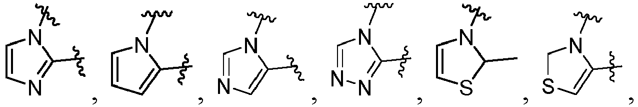 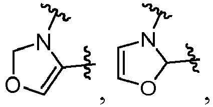 wherein " 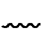 " represents a pair of adjacent carbon atoms shared as fused to another ring; the above structure is unsubstituted or substituted by 1, 2 or 3 substituent(s) each independently selected from the group S.

In one embodiment of the present invention, the A4 ring has a structure selected from the group consisting of: 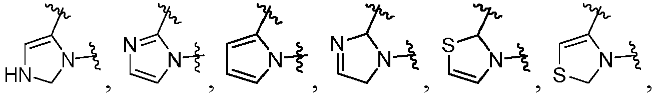 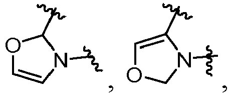 wherein " 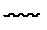 " represents a pair of adjacent carbon atoms shared as fused to another ring; the above structure is unsubstituted or substituted by 1, 2 or 3 substituent(s) each independently selected from the group S.

In one embodiment of the present invention, the substituent of the group S is selected from the group consisting of: hydroxy, halo, nitro, oxo, C₁₋₆ alkyl, hydroxy-substituted C₁₋₆ alkyl, benzyl, -S-C₁₋₆ alkyl, -S-halo C₁₋₆ alkyl, -(CH₂)ᵤ-cyano, - (CH₂)ᵤ-C₁₋₆ alkoxy, -(CH₂)ᵤ-halo C₁₋₆ alkoxy, -(CH₂)ᵤ-halo C₁₋₆alkyl, -(CH₂)ᵤ-C₃₋₆ monocyclic heterocyclyl, -(CH₂)ᵤ₋C₃₋₈ monocyclic cycloalkyl, -(CH₂)ᵤ-phenyl, - (CH₂)ᵤ-5- or 6-membered monoheteroaryl, -(CH₂)ᵤ-O-(CH₂)ᵥ-halo C₁₋₆alkyl, -(CH₂)ᵤ-O-(CH₂)ᵥ-C₃₋₈ monocyclic cycloalkyl, -(CH₂)ᵤ-O-(CH₂)ᵥ-C₃₋₆ monocyclic heterocyclyl, -(CH₂)ᵤ-O-(CH₂)ᵥ-phenyl, -(CH₂)ᵤ-O-(CH₂)ᵥ-5- or 6-membered monoheteroaryl, -(CH₂)ᵤ-S-(CH₂)ᵥ-phenyl, -(CH₂)ᵤ-SO₂-(CH₂)ᵥ-phenyl, -(CH₂)ᵤ-O-C(O)NRₐ₀R_{b0}, -(CH₂)ᵤ-O-(CH₂)ᵥ-C₁₋₆ alkoxy, -(CH₂)ᵤ-O-(CH₂)ᵥOH, -(CH₂)ᵤ-SO₂C₁₋₆ alkyl, -(CH₂)ᵤ-SO₂NRₐ₀R_{b0}, -(CH₂)ᵤ-C(O)NRₐ₀R_{b0}, -(CH₂)ᵤ-NRₐ₀R_{b0}, -(CH₂)ᵤ-C(O)C₁₋₆ alkyl, -C(O)OC₁₋₆alkyl, NRₐ₀C(O)-(CH₂)ᵤ-NRₐ₀R_{b0}, NRₐ₀C(O)-(CH₂)ᵤOH, NRₐ₀C(O)-halo C₁₋₆ alkyl; wherein the C₃₋₈monocyclic cycloalkyl, C₃₋₆ monocyclic heterocyclyl, phenyl, 5- or 6-membered monoheteroaryl are optionally substituted by 1, 2 or 3 substitute(s) selected from the group consisting of hydroxy, hydroxymethyl, hydroxyethyl, halo, cyano, cyanomethyl, cyanoethyl, C₁₋₃ alkyl, C₁₋₃ alkoxy and C₃₋₆ monocyclic cycloalkyl; u, v are each independently 0, 1, 2, 3 or 4; Rₐ₀, R_{b0} are each independently hydrogen or C₁₋₃alkyl; or Rₐ₀, R_{b0} together with the nitrogen atom attached thereto form a C₃₋₈ monocyclic heterocyclyl, the C₃₋₈ monocyclic heterocyclyl is optionally substituted by 1, 2 or 3 substitute(s) selected from the group consisting of halo or C₁₋₃ alkyl.

In one embodiment of the present invention, among the substitutes of the group S, the C₃₋₆ monocyclic heterocyclyl is selected from the group consisting of: aziridine, oxirane, azetidine, azetidin-2-one, oxetane, oxetan-2-one, oxazolidine, pyrrolidin-2-one, pyrrolidin-2,5-dione, 1,3-dioxolane, dihydrofuran-2(3H)-one, dihydrofuran-2,5-dione, piperidin-2-one, piperidin-2,6-dione, tetrahyro-2H-pyran-2-one, imidazolidine, tetrahydrofuran, tetrahydrothiophene, tetrahydropyrrole, 1,3-dioxolan-2-one, oxazolidin-2-one, imidazolidine-2-one, piperidine, piperazine, piperazin-2-one, morpholine, morpholin-3-one, morpholin-2-one, thiomorpholin-3-one 1,1-dioxide, thiomorpholine, thiomorpholine-1,1-dioxide, tetrahydropyran, 1,2-dihydroazacyclobutadiene, 1,2-dihydrooxetadiene, 2,5-dihydro-1H-pyrrole, 2,5-dihydrofuran, 2,3-dihydrofuran, 2,3-dihydro-1H-pyrrole, 3,4-dihydro-2H-pyran, 1,2,3,4-tetrahydropyridine, 3,6-dihydron-2H-pyran, 1,2,3,6-tetrahydropyridine, 1,3-oxazinane, hexahydropyrimidine, 1,4-dioxane, tetrahydropyrimidin-2(1H)-one, 1,4-dioxan-2-one, 5,6-dihydro-2H-pyran-2-one.

In one embodiment of the present invention, among the substitutes of the group S, the C₃₋₈ monocyclic cycloalkyl is selected from the group consisting of: cyclopropyl, cyclobutyl, cyclopentyl, cyclopentenyl, cyclohexyl, cyclohexenyl, cyclohexdienyl, cyclobutanone, cyclobutan-1,2-dione, cyclopentanone, cyclopentan-1,3-dione, cyclohexanone, cyclohexan-1,3-dione.

In one embodiment of the present invention, among the substitutes of the group S, the 5- or 6-membered monoheteroaryl is selected from the group consisting of: thiophene, furan, thiazole, isothiazole, imidazole, oxazole, pyrrole, pyrazole, triazole, 1,2,3-triazole, 1,2,4-triazole, 1,2,5-triazole, 1,3,4-triazole, tetrazole, isoxazole, oxadiazole, 1,2,3-oxadiazole, 1,2,4-oxadiazole, 1,2,5-oxadiazole, 1,3,4-oxadiazole, thiadiazole, pyridine, pyridazine, pyrimidine or pyrazine.

In one embodiment of the present invention, the C₆₋₁₄ aryl in R₀ is is phenyl, naphthyl, or a 9- or 10-membered aromatic fused bicyclic ring formed by fusing a phenyl to one non-aromatic ring, the non-aromatic ring is 3- to 6-membered saturated or partially unsaturated monocyclic heterocyclyl, or 3- to 6-membered saturated or partially unsaturated monocyclic cycloalkyl, wherein the 3- to 6-membered saturated or partially unsaturated monocyclic heterocyclyl is selected from the group consisting of: aziridine, oxirane, azetidine, azetidin-2-one, oxetane, oxetan-2-one, oxazolidine, pyrrolidin-2-one, pyrrolidin-2,5-dione, 1,3-dioxolane, dihydrofuran-2(3H)-one, dihydrofuran-2,5-dione, piperidin-2-one, piperidin-2,6-dione, tetrahyro-2H-pyran-2-one, imidazolidine, tetrahydrofuran, tetrahydrothiophene, tetrahydropyrrole, 1,3-dioxolan-2-one, oxazolidin-2-one, imidazolidine-2-one, piperidine, piperazine, piperazin-2-one, morpholine, morpholin-3-one, morpholin-2-one, thiomorpholin-3-one 1,1-dioxide, thiomorpholine, thiomorpholine-1,1-dioxide, tetrahydropyran, 1,2-dihydroazacyclobutadiene, 1,2-dihydrooxetadiene, 2,5-dihydro-1H-pyrrole, 2,5-dihydrofuran, 2,3-dihydrofuran, 2,3-dihydro-1H-pyrrole, 3,4-dihydro-2H-pyran, 1,2,3,4-tetrahydropyridine, 3,6-dihydro-2H-pyran, 1,2,3,6-tetrahydropyridine, 1,3-oxazinane, hexahydropyrimidine, 1,4-dioxane, tetrahydropyrimidin-2(1H)-one, 1,4-dioxan-2-one, 5,6-dihydro-2H-pyran-2-one; the 3- to 6-membered saturated or partially unsaturated monocyclic cycloalkyl is selected from the group consisting of: cyclopropyl, cyclobutyl, cyclopentyl, cyclopentenyl, cyclohexyl, cyclohexenyl, cyclohexdienyl, cyclobutanone, cyclobutan-1,2-dione, cyclopentanone, cyclopentan-1,3-dione, cyclohexanone, cyclohexan-1,3-dione; the 9- or 10-membered aromatic fused bicyclic ring is unsubstituted or substituted by 1, 2, 3 or 4 substituent(s) each independently selected from the group S.

In one embodiment of the present invention, the C₆₋₁₄ aryl in R₀ is phenyl.

In one embodiment of the present invention, the C₅₋₁₄ heteroaryl in R₀ is a 5- or 6-membered monoheteroaryl, wherein the 5- or 6-membered mono-heteroaryl is selected from the group consisting of: thiophene, furan, thiazole, isothiazole, imidazole, oxazole, pyrrole, pyrazole, triazole, 1,2,3-triazole, 1,2,4-triazole, 1,2,5-triazole, 1,3,4-triazole, tetrazole, isoxazole, oxadiazole, 1,2,3-oxadiazole, 1,2,4-oxadiazole, 1,2,5-oxadiazole, 1,3,4-oxadiazole, thiadiazole, pyridine, pyridazine, pyrimidine or pyrazine; the 5- or 6-membered monoheteroaryl is unsubstituted or substituted by 1, 2, 3 or 4 substituent(s) selected from the group S

In one embodiment of the present invention, the C₅₋₁₄ heteroaryl in R₀ is a 9- or 10-membered biheteroaryl formed by fusing a phenyl to a 5- or 6-membered monoheteroaryl, wherein the 5- or 6-membered monoheteroaryl is selected from the group consisting of: thiophene, furan, thiazole, isothiazole, imidazole, oxazole, pyrrole, pyrazole, triazole, 1,2,3-triazole, 1,2,4-triazole, 1,2,5-triazole, 1,3,4-triazole, tetrazole, isoxazole, oxadiazole, 1,2,3-oxadiazole, 1,2,4-oxadiazole, 1,2,5-oxadiazole, 1,3,4-oxadiazole, thiadiazole, pyridine, pyridazine, pyrimidine or pyrazine; the 9- or 10-membered biheteroaryl is unsubstituted or substituted by 1, 2, 3 or 4 substituent(s) selected from the group S.

In one embodiment of the present invention, the 9- or 10-membered biheteroaryl has a structure as represented by formula (A) or formula (B): 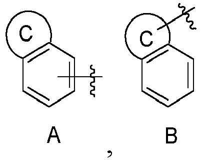 wherein, the C ring is a 5- or 6-membered monoheteroaryl; wherein the 5- or 6-membered monoheteroaryl is selected from the group consisting of: thiophene, furan, thiazole, isothiazole, imidazole, oxazole, pyrrole, pyrazole, triazole, 1,2,3-triazole, 1,2,4-triazole, 1,2,5-triazole, 1,3,4-triazole, tetrazole, isoxazole, oxadiazole, 1,2,3-oxadiazole, 1,2,4-oxadiazole, 1,2,5-oxadiazole, 1,3,4-oxadiazole, thiadiazole, pyridine, pyridazine, pyrimidine or pyrazine; the 9- or 10-membered biheteroaryl is unsubstituted or substituted by 1, 2, 3 or 4 substituent(s) selected from the group S.

In one embodiment of the present invention, the C ring has a structure selected from the group consisting of: 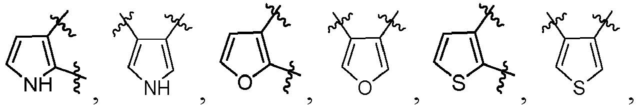 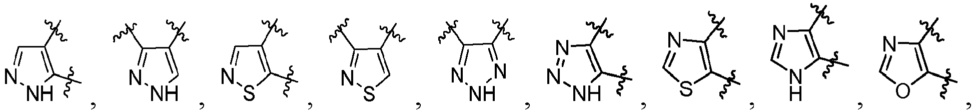 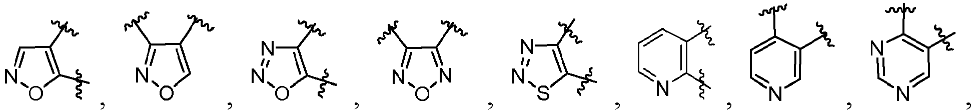 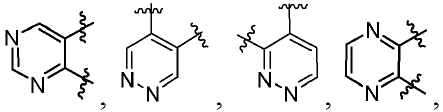 wherein " 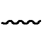 " represents a pair of adjacent carbon atoms shared as fused to phenyl.

In one embodiment of the present invention, the 9- or 10-membered biheteroaryl formed by fusing a phenyl to a 5- or 6-membered monoheteroaryl are selected from the group consisting of: benzoxazole, benzisoxazole, benzimidazole, benzothiazole, benzisothiazole, benzotriazole, benzofuran, benzothiophene, indole, indazole, isoindole, quinoline, isoquinoline, quinazoline, quinoxaline, cinnoline.

In one embodiment of the present invention, the C₅₋₁₄ heteroaryl in R₀ is a 8- to 10-membered biheteroaryl formed by fusing a 5- or 6-membered monoheteroaryl to a 5- or 6-membered monoheteroaryl, wherein the 5- or 6-membered monoheteroaryl is selected from the group consisting of: thiophene, furan, thiazole, isothiazole, imidazole, oxazole, pyrrole, pyrazole, triazole, 1,2,3-triazole, 1,2,4-triazole, 1,2,5-triazole, 1,3,4-triazole, tetrazole, isoxazole, oxadiazole, 1,2,3-oxadiazole, 1,2,4-oxadiazole, 1,2,5-oxadiazole, 1,3,4-oxadiazole, thiadiazole, pyridine, pyridazine, pyrimidine or pyrazine; the 8- to 10-membered biheteroaryl is unsubstituted or substituted by 1, 2, 3 or 4 substituent(s) selected from the group S.

In one embodiment of the present invention, the 8- to 10-membered biheteroaryl has a structure as represented by formula (C) or formula (D): 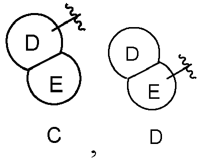 wherein,the D ring, E ring are each independently a 5- or 6-membered monoheteroaryl; wherein the 5- or 6-membered monoheteroaryl is selected from the group consisting of: thiophene, furan, thiazole, isothiazole, imidazole, oxazole, pyrrole, pyrazole, triazole, 1,2,3-triazole, 1,2,4-triazole, 1,2,5-triazole, 1,3,4-triazole, tetrazole, isoxazole, oxadiazole, 1,2,3-oxadiazole, 1,2,4-oxadiazole, 1,2,5-oxadiazole, 1,3,4-oxadiazole, thiadiazole, pyridine, pyridazine, pyrimidine or pyrazine; the 8- to 10-membered biheteroaryl is unsubstituted or substituted by 1, 2, 3 or 4 substituent(s) selected from the group S.

In one embodiment of the present invention, the D ring, E ring have a structure each independently selected from the group consisting of: 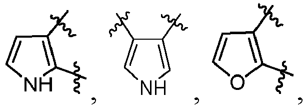 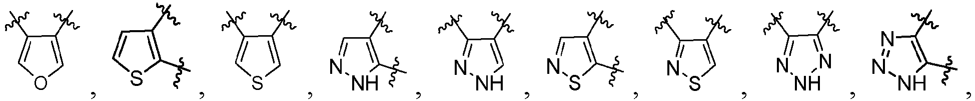 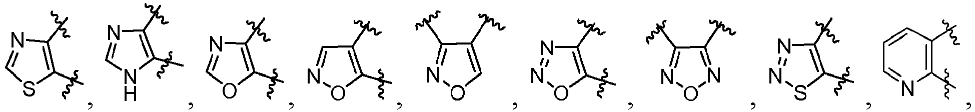 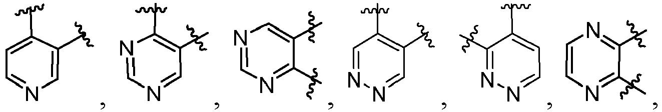 wherein " 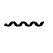 " represents a pair of adjacent carbon atoms shared as fused to another ring.

In one embodiment of the present invention, the 8- to 10-membered biheteroaryl formed by fusing a 5- or 6-membered monoheteroaryl to a 5- or 6-membered monoheteroaryl is selected from the group consisting of pyridopyrimidine and naphthyridine.

In one embodiment of the present invention, the 8- to 10-membered biheteroaryl formed by fusing a 5- or 6-membered monoheteroaryl to a 5- or 6-membered monoheteroaryl is selected from the group consisting of: pyrido[3,2-d]pyrimidine, pyrido[2,3-d]pyrimidine, pyrido[3,4-d]pyrimidine, pyrido[4,3-d]pyrimidine, 1,8-naphthyridine, 1,7-naphthyridine, 1,6-naphthyridine, 1,5-naphthyridine.

In one embodiment of the present invention, the 9- or 10-membered biheteroaryl has a structure selected from the groupo consisting of: 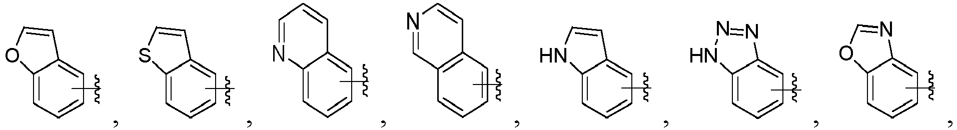 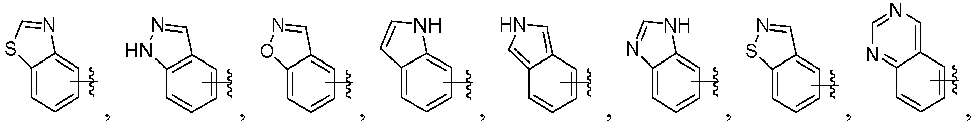 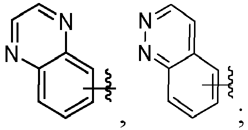 the above 9- or 10-membered biheteroaryl is unsubstituted or substituted by 1, 2, 3 or 4 substituent(s) selected from the group S.

In one embodiment of the present invention, the 8- to 10-membered biheteroaryl has a structure selected from the group consisting of: 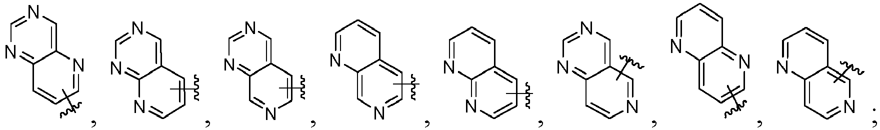 the above 8- to 10-membered biheteroaryl is unsubstituted or substituted by 1, 2, 3 or 4 substituent(s) selected from the group S.

In one embodiment of the present invention, the C₅₋₁₄ heteroaryl in R₀ is a 8- to 10-membered biheteroaryl formed by fusing a 5- or 6-membered monoheteroaryl to a non-aromatic ring, the non-aromatic ring is a 3- to 6-membered saturated or partially unsaturated monocyclic heterocyclyl, or 3- to 6-membered saturated or partially unsaturated monocyclic cycloalkyl, wherein the 5- or 6-membered monoheteroaryl is selected from the group consisting of: thiophene, furan, thiazole, isothiazole, imidazole, oxazole, pyrrole, pyrazole, triazole, 1,2,3-triazole, 1,2,4-triazole, 1,2,5-triazole, 1,3,4-triazole, tetrazole, isoxazole, oxadiazole, 1,2,3-oxadiazole, 1,2,4-oxadiazole, 1,2,5-oxadiazole, 1,3,4-oxadiazole, thiadiazole, pyridine, pyridazine, pyrimidine or pyrazine; the 3- to 6-membered saturated or partially unsaturated monocyclic heterocyclyl is selected from the group consisting of: aziridine, oxirane, azetidine, azetidin-2-one, oxetane, oxetan-2-one, oxazolidine, pyrrolidin-2-one, pyrrolidin-2,5-dione, 1,3-dioxolane, dihydrofuran-2(3H)-one, dihydrofuran-2,5-dione, piperidin-2-one, piperidin-2,6-dione, tetrahyro-2H-pyran-2-one, imidazolidine, tetrahydrofuran, tetrahydrothiophene, tetrahydropyrrole, 1,3-dioxolan-2-one, oxazolidin-2-one, imidazolidine-2-one, piperidine, piperazine, piperazin-2-one, morpholine, morpholin-3-one, morpholin-2-one, thiomorpholin-3-one 1,1-dioxide, thiomorpholine, thiomorpholine-1,1-dioxide, tetrahydropyran, 1,2-dihydroazacyclobutadiene, 1,2-dihydrooxetadiene, 2,5-dihydro-1H-pyrrole, 2,5-dihydrofuran, 2,3-dihydrofuran, 2,3-dihydro-1H-pyrrole, 3,4-dihydro-2H-pyran, 1,2,3,4-tetrahydropyridine, 3,6-dihydron-2H-pyran, 1,2,3,6-tetrahydropyridine, 1,3-oxazinane, hexahydropyrimidine, 1,4-dioxane, tetrahydropyrimidin-2(1H)-one, 1,4-dioxan-2-one, 5,6-dihydro-2H-pyran-2-one; the 3- to 6-membered saturated or partially unsaturated monocyclic cycloalkyl is selected from the group consisting of: cyclopropyl, cyclobutyl, cyclopentyl, cyclopentenyl, cyclohexyl, cyclohexenyl, cyclohexdienyl, cyclobutanone, cyclobutan-1,2-dione, cyclopentanone, cyclopentan-1,3-dione, cyclohexanone, cyclohexan-1,3-dione; the 8- to 10-membered biheteroaryl is unsubstituted or substituted by 1, 2, 3 or 4 substituent(s) selected from the group S.

In one embodiment of the present invention, R₀ is a substituted or unsubstituted phenyl, or a substituted or unsubstituted pyridyl, the "substituted" means that 1, 2, 3 or 4 hydrogen atom(s) in the group are substituted by a substituent each independently selected from the group S.

In one embodiment of the present invention, R₀ is selected from the group consisting of:

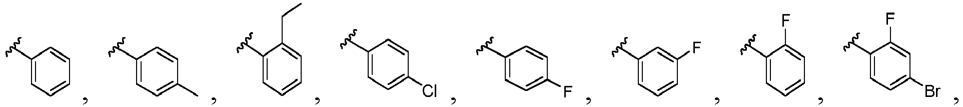

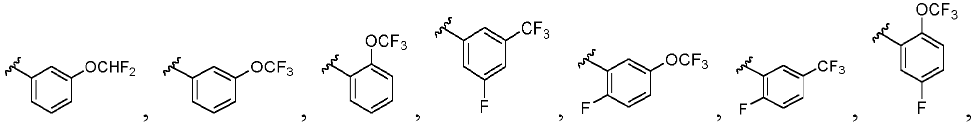

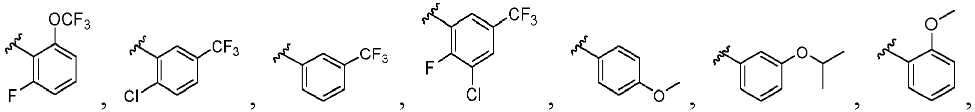

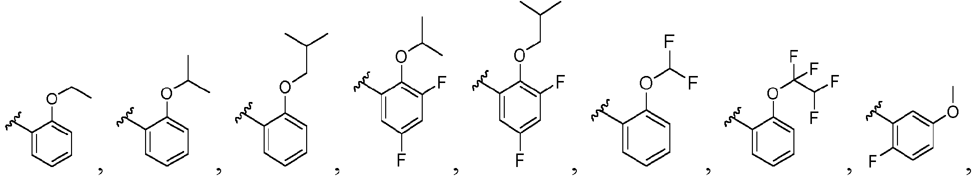

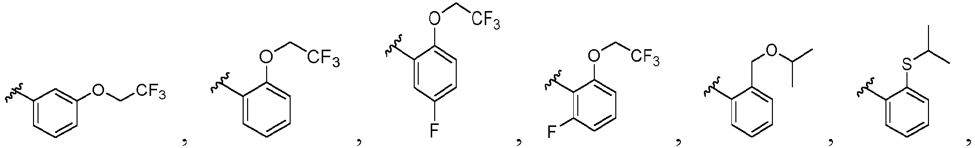

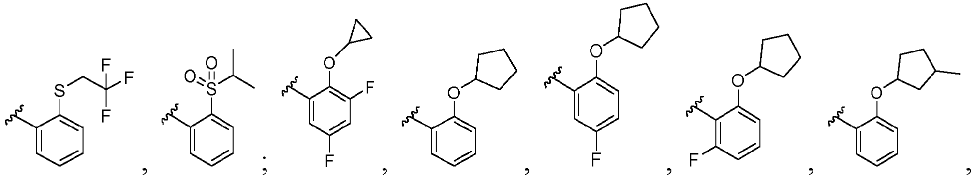

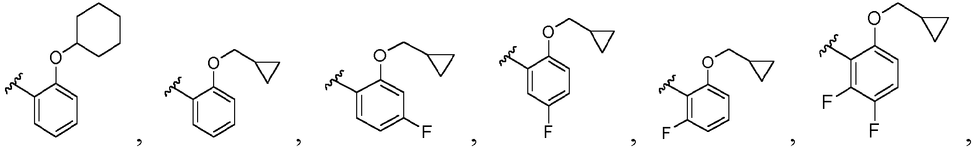

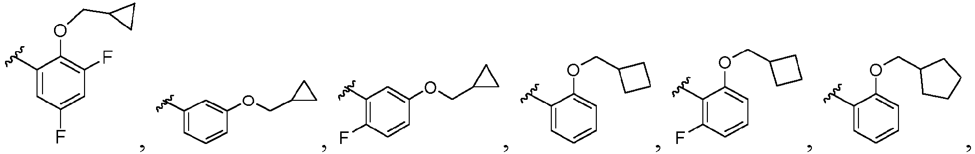

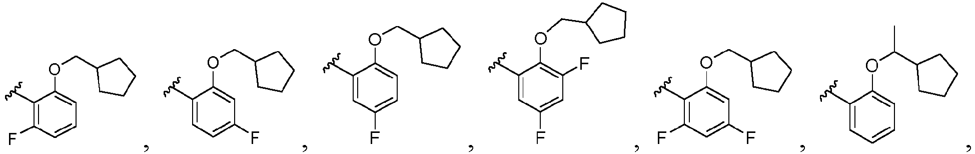

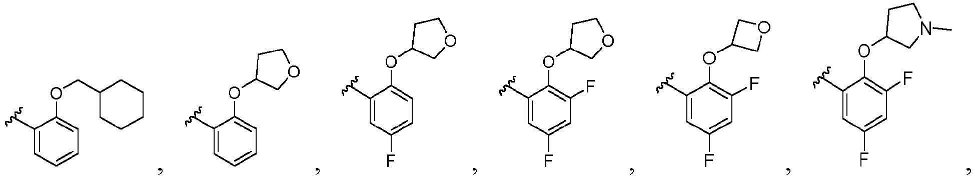

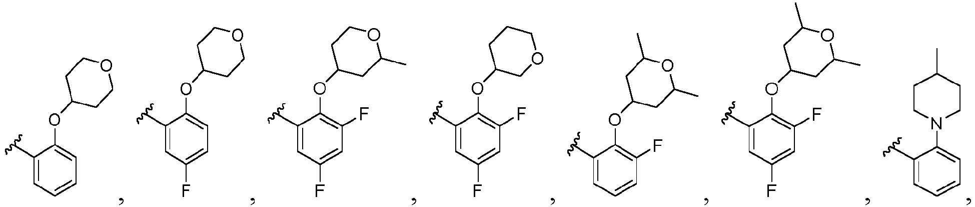

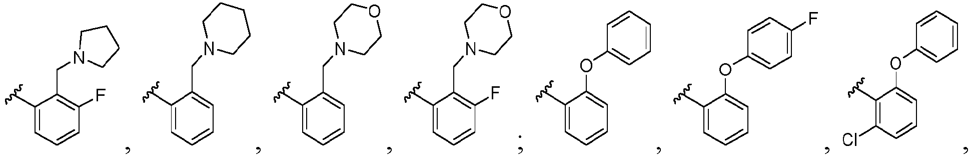

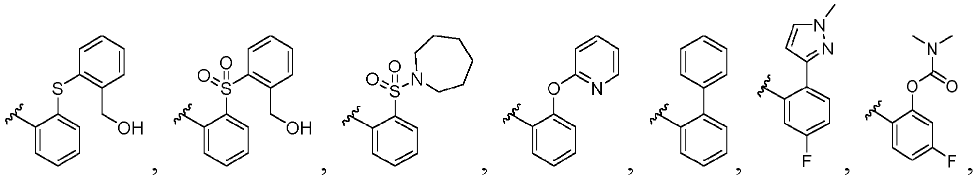

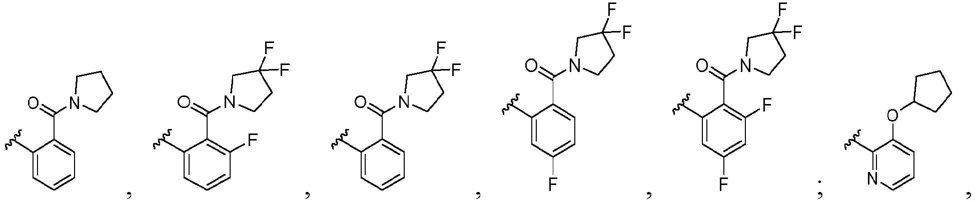

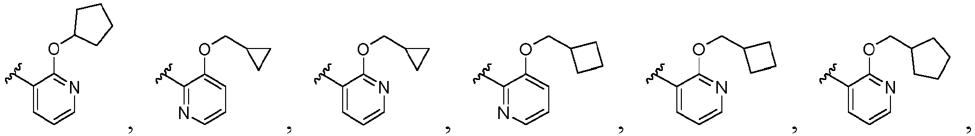

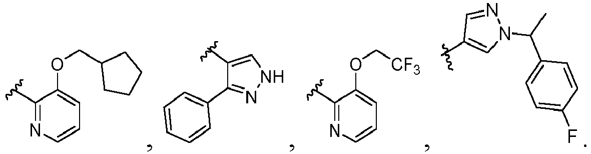

In one embodiment of the present invention, R₀₃ and R₂₁ are linked together to form a C₃₋₈ monocyclic heterocyclyl selected from the group consisting of: aziridine, oxirane, azetidine, oxetane, tetrahydrofuran, tetrahydrothiophene, tetrahydropyrrole, piperidine, piperazine, morpholine, thiomorpholine, thiomorpholine-1,1-dioxide and tetrahydropyran; the C₃₋₈ monocyclic heterocyclyl is unsubstituted or substituted by 1, 2, 3 or 4 substituent(s) each independently selected from the group S.

In one embodiment of the present invention, R₀₃ and R₃₁ are linked together to form a C₃₋₈ monocyclic heterocyclyl selected from the goup consisting of: aziridine, oxirane, azetidine, oxetane, tetrahydrofuran, tetrahydrothiophene, tetrahydropyrrole, piperidine, piperazine, morpholine, thiomorpholine, thiomorpholine-1,1-dioxide and tetrahydropyran; the C₃₋₈ monocyclic heterocyclyl is unsubstituted or substituted by 1, 2, 3 or 4 substituent(s) each independently selected from the group S.

In one embodiment of the present invention, the Rₐ₀, R_{b0} together with the nitrogen atom attached thereto form a C₃₋₈ monocyclic heterocyclyl having a structure selected from the group consisting of: 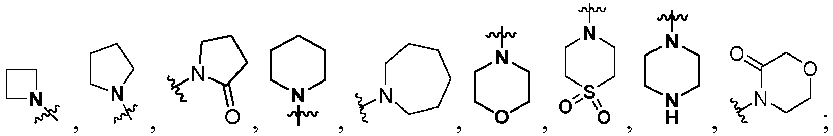 the above C₃₋₈ monocyclic heterocyclyl is unsubstituted or substituted by 1, 2, 3 or 4 substituent(s) each independently selected from the group S.

In one embodiment of the present invention, the R₀, R₁, R₂, R₃, R₀₁, R₀₂, R₀₃, A, B, L and the like are each independently the corresponding group in respective specific compound in the Examples.

In one embodiment of the present invention, the compound of formula (I) or formula (II) is selected from the group consisting of respective specific compounds as noted in the Examples, especially, any compound of Z1 to Z53.

In one embodiment of the present invention, the compound of formula (I) or formula (II) is selected from the group consisting of respective specific compounds as noted in the Examples, especially, any compound of Z54 to Z65.

In one embodiment of the present invention, the compound of formula (I) or formula (II) is selected from the group consisting of the compounds as prepared in the Examples of the present application.

In one embodiment of the present invention, the compound is selected from the group consisting of:

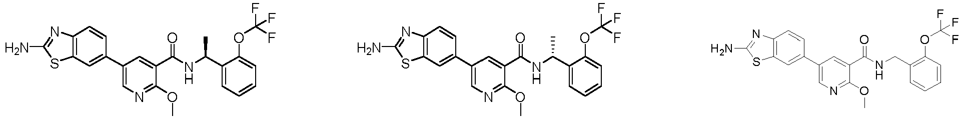

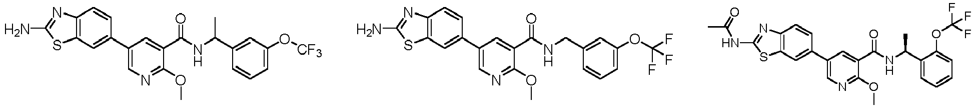

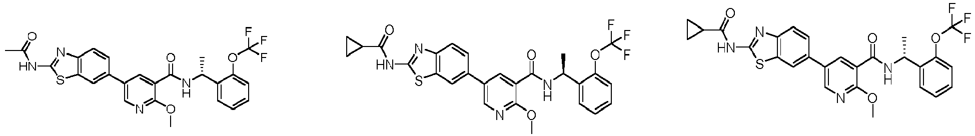

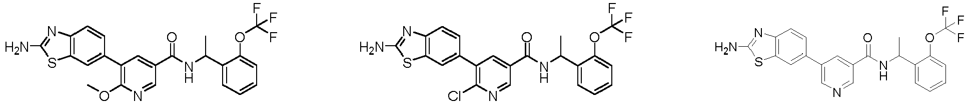

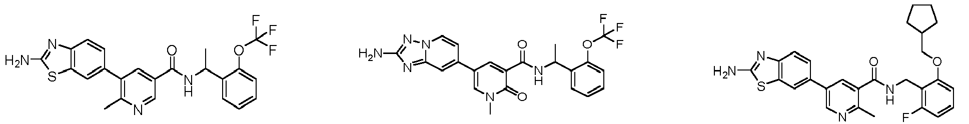

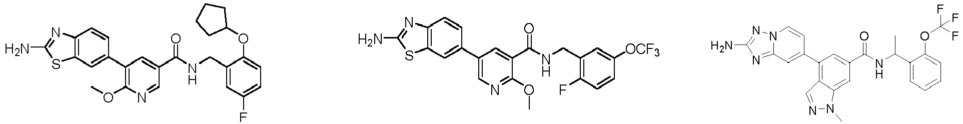

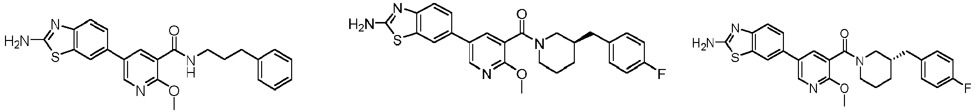

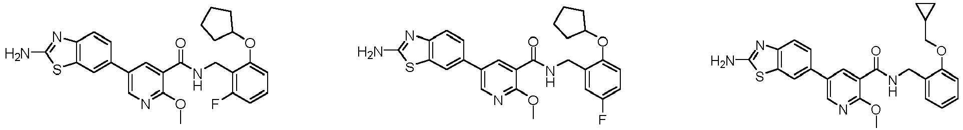

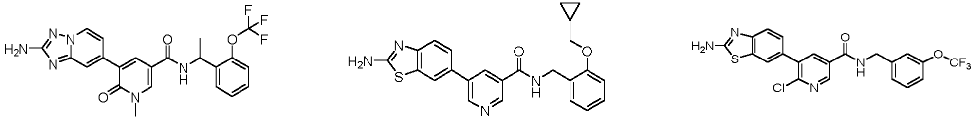

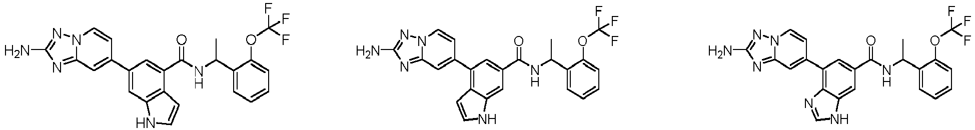

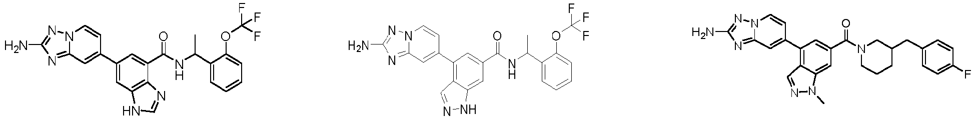

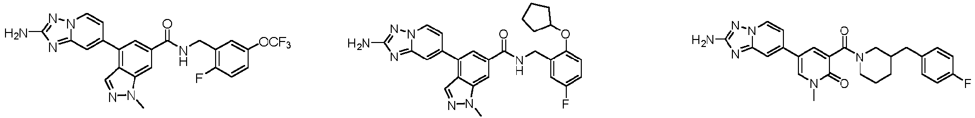

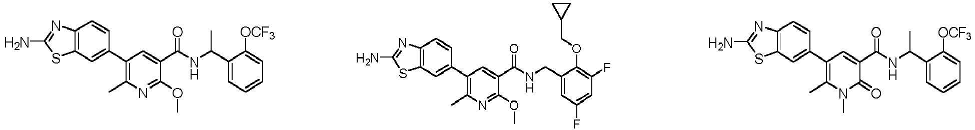

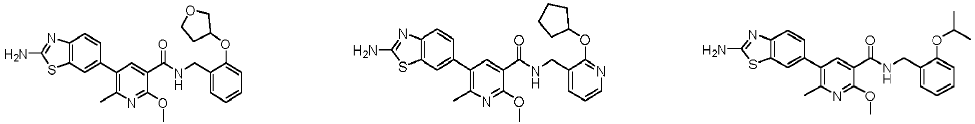

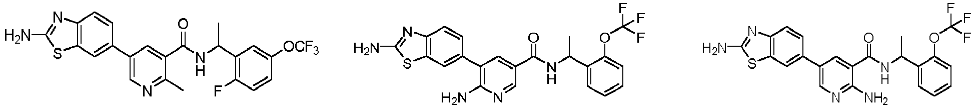

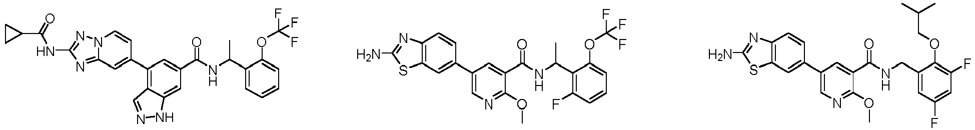

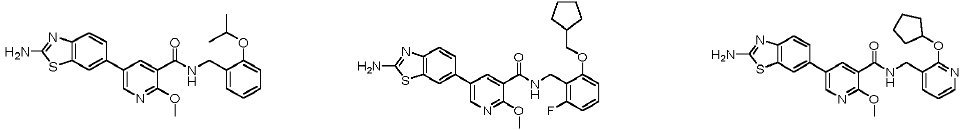

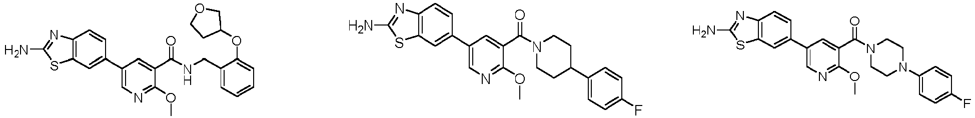

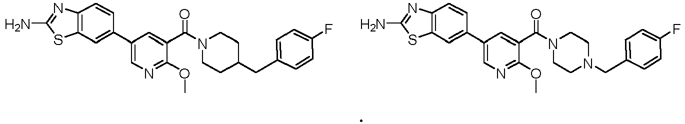

In one embodiment of the present invention, the compound is selected from the group consisting of:

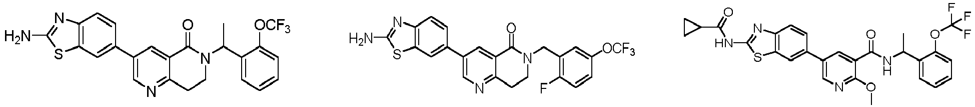

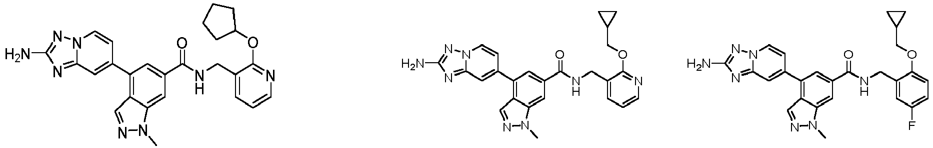

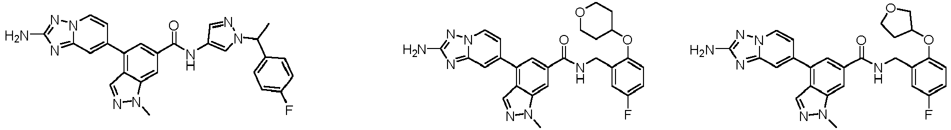

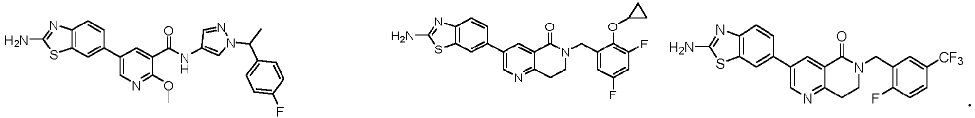

In another respect, the present invention provides a pharmaceutical composition, comprising the compound as decribed above or a pharmaceutically acceptable salt, stereoisomer, solvate or prodrug thereof; and a pharmaceutically acceptable carrier.

As used herein, the term "pharmaceutically acceptable carrier" means any formulation or carrier medium capable of delivering an effective amount of the active substance of the invention without interfering with the biological activity of the active substance and without causing adverse effects to the host or subject. Representative carriers include water, oil, vegetables and minerals, cream base, lotion base, ointment base and the like. These bases include suspension agents, viscosifiers, transdermal promoters and the like. Their formulations are known to those skilled in the field of cosmetic or topical medicine.

In an embodiment of the present invention, the pharmaceutical compoisiton may be administered in any form of oral, spray inhalation, rectal administration, nasal administration, buccal administration, topical administration, parenteral administration, such as, subcutaneous, intravenous, intramuscular, intraperitoneal, intrathecal, intraventricular, intrasternal and intracranial injection or infusion, or administered by means of an explanted reservoir. Among these, oral, intraperitoneal or intravenous administration is preferred. When administered orally, the compound of the present invention may be formulated into any orally acceptable dosage form, including but not limited to tablets, capsules, aqueous solutions or aqueous suspensions. Carriers used in tablets typically include lactose and cornstarch. Lubricants such as magnesium stearate may also be added. Diluents used in capsules typically include lactose and dried cornstarch. Aqueous suspensions are typically formulated by mixing an active ingredient with appropriate emulsifiers and suspension agents. Sweeteners, fragrances or colorants may be added to the oral dosage form as required. When topically administered, especially to the affected surface or organ readily accessible by topical application, such as eye, skin, or lower intestinal neuropathy, the compound of the present invention may be formulated into different topical dosage forms depending on the surface or organs. When topically administered to eyes, the compound of the present invention may be formulated into a dosage form of micronized suspension or solution using an isotonic sterile saline of a certain pH as the carrier, in which preservatives such as benzyl alkoxide chloride may or may not be added. For ocular administration, the compound may be formulated into a form of cream, such as, Vaseline cream. When administered topically to skin, the compound of the present invention may be formulated into a suitable dosage form of ointment, lotion or cream, in which an active ingredient is suspended or dissolved in one or more carriers. The carriers useful in an ointment formulation include but not limited to: mineral oils, liquid vaseline, white vaseline, propylene glycol, polyoxyethylene, polypropylene oxide, emulsified wax and water. The carriers useful in a lotion or cream include but not limited to: mineral oils, sorbitan monostearate, Tween 60, Cetyl ester wax, hexadecenyl aryl alcohol, 2-octyldodecanol, benzyl alcohol and water. The compound of the present invention may be administered in a dosage form of sterile injections, including sterial aqueous injection or oil suspension or sterile injection solution. Useful carriers and solvents include water, Ringer's solution and isotonic sodium chloride solution. Further, sterilized non-volatile oils can also be used as solvents or suspension media, such as monotriglycerides or diglycerides

In another respect, the present invention provides use of the above heterocyclic amide compound or a pharmaceutically acceptable salt, stereoisomer, solvate or prodrug thereof in the preparation of a medicament for preventing and/or treating a disease.

In another respect, the present invention provides a method for treating and/or preventing a RIPK1-related disease or disorder, comprising the step of administering to a patient in need thereof a therapeutically effective amount of compound, or a pharmaceutically acceptable salt, stereoisomer, solvate or prodrug thereof, or any combination thereof, or the above pharmaceutical compostition.

As used herein, the term "subject" refers to an aminal, especially a mammal, preferally a human being.

As used herein, the term "effective amount" or "therapeutically effective amount" refers to the sufficient amount of a drug or agent that is non-toxic but has the desired effect. In an embodiment of the present invention, when treating a patient in accordance with the present invention, the amount of a given drug depends on a number of factors, such as the particular dosage regimen, the type of disease or disorder and its severity, and the uniqueness of the subject or the host in need of treatment (e.g., body weight), however, depending on the particular circumstances, including, for example, the particular drug that has been employed, the route of administration, the condition being treated, and the subject or host being treated, the dosage administered can be decided by methods routinely known in the art. Generally, for use in the treatment for an adult, the dosage administered will typically range from 0.02 to 5000 mg/day, for example from about 1 to 1500 mg/day. The desired dose may conveniently be presented as a single dose, or concurrently (or in a short period of time) or in divided doses at appropriate intervals, such as two, three, four or more divided doses per day. It will be understood by those skilled in the art that although the above dosage ranges are given, the specific effective amount can be appropriately adjusted depending on the condition of the patient and in connection with the diagnosis of the physician.

In an embodiment of the present invention, the diease is inflammatory bowel disease, ulcerative colitis, Crohn's disease, psoriasis, rheumatoid arthritis, NASH and heart failure.

In another respect, the present invention provides use of the above heterocyclic amide compound or a pharmaceutically acceptable salt, stereoisomer, solvate or prodrug thereof in the preparation of a selective RIPK1 inhibitor, the selective RIPK1 inhibitor is useful in treating a RIPK1-related disease or disorder

In one embodiment of the present invention, the RIPK1-related disease or disorder includes, but are not limited to: inflammatory disease such as Crohn's disease and ulcerative colitis, inflammatory bowel disease, asthma, graft versus host disease, chronic obstructive pulmonary disease; autoimmune diseases such as Graves disease, rheumatoid arthritis, systemic lupus erythematosus, psoriasis; destructive bone diseases such as bone resorption diseases, osteoarthritis, osteoporosis, multiple myeloma-related bone diseases; hyperplastic diseases such as acute myeloid leukemia, chronic myelogenous leukemia; angiogenesis disorders such as angiogenesis disorders, include solid tumors, ocular neovascularization and infantile hemangiomas; infectious diseases such as sepsis, septic shock and shigellosis; neurodegenerative diseases such as Alzheimer's disease, Parkinson's disease, amyotrophic lateral sclerosis, cerebral ischemia or neurodegenerative diseases caused by or traumatic injury, tumors and viral diseases such as metastatic melanoma, Kaposi's sarcoma, multiple myeloma, HIV infection and CMV retinitis, AIDS.

In one embodiment of the present invention, the RIPK1-related disease or disorder includes, but is not limited to, pancreatitis (acute or chronic), asthma, allergies, adult respiratory distress syndrome, chronic obstructive pulmonary disease, glomerulonephritis, rheumatoid arthritis, systemic lupus erythematosus, scleroderma, Hashimoto's thyroiditis, Graves's disease, autoimmune gastritis, diabetes, autoimmune hemolytic anemia, autoimmune neutropenia, thrombocytopenia, atopic dermatitis, chronic active hepatitis, myasthenia gravis, amyotrophic lateral sclerosis, multiple sclerosis, inflammatory bowel disease, ulcerative colitis, Crohn's disease, psoriasis, graft versus host disease, inflammatory responses induced by endotoxins, tuberculosis, atherosclerosis, muscular degeneration, cachexia, psoriatic arthritis, Reiter's syndrome, gout, traumatic arthritis, rubella arthritis, acute synovitis, pancreatic beta cell disease; diseases characterized by extensive neutrophil infiltration; rheumatoid spondylitis, gouty arthritis and other arthritis conditions, cerebral malaria, chronic pulmonary inflammation, silicosis, pulmonary sarcomatosis, bone resorption diseases, allograft rejection, fever and myalgia induced by infections, cachexia secondary to infections, luteoid formation, scar tissue formation, ulcerative colitis, fever, influenza, osteoporosis, osteoarthritis, acute myeloid leukemia, chronic myelogenous leukemia, metastatic melanoma, Kaposi's sarcoma, multiple myeloma, sepsis, septic shock and Shigellosis; Alzheimer's disease, Parkinson's disease, cerebral ischemia or neurodegenerative diseases caused by traumatic injury; angiogenesis disorders, including solid tumors, ocular neovascularization, and infantile hemangiomas; viral diseases, including acute hepatitis infections (including Hepatitis A, B and C), HIV infection and CMV retinitis, AIDS, ARC or malignant tumor and herpes; stroke, myocardial ischemia, stroke heart attack, ischemia of organs, vascular proliferation, heart and kidney reperfusion injury, thrombosis, cardiac hypertrophy, the platelet aggregation induced by thrombin, entotoxemia and/or toxic shock syndrome, prostaglandin-endoperoxide synthase-2-related disorders and pemphigus vulgaris.

In one embodiment of the present invention, the RIPK1-related disease or disorder is selected from the group consisting of inflammatory bowel disease, Crohn's disease and ulcerative colitis, allograft rejection, rheumatoid arthritis, psoriasis, ankylosing spondylitis, psoriatic arthritis and pemphigus vulgaris. Alternatively, preferred diseases are selected from ischemia reperfusion injuries, including cerebral ischemia reperfusion injury induced by stroke and myocardial ischemia reperfusion injury induced by myocardial infarction.

As used therein, the term "pharmaceutically acceptable salts" refers to salts of the the compound of the compound which are pharmaceutically acceptable, and have the pharmacological activity of the parent compound. The type of pharmaceutical acceptable salts includes: acid addition salts formed with inorganic acids (such as hydrochloric acid, hydrobromic acid, sulfuric acid, nitric acid, phosphoric acid, and the like) or organic acids (such as acetic acid, propionic acid, hexanoic acid, cyclopentanepropionic acid, glycolic acid, pyruvic acid, trifluoroacetic acid, formic acid, lactic acid, malonic acid, succinic acid, malic acid, maleic acid, fumaric acid, tartaric acid, citric acid, benzoic acid, cinnamic acid, mandelic acid, methanesulfonic acid, ethanesulfonic acid, benzenesulfonic acid, naphthalene sulfonic acid, camphor sulfonic acid, gluconic acid, glutamic acid, hydroxynaphthalamic acid, salicylic acid, stearic acid, muconic acid and the like); or salts formed when an acidic proton present in the parent compound either is replaced by a metal ion such as an alkali metal ion or alkaline earth ion, or coordinates with an organic base such as ethanolamine, diethanolamine, triethanolamine, N-methylglucamine, and the like. The pharmaceutically acceptable salts of the present invention can be synthesized from the parent compound containing acid radicals or base radicals by conventional chemical methods. In general, such salts are prepared by the reaction of these compounds in a form of free acid or base with a stoichiometric amount of the appropriate base or acid in water or an organic solvent or a mixture of the both. In general, non-aqueous media such as ether, ethyl acetate, ethanol, isopropanol or acetonitrile are preferred. In addition to salt forms, the compounds provided herein also exist in prodrug forms. The prodrugs of the compounds described herein are readily chemically altered under physiological conditions to be converted into the compounds of the invention. In addition, prodrugs can be converted to the compounds of the present invention by chemical or biochemical methods in an *in vivo* environment.

As used herein, the term "solvate" refers to a substance formed by combining the compound of the invention with a pharmaceutically acceptable solvent. Pharmaceutical acceptable solvates include water, ethanol, acetic acid and the like. The solvates include stoichiometric solvates and non-stoichiometric solvates, preferably hydrates. Certain compounds of the present invention may be present in unsolvated or solvated forms, including hydrated forms. In general, solvated forms are equivalent to unsolvated forms and both are included within the scope of the present invention.

As used herein, the term "stereoisomers" include both conformational and configurational isomers, of which configurational isomers mainly include cis-trans isomers and optical isomers. The compound of the present invention may be present in a stereoisomeric form, and thereby cover all possible stereoisomeric forms, and any combination thereof or any mixture thereof, such as, a single enantiomer, a single non-enantiomer, or a mixture thereof. Where the compound of the invention contains an olefinic double bond, it includes a cis-isomer and trans-isomer, and any combination thereof, unless otherwise specified.

As used herein, the term "alkyl" refers to a liner or branched aliphatic hydrocarbon group having 1 to 20 carbon atoms. The term "C₁₋₁₀ alkyl" refers to a liner or branched alkyl group having 1 to 10 carbon atoms, more preferably 1, 2, 3, 4, 5 or 6 carbon atoms, i.e., C₁₋₆ alkyl, more preferably, C₁₋₄ alkyl, the most preferably, C₁₋₃ alkyl. Specific examples include, but are not limited to, methyl, ethyl, *n*-propyl, *iso*-propyl, *n*-butyl, *iso*-butyl, tert-butyl, sec-butyl, *n*-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl and various branched isomers thereof.

As used herein, the term "alkoxy" refers to a group having a structure of -O-alkyl, wherein the alkyl is defined as above. The term "C₁₋₁₀ alkoxy" refers to an alkoxy group having 1 to 10 carbon atoms, preferably, C₁₋₆ alkoxy, more preferably, C₁₋₄ alkoxy, the most preferably, C₁₋₃ alkoxy. Specific examples include, but are not limited to, methoxy, ethoxy, *n*-propoxy, iso-propoxy, *n*-butoxy, *tert*-butoxy, *sec-*butoxy, *n*-pentoxy and the like.

As used herein, the term "alkenyl" refers to an alkyl as defined above having one or more carbon-carbon double bond at any position of the chain. The term "C₂₋₈ alkenyl" refers to an alkenyl group having 2 to 8 carbon atoms and at least one carbon-carbon double bond, prefereably, an alkenyl group having 2 to 6 carbon atoms and 1 to 2 carbon-carbon double bond, i.e., C₂₋₆ alkenyl, more preferably, an alkenyl group having 2 to 4 carbon atoms and 1 to 2 carbon-carbon double bond, i.e., C₂₋₄ alkenyl. Specific examples include, but are not limited to, vinyl, 1-propenyl, 2-propenyl, 1-, 2- or 3-butenyl, pentenyl, hexenyl, butadienyl, and the like.

As used herein, the term "alkynyl" refers to an alkyl as defined above having one or more carbon-carbon triple bond at any position of the chain. The term "C₂₋₈ alkynyl" refers to an alkynyl group having 2 to 8 carbon atoms and at least one carbon-carbon triple bond, prefereably, an alkynyl group having 2 to 6 carbon atoms and 1 to 2 carbon-carbon triple bond, i.e., C₂₋₆ alkynyl, more preferably, an alkynyl group having 2 to 4 carbon atoms and 1 to 2 carbon-carbon triple bond, i.e., C₂₋₄ alkynyl. Specific examples include, but are not limited to, ethynyl, 1-propynyl, 2-propynyl, 1-, 2- or 3-butynyl, and the like.

As used herein, the term "halogen" refers to fluoro, chloro, bromo and iodine.

As used herein, the term "haloalkyl" refers to an alkyl as defined above which is substituted by one or more (1, 2, 3, 4 or 5) halogens. The term "halo C₁₋₁₀ alkyl" refers to a haloalkyl having 1 to 10 carbon atoms, preferably, halo C₁₋₆ alkyl, more preferably, halo C₁₋₄ alkyl, most preferably, halo C₁₋₃ alkyl. Specific examples include, but are not limited to, chloromethyl, dichloromethyl, trichloromethyl, chloroethyl, 1,2-dichloroethyl, trichloroethyl, bromoethyl, fluoromethyl, difluoromethyl, trifluoromethyl, fluoroethyl, difluoroethyl, trifluoroethyl, and the like.

As used herein, the term "haloalkoxy" refers to an alkoxy as defined above which is substituted by one or more (1, 2, 3, 4 or 5) halogens. The term "halo C₁₋₁₀ alkoxy" refers to a haloalkoxy having 1 to 10 carbon atoms, preferably, halo C₁₋₆ alkoxy, more preferably, halo C₁₋₄ alkoxy, most preferably, halo C₁₋₃ alkoxy. Specific examples include, but are not limited to, trifluoromethoxy, trifluoroethoxy, fluoromethoxy, fluoroethoxy, difluoromethoxy, difluoroethoxy, and the like.

As used herein, the term "cycloalkyl" and "cycloalkyl ring" are used exchangeably to refer to a saturated monocyclic or polycyclic fused cyclic hydrocarbyl. The term "C₃₋₂₀ cycloalkyl" refers to a cycloalkyl having 3 to 20 carbon atoms, including monocyclic cycloalkyl, spirocycloalkyl, fused cycloalkyl and briged cycloalkyl, preferably, C₃₋₁₂ cycloalkyl. In the present invention, a cyclic carbon atom in a cycloalkyl may be optionally replaced by 1, 2 or 3 oxo group(s) to form a cyclic ketone structure. The term "C₃₋₈ monocyclic cycloalkyl" and "C₃₋₈ cycloalkyl" refer to a saturated monocyclic cycloalkyl having 3 to 8 carbon atoms, preferably, C₃₋₆ monocyclic cycloalkyl (i.e., C₃₋₆ cycloalkyl), more preferably, C₃, C₄, C₅ or C₆ monocyclic cycloalkyl. Specific examples of monocyclic cycloalkyl include, but are not limited to, cyclopropy, cyclobutyl, cyclopentyl, cyclohexyl, cycloheptyl, cyclooctyl, and the like. As used herein, the term "spirocycloalkyl" and "spirocycloalkyl ring" refer to a polycyclic cycloalkyl formed by sharing a carbon atom (referred to as spiro atom) between two or more single rings. Depending on the number of the spiro atoms shared between the rings, the spirocycloalkyls are divided into monospirocycloalkyl, bispirocycloalkyl and polyspirocycloalkyl. As used herein, the term "fused cycloalkyl" and "fused cycloalkyl ring" refer to a polycyclic cycloalkyl formed by sharing a pair of adjacent carbon atoms between two or more single rings, and may be divided into bicyclic, tricyclic, tetracyclic or polycyclic fused cycloalkyl depending on the number of the formed ring(s). The cycloalkyl ring may be fused to an aryl ring, a heteroaryl ring or a heterocyclyl ring, wherein the ring attached to the parent structure is the cycloalkyl ring. Non-limiting examples include indanyl, tetralyl, benzocycloheptyl, and the like. In the present invention, each of the above types of cycloalkyl may be optionally substituted, where the substituent(s) are preferably one or more substituents as described in the present disclosure.

As used herein, the term "halocycloalkyl" refers to a cycloalkyl as defined above which is substituted by one or more (1, 2, 3, 4 or 5) halogens. The term "halo C₃₋₈ cycloalkyl" refers to a halocycloalkyl having 3 to 8 carbon atoms, preferably, halo C₃₋₆ cycloalkyl, more preferably, halo C₃, halo C₄, halo C₅, or halo C₆ cycloalkyl. Specific examples include, but are not limited to, trifluorocyclopropyl, fluorocyclopropyl, fluorocyclohexyl, difluorocyclopropyl, difluorocyclohexyl, and the like.

As used herein, the term "heterocyclyl" and "heterocyclyl ring" are used exchangeably to refer to a saturated or partially unsaturated monocyclic or polycyclic fused cyclic hydrocarbyl. The term "C₃₋₈ monocyclic heterocyclyl", "3- to 8-membered monocyclic heterocyclyl" and "3- to 8-membered monocyclic heterocyclyl ring" refer to a saturated or partially unsaturated monocyclic hydrocarbyl having 3 to 8 ring atoms wherein 1, 2 or 3 ring atoms are heteroatoms selected from the group consisting of nitrogen, oxygen or S(O)ₘ (wherein m is an integer from 0 to 2). The heterocyclyl ring does not contain a cyclic moity of -O-O-, -O-S- or -S-S-, and the remaining ring atoms are each carbon. Preferred is a 3- to 6-membered monocyclic heterocyclyl having 3 to 6 ring atoms, wherein 1 or 2 ring atoms are heteroatoms (i.e., C₃₋₆ monocyclic heterocyclyl or 3- to 6-membered saturated or partially unsaturated monocyclic heterocyclyl). More preferred is a 5- or 6-membered monocyclic heterocyclyl having 5 or 6 ring atoms, wherein 1 or 2 ring atoms are heteroatoms. If the heteroatom is a nitrogen atom, the nitrogen atom may be substituted or unsubstituted (i.e., N or NR, R is hydrogen or another substituent as defined herein). If the heteroatom is a sulfur atom, the sufur atom may be optionally oxidized (i.e., S(O)ₘ, m is an integer from 0 to 2). The carbon atom in the ring of the monocyclic heterocyclyl may be optionally replaced by 1, 2 or 3 oxo group(s) to form a cyclic ketone, cyclic lactone or cyclic lactam. Specific examples of the monocyclic heterocyclyl includes, but are not limited to, aziridine, oxirane, azetidine, azetidin-2-one, oxetane, oxetan-2-one, oxazolidine, pyrrolidin-2-one, pyrrolidin-2,5-dione, 1,3-dioxolane, dihydrofuran-2(3H)-one, dihydrofuran-2,5-dione, piperidin-2-one, piperidin-2,6-dione, tetrahyro-2H-pyran-2-one, imidazolidine, tetrahydrofuran, tetrahydrothiophene, tetrahydropyrrole, 1,3-dioxolan-2-one, oxazolidin-2-one, imidazolidine-2-one, piperidine, piperazine, piperazin-2-one, morpholine, morpholin-3-one, morpholin-2-one, thiomorpholin-3-one 1,1-dioxide, thiomorpholine, thiomorpholine-1,1-dioxide, tetrahydropyran, 1,2-dihydroazacyclobutadiene, 1,2-dihydrooxetadiene, 2,5-dihydro-1H-pyrrole, 2,5-dihydrofuran, 2,3-dihydrofuran, 2,3-dihydro-1H-pyrrole, 3,4-dihydro-2H-pyran, 1,2,3,4-tetrahydropyridine, 3,6-dihydron-2H-pyran, 1,2,3,6-tetrahydropyridine, 1,3-oxazinane, hexahydropyrimidine, 1,4-dioxane, tetrahydropyrimidin-2(1H)-one, 1,4-dioxan-2-one, 5,6-dihydro-2H-pyran-2-one, 5,6-dihydropyrimid-4(3H)-one, 3,4-dihydropyrid-2(1H)-one, 5,6-dihydropyrid-2(1H)-one, 5,6-dihydropyrimid-4(1H)-one, pyrimid-4(3H)-one, pyrimid-4(1H)-one, 4,5-dihydro-1H-imidazole, 2,3-dihydro-1H-imidazole, 2,3-dihydrooxazole, 1,3-dioxole, 2,3-dihydrothiophene, 2,5-dihydrothiophene, 3,4-dihydro-2H-1,4-oxazine, 3,4-dihydro-2H-1,4-thiazine 1,1-dioxide, 1,2,3,4-tetrahydropyrazine, 1,3-dihydro-2H-pyrrol-2-one, 1,5-dihydro-2H-pyrrol-2-one, 1H-pyrrol-2,5-dione, furan-2(3H)-one, furan-2(5H)-one, 1,3-dioxol-2-one, oxazole-2(3H)-one, 1,3-dihydro-2H-imidazole-2-one, furan-2,5-dione, 3,6-dihydropyrid-2(1H)-one, pyrid-2,6-(1H,3H)-dione, 5,6-dihydro-2H-pyran-2-one, 3,6-dihydro-2H-pyran-2-one, 3,4-dihydro-2H-1,3-oxazine, 3,6-dihydro-2H-1,3-oxazine , 1,2,3,4-tetrahydroopyrimidine, and the like. The adjacent two ring atoms in the above monocyclic heterocyclyl, including C-C, N-C, may be optionally fused to the cylcoalkyl, heterocyclyl, aryl or heteroaryl as defined herein, such as, monocyclic cycloalkyl ring, monocyclic heterocyclyl ring, monoaryl ring, 5- or 6-membered monoheteroaryl ring and the like, to form a fused polycyclyl. The adjacent two ring atoms in the above monocyclic heterocyclyl fused to another ring is preferably C-C. In the present invention, each of the above types of heterocyclyl may be optionally substituted. If substituted, the substituent(s) are preferably one or more substituents as described in the present disclosure.

As used herein, the terms "C₆₋₁₄ aryl", "C₆₋₁₄ aryl ring" and "C₆₋₁₄ aromatic ring" are used interchangeably to refer to all-carbon monocyclyl, all-carbon polycyclyl (a ring is linked to another by a covalent bond, non-fused) or all-carbon fused polycyclyl (i.e., a pair of adjacent carbon atoms are shared between the ring) groups having 6 to 14 ring atoms, wherein at least one ring is aromatic, that is, has a π electron conjugated system. The C₆₋₁₀ aryl is preferred. The C₆₋₁₄ aryl group in the present invention includes monocyclic aryl, polyocyclic aryl and aromatic fused polycyclyl. Among others, examples of monocyclic aryl group include phenyl, and examples of polycyclic aryl include biphenylyl, and the like.

In the present invention, if the C₆₋₁₄ aryl is an aromatic fused polycyclyl, the aromatic fused polycyclyl maybe a polycyclyl group formed by fusing a monoaryl ring to one or more monoaryl rings. Non-limiting examples include naphthyl, anthryl, and the like.

In certain embodiments of the present invention, the aromatic fused polycyclyl may be a polycyclyl group formed by fusing a monoaryl ring (such as phenyl) to one or more non-aromatic rings, wherein the ring attached to the parent structure is an aromatic or non-aromatic ring. The non-aromatic ring includes, but is not limited to, 3- to 6-membered monocyclic heterocyclyl ring (preferably a 5- or 6-membered monocyclic heterocyclyl ring, the carbon atom in the ring of the monocyclic heterocyclyl may be replace by 1 or 2 oxo groups to form a structure of cyclic lactam or cyclic lactone), 3- to 6-membered monocyclic cycloalkyl ring (preferably 5- or 6-membered monocyclic cycloalkyl ring, the carbon atom in the ring of the monocyclic cycloalkyl may be replace by 1 or 2 oxo goups to form a structure of cyclic ketone). The above polycyclyl group formed by fusing a monoaryl ring to one or more non-aromatic rings may be linked to other moiety or the parent structure through a nitrogen atom or carbon atom. The ring attached to the parent structure is an aromatic or non-aromatic ring.

In the present disclosure, the 9- or 10-membered aromatic fused dicyclyl formed by fusing a phenyl to a 5- or 6-membered monocyclic heterocyclyl ring means that the adjacent two substitutes in the phenyl together with the ring atom attached thereto form a fused 5- or 6-membered monocyclic heterocyclyl ring defined as above. The formed the 9- or 10-membered aromatic fused dicyclyl may also be referred to as a 9- or 10-membered phenylheterocyclyl ring.

In the present disclosure, the 9- or 10-membered aromatic fused dicyclyl formed by fusing a phenyl to a 5- or 6-membered monocyclic cycloalkyl ring means that the adjacent two substitutes in the phenyl together with the ring atom attached thereto form a fused 5- or 6-membered monocyclic cycloalkyl ring defined as above. The formed the 9- or 10-membered aromatic fused dicyclyl may also be referred to as a 9- or 10-membered phenylcycloalkyl ring. The non-limiting examples include:

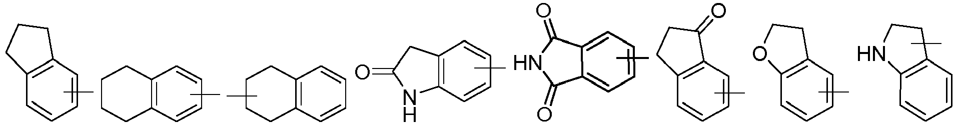

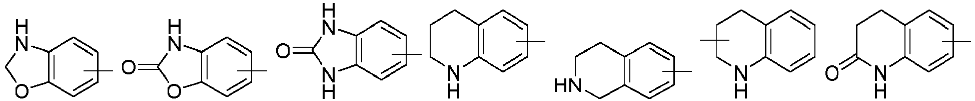

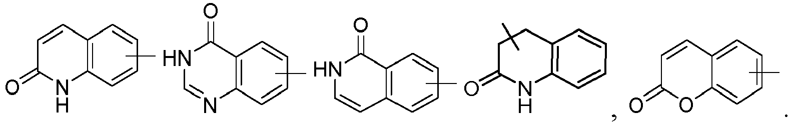

In the present invention, each of the above types of aryl may be substituted or unsubstituted. If substituted, the substituent(s) are preferably one or more substituents as described in the present disclosure.

As use herein, the tems "heteroaryl", "heteroaryl ring" and "heteroarylcycle" are used exchangably to refer to a monocyclic or fused polycyclic group having a ring atom being replaced by at least one heterotom selected from the group consisting of nitrogen, oxygen or sulfur (i.e., a pair of adjacent ring atoms, which may be C-C or N-C, are shared), wherein the nitrogen and sulfur atoms may be optionally oxidized, and the nitrogen atom may be optionally quaternized. The heteroaryl group has 6, 10 or 14 π electrons as shared, and in the group least one ring is aromatic. The terms "C₅₋₁₄ heteroaryl" and 5- to 14-membered heteroaryl" refer to a heteroaryl having 5 to 14 ring atoms wherein 1, 2, 3 or 4 ring atoms are heteroatoms, preferably, a heteroaryl having 5 to 10 ring atoms wherein 1, 2, 3 or 4 ring atoms are heteroatoms. The C₅₋₁₄ heteroaryl in the present invention may be a monoheteroaryl, fused dicyclic heteroaryl or fused tricyclic heteroaryl.

As used therein, the term "5- or 6-membered monoheteroaryl" refers to a monocyclic heteroaryl having 5 or 6 ring atoms, wherein 1, 2 or 3 ring atoms are heteroatom. Specific examples of monoheteroaryl include, but are not limited to, thiophene, furan, thiazole, isothiazole, imidazole, oxazole, pyrrole, pyrazole, triazole, 1,2,3-triazole, 1,2,4-triazole, 1,2,5-triazole, 1,3,4-triazole, tetrazole, isoxazole, oxadiazole, 1,2,3-oxadiazole, 1,2,4-oxadiazole, 1,2,5-oxadiazole, 1,3,4-oxadiazole, thiadiazole, pyridine, pyridazine, pyrimidine, pyrazine and the like.

As used herein, the term "8- to 10- membered biheteroaryl" refers to a fused bicyclic heteroaryl having 8 to 10 ring atoms, wherein 1, 2, 3, 4 or 5 ring atoms are heteroatoms. The fused bicyclic heteroaryl may be either a bicyclic group (prefererably, 9- or 10-membered biheteroaryl ring) formed by fusing a monoaryl ring (such as phenyl) to a monoheteroaryl ring (preferably, 5- or 6-membered monoheteroaryl ring), or a bicyclic group formed by fusing a monoheteroaryl ring (preferably, 5- or 6-membered monoheteroaryl ring) to a monoheteroaryl ring (preferably, 5- or 6-membered monoheteroaryl ring).

Any 2 adjacent ring atoms in the above monoheteroaryl ring, including C-C, N-C, N-N, may be fused to the cycloalkyl, heterocyclyl, aryl or heteroaryl such as the monocyclic cycloalkyl ring, monocyclic heterocyclyl ring, monoaryl ring, 5- or 6-membered monoheteroaryl ring and the like as define in the present disclosure, to form a fused polycyclyl. The 2 adjacent ring atoms in the monoheteroaryl ring, which are fused to another ring to form a fused ring, are preferably C-C, and include in a non-limiting way the forms of: 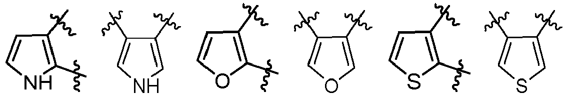 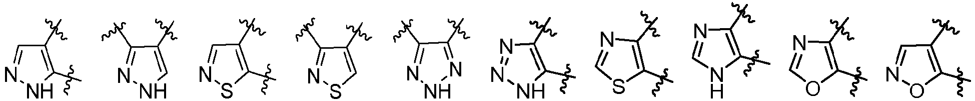 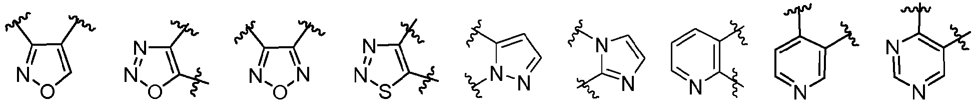 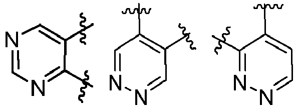 and

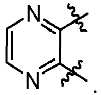

Non-limiting examples of 8- to 10- membered biheteroaryl include benzo[d]isoxazole, 1H-indole, isoindole, 1H-benzo[d]imidazole, benzo[d]isothiazole, 1H-benzo[d][1,2,3]triazole, benzo[d]oxazole, benzo[d]thiazole, indazole, benzofuran, benzo[b]thiophene, quinoline, isoquinoline, quinazoline, quinoxaline, cinnoline, pyrido[3,2-d]pyrimidine, pyrido[2,3-d]pyrimidine, pyrido[3,4-d]pyrimidine, pyrido[4,3-d]pyrimidine, 1,8-naphthyridine, 1,7-naphthyridine, 1,6-naphthyridine, 1,5-naphthyridine, pyrazolo[1,5-a]pyrimidine, imidazolo[1,2-b]pyridazine, and the like.

The above monoheteroaryl, or the biheteroaryl formed by fusing a phenyl to a monoheteroaryl ring, or the biheteroaryl formed by fusing a monoheteroaryl ring to a monoheteroaryl ring may be linked to other moiety or the parent structure through a nitrogen atom or carbon atom. In the case of a biheteroaryl, the ring linked to the parent structure is a monoheteroaryl ring or phenyl ring. Specific examples include, but are not limited to:

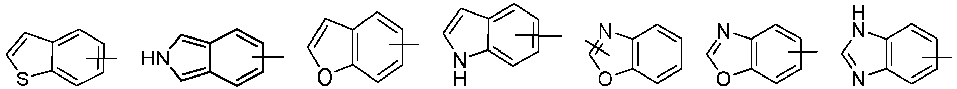

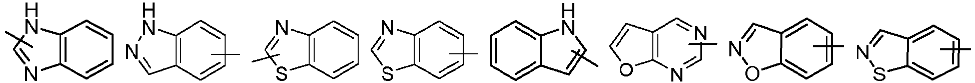

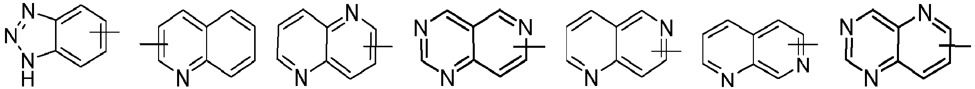

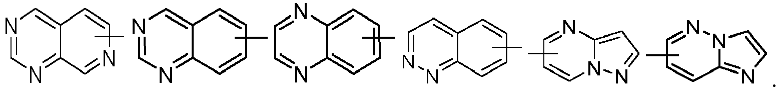

In certain embodiments of the present invention, the fused bicyclic heteroaryl or fused tricyclic heteroaryl may be a polycyclic group formed by fusing a monoheteroaryl ring (preferably, 5- or 6-membered monoheteroaryl ring) to one or more non-aromatic ring, wherein the ring linked to the parent structure is the monoheteroaryl ring or the non-aromatic ring. The non-aromatic ring includes, but is not limited to, 3- to 6- membered monocyclic heterocyclyl ring (preferably, 5- or 6-membered monocyclic heterocyclyl ring, the ring carbon atom in the monocyclic heterocyclyl ring may be replaced by 1 to 2 oxo groups, to form a structure of cyclic lactam or cyclic lactone), 3- to 6-membered monocyclic cycloalkyl ring (preferably, 5- or 6-membered monocyclic cycloalkyl ring, the ring carbon atom in the monocyclic cycloalkyl ring may be replace by 1 to 2 oxo, to form a structure of cyclic ketone) and the like. The polycyclic group formed by fusing a monoheteroaryl ring to one or more non-aromatic ring may be linked to other moiety or the parent structure through a nitrogen atom or carbon atom, and the ring linked to the parent structure is a monoheteroaryl ring or non-aromatic ring.

In the present disclosure, the 8- to 10-membered fused bicyclic heteroaryl formed by fusing a 5- or 6-membered monoheteroaryl ring to a 5- or 6-membered monocyclic heterocyclyl ring means that the adjacent two substitutes in the 5- or 6-membered monoheteroaryl together with the ring atom attached thereto form a fused 5- or 6-membered monocyclic heterocyclyl ring as defined above. The formed the 8-to 10-membered fused bicyclic heteroaryl may also be referred to as 8- to 10-membered heteroaryl heterocyclyl ring.

In the present disclosure, the 8- to 10-membered fused bicyclic heteroaryl formed by fusing a 5- or 6-membered monoheteroaryl ring to a 5- or 6-membered monocyclic cycloalkyl ring means that the adjacent two substitutes in the 5- or 6-membered monoheteroaryl together with the ring atoms attached thereto form a fused 5- or 6-membered monocyclic cycloalkyl ring as defined above. The formed the 8- to 10-membered fused bicyclic heteroaryl may also be referred to as 8- to 10-membered heteroaryl cycloalkyl ring. Non-limiting examples include:

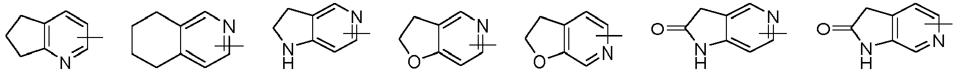

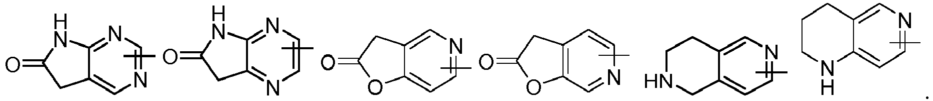

In the present invention, each of the above types of heteroaryl may be substituted or unsubstituted. When substituted, the substituent(s) are preferably one or more substituents as described in the present disclosure.

As used herein, the term "3- to 6-membered saturated or partially unsaturated monocyclic cycloalkyl" refers to a saturated or partially unsaturated (such as, including one or two double bonds) all-carbon monocyclic group having 3 to 6 ring atoms. The ring carbon atoms in the monocyclic cycloalkyl may be optionally replaced by 1, 2 or 3 oxo groups, to form a structure of cyclic ketone. Examples of 3-to 6-membered saturated or partially unsaturated monocyclic cycloalkyl include (but are not limited to): cyclopropyl, cyclobutyl, cyclopentyl, cyclopentenyl, cyclohexyl, cyclohexenyl, cyclohexdienyl, cyclobutanone, cyclobutan-1,2-dione, cyclopentanone, cyclopentan-1,3-dione, cyclohexanone, cyclohexan-1,3-dione, and the like.

As used herein, the term "hydroxyl" refers to -OH.

As used herein, the term "hydroxylmethyl" refers to -CH₂OH, and "hydroxyethyl" refers to -CH₂CH₂OH or -CH(OH)CH₃.

As used herein, the term "cyanomethyl" refers to -CH₂CN, and "cyanoethyl" refers to -CH₂CH₂CN or -CHCNCH₃.

As used herein, the term "aminio" refers to -NH₂.

As used herein, the term "cyano" refers to -CN.

As used herein, the term "nitro" refers to -NO₂.

As used herein, the term "benzyl"refers to -CH₂-phenyl.

As used herein, the term "oxo group"refers to =O.

As used herein, the term "carboxyl"refers to -C(O)OH.

As used herein, the term "carboxylic ester group"refers to -C(O)O(alkyl) or - C(O)O(cycloalkyl).

As used herein, the term "acetyl"refers to -COCH₃.

As used herein, the term "substituted" means that any one or more hydrogen atoms on a particular atom are replaced with substituents, including deuterium and hydrogen variants, as long as the valence of a particular atom is normal and the substituted compound is stable. When the substituent is an oxo group (i.e., =O), it means that two hydrogen atoms are replaced. Replacement of an oxo group does not occur on aromatic groups. The term "optionally substituted" means that it may or may not be substituted. Unless otherwise specified, the type and number of substituents may be arbitrary on the basis of being chemically achievable.

When any variant (e.g., R) occurs more than once in the constitution or structure of a compound, its definition in each case is independent. Thus, for example, if a group is substituted by 0-2 R, the group may optionally be substituted with up to two R, and R in each case has an independent option. In addition, combinations of substituents and/or variants thereof are permissible only if such combinations result in stable compounds.

### Detailed Description of the Invention

The test results show that the compound of the present invention as an RIPK1 inhibitor has high inhibitory activity on both enzymes and cells, wherein the IC₅₀ value for U937 cells is 0.001µM - 1µM, or 0.001µM - 0.3µM, or 0.001µM - 0.1µM. Therefore, the compound of the present invention demonstrates great potential for the development as a therapeutic drug and is very promising to be developed into a drug.

The compound of the present invention can be prepared by a variety of synthetic methods well known to those skilled in the art, including the specific embodiments listed below, the embodiments obtained by combining those listed below with other chemical synthesis methods, and the equivalent alternatives well known to those skilled in the art. Preferred embodiments includ, but are not limited to, the Examples of the present invention.

The present invention is illustrated in details by means of the Examples, but is not meant to be unfavorably limited thereto. In the present disclosure, the present invention has been described in details, wherein specific embodiments thereof are also disclosed. It will be apparent to those skilled in the art that various changes and modifications can be made to the specific embodiments of the present invention without departing from the spirit and scope of the present invention. If specific conditions are not indicated in the Examples, it shall be carried out in accordance with conventional conditions or those recommended by the manufacturer. The reagents or instruments without their manufacturers indicated are all conventional products that can be purchased commercially. PTLC is preparative thin layer chromatography.

### Synthesis of Methyl 5-bromo-1-methyl-2-oxo-1,2-dihydropyridine-3-carboxylate

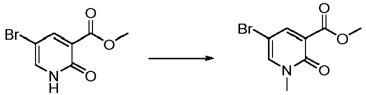

Step 1: Methyl 5-bromo-2-oxo-1,2-dihydropyridine-3-carboxylate (1.0g, 4.31mmol), methyl iodide (0.81ml, 12.93mmol), potassium carbonate (1.19g, 8.62mmol), methanol (12ml) and *N,N*-dimethylformamide (12ml) were added into a 100mL round-bottomed flask. The reaction was carried out overnight at room temperature. Thereafter, 50ml water was added. The resultant mixture was extracted with 50ml ethyl acetate for three times. The organic phases were combined, dried over anhydrous sodium sulfate, and concentrated to give methyl 5-bromo-1-methyl-2-oxo-1,2-dihydropyridine-3-carboxylate (404mg, yield (hereinafter referred to as Y): 31%). ES-API: [M+H]⁺=247.0.

### Synthesis of (2-(cyclopentylmethoxy)-6-fluorophenyl)methylamine

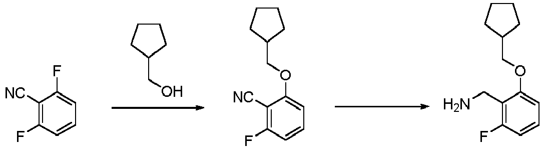

Step 1: 20mL of anhydrous *N,N-*dimethylformamide and sodium hydride (790mg, 18.10mmol), and finally 2,6-difluorobenzene (2.0g, 14.48mmol) were added into a 50mL three-necked round-bottomed flask under the protection of nitrogen gas. The mixture was stirred at room temperature for 30 minutes. Then, cyclopentyl methanol (1.45g, 14.48 mmol) was added in batches. After the addition, the mixtue was slowly warmed up to 50°C, and stirred for another 5 hours. After the reaction mixture was cooled to room temperature, 80mL of ethyl acetate was added into the system. The resultant mixture was washed twice with saturated ammonium chloride and sodium chloride sequentially (60mL×2). The phase of ethyl acetate was dried over anhydrous sodium sulfate, and then filtered. The filtrate was spin-dred to give the target compound of 2-(cyclopentylmethoxy)-6-fluorobenzonitrile (3.0g, Y: 95%). ES-API: [M+H]⁺=219.

Step 2: 10mL Anhydrous tetrahydrofuran, 2-(cyclopentylmethoxy)-6-fluorobenzonitrile (500mg, 2.293mmol), and finally borane-dimethyl sulfide complex (11.46ml, 22.93 mmol) were added in a 50mL three-necked round-bottomed flask under the protection of nitrogen gas. The resultant mixture was stirred at room temperature for 1 hour, and then was heated under reflux for another 3 hours. After the reaction mixture was cooled to room temperature, 40mL 1M hydrochloric acid solution was slowly added dropwise. The resultant mixture was concentrated under reduced pressure so that about 20mL of solvant was left. The concentrate was washed once with 40mL dichloromethane. The aqueous phase was adjusted to be basic with sodium bicarbonate, and then was extracted twice with 60mL of ethyl acetate (60mL×2). The phase of ethyl acetate was dried over anhydrous sodium sulfate and filtered. The filtrate was spin-dried, and purified with PTLC [petroleum ether: ethyl acetate=1:9, volume ratio (referred to as v/v hereinafter)] to give the target compound of (2-(cyclopentylmethoxy)-6-fluorophenyl)methylamine (602mg, crude product). ES-API: [M+H]⁺=224.

### Synthesis of (2-(cyclopentyloxy)-5-fluorophenyl)methylamine

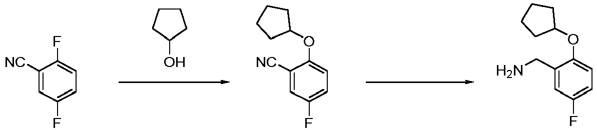

Step 1: 12ml of anhydrous *N,N*-dimethylformamide, sodium hydride (395mg, 9.051mmol), and finally 2,5-difluorobenzene (1.0g, 7.241mmol) were added into a 50mL three-necked round-bottomed flask under the protection of nitrogen gas. The mixture was stirred at room temperature for 30 minutes. Then, cyclopentanol (1.45 g, 16.95 mmol) was added in batches. After the addition, the mixtue was slowly warmed up to 50°C, and stirred for another 5 hours. After the reaction mixture was cooled to room temperature, 80mL of ethyl acetate was added into the system. The resultant mixture was washed twice with saturated ammonium chloride and sodium chloride sequentially (60mL×2). The phase of ethyl acetate was dried over anhydrous sodium sulfate, and then filtered. The filtrate was spin-dred to give the target compound of 2-(cyclopentyloxy)-5-fluorobenzonitrile (1.52 g, crude product). ES-API: [M+H]⁺=206.1.

Step 2: 10mL of anhydrous tetrahydrofuran, 2-(cyclopentyloxy)-5-fluorobenzonitrile (600mg, 2.353mmol), and finally borane-dimethyl sulfide complex (11.7mL, 23.53mmol) were added into a 50mL three-necked round-bottomed flask under the protection of nitrogen gas. The resultant mixture was stirred at room temperature for 1 hour, and then was heated under reflux for another 3 hours. After the reaction mixture was cooled to room temperature, 50mL of 1M hydrochloric acid solution was slowly added dropwise. The resultant mixture was concentrated under reduced pressure so that about 30mL of solvant was left. The concentrate was washed once with 50mL of dichloromethane. The aqueous phase was adjusted to be basic with sodium bicarbonate, and then was extracted twice with 70mL of ethyl acetate (70mL×2). The phase of ethyl acetate was dried over anhydrous sodium sulfate, and then filtered. The filtrate was spin-dried, and purified with PTLC [petroleum ether:ethyl acetate=1:9, v/v] to give the target compound of (2-(cyclopentyloxy)-5-fluorophenyl)methylamine (200mg, Y: 37%). ES-API:[M+H]⁺= 210.

### Synthesis of 3-(4-fluorobenzyl)piperidine

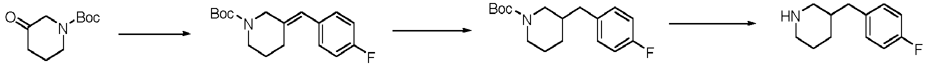

Step 1: (4-Fluorobenzyl)triphenylphosphine chloride (4.9g, 12.045mmol) was added to a solution of potassium *tert*-butoxide (1.239g, 11.042mmol) in tetrahydrofuran (30ml) in batches. The reaction mixture was stirred at room temperature for 45 minutes. Next, A solution of tert-butyl 3-oxopiperidine-1-carboxylate (2g, 10.038mmol) in tetrahydrofuran (20ml) was slowly added dropwise to the above solution while maintaining internal temperature of the reaction solution at 20°C. After the addition, the reaction solution was stirred overnight at room temperature. As the LCMS shows that the reaction was completed, the reaction solution was added to ethyl acetate (50 ml) and water (50 ml) in batches. The aqueous phase was extracted with ethyl acetate (60 ml) for 3 times. The organic phases were combined, dried and filtered. The filtrate was concentrated to obtain a crude product, which was purified by column chromatography (ethyl acetate : petroleum ether = 1:50~1:10) to give tert-butyl 3-(4-fluorobenzylidene)piperidine-1-carboxylate as a yellow liquid (335mg, Y: 10%). ES-API: [M+H]⁺= 292.1.

Step 2: 72 mg of 10% Pd/C was added to a solution of tert-butyl 3-(4-fluorobenzylidene)piperidine-1-carboxylate (335mg, 1.151 mmol) in ethanol (15 ml) under the protection of hydrogen gas. The reaction solution was stirred overnight at room temperature. The resultant mixture was filtered through celite, and concentrated to give a crude product of tert-butyl 3-(4-fluorobenzyl)piperidine-1-carboxylate (334 mg, Y: 73%). ES-API: [M+H]⁺= 294.1.

Step 3: tert-Butyl 3-(4-fluorobenzyl)piperidine-1-carboxylate (334 mg, 1.140 mmol) was dissolved in 6 mL of 4N hydrogen chloride in dioxane. The reaction solution was stirred at room temperature for 2 hours, and then concentrated to give a crude product of 3-(4-fluorobenzyl)piperidine (220 mg, Y: 100%). ES-API: [M+H]⁺= 194.1.

### Synthesis of (2-(cyclopentyloxy)-6-fluorophenyl)methylamine

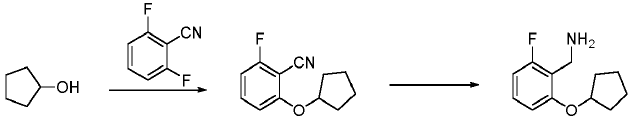

Step 1: A solution obtained by dissolving cyclopentanol (1.5g, 17.416mmol) in 30 ml of tetrahydrofuran was cooled to 0°C in an ice-water bath. Sodium hydride (766mg, 19.157mmol) was added to the above solution in batches. The reaction solution was stirred at 0°C for 1 hour. Next, 2,6-difluorobenzonitrile (2.423g, 17.416mmol) was added. The reaction solution was heated to 50°C and stirred overnight. As the LCMS shows that the reaction was completed, the reaction was quenched in an ice-water bath. The resultant mixture was extracted with 50 mL of ethyl acetate for three times. The organic phases were combined, washed with 30 mL of saturated brine for three times, dried, and concentrated to give a crude product of 2-(cyclopentyloxy)-6-fluorobenzonitrile (2.6 g, Y: 73%). ES-API: [M+H]⁺= 206.1.

Step 2: 2-(Cyclopentyloxy)-6-fluorobenzonitrile (1.1g, 5.366mmol) was dissolved in tetrahydrofuran (25 mL). A solution of 2 M borane in tetrahydrofuran (13.5mL) was added at room temperature. The reaction solution was heated under reflux overnight. As the LCMS shows that the reaction was completed, the reaction solution was cooled to room temperature. Methanol was slowly added dropwise to quench the reaction. The reaction solution was concentrated. The crude product was dissolved in IN hydrochloric acid (25mL). The resultant solution was extracted with 30 mL of dichloromethane for three times, and concentrated to give a crude product of (2-(cyclopentyloxy)-6-fluorophenyl)methylamine (680mg, Y: 61%). ES-API: [M+H]⁺= 210.1.

### Synthesis of (2-(cyclopropylmethoxy)phenyl)methanamine hydrochloride

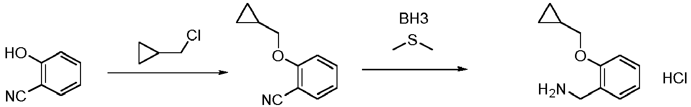

Step 1: 2-Hydroxybenzonitrile (2.0g, 16.79mmol) and acetonitrile (100ml) were added to a 250mL round-bottomed flask. The mixtue was stirred at room temperature. Next, (chloromethyl)cyclopropane (2.13g, 23.51mmol) and potassium carbonate (9.28g, 67.16mmol) were added. The reaction was carried out overnight at 75°C. Then, the reaction mixture was cooled to room temperature, and was suction filtered. The filtrate obtained by washing the filter cake with 100ml of ethyl acetate for three times was concentrated to give a crude product of 2-(cyclopropylmethoxy)benzonitrile (596mg , Y: 20%).

Step 2: 2-(Cyclopropylmethoxy)benzonitrile (596mg, 3.45mmol) and tetrahydrofuran (20ml) were added to a 100mL three-necked round-bottomed flask, and stirred at room temperature. Next, borane-dimethyl sulfide complex (2M, 5.72ml, 11.44mmol) were slowly added dropwise. The reaction was carried out at 85°C for 1 hour. After the reaction mixture was cooled to room temperature, diluted hydrochloric acid (1M, 11.44ml, 11.44mmol) was added dropwise. The reaction was carried out at 85°C for another 30 minutes. The resultant reaction mixture was cooled to room temperatue, and concentrated at reduce pressure to give a crude product of (2-(cyclopropylmethoxy)phenyl)methylamine hydrochloride (350 mg, Y: 50%).

### Example 1: Synthesis of (S or R)-5-(2-aminobenzothiazol-6-yl)-2-methoxy-N-(1-(2-(trifluoromethoxy)phenyl)ethyl)nicotinamide

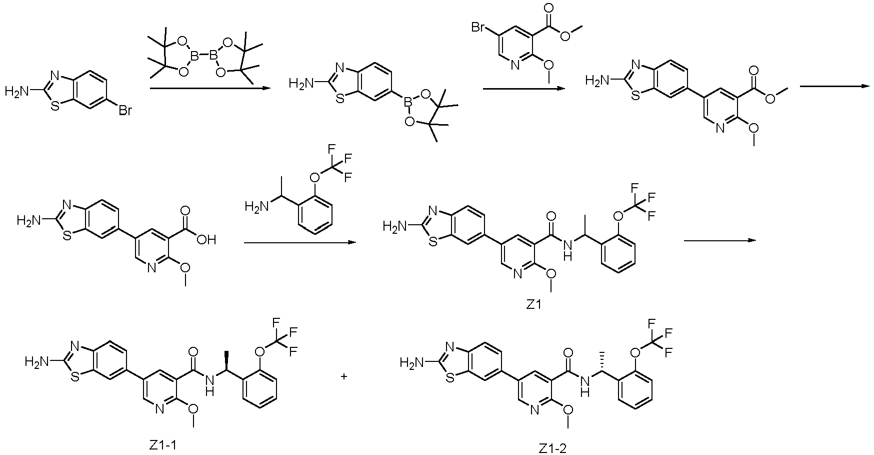

Step 1: 2-Amino-6-bromobenzothiazole (3.0g, 13.1mmol), bis(pinacolato)diboron (6.65g, 26.2mmol), potassium acetate (3.21g, 32.74mmol), [1,1'-bis(diphenylphosphino)ferrocene]dichloropalladium-dichlorom ethane complex (1g, 1.31mmol), and 1,4-dioxane (60ml) were added to a 250mL round-bottomed flask. The mixture was nitrogen-purged for three times. The reaction was carried out at 100°C for 8 hours. Then, the reaction mixture was cooled to room temperature, and was suction filtered. The filtrate obtained by washing the filter cake with ethyl acetate for three times was concentrated to give a crude product of 6-(4,4,5,5-tetramethyl-1,3,2-dioxaborolan-2-yl)benzothiazol-2-amine (3.8g, Y: 100%). ES-API: [M+H]⁺=277.2

Step 2: Methyl 5-bromo-2-methoxynicotinate (2.0g, 8.13mmol), 6-(4,4,5,5-tetramethyl-1,3,2-dioxaborolan-2-yl)benzothiazol-2-amine (3.37g, 12.19mmol), sodium carbonate (2.15g, 20.32mmol), [1,1'-bis(diphenylphosphino)ferrocene]dichloropalladium-dichloromethane complex (663mg, 0.813mmol), dimethoxyethane (100ml), and water (20ml) were added to a 250mL round-bottomed flask. The mixture was nitrogen-purged for three times. The reaction was carried out overnight at 80°C. Then, the reaction mixture was cooled to room temperature, and was suction filtered. 100mL water was added to a filtrate obtained by washing the filter cake with ethyl acetate for three times. The resultant mixture was exracted with 100mL of ethyl acetate for 3 times. The organic phase was dried, concentrated, and subjected to column chromatography (dichloromethane:methanol=15:1) to give methyl 5-(2-aminobenzo[d]thiazol-6-yl)-2-methoxynicotinate (1.8g, Y: 73%), ES-API: [M+H]⁺=316.1.

Step 3: Methyl 5-(2-aminobenzo[d]thiazol-6-yl)-2-methoxynicotinate (800mg, 2.54mmol), lithium hydroxide monohydrate (533mg, 12.7mmol), tetrahydrofuran (10ml), methanol (2ml), and water (2ml) were added to a 100mL round-bottomed flask. The reaction was carried out at room temperature for 3 hours. After concentrating the solvent, 10 ml water was added to the flask. Next, the reaction was adjusted to pH=5 by adding 1M diluted hydrochloric acid. The solvent was concentrated. The concentrate was vacuum-dried to give a crude product of 5-(2-aminobenzo[d]thiazol-6-yl)-2-methoxynicotinic acid (912mg, Y:100%), ES-API: [M+H]⁺=302.1.

Step 4: 5-(2-Aminobenzo[d]thiazol-6-yl)-2-methoxynicotinic acid (912mg, 3mmol), 1-(2-(trifluoromethoxy)phenyl)ethyl-1-amine (738mg, 3.6mmol), ((benzotriazol-1-yl)oxy)tris(dimethylamino)phosphonium hexafluorophosphate (2.0g, 4.5mmol), triethylamine (1.2 ml, 9mmol), and *N,N*-dimethylformamide (10ml) were added to a 100mL round-bottomed flask. The reaction was carried out overnight at room temperature. 50mL of ethyl acetate was added to the reaction mixture. The resultant mixture was washed with 30mL of water for three times. The organic phases were combined, dried over anhydrous sodium sulfate, and concentrated. The obtained concentrate was subjected to a column chromatography (dichloromethane:methanol=15:1) to give 5-(2-aminobenzothiazol-6-yl)-2-methoxy-*N*-(1-(2-(trifluoromethoxy)phenyl)ethyl)nicotinamide (380mg, Y:32%). ES-API: [M+H]⁺=489.0, ¹H NMR (400 MHz, CD₃OD) δ 8.53 (d, *J* = 2.6 Hz, 1H), 8.40 (d, *J* = 2.6 Hz, 1H), 7.84 (d, *J* = 1.8 Hz, 1H), 7.61 - 7.54 (m, 1H), 7.50 (d, *J* = 1.9 Hz, 0H), 7.48 (d, *J* = 1.9 Hz, 1H), 7.43 (d, *J* = 8.4 Hz, 1H), 7.39 - 7.34 (m, 2H), 7.31 (ddd, *J* = 5.8, 4.5, 2.7 Hz, 1H), 5.51 (q, *J* = 7.0 Hz, 1H), 4.58 (s, 1H), 4.12 (s, 3H), 1.55 (d, *J* = 7.0 Hz, 3H).

Step 5: The above compound of 5-(2-aminobenzothiazol-6-yl)-2-methoxy-*N*-(1-(2-(trifluoromethoxy)phenyl)ethyl)nicotinamide Z1 (100mg, 0.2mmol) was subjected to chiral resolution by SFC (co-solvent: methanol (ammonia containing 0.2% methanol); column type: (R,R)-Whelk-O1 (4.6^{∗}100mm^{∗}5um); flow rate: 1.4 ml/min; temperature: 40°C) to give the compounds (Z1-1 and Z1-2): (S)-5-(2-aminobenzothiazol-6-yl)-2-methoxy-*N*-(1-(2-(trifluoromethoxy)phenyl)ethyl) nicotinamide (an arbitrarily assigned absolute configuration, 33mg, ee value: >99%, peak 1, retention time: 2.15 min). ES-API: [M+H]⁺=489.1. (R)-5-(2-aminobenzothiazol-6-yl)-2-methoxy-*N*-(1-(2-(trifluoromethoxy)phenyl) ethyl) nicotinamide (an arbitrarily assigned absolute configuration, 28mg, ee value: >99%, peak 2, retention time: 3.09 min). ES-API: [M+H]⁺=489.1.

### Example 2: Synthesis of 5-(2-aminobenzo[d]thiazol-6-yl)-2-methoxy-N-(2-(trifluoromethoxy)benzyl)nicotinamide

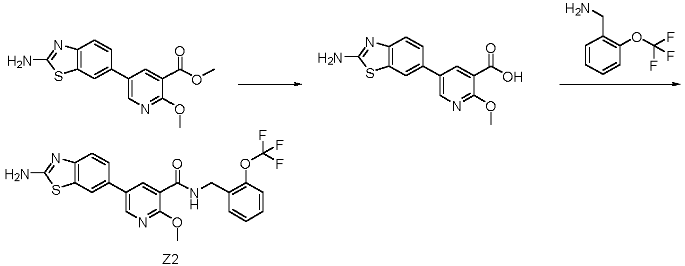

Step 1: Methyl 5-(2-aminobenzo[d]thiazol-6-yl)-2-methoxynicotinate (600mg, 1.905mmol), lithium hydroxide monohydrate (762mg, 19.05mmol), 10mL of tetrahydrofuran, 10mL of methanol, and 5mL of water were added to a 50mL round-bottomed flask. The reaction was carried out at room temperature for 3 hours. As monitored by TLC [dichloromethane:methanol=10:1, v/v] when the spot for the starting materials disappeared, the reaction was terminated. The reaction system was spin-dried under reduced pressure; thereafter, 6mL water was added. The reaction system was cooled to 0°C in an ice-water bath. Hydrogen chloride solution (4M in dioxane) was added dropwise to adjust the pH to 5-6. 40ml of toluene was added. The resultant mixture was spin-dried under reduced pressure, to give a crude product of 5-(2-aminobenzo[d]thiazol-6-yl)-2-methoxynicotinic acid (900 mg, crude product) ES-API: [M+H]⁺=302.1.

Step 2: 5-(2-Aminobenzo[d]thiazol-6-yl)-2-methoxynicotinic acid (225mg, 0.4762mmol) and 15mL *N,N-*dimethylformamide were added to a 50mL round-bottomed flask. Next, (2-(trifluoromethoxy)phenyl)methylamine (137mg, 0.7413mmol), triethylamine (480mg, 4.672mmol), and BOP reagent (330mg, 0.7467mmol) were added sequentially. The reaction was carried out overnight at room temperature under the protection of nitrogen gas. 80mL of ethyl acetate was added to the system. The resultant mixture was washed with saturated brine for 5 times (50mL×5). The organic phase of ethyl acetate was dried over anhydrous sodium sulfate and filtered, and the filtrate was spin-dried to give 5-(2-aminobenzo[d]thiazol-6-yl)-2-methoxy-*N*-(2-(trifluoromethoxy)benzyl)nlcotinamide Z2 (54.1mg, Y: 23%). ES-API:[M+H]⁺=475.1.¹H NMR (400 MHz, DMSO-d6) δ 8.87 (t, *J* = 6.0 Hz, 1H), 8.58 (d, *J* = 2.5 Hz, 1H), 8.33 (d, *J* = 2.5 Hz, 1H), 8.00 (d, *J* = 1.8 Hz, 1H), 7.55 (s, 2H), 7.49 (td, *J* = 9.1, 2.5 Hz, 2H), 7.42 - 7.31 (m, 4H), 4.56 (d, *J* = 6.0 Hz, 2H), 4.00 (s, 3H).

### Example 3: Synthesis of 5-(2-aminobenzo[d]thiazol-6-yl)-2-methoxy-N-(1-(3-(trifluoromethoxy)phenyl)ethyl)nicotinamide

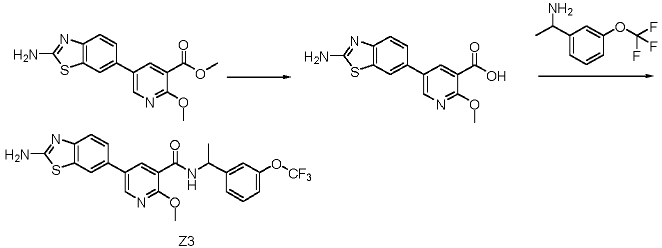

Step 1: Methyl 5-(2-aminobenzo[d]thiazol-6-yl)-2-methoxynicotinate (600mg, 1.905mmol), lithium hydroxide monohydrate (762mg, 19.05mmol), 10mL of tetrahydrofuran, 10mL of methanol, and 5mL of water were added to a 50mL round-bottomed flask. The mixture was stirred for reaction at room temperature for 3 hours. As monitored by TLC [dichloromethane:methanol=10:1, v/v] when the spot for the starting materials disappeared, the reaction was terminated. The reaction system was spin-dried under reduced pressure; thereafter, 6mL of water was added. The reaction system was cooled to 0°C in an ice-water bath. Hydrogen chloride solution (4M in dioxane) was added dropwise to adjust the pH to 5-6. 40ml of toluene was added. The resultant mixture was spin-dried under reduced pressure, to give a crude product of 5-(2-aminobenzo[d]thiazol-6-yl)-2-methoxynicotinic acid (900 mg, crude product). ES-API: [M+H]⁺=302.1.

Step 2: 5-(2-Aminobenzo[d]thiazol-6-yl)-2-methoxynicotinic acid (225mg, 0.4762mmol) and 15mL of *N,N*-dimethylformamide were added to a 50mL round-bottomed flask. Next, 1-(3-(trifluoromethoxy)phenyl)ethyl-1-amine (146.54mg, 0.7143mmol), triethylamine (480mg, 4.672mmol), and BOP reagent (330mg, 0.7467mmol) were added sequentially. The reaction was carried out overnight at room temperature under the protection of nitrogen gas. 80mL of ethyl acetate was added to the system. The resultant mixture was washed with saturated brine for 5 times (50mL×5). The organic phase of ethyl acetate was dried over anhydrous sodium sulfate and filtered, and the filtrate was spin-dried to give 5-(2-aminobenzo[d]thiazol-6-yl)-2-methoxy-*N*-(1-(3-(trifluoromethoxy)phenyl)ethyl) nicotinamide Z3 (40mg, Y:17%). ES-API:[M+H]⁺=489.1. ¹H NMR (400 MHz, DMSO-d6) δ 8.73 (d, *J* = 7.9 Hz, 1H), 8.55 (d, *J* = 2.5 Hz, 1H), 8.15 (d, *J* = 2.5 Hz, 1H), 7.99 (d, J= 1.8 Hz, 1H), 7.61 - 7.33 (m, 7H), 7.21 (d, *J* = 7.2 Hz, 1H), 5.16 (p, *J* = 7.0 Hz, 1H), 3.97 (s, 3H), 1.44 (d, *J* = 7.0 Hz, 3H).

### Example 4: Synthesis of 5-(2-aminobenzo[d]thiazol-6-yl)-2-methoxy-N-(3-(trifluoromethoxy)benzyl)nicotinamide

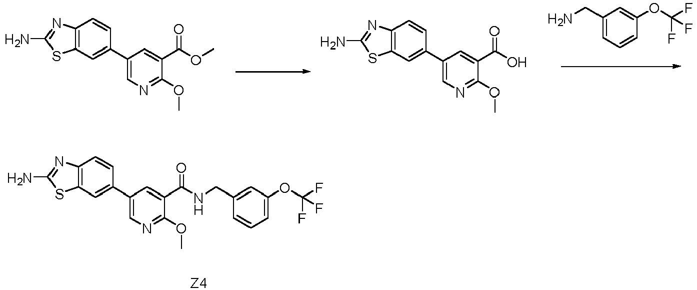

Step 1: Methyl 5-(2-aminobenzo[d]thiazol-6-yl)-2-methoxynicotinate (600mg, 1.905mmol), lithium hydroxide monohydrate (762mg, 19.05mmol), 10mL of tetrahydrofuran, 10mL of methanol, and 5mL of water were added to a 50mL round-bottomed flask. The mixture was stirred for reaction at room temperature for 3 hours. As monitored by TLC [dichloromethane:methanol=10:1, v/v] when the spot for the starting materials disappeared, the reaction was terminated. The reaction system was spin-dried under reduced pressure; thereafter, 6mL of water was added. The reaction system was cooled to 0°C in an ice-water bath. Hydrogen chloride solution (4M in dioxane) was added dropwise to adjust the pH to 5-6. 40ml of toluene was added. The resultant mixture was spin-dried under reduced pressure, to give a crude product of 5-(2-aminobenzo[d]thiazol-6-yl)-2-methoxynicotinic acid (900 mg, crude product). ES-API: [M+H]⁺=302.1.

Step 2: 5-(2-Aminobenzo[d]thiazol-6-yl)-2-methoxynicotinic acid (225mg, 0.4762mmol) and 15mL of *N,N*-dimethylformamide were added to a 50mL round-bottomed flask. Next, (3-(trifluoromethoxy)phenyl)methylamine (137mg, 0.7413mmol), triethylamine (480mg, 4.672mmol), and BOP reagent (330mg, 0.7467mmol) were added sequentially. The reaction was carried out overnight at room temperature under the protection of nitrogen gas. 80mL of ethyl acetate was added to the system. The resultant mixture was washed with saturated brine for 5 times (50mL×5). The organic phase of ethyl acetate was dried over anhydrous sodium sulfate and filtered, and the filtrate was spin-dried to give 5-(2-aminobenzo[d]thiazol-6-yl)-2-methoxy-*N*-(3-(trifluoromethoxy)benzyl)nicotinamide Z4 (56mg, Y: 25%). ES-API:[M+H]⁺=475.1. ¹H NMR (400 MHz, dmso-d6) δ 8.94 (t, *J* = 5.9 Hz, 1H), 8.57 (d, *J* = 2.5 Hz, 1H), 8.29 (d, *J* = 2.5 Hz, 1H), 8.00 (d, *J* = 1.5 Hz, 1H), 7.63 - 7.42 (m, 4H), 7.41 - 7.28 (m, 3H), 7.22 (d, *J* = 7.8 Hz, 1H), 4.54 (d, *J* = 5.9 Hz, 2H), 3.98 (s, 3H).

### Example 5: Synthesis of (S or R)-5-(2-acetylaminobenzothiazol-6-yl)-2-methoxy-N-(1-(2-(trifluoromethoxy)phenyl)ethyl)nicotinamide

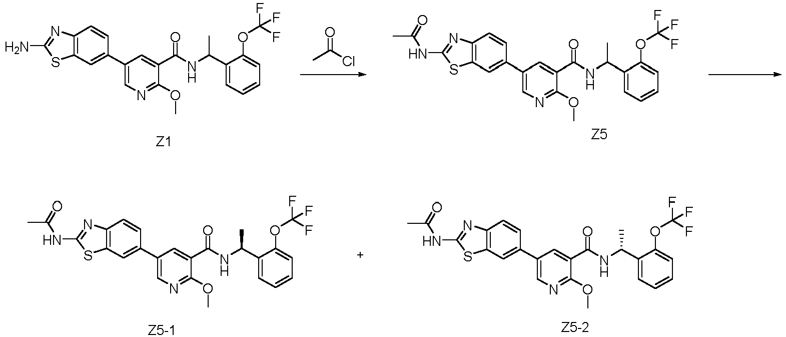

Step 1: 5-(2-Aminobenzothiazol-6-yl)-2-methoxy-*N*-(1-(2-(trifluoromethoxy) phenyl)ethyl)nicotinamide (180mg, 0.37mmol), acetyl chloride (44mg, 0.56mmol), triethylamine (112mg, 1.11mmol), and dichloromethane (5ml) were added to a 50mL round-bottomed flask. The reaction was carried out in an ice-water bath for 5 minutes. 10ml of water was added. The resultant mixture was extracted with 20ml of dichloromethane for three times. The organic phases were combined, dried over anhydrous sodium sulfate. The organic phase was concentrated, and separated by thin layer chromatography (dichloromethane:methanol=15:1) to give 5-(2-acetylaminobenzothiazol-6-yl)-2-methoxy-*N*-(1-(2-(trifluoromethoxy)phenyl)ethyl) nicotinamide Z5 (141mg, Y: 71.9 %). ES-API:[M+H]⁺=531.2, ¹H NMR (400 MHz, DMSO) δ 12.38 (s, 1H), 8.76 (d, *J* = 7.6 Hz, 1H), 8.63 (d, *J* = 2.5 Hz, 1H), 8.30 (s, 1H), 8.25 (d, *J* = 2.5 Hz, 1H), 7.77 (d, *J* = 8.4 Hz, 1H), 7.74 - 7.69 (m, 1H), 7.67-7.57 (m, 1H), 7.38 (dd, *J* = 6.5, 2.8 Hz, 2H), 7.33 (s, 1H), 5.44 - 5.34 (m, 1H), 4.00 (s, 3H), 2.18 (s, 3H), 1.42 (d, *J* = 7.0 Hz, 3H).

Step 2: The above compound of 5-(2-acetylaminobenzothiazol-6-yl)-2-methoxy-*N*-(1-(2-(trifluoromethoxy)phenyl)ethyl)nicotinamide Z5 (125mg, 0.24mmol) was subjected to chiral resolution by SFC (Co-solvent: methanol (ammonia containing 0.2% methanol); column type: (S,S)-WHELK -O1 (4.6^{∗}100mm^{∗}5um); flow rate: 1.4 ml/min; temperature: 40°C) to give the compounds (Z5-1 and Z5-2): (S)-5-(2-acetylaminobenzothiazol-6-yl)-2-methoxy-*N*-(1-(2-(trifluoromethoxy) phenyl) ethyl) nicotinamide (an arbitrarily assigned absolute configuration, 41mg, ee value: 99%, peak 1, retention time: 2.77 min). ES-API: [M+H]⁺= 531.2. (R)-5-(2-acetylaminobenzothiazol-6-yl)-2-methoxy-*N*-(1-(2-(trifluoromethoxy) phenyl) ethyl) nicotinamide (an arbitrarily assigned absolute configuration, 39mg, ee value: 98.9%, peak 2, retention time: 3.72 min). ES-API: [M+H]⁺= 531.2.

### Example 6: Synthesis of (S or R)-5-(2-(cyclopropylcarbonylamino)benzo[d] thiazol-6-yl)-2-methoxy-N-(1-(2-(trifluoromethoxy)phenyl)ethyl)nicotinamide

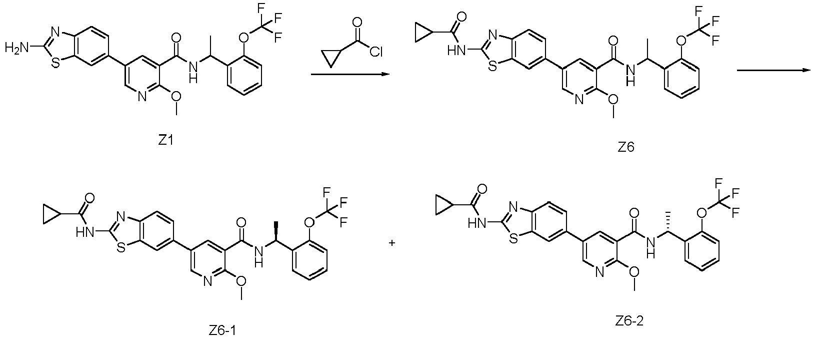

Step 1: 5-(2-Aminobenzothiazol-6-yl)-2-methoxy-*N*-(1-(2-(trifluoromethoxy)phenyl)ethyl)nicotinamide (190mg, 0.39mmol), cyclopropanecarbonyl chloride (61mg, 0.59mmol), triethylamine (118mg, 1.17mmol), and dichloromethane (5ml) were added to a 50mL round-bottomed flask. The reaction was carried out for 5 minutes in an ice-water bath. 10ml of water was added. The resultant mixture was extracted with 20ml of dichloromethane for three times. The organic phases were combined, dried over anhydrous sodium sulfate, and concentrated to give 5-(2-(cyclopropylcarbonylamino)benzo[d]thiazol-6-yl)-2-methoxy-*N*-(1-(2-(trifluoromethoxy)phenyl)ethyl)nicotinamide (145 mg, Y: 66%). ES-API: [M+H]⁺=557.2.

Step 2: The above compound of 5-(2-(cyclopropylcarbonylamino)benzo[d] thiazol-6-yl)-2-methoxy-*N*-(1-(2-(trifluoromethoxy) phenyl) ethyl) nicotinamide Z6 (121mg, 0.22mmol) was subjected to chiral resolution by SFC (Co-solvent: methanol (ammonia containing 0.2% methanol); column type: S,S-WHELK-O1 (4.6^{∗}100mm^{∗}5um); flow rate: 1.4 ml/min; temperature: 40°C) to give the compounds (Z6-1 and Z6-2): (S)-5-(2-(cyclopropylcarbonylamino)benzothiazol-6-yl)-2-methoxy-*N*-(1-(2-(trifluoromethoxy)phenyl)ethyl)nicotinamide (an arbitrarily assigned absolute configuration, 39mg, ee value: 99%, peak 1, retention time: 2.94 min). ES-API: [M+H]⁺=557.2. (R)-5-(2-(cyclopropylcarbonylamino)benzothiazol-6-yl)-2-methoxy-*N*-(1-(2-(trifluoromethoxy) phenyl) ethyl) nicotinamide (an arbitrarily assigned absolute configuration, 38mg, ee value: 98%, peak 2, retention time: 3.99 min). ES-API: [M+H]⁺= 557.2.

### Example 7: Synthesis of 5-(2-aminobenzo[d]thiazol-6-yl)-6-methoxy-N-(1-(2-(trifluoromethoxy)phenyl)ethyl)nicotinamide

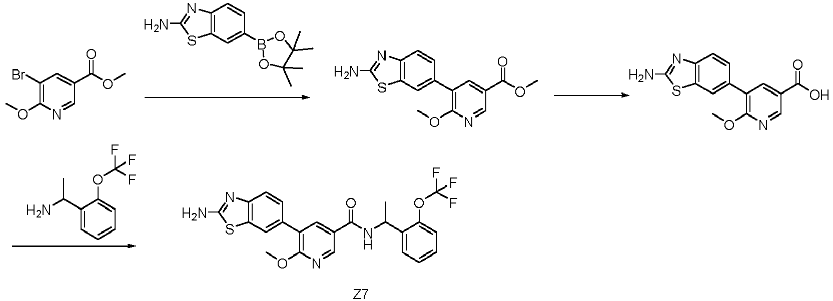

Step 1: Methyl 5-bromo-6-methoxynicotinate (150mg, 0.60971mmol), 6-(4,4,5,5-tetramethyl-1,3,2-dioxaborolan-2-yl)benzo[d]thiazol-2-amine (180mg, 0.6521mmol), [1,1'-bis(diphenylphosphino)ferrocene] dichloropalladium-dichloromethane complex (99.8mg, 0.1219mmol), and sodium carbonate (260mg, 2.452mmol), and finally 15mL of dioxane and 3mL of water were added to a 20mL microwave tube. The tube was purged with nitrogen gas for about 1.5 minutes. The reaction was carried out under microwave at 120°C for 30 minutes. 80mL of ethyl acetate was added to the system. The resultant mixture was washed with saturated brine for 5 times (60mL×5). The phase of ethyl acetate was dried over anhydrous sodium sulfate and filtered. The filtrate was spin-dried and purified by PTLC [dichloromethane:methanol=10:1, v/v] to give methyl 5-(2-aminobenzo[d]thiazol-6-yl)-6-methoxynicotinate (108 mg, Y: 56%). ES-API: [M+H]⁺=315.8.

Step 2: Methyl 5-(2-aminobenzo[d]thiazol-6-yl)-6-methoxynicotinate (108mg, 0.3428mmol), lithium hydroxide monohydrate (137.14mg, 3.428mmol), 5mL of tetrahydrofuran, 5mL of methanol, and 5mL of water were added to a 50mL round-bottomed flask. The mixture was stirred for reaction at room temperature for 3 hours. As monitored by TLC [dichloromethane:methanol=10:1, v/v] when the spot for the starting materials disappeared, the reaction was terminated. The reaction system was spin-dried under reduced pressure; thereafter, 6mL water was added. The reaction system was cooled to 0°C in an ice-water bath. Hydrogen chloride solution (4M in dioxane) was added dropwise to adjust the pH to 5-6. 40ml of toluene was added. The resultant mixture was spin-dried under reduced pressure, to give a crude product of 5-(2-aminobenzo[d]thiazol-6-yl)-6-methoxynicotinic acid (97 mg, crude product). ES-API: [M+H]⁺=301.8.

Step 3: 5-(2-Aminobenzo[d]thiazol-6-yl)-6-methoxynicotinic acid (97mg, 0.3222mmol) and 15mL anhydrous *N,N*-dimethylformamide were added to a 50mL round-bottomed flask. Next, 1-(2-(trifluoromethoxy)phenyl)ethyl-1-amine (99.10mg, 0.4834mmol), triethylamine (325mg, 3.222mmol) and BOP reagent (213.6mg, 0.4834mmol) were added sequentially. The reaction was carried out overnight at room temperature under the protection of nitrogen gas. 90mL Ethyl acetate was added to the system. The resultant mixture was washed with saturated brine for 5 times (60mL×5). The organic phase of ethyl acetate was dried over anhydrous sodium sulfate and filtered, and the filtrate was spin-dried to give 5-(2-aminobenzo[d]thiazol-6-yl)-6-methoxy-*N*-(1-(2-(trifluoromethoxy)phenyl)ethyl)nicotinamide Z7 (36mg, Y:25%). ES-API: [M+H]⁺=488.5. ¹H NMR (400 MHz,DMSO-d6) δ 8.90 (d, *J* = 7.3 Hz, 1H), 8.59 (d, J = 2.3 Hz, 1H), 8.22 (d, *J* = 2.3 Hz, 1H), 7.87 (d, *J* = 1.5 Hz, 1H), 7.61 - 7.54 (m, 3H), 7.42 (dd, *J* = 8.3, 1.8 Hz, 1H), 7.38 - 7.33 (m, 3H), 7.31 (d, *J* = 1.6 Hz, 1H), 5.40 (dd, *J* = 14.4, 7.3 Hz, 1H), 3.91 (s, 3H), 1.42 (d, *J* = 7.0 Hz, 3H).

### Example 8: Synthesis of 5-(2-aminobenzo[d]thiazol-6-yl)-6-chloro-N-(1-(2-(trifluoromethoxy)phenyl)ethyl)nicotinamide

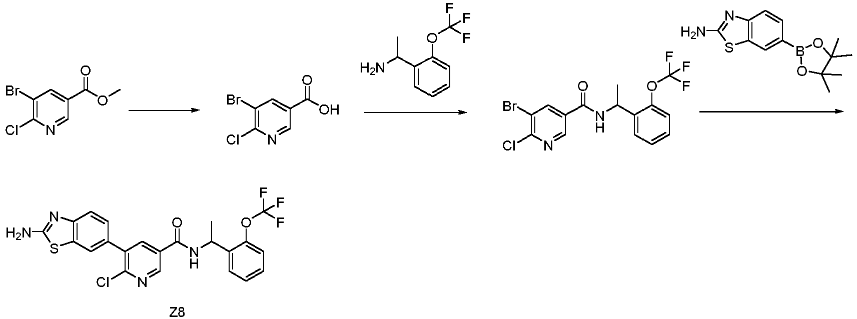

Step 1: Methyl 5-bromo-6-chloronicotinate (200mg, 0.7985mmol), lithium hydroxide monohydrate (319.4mg, 7.985mmol), 3mL of tetrahydrofuran, 3mL of methanol and 3mL of water were added to a 50mL round-bottomed flask. The mixture was stirred overnight at room temperature. As monitored by TLC [dichloromethane: methanol=10:1, v/v] when the spot for the starting materials disappeared, the reaction was terminated. The reaction system was spin-dried under reduced pressure; thereafter, 7mL of water was added. The reaction system was cooled to 0°C in an ice-water bath. Hydrogen chloride solution (4M in dioxane) was added dropwise to adjust the pH to 5-6. 50ml of toluene was added. The resultant mixture was spin-dried under reduced pressure, to give a crude product of 5-bromo-6-chloronicotinic acid (233 mg, crude product). ES-API: [M+H]⁺=201.9.

Step 2: 5-Bromo-6-chloronicotinic acid (230mg, 0.7985mmol) and 20mL of anhydrous *N,N-*dimethylformamide were added to a 50mL round-bottomed flask. Next, 1-(2-(trifluoromethoxy)phenyl)ethyl-1-amine (245.5 mg, 1.197 mmol), triethylamine (806 mg, 7.985 mmol) and BOP reagent (529.38 mg, 1.1977 mmol) were added sequentially. The reaction was carried out at room temperature under the protection of nitrogen gas for 2 hours. After the reacton was completed, 100mL of ethyl acetate was added to the system. The resultant mixture was washed with saturated brine for 5 times (80mL×5). The organic phase of ethyl acetate was dried over anhydrous sodium sulfate and filtered, and the filtrate was spin-dried, followed by PTLC [dichloromethane:methanol=10:1, v/v] to give 5-bromo-6-chloro-*N*-(1-(2-(trifluoromethoxy)phenyl)ethyl)nicotinamide (244mg, Y:72%).

Step 3: 5-Bromo-6-chloro-*N*-(1-(2-(trifluoromethoxy)phenyl)ethyl)nicotinamide (100mg, 0.2358mmol), 6-(4,4,5,5-tetramethyl-1,3,2-dioxaborolan-2-yl)benzo[d] thiazol-2-amine (165mg, 0.2358mmol), [1,1'-bis(diphenylphosphino)ferrocene] dichloropalladium (38mg, 0.05184mmol), and sodium carbonate (75mg, 0.7075mmol), and finally 10mL of dioxane and 2mL of water were added to a 20mL microwave tube. The tube was purged with nitrogen gas for about 2 minutes. The reaction was carried out under microwave at 120°C for 30 minutes. 50mL of ethyl acetate was added to the system. The resultant mixture was washed with saturated brine for 5 times (30mL×5). The phase of ethyl acetate was dried over anhydrous sodium sulfate and filtered. The filtrate was spin-dried to give the target compound 5-(2-aminobenzo[d]thiazol-6-yl)-6-chloro-*N*-(1-(2-(trifluoromethoxy)phenyl)ethyl) nicotinamide Z8 (4.1 mg, Y: 3.56%). ES-API:[M+H]⁺=492.5. ¹H NMR (400 MHz, DMSO-d6) δ 9.15 (d, *J* = 7.1 Hz, 1H), 8.77 (d, *J* = 2.1 Hz, 1H), 8.32 (d, *J* = 2.1 Hz, 1H), 7.82 (s, 1H), 7.64 (s, 2H), 7.60 - 7.55 (m, 1H), 7.44 - 7.26 (m, 5H), 5.48 - 5.33 (m, 1H), 1.42 (d, *J* = 7.1 Hz, 3H).

### Example 9: Synthesis of 5-(2-aminobenzo[d]thiazol-6-yl)-N-(1-(2-(trifluoromethoxy)phenyl)ethyl)nicotinamide

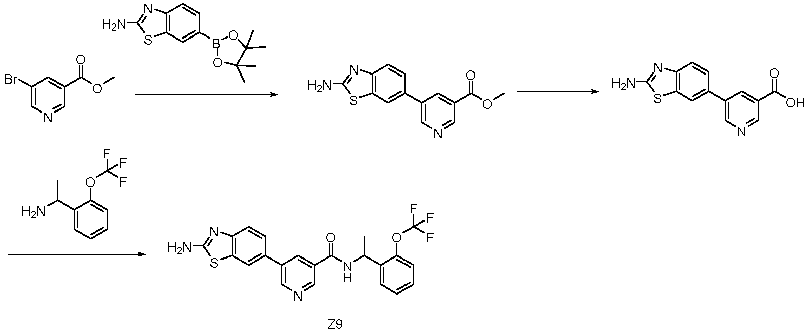

Step 1: Methyl 5-bromonicotinate (300mg, 1.388mmol), 6-(4,4,5,5-tetramethyl-1,3,2-dioxaborolan-2-yl)benzo[d]thiazol-2-amine (462mg, 1.674mmol), [1,1'-bis(diphenylphosphino)ferrocene] dichloropalladium (227mg, 0.9096mmol), and sodium carbonate (441mg, 4.160mmol), and finally 15mL of dioxane and 3mL of water were added to a 20mL microwave tube. The tube was purged with nitrogen gas for about 1.5 minutes. The reaction was carried out under microwave at 110°C for 30 minutes. 80mL of ethyl acetate was added to the system. The resultant mixture was washed with saturated brine for 5 times (60mL×5). The phase of ethyl acetate was dried over anhydrous sodium sulfate and filtered. The filtrate was spin-dried and purified by PTLC [dichloromethane:methanol=10:1, v/v] to give methyl 5-(2-aminobenzo[d]thiazol-6-yl)nicotinate (100 mg, Y: 25%). ES-API: [M+H]⁺=286.1.

Step 2: Methyl 5-(2-aminobenzo[d]thiazol-6-yl)nicotinate (100mg, 0.3508mmol), lithium hydroxide monohydrate (140.3mg, 3.508mmol), 3mL of tetrahydrofuran, 3mL of methanol, and 3mL of water were added to a 50mL round-bottomed flask. The mixture was stirred for reaction at room temperature for 8 hours. As monitored by TLC [dichloromethane:methanol=10:1, v/v] when the spot for the starting materials disappeared, the reaction was terminated. The reaction system was spin-dried under reduced pressure; thereafter, 7mL of water was added. The reaction system was cooled to 0°C in an ice-water bath. Hydrogen chloride solution (4M in dioxane) was added dropwise to adjust the pH to 5-6. 52mL of toluene was added. The resultant mixture was spin-dried under reduced pressure, to give a crude product of 5-(2-aminobenzo[d]thiazol-6-yl)nicotinic acid (83 mg, Y: 87%), ES-API: [M+H]⁺=272.1.

Step 3: 5-(2-aminobenzo[d]thiazol-6-yl)nicotinic acid (83mg, 0.3062mmol) and 16mL of anhydrous *N,N*-dimethylformamide were added to a 50mL round-bottomed flask. Next, 1-(2-(trifluoromethoxy)phenyl)ethan-1-amine (94.2mg, 0.4594mmol), triethylamine (309mg, 3.062mmol), and BOP reagent (203mg, 0.4594mmol) were added sequentially. The reaction was carried out at room temperature under the protection of nitrogen gas for 3 hours. 60mL of ethyl acetate was added to the system. The resultant mixture was washed with saturated brine for 5 times (40mL×5). The organic phase of ethyl acetate was dried over anhydrous sodium sulfate and filtered, and the filtrate was spin-dried to give 5-(2-aminobenzo[d]thiazol-6-yl)-*N*-(1-(2-(trifluoromethoxy) phenyl)ethyl)nicotinamide Z9 (25 mg, Y: 17.8%). ES-API:[M+H]⁺=458.6. ¹H NMR (400 MHz,DMSO-d6)δ 9.12 (d, *J* = 7.3 Hz, 1H), 8.99 (d, *J* = 1.9 Hz, 1H), 8.90 (d, *J* = 1.6 Hz, 1H), 8.46 (s, 1H), 8.11 (s, 1H), 7.62 (d, *J* = 7.9 Hz, 4H), 7.42 (d, *J* = 8.3 Hz, 1H), 7.35 (dd, *J* = 12.7, 10.0 Hz, 3H), 5.50 - 5.37 (m, 1H), 1.46 (d, *J* = 7.0 Hz, 3H).

### Example 10: Synthesis of 5-(2-aminobenzo[d]thiazol-6-yl)-6-methyl-N-(1-(2-(trifluoromethoxy)phenyl)ethyl)nicotinamide

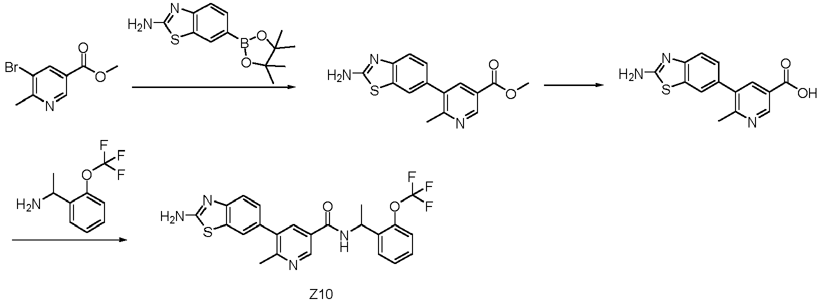

Step 1: Methyl 5-bromo-6-methylnicotinate (300mg, 1.304mmol), 6-(4,4,5,5-tetramethyl-1,3,2-dioxaborolan-2-yl)benzo[d]thiazol-2-amine (540mg, 1.956mmol), [1,1'-bis(diphenylphosphino)ferrocene] dichloropalladium (150mg, 0.2046mmol), sodium carbonate (414.7mg, 3.912mmol), and finally 15mL of dioxane and 3mL of water were added to a 20mL microwave tube. The tube was purged with nitrogen gas for about 1.5 minutes. The reaction was carried out under microwave at 120°C for 30 minutes. 90mL of ethyl acetate was added to the system. The resultant mixture was washed with saturated brine for 5 times (70mL×5). The phase of ethyl acetate was dried over anhydrous sodium sulfate and filtered. The filtrate was spin-dried and purified by PTLC [dichloromethane:methanol=10:1, v/v] to give methyl 5-(2-aminobenzo[d]thiazol-6-yl)-6-methylnicotinate (216 mg, Y: 55.38%). ES-API: [M+H]⁺=300.1.

Step 2: Methyl 5-(2-aminobenzo[d]thiazol-6-yl)-6-methylnicotinate (216mg, 0.7224mmol), lithium hydroxide monohydrate (288.9 mg, 7.224mmol), 5mL of tetrahydrofuran, 5mL of methanol, and 5mL of water were added to a 50mL round-bottomed flask. The mixture was stirred for reaction overnight at room temperature. As monitored by TLC [dichloromethane:methanol=10:1, v/v] when the spot for the starting materials disappeared, the reaction was terminated. The reaction system was spin-dried under reduced pressure; thereafter, 10mL of water was added. The reaction system was cooled to 0°C in an ice-water bath. Hydrogen chloride solution (4M in dioxane) was added dropwise to adjust the pH to 5-6. 60mL of toluene was added. The resultant mixture was spin-dried under reduced pressure, to give a crude product of 5-(2-aminobenzo[d]thiazol-6-yl)-6-methylnicotinic acid (200mg, 97.5%). ES-API: [M+H]⁺=286.1.

Step 3: 5-(2-aminobenzo[d]thiazol-6-yl)-6-methylnicotinic acid (200mg, 0.7017mmol) and 20mL of anhydrous *N,N*-dimethylformamide were added to a 50mL round-bottomed flask. Next, 1-(2-(trifluoromethoxy)phenyl)ethan-1-amine (220mg, 1.073mmol), triethylamine (709mg, 7.017mmol), and BOP reagent (676mg, 1.073mmol) were added sequentially. The reaction was carried out at room temperature under the protection of nitrogen gas for 2 hours. 80mL of ethyl acetate was added to the system. The resultant mixture was washed with saturated brine for 5 times (60mL×5). The organic phase of ethyl acetate was dried over anhydrous sodium sulfate and filtered, and the filtrate was spin-dried to give 5-(2-aminobenzo[d]thiazol-6-yl)-6-methyl-*N*-(1-(2-(trifluoromethoxy)phenyl)ethyl)nicotinamide Z10 (14 mg, Y: 4.2%). ES-API: [M+H]⁺=473.2. ¹H NMR (400 MHz, DMSO-d6) δ 9.02 (d, *J* = 7.2 Hz, 1H), 8.84 (d, *J* = 1.7 Hz, 1H), 8.10 (d, *J* = 1.7 Hz, 1H), 7.74 (d, *J* = 1.1 Hz, 1H), 7.58 (s, 3H), 7.41 - 7.24 (m, 5H), 5.50 - 5.35 (m, 1H), 3.32 (s, 3H), 1.42 (d, *J* = 7.0 Hz, 3H).

### Example 11: Synthesis of 5-(2-amino-[1,2,4]triazolo[1,5-α]pyrid-7-yl)-1-methyl-2-oxo-N-(1-(2-(trifluoromethoxy)phenyl)ethyl)-1,2-dihydropyridine-3-carboxamide

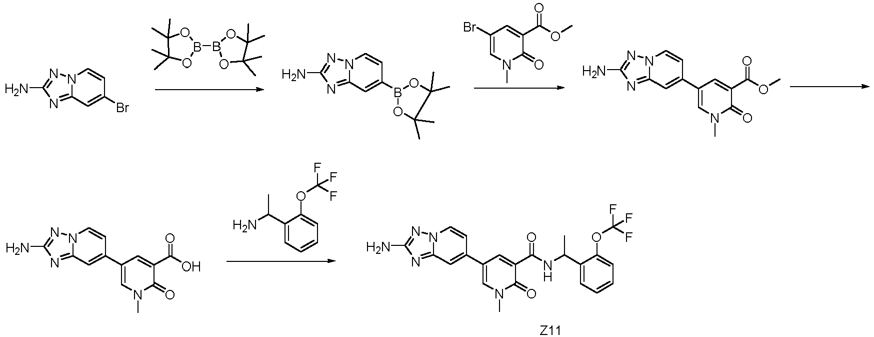

Step 1: 7-Bromo-[1,2,4]triazolo[1,5-*a*]pyridine-2-amine (2.0g, 9.39mmol), bis(pinacolato)diboron (3.58g, 14.08mmol), potassium acetate (1.84g, 18.78mmol), [1,1'-bis(diphenylphosphino)ferrocene]dichloropalladium-dichlorom ethane complex (766mg, 0.939 mmol), and 1,4-dioxane (50ml) were added to a 100mL round-bottomed flask. The mixture was nitrogen-purged for three times. The reaction was carried out at 100°C for 8 hours. Then, the reaction mixture was cooled to room temperature, and was suction filtered. The filtrate obtained by washing the filter cake with ethyl acetate for three times was concentrated to give a crude product of 7-(4,4,5,5-tetramethyl-1,3,2-dioxaborolan-2-yl)-[1,2,4]triazolo[1,5-a]pyridine-2-amine (2.9 g, Y: 100%). ES-API: [M+H]⁺=261.2.

Step 2: Methyl 5-bromo-1-methyl-2-oxo-1,2-dihydropyridine-3-carboxylate (200mg, 0.812mmol), 7-(4,4,5,5-tetramethyl-1,3,2-dioxaborolan-2-yl)-[1,2,4]triazolo [1,5-a]pyridine-2-amine (423mg, 1.63mmol), sodium carbonate (215mg, 2.03mmol), [1,1'-bis(diphenylphosphino)ferrocene]dichloropalladium-dichloromethane complex (62.2mg, 0.0812mmol), 1,4-dioxane (10ml) and water (2ml) were added to a 50mL round-bottomed flask. The mixture was nitrogen-purged for three times. The reaction was carried out overnight at 80°C. Then, the reaction mixture was cooled to room temperature, and was suction filtered. 100mL water was added to a filtrate obtained by washing the filter cake with ethyl acetate for three times. The resultant mixture was exracted with 100mL of ethyl actate for 3 times. The organic phase was dried, concentrated, and separated by thin layer chromatography (dichloromethane:methanol=15:1) to give methyl 5-(2-amino-[1,2,4]triazolo[1,5-a] pyridin-7-yl)-1-methyl-2-oxo-1,2-dihydropyridine-3-carboxylate (78mg, Y: 32%), ES-API: [M+H]⁺=300.2.

Step 3: methyl 5-(2-amino-[1,2,4]triazolo[1,5-a]pyridin-7-yl)-1-methyl-2-oxo-1,2-dihydropyridine-3-carboxylate (78mg, 0.26mmol), lithium hydroxide monohydrate (53mg, 1.3mmol), tetrahydrofuran (5ml), methanol (1ml), and water (1ml) were added to a 50mL round-bottomed flask. The reacton was carried out at room temperature for 3 hours. After concentrating the solvent, 10 ml water was added to the flask. Next, the reaction was adjusted to pH=5 by adding 1M diluted hydrochloric acid. The resultant mixture was concentrated. The concentrate was vacuum-dried to give a crude product of 5-(2-amino-[1,2,4]triazolo[1,5-*a*]pyridin-7-yl)-1-methyl-2-oxo-1,2-dihydropyridine-3-carboxylic acid (79mg, Y:100%). ES-API: [M+H]⁺=286.2.

Step 4: 5-(2-amino-[1,2,4]triazolo[1,5-a]pyridin-7-yl)-1-methyl-2-oxo-1,2-dihydropyridine-3-carboxylic acid (79mg, 0.28mmol), 1-(2-(trifluoromethoxy)phenyl)ethan-1-amine (53mg, 0.26mmol), ((benzotriazol-1-yl)oxy)tris(dimethylamino)phosphonium hexafluorophosphate (219mg, 0.52mmol), triethylamine (0.1 ml, 0.78mmol), and *N,N-*dimethylformamide (5ml) were added to a 50mL round-bottomed flask. The reacton was carried out overnight at room temperature untile the reaction was completed. 50mL of ethyl acetate was added to the reaction mixture. The resultant mixture was washed with 30mL of water for three times. The organic phases were combined, dried over anhydrous sodium sulfate, and concentrated. The obtained concentrate was separated by Prep-HPLC to give 5-(2-amino-[1,2,4]triazolo[1,5-a]pyridin-7-yl)-1-methyl-2-oxo-*N*-(1-(2-(trifluoro methoxy)phenyl)ethyl)-1,2-dihydropyridine-3-carboxamide Z11(38mg, Y:31%). ES-API:[M+H]⁺=473.2, ¹H NMR (400 MHz, CD₃OD) δ 8.74 (d, *J* = 2.8 Hz, 1H), 8.49 (d, *J* = 2.8 Hz, 1H), 8.43 (d, *J* = 7.1 Hz, 1H), 7.58 - 7.49 (m, 2H), 7.40 - 7.28 (m, 3H), 7.18 (dd, *J* = 7.1, 2.0 Hz, 1H), 5.51 (q, *J* = 6.8 Hz, 1H), 3.75 (s, 3H).

### Example 12: Synthesis of 5-(2-Aminobenzo[d]thiazol-6-yl)-N-(2-(cyclopentylmethoxy)-6-fluorobenzyl)-2-methylnicotinamid

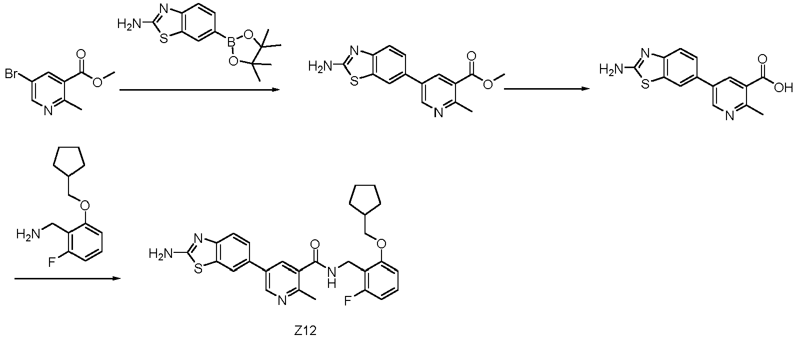

Step 1: Methyl 5-bromo-2-methylnicotinate (300mg, 1.304mmol), 6-(4,4,5,5-tetramethyl-1,3,2-dioxaborolan-2-yl)benzo[d]thiazol-2-amine (432mg, 1.565mmol), [1,1'-bis(diphenylphosphino)ferrocene] dichloropalladium (191mg, 0.2608mmol), sodium carbonate (415mg, 3.912mmol), and finally 15mL of dioxane and 3mL of water were added to a 20mL microwave tube. The tube was purged with nitrogen gas for about 1.5 minutes. The reaction was carried out under microwave at 120°C for 35 minutes. 90mL of ethyl acetate was added to the system. The resultant mixture was washed with saturated brine for 5 times (70mL×5). The phase of ethyl acetate was dried over anhydrous sodium sulfate and filtered. The filtrate was spin-dried and purified by PTLC [dichloromethane:methanol=10:1, v/v] to give methyl 5-(2-aminobenzo[d]thiazol-6-yl)-2-methylnicotinate (217 mg, Y: 55.6%). ES-API: [M+H]⁺ =300.1.

Step 2: Methyl 5-(2-aminobenzo[d]thiazol-6-yl)-2-methylnicotinate(217mg, 0.7257mmol), lithium hydroxide monohydrate (290.3 mg, 7.257mmol), 5mL of tetrahydrofuran, 5mL of methanol, and 5mL of water were added to a 50mL round-bottomed flask. The mixture was stirred for reaction overnight at room temperature. As monitored by TLC [dichloromethane:methanol=10:1, v/v] when the spot for the starting materials disappeared, the reaction was terminated. The reaction system was spin-dried under reduced pressure; thereafter, 10mL of water was added. The reaction system was cooled to 0°C in an ice-water bath. Hydrogen chloride solution (4M in dioxane) was added dropwise to adjust the pH to 5-6. 60mL of toluene was added. The resultant mixture was spin-dried under reduced pressure, to give a crude product of 5-(2-aminobenzo[d]thiazol-6-yl)-2-methylnicotinic acid (252 mg, crude product), ES-API: [M+H]⁺=286.1.

Step 3: 5-(2-aminobenzo[d]thiazol-6-yl)-2-methylnicotinic acid (110mg, 0.3860mmol) and 4.0mL anhydrous of *N,N*-dimethylformamide were added to a 50mL round-bottomed flask. Next, (2-(cyclopentylmethoxy)-6-fluorophenyl)methylamine (164mg, 0.7354mmol), triethylamine (390mg, 3.865mmol), and BOP reagent (324mg, 0.7354mmol) were added sequentially. The reaction was carried out at room temperature under the protection of nitrogen gas for 2 hours. 85mL of ethyl acetate was added to the system. The resultant mixture was washed with saturated brine for 6 times (60mL×6). The phase of ethyl acetate was dried over anhydrous sodium sulfate and filtered, and the filtrate was spin-dried to give 5-(2-aminobenzo[d]thiazol-6-yl)-*N*-(2-(cyclopentylmethoxy)-6-fluorobenzyl)-2-methylnicotinamide Z12 (12.5mg, Y: 6%). ES-API:[M+H]⁺=491.3. ¹H NMR (400 MHz, DMSO-d6)δ 8.73 (d, *J* = 2.3 Hz, 1H), 8.54 (t, *J* = 4.3 Hz, 1H), 8.00 (d, *J* = 1.7 Hz, 1H), 7.83 (d, *J* = 2.2 Hz, 1H), 7.57 (s, 2H), 7.52 (dd, *J* = 8.4, 1.9 Hz, 1H), 7.37 (d, *J* = 8.3 Hz, 1H), 7.26 (dd, *J* = 15.3, 8.3 Hz, 1H), 6.84 (d, *J* = 8.4 Hz, 1H), 6.76 (t, *J* = 8.9 Hz, 1H), 4.46 (d, *J* = 3.9 Hz, 2H), 3.88 (d, *J* = 6.8 Hz, 2H), 2.49 (s, 3H), 2.29 (dt, *J* = 14.7, 7.4 Hz, 1H), 1.74 (d, *J* = 7.8 Hz, 2H), 1.58 - 1.41 (m, 4H), 1.38 - 1.26 (m, 2H).

### Example 13: Synthesis of 5-(2-aminobenzo[d]thiazol-6-yl)-N-(2-(cyclopentyloxy)-5-fluorobenzyl)-6-methoxynicotinamide

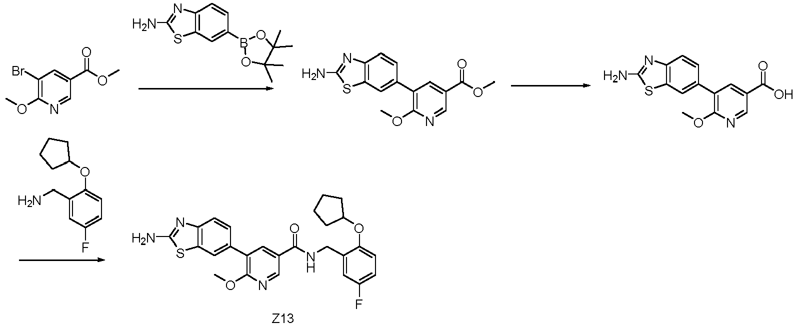

Step 1: Methyl 5-bromo-6-methoxynicotinate (200mg, 0.8130mmol), 6-(4,4,5,5-tetramethyl-1,3,2-dioxaborolan-2-yl)benzo[d]thiazol-2-amine (270mg, 0.9782mmol), [1,1'-bis(diphenylphosphino)ferrocene]dichloropalladium (121.8mg, 0.1660mmol), and sodium carbonate (260mg, 2.440mmol), and finally 12mL of dioxane and 3mL of water were added to a 20mL microwave tube. The tube was purged with nitrogen gas for about 1.5 minutes. The reaction was carried out under microwave at 120°C for 35 minutes. 100mL of ethyl acetate was added to the system. The resultant mixture was washed with saturated brine for 5 times (80mL×5). The phase of ethyl acetate was dried over anhydrous sodium sulfate and filtered. The filtrate was spin-dried and purified by PTLC [dichloromethane:methanol=10:1, v/v] to give methyl 5-(2-amino benzo[d]thiazol-6-yl)-6-methoxynicotinate (174mg, Y: 67.7%). ES-API: [M+H]⁺ =316.

Step 2: Methyl 5-(2-aminobenzo[d]thiazol-6-yl)-6-methoxynicotinate (174mg, 0.5506mmol), lithium hydroxide monohydrate (220mg, 5.506mmol), 5mL of tetrahydrofuran, 5mL of methanol and 5mL of water were added to a 50mL round-bottomed flask. The mixture was stirred for reaction overnight at room temperature. As monitored by TLC [dichloromethane:methanol=10:1, v/v] when the spot for the starting materials disappeared, the reaction was terminated. The reaction system was spin-dried under reduced pressure; thereafter, 10mL of water was added. The reaction system was cooled to 0°C in an ice-water bath. Hydrogen chloride solution (4M in dioxane) was added dropwise to adjust the pH to 5-6. 70mL of toluene was added. The resultant mixture was spin-dried under reduced pressure, to give a crude product of 5-(2-aminobenzo[d]thiazol-6-yl)-6-methoxynicotinic acid (190 mg, crude product), ES-API: [M+H]⁺=302.1.

Step 3: 5-(2-aminobenzo[d]thiazol-6-yl)-6-methoxynicotinic acid (30mg, 0.09966mmol) and 1.0mL of anhydrous *N,N*-dimethylformamide were added to a 50mL round-bottomed flask. Next, (2-(cyclopentyloxy)-5-fluorophenyl)methylamine (31.24mg, 0.150mmol), triethylamine (101mg, 0.9966mmol) and BOP reagent (66.3 mg, 0.150mmol) were added sequentially. The reaction was carried out at room temperature under the protection of nitrogen gas for 12 hours. 50mL of ethyl acetate was added to the system. The resultant mixture was washed with saturated brine for 6 times (30mL×6). The phase of ethyl acetate was dried over anhydrous sodium sulfate and filtered, and the filtrate was spin-dried to give 5-(2-aminobenzo[d]thiazol-6-yl)-*N*-(2-(cyclopentyloxy)-5-fluorobenzyl)-6-methoxynicotinamide Z13 (7.6mg, Y:15%). ES-API:[M+H]⁺=493.3. ¹H NMR (400 MHz, DMSO-d6) δ 8.86 (d, *J* = 5.7 Hz, 1H), 8.63 (d, *J* = 2.3 Hz, 1H), 8.20 (d, *J* = 2.3 Hz, 1H), 7.88 (d, *J* = 1.5 Hz, 1H), 7.55 (s, 2H), 7.44 (dd, *J* = 8.4, 1.7 Hz, 1H), 7.35 (d, *J* = 8.3 Hz, 1H), 7.06 - 6.90 (m, 3H), 4.81 (s, 1H), 4.38 (d, *J* = 5.6 Hz, 2H), 3.92 (s, 3H), 1.83 (d, *J* = 5.4 Hz, 2H), 1.70 (d, *J* = 11.6 Hz, 4H), 1.54 (d, *J* = 2.0 Hz, 2H).

### Example 14: Synthesis of 5-(2-aminobenzo[d]thiazol-6-yl)-N-(2-fluoro-5-(trifluoromethoxy)benzyl)-2-methoxynicotinamide

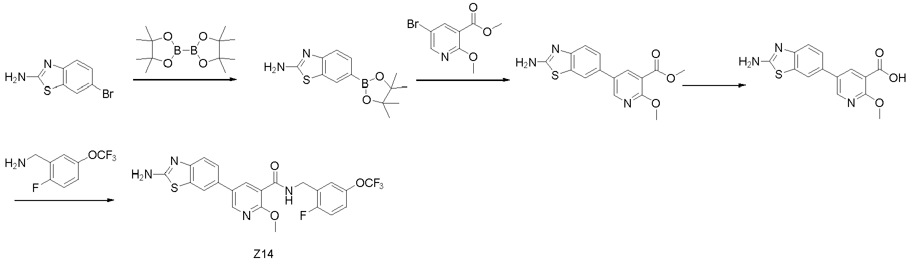

Step 1: 2-Amino-6-bromobenzothiazole (3.0 g, 13.1mmol), bis(pinacolato)diboron (6.65 g, 26.2mmol), potassium acetate (3.21 g, 32.74mmol), [1,1'-bis(diphenylphosphino)ferrocene]dichloropalladium-dichlorom ethane complex (1g, 1.31mmol), and 1,4-dioxane (60ml) were added to a 250mL round-bottomed flask. The mixture was nitrogen-purged for three times. The reaction was carried out at 100°C for 8 hours. Then, the reaction mixture was cooled to room temperature, and was suction filtered. The filtrate obtained by washing the filter cake with ethyl acetate for three times was concentrated to give a crude product of 6-(4,4,5,5-tetramethyl-1,3,2-dioxaborolan-2-yl)benzothiazol-2-amine (3.8g, Y: 100%). ES-API: [M+H]⁺=277.2.

Step 2: Methyl 5-bromo-2-methoxynicotinate (2.0 g, 8.13 mmol), 6-(4,4,5,5-tetramethyl-1,3,2-dioxaborolan-2-yl)benzothiazol-2-amine (3.37 g, 12.19 mmol), sodium carbonate (2.15 g, 20.32 mmol), [1,1'-bis(diphenylphosphino)ferrocene] dichloropalladium-dichloromethane complex (663mg, 0.813mmol), dimethoxyethane (100ml) and water (20ml) were added to a 250mL round-bottomed flask. The mixture was nitrogen-purged for three times. The reaction was carried out overnight at 80°C. Then, the reaction mixture was cooled to room temperature, and was suction filtered. 100mL of water was added to a filtrate obtained by washing the filter cake with 100mL of ethyl acetate for three times. The resultant mixture was extracted with 100 mL of ethyl acetate for 3 times. The organic phase was dried, concentrated, and subjected to column chromatography (dichloromethane:methanol=0~10%) to give methyl 5-(2-aminobenzo[d]thiazol-6-yl)-2-methoxynicotinate (1.8g, Y: 73%), ES-API: [M+H]⁺= 316.1.

Step 3: Methyl 5-(2-aminobenzo[d]thiazol-6-yl)-2-methoxynicotinate (800mg, 2.54mmol), lithium hydroxide monohydrate (533mg, 12.7mmol), tetrahydrofuran (10ml), methanol (2ml) and water (2ml) were added to a 100mL round-bottomed flask. The reacton was carried out at room temperature for 3 hours. After concentrating the solvent, 10 ml water was added to the flask. Next, the reaction was adjusted to pH=5 by adding IN diluted hydrochloric acid. The solvent was concentrated. The concentrate was vacuum-dried to give a crude product of 5-(2-aminobenzo[d]thiazol-6-yl)-2-methoxynicotinic acid (912mg, Y:100%), ES-API: [M+H]⁺ = 302.1.

Step 4: 5-(2-aminobenzo[d]thiazol-6-yl)-2-methoxynicotinic acid (80 mg, 0.266 mmol), (2-fluoro-5-(trifluoromethoxy)phenyl)methylamine (56 mg, 0.266 mmol), ((benzotriazol-1-yl)oxy)tris(dimethylamino)phosphonium hexafluorophosphate (176 mg, 0.399 mmol), triethylamine (80 mg, 0.798 mmol) and *N,N*-dimethylformamide (8 ml) were added to a 100mL round-bottomed flask. The reaction was carried out at room temperature for 2 hours. 15mL of water was added to the reaction mixture. The resultant mixture was extracted with 30mL of ethyl acetate for 3 times. The organic phases were combined, dried over anhydrous sodium sulfate, and filtered. The concentrated organic phase was purified by alkaline HPLC to give 5-(2-aminobenzo[d]thiazol-6-yl)-*N*-(2-fluoro-5-(trifluoromethoxy)benzyl)-2-methoxynicotinamide Z14 as an off-white powder (15mg, Y: 11%). ES-API: [M+H]⁺=493.1. ¹H NMR (400 MHz, DMSO-d6) δ 8.97 (t, *J* = 6.0 Hz, 1 H), 8.62 (d, *J* = 2.4 Hz, 1 H), 8.30 (d, *J* = 2.4 Hz, 1 H), 8.03 (d, *J* = 1.6 Hz, 1 H), 7.59 (s, 2 H), 7.54 (dd, *J1* = 2.0 Hz, *J2* = 8.4 Hz, 1 H), 7.41- 7.36 (m, 4 H), 4.58 (d, *J* = 6.0 Hz, 2 H), 4.02 (s, 3H).

### Example 15: Synthesis of 4-(2-amino-[1,2,4]triazolo[1,5-a]pyridyl-7-yl)-1-methyl-N-(1-(2-(trifluoromethoxy)phenyl)ethyl)-1H-indazole-6-carboxamide

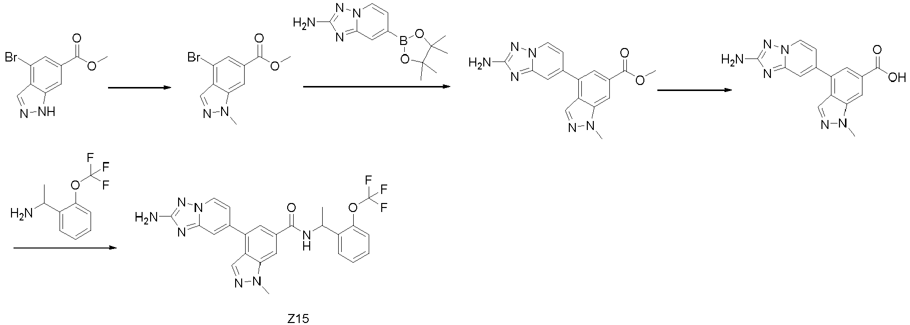

Step 1: Methyl 4-bromo-1*H*-indazole-6-carboxylate (200mg, 0.78mmol), methyl iodide (0.145ml, 2.34mmol), potassium carbonate (215mg, 1.56mmol), methanol (5ml), and *N,N*-dimethylformamide (5ml) were added to a 50mL round-bottomed flask. The reaction was carried out overnight at room temperature. 30mL of water was added. The resultant mixture was extracted with 30ml of ethyl acetate for three times. The organic phases were combined, dried over anhydrous sodium sulfate, and concentrated. The concentrate was separated by thin-layer chromatography (petroleum ether/ethyl acetate=7/1) to give methyl 4-bromo-1-methyl-1*H*-indazole-6-carboxylate (101mg, Y: 48%), ES-API: [M+H]⁺=271.1.

Step 2: Methyl 4-bromo-1-methyl-1*H*-indazole-6-carboxylate (101mg, 0.38mmol), 7-(4,4,5,5-tetramethyl-1,3,2-dioxaborolan-2-yl)-[1,2,4]triazolo[1,5-a] pyridine-2-amine (148mg, 0.57mmol), sodium carbonate (101mg, 0.95mmol), [1,1'-bis(diphenylphosphino)ferrocene]dichloropalladium-dichloromethane complex (31mg, 0.038mmol), 1,4-dioxane (6ml), and water (1.2ml) were added to a 50mL round-bottomed flask. The mixture was nitrogen-purged for three times. The reaction was carried out overnight at 80°C. Then, the reaction mixture was cooled to room temperature, and was suction filtered. 30mL of water was added to a filtrate obtained by washing the filter cake with ethyl acetate for three times. The resultant mixture was extracted with 30mL of ethyl acetate for 3 times. The organic phases were combined, dried over anhydrous sodium sulfate, and concentrated. The concentrate was seperated by thin layer chromatography (dichloromethane:methanol=15/1) to give methyl 4-(2-amino-[1,2,4]triazolo[1,5-*a*]pyridin-7-yl)-1-methyl-1*H*-indazole-6-carboxylate (86mg, Y:70%), ES-API: [M+H]⁺=323.2.

Step 3: Methyl 4-(2-amino-[1,2,4]triazolo[1,5-*a*]pyridin-7-yl)-1-methyl-1*H-*indazole-6-carboxylate (86mg, 0.27mmol), lithium hydroxide monohydrate (55mg, 1.35mmol), tetrahydrofuran (5ml), methanol (1ml), and water (1ml) were added to a 50mL round-bottomed flask. The reaction was carried out at room temperature for 3 hours. After concentrating the solvent, 5 ml of water was added to the flask. Next, the reaction was adjusted to pH=5 by adding 1M diluted hydrochloric acid. The resultant mixture was concentrated. The concentrate was vacuum-dried to give a crude product of 4-(2-amino-[1,2,4]triazolo[1,5-*a*]pyridin-7-yl)-1-methyl-1*H-*indazole-6-carboxylic acid (96mg, Y: 100%), ES-API: [M+H]⁺=309.2.

Step 4: 4-(2-amino-[1-,2,4]triazolo[1,5-*a*]pyridin-7-yl)-1-methyl-1*H*-indazole-6 carboxylic acid (96mg, 0.31mmol), 1-(2-(trifluoromethoxy)phenyl)ethan-1-amine (64mg, 0.31mmol), ((benzotriazol-1-yl)oxy)tris(dimethylamino)phosphonium hexafluorophosphate (196mg, 0.465mmol), triethylamine (100mg, 0.93mmol) and *N,N*-dimethylformamide (5ml) were added to a 50mL round-bottomed flask. The reacton was carried out overnight at room temperature untile the reaction was completed. 50mL of ethyl acetate was added to the reaction mixture. The resultant mixture was washed with 30mL of water for three times. The organic phases were combined, dried over anhydrous sodium sulfate, and concentrated. The obtained concentrate was separated by Prep-HPLC to give 4-(2-amino-[1,2,4]triazolo[1,5-*a*]pyridin-7-yl)-1-methyl-*N*-(1-(2-(trifluoromethoxy)phenyl)ethyl)-1*H*-indazole-6-carboxamide Z15 (21mg,Y:13.7%), ES-API:[M+H]⁺=496.3,¹H NMR (400 MHz, DMSO, ppm) , δ 9.06 (d, *J* = 7.4 Hz, 1H), 8.65 (d, *J* = 6.7 Hz, 1H), 8.24 (d, *J* = 10.7 Hz, 2H), 7.86 (s, 1H), 7.71 (s, 1H), 7.67 - 7.58 (m, 1H), 7.40 - 7.29 (m, 3H), 7.26 (dd, *J* = 6.9, 1.9 Hz, 1H), 6.08 (s, 2H), 5.55 - 5.40 (m, 1H), 4.13 (s, 3H), 1.49 (d, *J* = 7.0 Hz, 3H).

### Example 16: Synthesis of 5-(2-aminobenzo[d]thiazol-6-yl)-2-methoxy-N-(3-phenylpropyl) nicotinamide

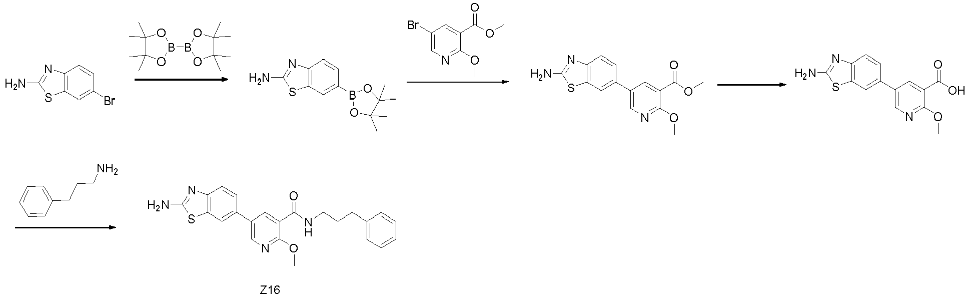

Step 1: 2-Amino-6-bromobenzothiazole (3.0 g, 13.1mmol), bis(pinacolato) diboron (6.65 g, 26.2mmol), potassium acetate (3.21 g, 32.74mmol), [1,1'-bis(diphenyl phosphino)ferrocene]dichloropalladium-dichloromethane complex (1g, 1.31mmol), and 1,4-dioxane (60ml) were added to a 250mL round-bottomed flask. The mixture was nitrogen-purged for three times. The reaction was carried out at 100°C for 8 hours. Then, the reaction mixture was cooled to room temperature, and was suction filtered. The filtrate obtained by washing the filter cake with ethyl acetate for three times was concentrated to give a crude product of 6-(4,4,5,5-tetramethyl-1,3,2-dioxaborolan-2-yl)benzo[d]thiazol-2-amine (3.8g, Y: 100%). ES-API: [M+H]⁺= 277.2.

Step 2: Methyl 5-bromo-2-methoxynicotinate (2.0 g, 8.13 mmol), 6-(4,4,5,5-tetramethyl-1,3,2-dioxaborolan-2-yl)benzothiazol-2-amine (3.37 g, 12.19 mmol), sodium carbonate (2.15 g, 20.32 mmol), [1,1'-bis(diphenylphosphino)ferrocene] dichloropalladium-dichloromethane complex (663mg, 0.813mmol), dimethoxyethane (100ml), and water (20ml) were added to a 250mL round-bottomed flask. The mixture was nitrogen-purged for three times. The reaction was carried out overnight at 80°C. Then, the reaction mixture was cooled to room temperature, and was suction filtered. 100mL of water was added to a filtrate obtained by washing the filter cake with ethyl acetate for three times. The resultant mixture was exracted with 100 mL of ethyl acetate for 3 times. The organic phase was dried, concentrated, and subjected to column chromatography (methanol:dichloromethane =0~10%) to give methyl 5-(2-aminobenzo[d]thiazol-6-yl)-2-methoxynicotinate (1.8g, Y: 73%), ES-API: [M+H]⁺= 316.1.

Step 3: Methyl 5-(2-aminobenzo[d]thiazol-6-yl)-2-methoxynicotinate (800mg, 2.54mmol), lithium hydroxide monohydrate (533mg, 12.7mmol), tetrahydrofuran (10ml), methanol (2ml), and water (2ml) were added to a 100mL round-bottomed flask. The reacton was carried out at room temperature for 3 hours. After concentrating the solvent, 10 ml of water was added to the flask. Next, the reaction was adjusted to pH=5 by adding IN diluted hydrochloric acid. The solvent was concentrated. The concentrate was vacuum-dried to give a crude product of 5-(2-aminobenzo[d]thiazol-6-yl)-2-methoxynicotinic acid (912mg, Y:100%), ES-API: [M+H]⁺ = 302.1.

Step 4: 5-(2-aminobenzo[d]thiazol-6-yl)-2-methoxynicotinic acid (80 mg, 0.266 mmol), 3-phenylpropan-1-amine (36 mg, 0.266 mmol), ((benzotriazol-1-yl)oxy)tris(dimethylamino)phosphonium hexafluorophosphate (176 mg, 0.399 mmol), triethylamine (80 mg, 0.798 mmol), and *N,N*-dimethylformamide (8 ml) were added to a 100mL round-bottomed flask. The reaction was carried out at room temperature for 2 hours. 15mL of water was added to the reaction mixture. The resultant mixture was extracted with 30mL of ethyl acetate for 3 times. The organic phases were combined, dried over anhydrous sodium sulfate, filtered, and concentrated to obtain a crude product, which was purified by alkaline HPLC to give 5-(2-aminobenzo[d]thiazol-6-yl)-2-methoxy-*N*-(3-phenylpropyl)nicotinamide Z16 as an off-white powder (21.5 mg, Y:19 %). ES-API:[M+H]⁺= 419.1. ¹H NMR (400 MHz, DMSO-d6) δ 8.58 (d, *J* = 2.8 Hz, 1 H), 8.36 (t, *J* = 6.0 Hz, 1H), 8.28 (d, *J* = 2.8 Hz, 1H), 8.03 (d, *J* = 1.6 Hz, 1 H), 7.58 (s, 2 H),7.53 (dd, *J1* = 2.0 Hz, *J2* = 8.4 Hz, 1 H), 7.40 (d, *J* = 8.4 Hz, 1 H), 7.31- 7.23 (m, 4 H), 7.20-7.16 (m, 1 H),4.00 (s, 3 H),3.31 (t, *J* = 7.6 Hz, 2 H), 2.66 (t, *J* = 7.6 Hz, 2 H), 1.88-1.81 (m, 2 H).

### Example 17: Synthesis of (5-(2-aminobenzo[d]thiazol-6-yl)-2-methoxypyridin-3-yl)(3-(4-fluorobenzyl)piperidin-1-yl)methanone

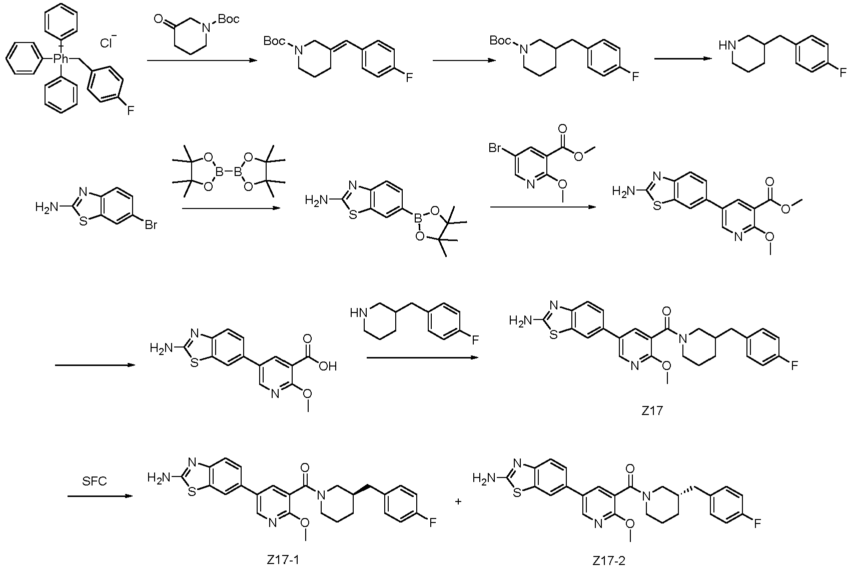

Step 1: (4-Fluorobenzyl)triphenylphosphine chloride (4.9g, 12.045mmol) was added to a solution of potassium *tert*-butoxide (1.239g, 11.042mmol) in tetrahydrofuran (30ml) in batches. The reaction mixture was stirred at room temperature for 45 minutes. Next, A solution of tert-butyl 3-oxopiperidine-1-carboxylate (2g, 10.038mmol) in tetrahydrofuran (20ml) was slowly added dropwise to the above solution while maintaining internal temperature of the reaction solution at 20°C. After the addition, the reaction solution was stirred overnight at room temperature. As the LCMS shows that the reaction was completed, the reaction solution was added to ethyl acetate (50 ml) and water (50 ml) in batches. The aqueous phase was extracted with ethyl acetate (60 ml) for 3 times. The organic phases were combined, dried and filtered. The filtrate was concentrated to obtain a crude product, which was purified by column chromatography (ethyl acetate : petroleum ether = 1:50~1:10) to give tert-butyl 3-(4-fluorobenzylidene)piperidine-1-carboxylate as a yellow liquid (335mg, Y: 10%). ES-API: [M+H]⁺= 292.1.

Step 2: 72 mg of 10% Pd/C was added to a solution of *tert*-butyl 3-(4-fluorobenzylidene)piperidine-1-carboxylate (335mg, 1.151 mmol) in ethanol (15 ml) under the protection of hydrogen gas. The reaction solution was stirred overnight at room temperature. The resultant mixture was filtered through celite, and concentrated to give a crude product of *tert*-butyl 3-(4-fluorobenzyl)piperidine-1-carboxylate (334 mg, Y: 73%). ES-API: [M+H]⁺= 294.1.

Step 3: *tert*-Butyl 3-(4-fluorobenzyl)piperidine-1-carboxylate (334 mg, 1.140 mmol) was dissolved in 6 mL of 4N hydrogen chloride in dioxane. The reaction solution was stirred at room temperature for 2 hours, and then concentrated to give a crude product of 3-(4-fluorobenzyl)piperidine (220 mg, Y: 100%). ES-API: [M+H]⁺= 194.1.

Step 4: 2-Amino-6-bromobenzothiazole (3.0 g, 13.1mmol), bis(pinacolato) diboron (6.65 g, 26.2mmol), potassium acetate (3.21 g, 32.74), [1,1'-bis(diphenyl phosphino)ferrocene]dichloropalladium-dichloromethane complex (1g, 1.31mmol), and 1,4-dioxane (60ml) were added to a 250mL round-bottomed flask. The mixture was nitrogen-purged for three times. The reaction was carried out at 100°C for 8 hours. Then, the reaction mixture was cooled to room temperature, and was suction filtered. The filtrate obtained by washing the filter cake with ethyl acetate for three times was concentrated to give a crude product of 6-(4,4,5,5-tetramethyl-1,3,2-dioxaborolan-2-yl)benzothiazol-2-amine (3.8g, Y: 100%). ES-API: [M+H]⁺= 277.2.

Step 5: Methyl 5-bromo-2-methoxynicotinate (2.0 g, 8.13 mmol), 6-(4,4,5,5-tetramethyl-1,3,2-dioxaborolan-2-yl)benzothiazol-2-amine (3.37 g, 12.19 mmol), sodium carbonate (2.15 g, 20.32 mmol), [1,1'-bis(diphenylphosphino)ferrocene] dichloropalladium-dichloromethane complex (663mg, 0.813mmol), dimethoxyethane (100ml), and water (20ml) were added to a 250mL round-bottomed flask. The mixture was nitrogen-purged for three times. The reaction was carried out overnight at 80°C. Then, the reaction mixture was cooled to room temperature, and was suction filtered. 100mL of water was added to a filtrate obtained by washing the filter cake with ethyl acetate for three times. The resultant mixture was extracted with 100 mL of ethyl acetate for 3 times. The organic phase was dried, concentrated, and subjected to column chromatography (methanol:dichloromethane =0~10%) to give methyl 5-(2-aminobenzo[d]thiazol-6-yl)-2-methoxynicotinate (1.8g, Y: 73%), ES-API: [M+H]⁺= 316.1.

Step 6: Methyl 5-(2-aminobenzo[d]thiazol-6-yl)-2-methoxynicotinate (800mg, 2.54mmol), lithium hydroxide monohydrate (533mg, 12.7mmol), tetrahydrofuran (10ml), methanol (2ml), and water (2ml) were added to a 100mL round-bottomed flask. The reacton was carried out at room temperature for 3 hours. After concentrating the solvent, 10 ml of water was added to the flask. Next, the reaction was adjusted to pH=5 by adding IN diluted hydrochloric acid. The solvent was concentrated. The concentrate was vacuum-dried to give a crude product of 5-(2-aminobenzo[d]thiazol-6-yl)-2-methoxynicotinic acid (912mg, Y:100%), ES-API: [M+H]⁺ = 302.1.

Step 7: 5-(2-aminobenzo[d]thiazol-6-yl)-2-methoxynicotinic acid (80 mg, 0.266 mmol), 3-(4-fluorobenzyl)piperidine (51mg, 0.266 mmol), ((benzotriazol-1-yl)oxy)tris(dimethylamino)phosphonium hexafluorophosphate (176 mg, 0.399 mmol), triethylamine (80 mg, 0.798 mmol), and *N,N*-dimethylformamide (8 ml) were added to a 100mL round-bottomed flask. The reaction was carried out at room temperature for 2 hours. 15mL of water was added to the reaction mixture. The resultant mixture was extracted with 30mL ethyl acetate for 3 times. The organic phases were combined, dried over anhydrous sodium sulfate, filtered, and concentrated to obtain a crude product, whichwas purified by alkaline HPLC to give (5-(2-aminobenzo[d]thiazol-6-yl)-2-methoxypyrid-3-yl)(3-(4-fluorobenzyl)piperid-1-yl)methanone Z17 as an off-white powder (20.2mg, Y:16%). ES-API:[M+H]⁺= 477.1. ¹H NMR (400 MHz, DMSO-d6) δ 8.52-8.44(m, 1 H), 8.02-7.85 (m, 2H), 7.59-7.48 (m, 3H), 7.44-7.37 (m, 1 H), 7.28-7.27 (m, 1H),7.16-7.11 (m, 1 H), 7.06-6.67 (m, 2 H), 4.43-4.17 (m, 1H), 3.92-3.81(m, 3 H),3.31-2.52(m, 4H),2.33-2.23(m,1 H), 1.75-1.19(m, 5 H).

Step 8: The compound Z17 was seperated by SFC (column type: OX-H 4.6^{∗}100mm 5µm; Co-solvent: ethanol (ammonia containing 1% methanol); column temperature: 40.5°C; flow rate: 1.8 ml/min) to give the compound Z17-1 as a white powder (an arbitrarily assigned absolute configuration, peak 1, retention time: 2.36 min, 16.48 mg, Y: 16%). ES-API: [M+H]⁺= 477.1. ¹H NMR (400 MHz, DMSO-d6) δ 8.52-8.44 (m, 1 H), 8.02-7.85 (m, 2 H), 7.59-7.48 (m, 3 H), 7.44-7.37 (m, 1 H), 7.28-7.27 (m, 1 H), 7.16-7.11 (m, 1 H), 7.06-6.67 (m, 2 H), 4.43-4.17 (m, 1 H), 3.92-3.81 (m, 3 H), 3.31-2.52 (m, 4 H), 2.33-2.23 (m, 1 H), 1.75-1.19 (m, 5 H) and the compound Z17-2 (an arbitrarily assigned absolute configuration, peak 2, retention time: 3.84 min, 16.73 mg, Y: 40%), ES-API: [M+H]⁺=477.1.

### Example 18: Synthesis of 5-(2-aminobenzo[d]thiazol-6-yl)-N-(2-(cyclopentyloxy)-6-fluorobenzyl)-2-methoxynicotinamide

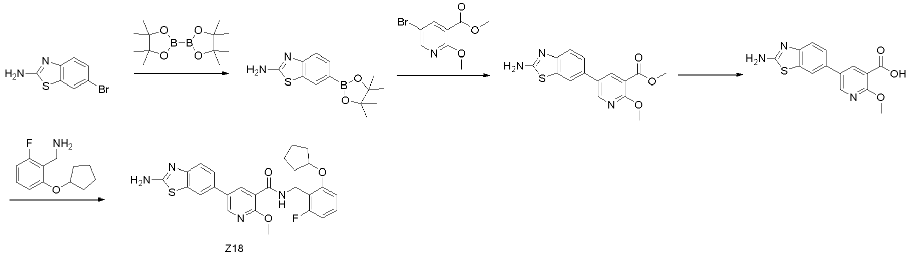

Step 1: 2-Amino-6-bromobenzothiazole (3.0 g, 13.1mmol), bis(pinacolato) diboron (6.65 g, 26.2mmol), potassium acetate (3.21 g, 32.74), [1,1'-bis(diphenyl phosphino)ferrocene]dichloropalladium-dichloromethane complex (1g, 1.31mmol), and 1,4-dioxane (60ml) were added to a 250mL round-bottomed flask. The mixture was nitrogen-purged for three times. The reaction was carried out at 100°C for 8 hours. Then, the reaction mixture was cooled to room temperature, and was suction filtered. The filtrate obtained by washing the filter cake with ethyl acetate for three times was concentrated to give a crude product of 6-(4,4,5,5-tetramethyl-1,3,2-dioxaborolan-2-yl)benzothiazol-2-amine (3.8g, Y: 100%). ES-API: [M+H]⁺= 277.2.

Step 2: Methyl 5-bromo-2-methoxynicotinate (2.0 g, 8.13 mmol), 6-(4,4,5,5-tetramethyl-1,3,2-dioxaborolan-2-yl)benzothiazol-2-amine (3.37 g, 12.19 mmol), sodium carbonate (2.15 g, 20.32 mmol), [1,1'-bis(diphenylphosphino)ferrocene] dichloropalladium-dichloromethane complex (663mg, 0.813mmol), dimethoxyethane (100ml), and water (20ml) were added to a 250mL round-bottomed flask. The mixture was nitrogen-purged for three times. The reaction was carried out overnight at 80°C. Then, the reaction mixture was cooled to room temperature, and was suction filtered. 100mL of water was added to a filtrate obtained by washing the filter cake with ethyl acetate for three times. The resultant mixture was extracted with 100 mL of ethyl actate for 3 times. The organic phase was dried, concentrated, and subjected to column chromatography (methanol:dichloromethane=0~10%) to give methyl 5-(2-aminobenzo[d]thiazol-6-yl)-2-methoxynicotinate (1.8g, Y: 73%), ES-API: [M+H]⁺= 316.1.

Step 3: Methyl 5-(2-aminobenzo[d]thiazol-6-yl)-2-methoxynicotinate (800mg, 2.54mmol), lithium hydroxide monohydrate (533mg, 12.7mmol), tetrahydrofuran (10ml), methanol (2ml), and water (2ml) were added to a 100mL round-bottomed flask. The reacton was carried out at room temperature for 3 hours. After concentrating the solvent, 10 ml of water was added to the flask. Next, the reaction was adjusted to pH=5 by adding IN diluted hydrochloric acid. The solvent was concentrated. The concentrate was vacuum-dried to give a crude product of 5-(2-aminobenzo[d]thiazol-6-yl)-2-methoxynicotinic acid (912mg, Y:100%), ES-API: [M+H]⁺ = 302.1.

Step 4: 5-(2-aminobenzo[d]thiazol-6-yl)-2-methoxynicotinic acid (80 mg, 0.266 mmol), (2-(cyclopentyloxy)-6-fluorophenyl)methylamine (56mg, 0.266 mmol), ((benzotriazol-1-yl)oxy)tris(dimethylamino)phosphonium hexafluorophosphate (176 mg, 0.399 mmol), triethylamine (80 mg, 0.798 mmol), and *N,N*-dimethylformamide (8 ml) were added to a 100mL round-bottomed flask. The reaction was carried out at room temperature for 2 hours. 15mL of water was added to the reaction mixture. The resultant mixture was extracted with 30mL of ethyl acetate for 3 times. The organic phases were combined, dried over anhydrous sodium sulfate, and filtered. The concentrated organic phase was purified by alkaline HPLC to give 5-(2-aminobenzo[d]thiazol-6-yl)-*N*-(2-(cyclopentyloxy)-6-(fluorobenzyl)-2-methoxynicotinamide Z18 as an off-white powder (13mg, Y: 10%). ES-API:[M+H]⁺= 493.2. ¹H NMR (400 MHz, DMSO-d6) δ 8.60(d, *J* = 2.4 Hz, 1 H), 8.37-8.34 (m, 2H), 8.01 (d, *J=* 1.6 Hz, 1H), 7.58 (s, 2 H), 7.51 (dd, *J1* = 2.0 Hz, *J2* = 8.4 Hz, 1H),7.40 (d, *J=* 8.4 Hz, 1 H), 7.32-7.26 (m, 1 H), 6.88 (d, *J=* 9.2 Hz, 1H), 6.78(t, *J*= 8.8 Hz, 1 H),4.95-4.92(s, 1 H),4.54(d, *J=* 5.2 Hz, 2 H), 4.00(s, 3 H),1.94-1.57 (m, 8 H).

### Example 19: Synthesis of 5-(2-aminobenzo[d]thiazol-6-yl)-N-(2-(cyclopentyloxy)-5-fluorobenzyl)-2-methoxynicotinamide

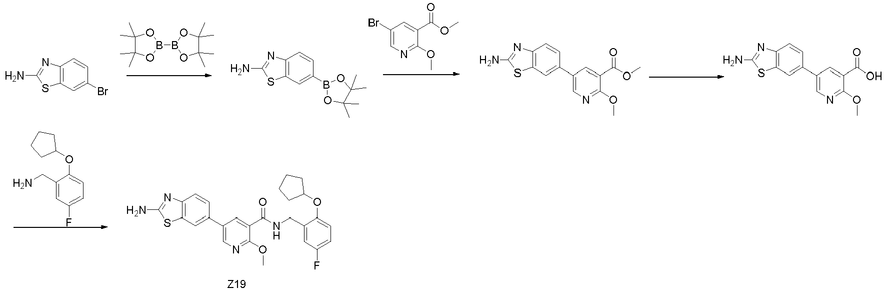

Step 1: 2-Amino-6-bromobenzothiazole (3.0 g, 13.1mmol), bis(pinacolato) diboron (6.65 g, 26.2mmol), potassium acetate (3.21 g, 32.74), [1,1'-bis(diphenyl phosphino)ferrocene]dichloropalladium-dichloromethane complex (1g, 1.31mmol), and 1,4-dioxane (60ml) were added to a 250mL round-bottomed flask. The mixture was nitrogen-purged for three times. The reaction was carried out at 100°C for 8 hours. Then, the reaction mixture was cooled to room temperature, and was suction filtered. The filtrate obtained by washing the filter cake with ethyl acetate for three times was concentrated to give a crude product of 6-(4,4,5,5-tetramethyl-1,3,2-dioxaborolan-2-yl)benzothiazol-2-amine (3.8g, Y: 100%). ES-API: [M+H]⁺= 277.2.

Step 2: Methyl 5-bromo-2-methoxynicotinate (2.0 g, 8.13 mmol), 6-(4,4,5,5-tetramethyl-1,3,2-dioxaborolan-2-yl)benzothiazol-2-amine (3.37 g, 12.19 mmol), sodium carbonate (2.15 g, 20.32 mmol), [1,1'-bis(diphenylphosphino)ferrocene] dichloropalladium-dichloromethane complex (663mg, 0.813mmol), dimethoxyethane (100ml), and water (20ml) were added to a 250mL round-bottomed flask. The mixture was nitrogen-purged for three times. The reaction was carried out overnight at 80°C. Then, the reaction mixture was cooled to room temperature, and was suction filtered. 100mL of water was added to a filtrate obtained by washing the filter cake with ethyl acetate for three times. The resultant mixture was extracted with 100 mL of ethyl actate for 3 times. The organic phase was dried, concentrated, and subjected to column chromatography (methanol:dichloromethane =0~10%) to give methyl 5-(2-aminobenzo[d]thiazol-6-yl)-2-methoxynicotinate (1.8g, Y: 73%), ES-API: [M+H]+= 316.1.

Step 3: Methyl 5-(2-aminobenzo[d]thiazol-6-yl)-2-methoxynicotinate (800mg, 2.54mmol), lithium hydroxide monohydrate (533mg, 12.7mmol), tetrahydrofuran (10ml), methanol (2ml), and water (2ml) were added to a 100mL round-bottomed flask. The reacton was carried out at room temperature for 3 hours. After concentrating the solvent, 10 ml water was added to the flask. Next, the reaction was adjusted to pH=5 by adding IN diluted hydrochloric acid. The solvent was concentrated. The concentrate was vacuum-dried to give a crude product of 5-(2-aminobenzo[d]thiazol-6-yl)-2-methoxynicotinic acid (912mg, Y:100%), ES-API: [M+H]⁺ = 302.1.

Step 4: 5-(2-aminobenzo[d]thiazol-6-yl)-2-methoxynicotinic acid (80 mg, 0.266 mmol), (2-(cyclopentyloxy)-5-fluorophenyl)methylamine (56mg, 0.266 mmol), ((benzotriazol-1-yl)oxy)tris(dimethylamino)phosphonium hexafluorophosphate (176 mg, 0.399 mmol), triethylamine (80 mg, 0.798 mmol), and *N,N*-dimethylformamide (8 ml) were added to a 100mL round-bottomed flask. The reacton was carried out at room temperature for 2 hours. 15mL of water was added to the reaction mixture. The resultant mixture was extracted with 30mL of ethyl acetate for 3 times. The organic phases were combined, dried over anhydrous sodium sulfate, filtered, and concentrated to obtain crude product, which was purified by alkaline HPLC to give 5-(2-aminobenzo[d]thiazol-6-yl)-N-(2-(cyclopentyloxy)-5-(fluorobenzyl)-2-methoxynicotinamide Z19 as an off-white powder (10.2mg, Y: 8%). ES-API:[M+H]⁺= 493.2. ¹H NMR (400 MHz, DMSO-d6) δ 8.71(t, *J* = 5.6 Hz, 1 H), 8.62 (d, *J* = 2.8 Hz, 1H), 8.35 (d, *J=* 2.4 Hz, 1H), 8.04 (d,*J* = 2.0 Hz,1 H), 7.59 (s, 2H),7.54 (dd, *J1*= 2.0 Hz,*J2* = 8.4 Hz,1 H), 7.41 (d,*J*= 8.4 Hz, 1 H), 7.09-6.98 (m, 3H), 4.89-4.86 (m, 1 H),4.43(d, *J*= 6.0 Hz, 2 H),4.03(s,3 H), 1.91-1.59 (m, 8 H).

### Example 20: Synthesis of 5-(2-aminobenzo[d]thiazol-6-yl)-N-(2-(cyclopropylmethoxy)benzyl)-2-methoxynicotinamide

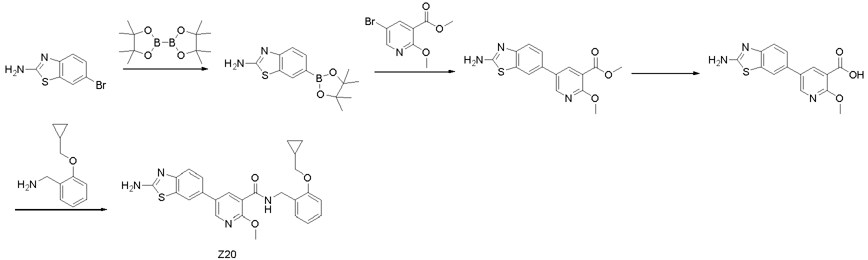

Step 1: 2-Amino-6-bromobenzothiazole (3.0 g, 13.1mmol), bis(pinacolato) diboron (6.65 g, 26.2mmol), potassium acetate (3.21 g, 32.74), [1,1'-bis(diphenyl phosphino)ferrocene]dichloropalladium-dichloromethane complex (1g, 1.31mmol), and 1,4-dioxane (60ml) were added to a 250mL round-bottomed flask. The mixture was nitrogen-purged for three times. The reaction was carried out at 100°C for 8 hours. Then, the reaction mixture was cooled to room temperature, and was suction filtered. The filtrate obtained by washing the filter cake with ethyl acetate for three times was concentrated to give a crude product of 6-(4,4,5,5-tetramethyl-1,3,2-dioxaborolan-2-yl)benzothiazol-2-amine (3.8g, Y: 100%). ES-API: [M+H]⁺= 277.2.

Step 2: Methyl 5-bromo-2-methoxynicotinate (2.0 g, 8.13 mmol), 6-(4,4,5,5-tetramethyl-1,3,2-dioxaborolan-2-yl)benzothiazol-2-amine (3.37 g, 12.19 mmol), sodium carbonate (2.15 g, 20.32 mmol), [1,1'-bis(diphenylphosphino)ferrocene] dichloropalladium-dichloromethane complex (663mg, 0.813mmol), dimethoxyethane (100ml), and water (20ml) were added to a 250mL round-bottomed flask. The mixture was nitrogen-purged for three times. The reaction was carried out overnight at 80°C. Then, the reaction mixture was cooled to room temperature, and was suction filtered. 100mL water was added to a filtrate obtained by washing the filter cake with ethyl acetate for three times. The resultant mixture was extracted with 100 mL of ethyl actate for 3 times. The organic phase was dried, concentrated, and subjected to column chromatography (methanol:dichloromethane =0~10%) to give methyl 5-(2-aminobenzo[d]thiazol-6-yl)-2-methoxynicotinate (1.8g, Y: 73%), ES-API: [M+H]⁺= 316.1.

Step 3: Methyl 5-(2-aminobenzo[d]thiazol-6-yl)-2-methoxynicotinate (800mg, 2.54mmol), lithium hydroxide monohydrate (533mg, 12.7mmol), tetrahydrofuran (10ml), methanol (2ml), and water (2ml) were added to a 100mL round-bottomed flask. The reacton was carried out at room temperature for 3 hours. After concentrating the solvent, 10 ml of water was added to the flask. Next, the reaction was adjusted to pH=5 by adding IN diluted hydrochloric acid. The solvent was concentrated. The concentrate was vacuum-dried to give a crude product of 5-(2-aminobenzo[d]thiazol-6-yl)-2-methoxynicotinic acid (912mg, Y:100%), ES-API: [M+H]⁺ = 302.1.

Step 4: 5-(2-aminobenzo[d]thiazol-6-yl)-2-methoxynicotinic acid (80 mg, 0.266 mmol), (2-(cyclopropylmethoxy)phenyl)methylamine (47mg, 0.266 mmol), ((benzotriazol-1-yl)oxy)tris(dimethylamino)phosphonium hexafluorophosphate (176 mg, 0.399 mmol), triethylamine (80 mg, 0.798 mmol), and *N,N*-dimethylformamide (8 ml) were added to a 100mL round-bottomed flask. The reaction was carried out at room temperature for 2 hours. 15mL of water was added to the reaction mixture. The resultant mixture was extracted with 30mL of ethyl acetate for 3 times. The organic phases were combined, dried over anhydrous sodium sulfate, and filtered. The crude product obtained by concentrating the organic phase was purified by alkaline HPLC to give 5-(2-aminobenzo[d]thiazol-6-yl)-*N*-(2-(cyclopropylmethoxy)benzyl)-2-methoxynicotinamide Z20 as an off-white powder (13 mg, Y: 11%). ES-API: [M+H]⁺= 461.2. ¹H NMR (400 MHz, DMSO-d6) δ 8.68(t, *J* = 5.6 Hz, 1 H), 8.62 (d, *J* = 2.4 Hz, 1H), 8.39 (d, *J=* 2.4 Hz, 1H), 8.04 (d,*J* = 1.6 Hz,1 H), 7.62 (s, 2H),7.54 (dd, *J1*= 1.6 Hz,*J2* = 8.0 Hz,1 H), 7.41 (d,*J*= 8.0 Hz, 1 H), 7.27-7.20 (m, 2H), 6.98(d,*J*= 7.6 Hz,1 H),6.92(t, *J*= 7.6 Hz, 1 H),4.53 (d, *J=* 6.0 Hz, 2 H),4.04 (s, 3 H), 3.91 (d, *J=* 6.8 Hz, 2 H),1.29-1.23(m,1 H),0.61-0.57 (m, 2 H),0.39-0.35 (m, 2 H).

### Example 21: Synthesis of 5-(2-amino-[1,2,4]triazolo[1,5-a]pyrid-7-yl)-1-methyl-6-oxo-N-(1-(2-(trifluoromethoxy)phenyl)ethyl)-1,6-dihydropyridine-3-carboxamide

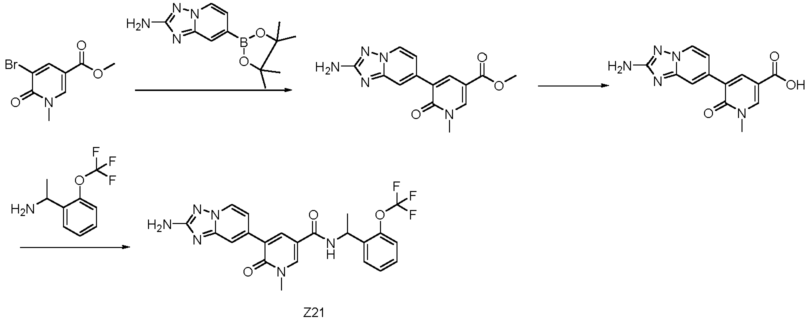

Step 1: Methyl 5-bromo-1-methyl-6-oxo-1,6-dihydropyridine-3-carboxylate (150mg, 0.61mmol), 7-(4,4,5,5-tetramethyl-1,3,2-dioxaborolan-2-yl)-[1,2,4]triazolo [1,5-a]pyridine-2-amine (237mg, 0.91mmol), sodium carbonate (162mg, 1.52mmol), [1,1'-bis(diphenylphosphino)ferrocene]dichloropalladium-dichloromethane complex (50mg, 0.061mmol), 1,4-dioxane (10ml), and water (2ml) were added to a 50mL round-bottomed flask. The mixture was nitrogen-purged for three times. The reaction was carried out overnight at 80°C. Then, the reaction mixture was cooled to room temperature, and was suction filtered. 30mL of water was added to a filtrate obtained by washing the filter cake with ethyl acetate for three times. The resultant mixture was exracted with 30mL of ethyl acetate for 3 times. The organic phase was dried, concentrated, and separated by thin layer chromatography (dichloromethane:methanol=15:1) to give methyl 5-(2-amino-[1,2,4]triazolo[1,5-a] pyridin-7-yl)-1-methyl-6-oxo-1,6-dihydropyridine-3-carboxylate (120mg, Y: 66%), ES-API: [M+H]⁺=300.1.

Step 2: Methyl 5-(2-amino-[1,2,4]triazolo[1,5-a]pyridin-7-yl)-1-methyl-6-oxo-1,6-dihydropyridine-3-carboxylate (67mg, 0.22mmol), lithium hydroxide monohydrate (45mg, 1.1mmol), tetrahydrofuran (5ml), methanol (1ml), and water (1ml) were added to a 50mL round-bottomed flask. The reacton was carried out at room temperature for 3 hours. After concentrating the solvent, 5 ml of water was added to the flask. Next, the reaction was adjusted to pH=5 by adding 1M diluted hydrochloric acid dropwise. The resultant mixture was concentrated. The concentrate was vacuum-dried to give a crude product of 5-(2-amino-[1,2,4]triazolo[1,5-*a*]pyridin-7-yl)-1-methyl-6-oxo-1,6-dihydropyridine-3-carboxylic acid (98mg, Y:100%). ES-API: [M+H]⁺=286.1.

Step 3: 5-(2-Amino-[1,2,4]triazolo[1,5-a]pyridin-7-yl)-1-methyl-6-oxo-1,6-dihydropyridine-3-carboxylic acid (98mg, 0.34mmol), 1-(2-(trifluoromethoxy)phenyl)ethan-1-amine (70mg, 0.34mmol), ((benzotriazol-1-yl)oxy)tris(dimethylamino)phosphonium hexafluorophosphate (225mg, 0.51mmol), triethylamine (103mg, 1.02mmol), and *N,N*-dimethylformamide (5ml) were added to a 50mL round-bottomed flask. The reaction was carried out overnight at room temperature untile the reaction was completed. 50mL of ethyl acetate was added to the reaction mixture. The resultant mixture was washed with 30mL of water for three times. The organic phases were combined, dried over anhydrous sodium sulfate, and concentrated. The obtained concentrate was separated by Prep-HPLC to give 5-(2-amino-[1,2,4]triazolo[1,5-a]pyridin-7-yl)-1-methyl-6-oxo-*N*-(1-(2-(trifluoro methoxy)phenyl)ethyl)-1,6-dihydropyridine-3-carboxamide Z21 (9mg,Y:5.6%), ES-API:[M+H]⁺=473.2,¹H NMR (400 MHz, DMSO) δ 8.72 (d, *J* = 7.1 Hz, 1H), 8.52 (d, *J=* 7.1 Hz, 1H), 8.45 (s, 1H), 8.30 (s, 1H), 7.84 (s, 1H), 7.64 - 7.53 (m, 1H), 7.35 (d, *J* = 9.3 Hz, 2H), 7.33 - 7.24 (m, 3H), 5.99 (s, 2H), 5.46 - 5.33 (m, 1H), 3.56 (s, 3H), 1.43 (d, *J* = 7.1 Hz, 3H).

### Example 22: Synthesis of 5-(2-aminobenzo[d]thiazol-6-yl)-N-(2-(cyclopropylmethoxy)benzyl)nicotinamide

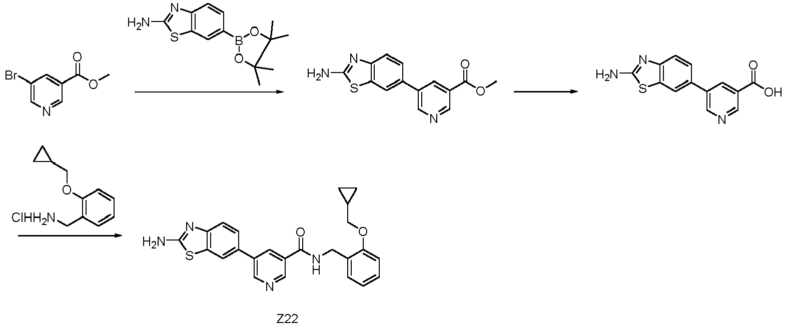

Step 1: Methyl 5-bromo-nicotinate (500mg, 2.31mmol), 6-(4,4,5,5-tetramethyl-1,3,2-dioxaborolan-2-yl)benzo[d]thiazol-2-amine (959mg, 3.47mmol), sodium carbonate (613mg, 5.79mmol), [1,1'-bis(diphenylphosphino)ferrocene] dichloropalladium-dichloromethane complex (188mg, 0.231mmol), 1,4-dioxane (15ml) and water (3ml) were added to a 50mL round-bottomed flask. The mixture was purged with nitrogen gas for three times. The reaction was carried out overnight at 80°C. Then, the reaction mixture was cooled to room temperature, and was suction filtered. 30mL of water was added to a filtrate obtained by washing the filter cake with ethyl acetate for three times. The resultant mixture was extracted with 30mL ethyl actate for 3 times. The organic phase was dried, concentrated, and separated by thin layer chromatography (dichloromethane:methanol=15:1) to give methyl 5-(2-aminobenzo[d]thiazol-6-yl)nicotinate (356 mg, Y: 54%), ES-API: [M+H]⁺=286.1.

Step 2: Methyl 5-(2-aminobenzo[d]thiazol-6-yl)nicotinate (300mg, 1.05mmol), lithium hydroxide monohydrate (215mg, 5.25mmol), tetrahydrofuran (5ml), methanol (1ml), and water (1ml) were added to a 50mL round-bottomed flask. The reacton was carried out at room temperature for 3 hours. After concentrating the solvent, 5 ml of water was added to the flask. Next, the reaction was adjusted to pH=5 by adding 1M diluted hydrochloric acid. The resultant mixture was concentrated. The concentrate was vacuum-dried to give a crude product of 5-(2-aminobenzo[d]thiazol-6-yl) nicotinic acid (361mg, Y: 100%), ES-API: [M+H]⁺=272.2.

Step 3: 5-(2-Aminobenzo[d]thiazol-6-yl)nicotinic acid (152mg, 0.56mmol), (2-(cyclopropylmethoxy)phenyl)methylamine hydrochloride (100mg, 0.56mmol), ((benzotriazol-1 -yl)oxy)tris(dimethylamino)phosphonium hexafluorophosphate (351mg, 0.84mmol), triethylamine (170mg, 1.68mmol), and *N,N*-dimethylformamide (5ml) were added to a 50mL round-bottomed flask. The reaction was carried out overnight at room temperature untile the reaction was completed. 50mL of ethyl acetate was added to the reaction mixture. The resultant mixture was washed with 30mL of water for three times. The organic phases were combined, dried over anhydrous sodium sulfate, and concentrated. The obtained concentrate was separated by Prep-HPLC to give 5-(2-aminobenzo[d]thiazol-6-yl)-*N*-(2-(cyclopropylmethoxy) benzyl) nicotinamide Z22 (10mg,Y:4.1%), ES-API:[M+H]⁺=431.2,¹H NMR (400 MHz, DMSO, ppm) δ 9.10 - 9.04 (m, 0H), 9.01 - 8.98 (m, 0H), 8.96 - 8.93 (m, 0H), 8.49 (s, 0H), 8.12 (s, 0H), 7.69 - 7.58 (m, 1H), 7.41 *(d, J =* 8.3 Hz, 0H), 7.19 (dd, *J* = 13.9, 7.1 Hz, 1H), 6.94 (d, *J* = 8.4 Hz, 0H), 6.87 (t, *J* = 7.2 Hz, 1H), 4.50 (d, *J* = 5.7 Hz, 1H), 3.86 (d, *J* =6.8 Hz, 1H), 1.29 - 1.15 (m, 1H), 0.58 - 0.47 (m, 1H), 0.31 (d, *J* = 4.6 Hz, 1H).

### Example 23: Synthesis of 5-(2-aminobenzo[d]thiazol-6-yl)-6-chloro-N-(3-(trifluoromethoxy)benzyl)nicotinamide

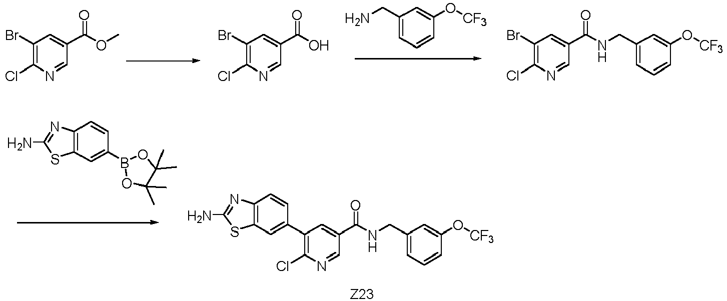

Step 1: Methyl 5-bromo-6-chloronicotinate (150mg, 0.5988mmol), lithium hydroxide monohydrate (239mg, 5.988mmol), 5mL of tetrahydrofuran, 5mL of methanol and 5mL of water were added to a 50mL round-bottom flask. The mixture was stirred for reaction overnight at room temperature. As monitored by TLC [dichloromethane: methanol=10:1, v/v] when the spot for the starting materials disappeared, the reaction was terminated. The reaction system was spin-dried under reduced pressure; thereafter, 8mL of water was added. The reaction system was cooled to 0°C in an ice-water bath. Hydrogen chloride solution (4M in dioxane) was added dropwise to adjust the pH to 5-6. 50ml of toluene was added. The resultant mixture was spin-dried under reduced pressure, to give a crude product of 5-bromo-6-chloronicotinic acid (201 mg, crude product). ES-API: [M+H]⁺=201.9.

Step 2: 5-Bromo-6-chloronicotinic acid (201mg, 0.5988mmol) and 20mL of anhydrous *N,N*-dimethylformamide were added to a 50mL round-bottomed flask. Next, (3-(trifluoromethoxy)phenyl)methylamine (171.5mg, 0.8982mmol), triethylamine (605mg, 5.988mmol), and BOP reagent (397mg, 0.8982mmol) were added sequentially. The reaction was carried out at room temperature under the protection of nitrogen gas for 2 hours. After the reaction was completed, 120mL of ethyl acetate was added to the system. The resultant mixture was washed with saturated brine for 5 times (90mLx5). The organic phase of ethyl acetate was dried over anhydrous sodium sulfate and filtered, and the filtrate was spin-dried, followed by PTLC [dichloromethane:methanol=10:1, v/v] to give 5-bromo-6-chloro-*N*-(3-(trifluoro methoxy)benzyl)nicotinamide (171mg, Y:70%), ES-API: M+H]⁺=409.2.

Step 3: 5-Bromo-6-chloro-*N*-(3-(trifluoromethoxy)benzyl)nicotinamide (171mg, 0.4190mmol), 6-(4,4,5,5-tetramethyl-1,3,2-dioxaborolan-2-yl)benzo[d]thiazol-2-amine (173mg, 0.628mmol) [1,1'-bis(diphenylphosphino)ferrocene] dichloropalladium-dichloromethane complex (20mg, 0.02095mmol), and sodium carbonate (133mg, 1.257mmol), and finally 15mL of dioxane and 3mL of water were added to a 20mL microwave tube. The tube was purged with nitrogen gas for about 2 minutes. The reaction was carried out in an oil bath at 110°C for 4 hours. After the reaction was cooled to room temperature, 50mL of ethyl acetate was added to the system. The resultant mixture was washed with saturated brine for 5 times (30mLx5). The phase of ethyl acetate was dried over anhydrous sodium sulfate and filtered. The filtrate was spin-dried to give the target compound of 5-(2-aminobenzo[d]thiazol-6-yl)-6-chloro-*N*-(3-(trifluoromethoxy)benzyl)nicotinamide Z23 (2.4mg, Y:1.2%). ES-API:[M+H]⁺=479.6. ¹H NMR (400 MHz, DMSO-d6) δ 9.37 (t, *J* = 5.9 Hz, 1H), 8.80 (d, *J* = 2.3 Hz, 1H), 8.29 (d, *J* = 2.3 Hz, 1H), 7.82 (d, *J* = 1.5 Hz, 1H), 7.63 (s, 2H), 7.46 - 7.21 (m, 6H), 4.52 (d,*J*= 5.7 Hz, 2H).

### Example 24: Synthesis of 6-(2-amino-[1,2,4]triazolo[1,5-a]pyridin-7-yl)-N-(1-(2-(trifluoromethoxy)phenyl)ethyl)-1H-indole-4-carboxamide

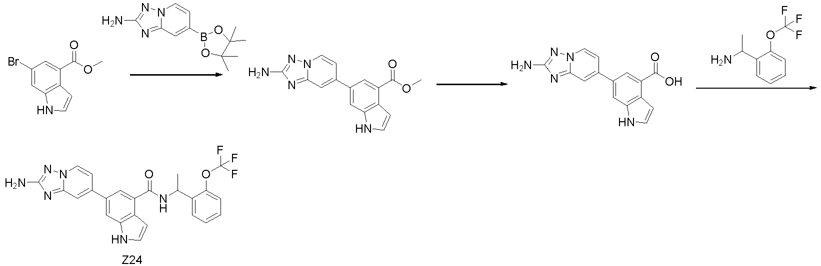

Step 1: A compound of methyl 6-bromo-4-indole-carboxylate (200mg, 0.787mmol) was charged into a microwave tube. 7-(4,4,5,5-tetramethyl-1,3,2-dioxaborolan-2-yl)-[1,2,4]triazolo[1,5-*a*]pyridin-2-amine (406mg, 1.57mmol), [1,1'-bis(diphenylphosphino)ferrocene]dichloropalladium-dichloromethane complex (64mg, 0.0787mmol), sodium carbonate (208mg, 1.97mmol), 3 mL of dioxaneand 0.6mL of water were added sequentially. The reaction was carried out in a microwave reactor at 80°C for 30 minutes. After the mixture was cooled to room temperature , 15 mL of water was added to the mixture. The resultant mixture was extracted with 20 mL of ethyl acetate twice. The organic phases were combined, washed with 15 mL of saturated brine, dried over anhydrous sodium sulfate, and concentrated under reduced pressure to remove the solvent. The residue was purified by silica gel column chromatography (dichloromethane:methanol=100:1-10:1) to give methyl 6-(2-amino-[1,2,4]triazolo[1,5-a]pyridin-7-yl)-1*H*-indole-4-carboxylate (156 mg, Y: 65%). ES-API: [M+H]⁺=308.7.

Step 2: Methyl 6-(2-Amino-[1,2,4]triazolo[1,5-a]pyridin-7-yl)-1*H*-indole-4-carboxylate (156mg, 0.51mmol) was added to a 50ml round-bottomed flask. Lithium hydroxide (61mg, 2.55mmol), tetrahydrofuran (5ml), methanol (2ml), and water (2ml) were added sequentially. The reaction was carried out at room temperature for 3 hours. The reaction was adjusted to pH=6 by adding 1M diluted hydrochloric acid. The solvent was concentrated to give the compound of 6-(2-amino-[1,2,4]triazolo[1,5-a]pyridin-7-yl)-1H-indole-4-carboxylic acid (150 mg, Y: 100%), which was used in the reaction of the next step directly. ES-API: [M+H]⁺=294.0.

Step 3: The compound of 6-(2-amino-[1,2,4]triazolo[1,5-a]pyridin-7-yl)-1H-indole-4-carboxylic acid (100mg, 0.34mmol) was add to a 50ml round-bottomed flask. 1-(2-(trifluoromethoxy)phenyl)ethyl-1-amine (70mg, 0.34mmol), ((benzotriazol-1-yl)oxy)tris(dimethylamino)phosphonium hexafluorophosphate (225mg, 0.51mmol), triethylamine (0.2 ml) and *N,N*-dimethylformamide (5ml) were added sequentially. The mixture was reacted at room temperature for 2 hours. 15 mL of water was added. The resultant mixture was extracted with 30 mL of ethyl acetate for 3 times. The organic phases were combined, dried over anhydrous sodium sulfate, and filtered. The crude product obtained by concentrating the organic phase was purified by alkaline HPLC to give the compound of 6-(2-amino-[1,2,4]triazolo[1,5-*a*]pyridin-7-yl)-*N*-(1-(2-(trifluoromethoxy)pheny1)ethyl)-1*H*-indole-4-carboxamide (10mg, Y:6%). ES-API: [M+H]⁺=481.0.

### Example 25: Synthesis of 4-(2-amino-[1,2,4]triazolo[1,5-a]pyridin-7-yl)-N-(1-(2-(trifluoromethoxy)phenyl)ethyl)-1H-indole-6-carboxamide

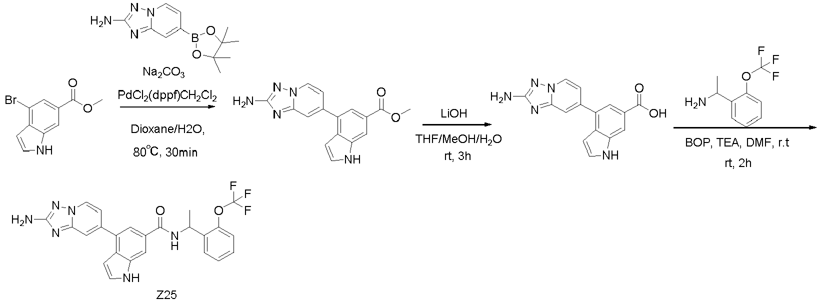

The preparation method and experimental conditions were similar to those in Example 24. ES-API: [M+H]⁺=481.0.

### Example 26: Synthesis of 4-(2-amino-[1,2,4]triazolo[1,5-a]pyridin-7-yl)-N-(1-(2-(trifluoromethoxy)phenyl)ethyl)-1H-benzo[d]imidazole-6-carboxamide

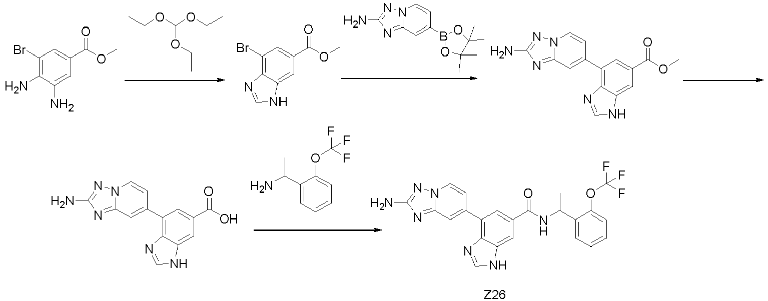

Step 1: 10 mL of Anhydrous tetrahydrofuran, then methyl 3,4-diamino-5-bromobenzoate (280mg, 1.1425mmol) and triethoxymethane (345.0mg, 2.3279mmol), and finally methanesulfonic acid monohydrate (22.0mg, 0.1156mmol) were added to a 100mL single-necked flask. The reaction was carried out overnight at room temperature. 100mL of ethyl acetate was added to the system. The resultant mixture was washed with saturated brine for 3 times (80mL×3). The phase of ethyl acetate was dried over anhydrous sodium sulfate, and filtered. The filtrate was spin-dried to give a crude product of methyl 4-bromo-1*H*-benzo[d]imidazole-6-carboxylate (297 mg, crude product). ES-API: [M+H]⁺=257.0.

Step 2: Methyl 4-bromo-1*H*-benzo[d]imidazole-6-carboxylate (200mg, 0.7842mmol), 7-(4,4,5,5-tetramethyl-1,3,2-dioxaborolan-2-yl)-[1,2,4]triazolo[1,5-a]pyridin-2-amine (406.0mg, 7.5614mmol), [1,1'-bis(diphenylphosphino)ferrocene] dichloropalladium-dichloromethane complex (140.0mg, 0.1708 mmol) and sodium carbonate (250.0mg, 2.360mmol), and finally 20mL of dioxane and 4mL of water were added to a 100mL single-neck flask. The mixture was purged with nitrogen gas for about 1.5 minutes. The reaction was carried out in an oil bath at 110°C for 5 hours. 100mL of ethyl acetate was added to the system. The resultant mixture was washed with saturated brine for 5 times (80mL×5). The phase of ethyl acetate was dried over anhydrous sodium sulfate and filtered. The filtrate was spin-dried, and the concentrate was purified by PTLC [dichloromethane:methanol=10:1, v/v] to give methyl 4-(2-amino-[1,2,4]triazolo[1,5-*a*]pyridin-7-yl)-1*H*-benzo[d]imidazole-6-carboxylate (233.0 mg, Y: 64%). ES-API: [M+H]⁺=309.0.

Step 3: Methyl 4-(2-amino-[1,2,4]triazolo[1,5-*a*]pyridin-7-yl)-1*H*-benzo[d] imidazole-6-carboxylate (233.0 mg, 0.7565 mmol), lithium hydroxide monohydrate (605 mg, 15.13 mmol), 10mL of tetrahydrofuran, 10mL of methanol, and 5mL of water were added to a 50mL round-bottomed flask. The reaction was carried out overnight at room temperature. As monitored by TLC [dichloromethane:methanol=10:1, v/v] when the spot for the starting materials disappeared, the reaction was terminated. The reaction system was spin-dried under reduced pressure; thereafter, 10mL of water was added. The reaction system was cooled to 0°C in an ice-water bath. Hydrogen chloride solution (4M in dioxane) was added dropwise to adjust the pH to 5-6. 70ml of toluene was added. The resultant mixture was spin-dried under reduced pressure, to give 4-(2-amino-[1,2,4]triazolo[1,5-*a*]pyridin-7-yl)-1*H*-benzo[d]imidazole-6-carboxylic acid (250mg, crude product). ES-API: [M+H]⁺= 295.0.

Step 4: 4-(2-amino-[1,2,4]triazolo[1,5-*a*]pyridin-7-yl)-1*H*-benzo[d]imidazole-6-carboxylic acid (250mg, 0.7565mmol) and 10.0mL of anhydrous *N,N-*dimethylformamide were added to a 50mL round-bottomed flask. Next, 1-(2-(trifluoromethoxy)phenyl)ethan-1-amine (232.0 mg, 1.1347mmol), triethylamine (764.06mg, 7.565mmol), and BOP reagent (501.5mg, 1.1347mmol) were added sequentially. The reaction was carried out at room temperature under the protection of nitrogen gas for 2 hours. 50mL of ethyl acetate was added to the system. The resultant mixture was washed with saturated brine for 4 times (30mLx4). The phase of ethyl acetate was dried over anhydrous sodium sulfate and filtered, and the filtrate was spin-dried to give 4-(2-amino-[1,2,4]triazolo[1,5-*a*]pyridin-7-yl)-*N-*(1-(2-(trifluoromethoxy)phenyl)ethyl)-1H-benzo[d]imidazole-6-carboxamide (31 mg, Y: 8.3%). ES-API:[M+H]⁺=481.6. ¹H NMR (400 MHz, DMSO-d6) δ 9.03 (d, *J* = 7.4 Hz, 1H), 8.66 (d, *J* = 7.0 Hz, 1H), 8.51 (s, 1H), 8.26 (s, 1H), 8.14 (d, *J* = 9.7 Hz, 2H), 7.77 - 7.61 (m, 2H), 7.39 - 7.29 (m, 3H), 6.17 (s, 2H), 5.46 (dd, *J* = 14.3, 7.1 Hz, 1H), 1.48 (d, *J* = 7.0 Hz, 3H).

### Example 27: Synthesis of 6-(2-amino-[1,2,4]triazolo[1,5-a]pyridin-7-yl)-N-(1-(2-(trifluoromethoxy)phenyl)ethyl)-1H-benzo[d]imidazole-4-carboxamide

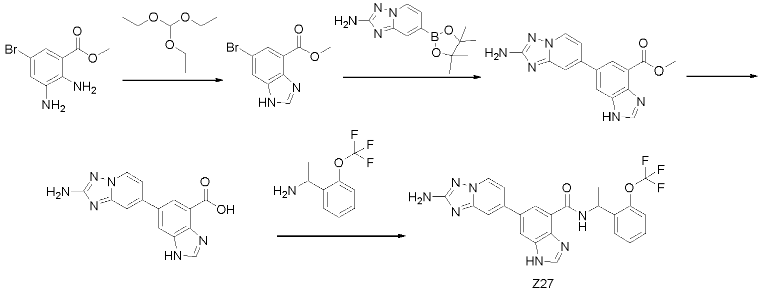

Step 1: 10 mL of anhydrous tetrahydrofuran, then methyl 2,3-diamino-5-bromobenzoate (280mg, 1.1425mmol) and triethoxymethane (345.0mg, 2.3279mmol), and finally methanesulfonic acid monohydrate (22.0mg, 0.1156mmol) were added to a 100mL single-necked flask. The reaction was carried out overnight at room temperature. 100mL of ethyl acetate was added to the system. The resultant mixture was washed with saturated brine for 3 times (80mL×3). The phase of ethyl acetate was dried over anhydrous sodium sulfate, and filtered. The filtrate was spin-dried to give a crude product of methyl 6-bromo-1*H*-benzo[d]imidazole-4-carboxylate (313 mg, crude product). ES-API: [M+H]⁺=257.0.

Step 2: Methyl 6-bromo-1*H*-benzo[d]imidazole-4-carboxylate (200mg, 0.7842mmol), 7-(4,4,5,5-tetramethyl-1,3,2-dioxaborolan-2-yl)-[1,2,4]triazolo[1,5-a]pyridin-2-amine (406.0mg, 7.5614mmol), [1,1'-bis(diphenylphosphino)ferrocene] dichloropalladium (140.0mg, 0.1708 mmol) and sodium carbonate (250.0mg, 2.360mmol), and finally 20mL of dioxane and 4mL of water were added to a 100mL single-neck flask. The mixture was purged with nitrogen gas for about 1.5 minutes. The reaction was carried out in an oil bath at 110°C for 5 hours. 100mL of ethyl acetate was added to the system. The resultant mixture was washed with saturated brine for 5 times (80mL×5). The phase of ethyl acetate was dried over anhydrous sodium sulfate and filtered. The filtrate was spin-dried, and the concentrate was purified by PTLC [dichloromethane:methanol=10:1, v/v] to give methyl 6-(2-amino-[1,2,4]triazolo[1,5-*a*]pyridin-7-yl)-1*H*-benzo[d]imidazole-4-carboxylate (97mg, Y: 25%). ES-API: [M+H]⁺=309.0.

Step 3: Methyl 6-(2-amino-[1,2,4]triazolo[1,5-*a*]pyridin-7-yl)-1*H*-benzo[d] imidazole-4-carboxylate (97.0 mg, 0.3149 mmol), lithium hydroxide monohydrate (63.0mg, 1.5746 mmol), 3mL of tetrahydrofuran, 3mL of methanol, and 3mL of water were added to a 50mL round-bottomed flask. The reaction was carried out overnight at room temperature. As monitored by TLC [dichloromethane:methanol=10:1, v/v] when the spot for the starting materials disappeared, the reaction was terminated. The reaction system was spin-dried under reduced pressure; thereafter, 10mL of water was added. The reaction system was cooled to 0°C in an ice-water bath. Hydrogen chloride solution (4M in dioxane) was added dropwise to adjust the pH to 5-6. 70ml of toluene was added. The resultant mixture was spin-dried under reduced pressure, to give 6-(2-amino-[1,2,4]triazolo[1,5-*a*]pyridin-7-yl)-1*H*-benzo[d]imidazole-4-carboxylic acid (103.0mg, crude product). ES-API: [M+H]⁺= 295.0.

Step 4: 6-(2-amino-[1,2,4]triazolo[1,5-*a*]pyridin-7-yl)-1*H*-benzo[d]imidazole-4-carboxylic acid (103.0mg, 0.3149mmol) and 10.0mL of anhydrous *N,N-*dimethylformamide were added to a 50mL round-bottomed flask. Next, 1-(2-(trifluoromethoxy)phenyl)ethan-1-amine (97.0mg, 0.4723mmol), triethylamine (318.0mg, 3.149mmol), and BOP reagent (154.0mg, 0.3480mmol) were added sequentially. The reaction was carried out at room temperature under the protection of nitrogen gas for 2 hours. 50mL of ethyl acetate was added to the system. The resultant mixture was washed with saturated brine for 4 times (30mLx4). The phase of ethyl acetate was dried over anhydrous sodium sulfate and filtered, and the filtrate was spin-dried to give 6-(2-amino-[1,2,4]triazolo[1,5-*a*]pyridin-7-yl)-*N*-(1-(2-(trifluoromethoxy)phenyl)ethyl)-1*H*-benzo[d]imidazole-4-carboxamide (30.0 mg, Y: 20%). ES-API:[M+H]⁺=481.5. ¹H NMR (400 MHz, DMSO-d6) δ 13.14 (s, 1H), 10.36 (s, 1H), 8.58 (d, *J* = 6.8 Hz, 1H), 8.14 (s, 2H), 7.73 - 7.51 (m, 2H), 7.44 - 7.15 (m, 5H), 6.50 (d, *J* = 62.6 Hz, 1H), 6.02 (s, 2H), 5.51 (t, *J* = 7.0 Hz, 1H), 1.53 (d, *J* = 7.0 Hz, 3H).

### Example 28: Synthesis of 4-(2-amino-[1,2,4]triazole[1,5-a]pyridin-7-yl)-N-(1-(2-(trifluoromethoxy)phenyl)ethyl)-1H-indazole-6-carboxamide

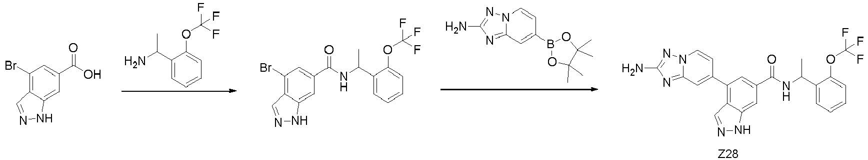

Step 1: 4-Bromo-1*H*-indazole-6-carboxylic acid (600 mg, 2.490 mmol) and 10 mL of *N,N*-dimethylformamide were added to a 50 mL round-bottomed flask. Next, 1-(2-(trifluoromethoxy)phenyl)ethan-1-amine (766 mg, 3.734 mmol), *N,N-*diisopropylethylamine (642 mg, 4.980 mmol), and 0-(7-azabenzotriazol-1-yl)-N,N,N',N'-tetramethyluronium hexafluorophosphate (1135 mg, 2.988 mmol) were added sequentially. The reaction mixture was reacted at room temperature for 4 hours. After the reaction was completed, 20 mL of ethyl acetate was added to the reaction solution. The resultant mixture was washed with saturated brine (20 mL^{∗}3). The organic phase was dried, filtered, and concentrated under reduced pressure. The residue was purified by combiflash to give 4-bromo-*N*-(1-(2-(trifluoromethoxy)phenyl)ethyl)-1*H*-indazole-6-carboxamide (320 mg, Y: 30%). ES-API: [M+H]⁺= 428.1

Step 2: 4-bromo-*N*-(1-(2-(trifluoromethoxy)phenyl)ethyl)-1*H*-indazole-6-carboxamide (50 mg, 0.117 mmol), 7-(4,4,5,5-tetramethyl-1,3,2-dioxaborolan-2-yl)-[1,2,4]triazolo[1,5-a]pyridine-2-amine (61 mg, 0.234 mmol), sodium carbonate (31 mg, 0.293 mmol), [1,1'-bis(diphenylphosphino)ferrocene]dichloropalladium (4 mg, 0.00585 mmol), dioxane (5 mL), and water (1 mL) were added to a 50mL single-necked flask. The reaction solution was heated to 110°C under the protection of nitrogen gas and stirred for 2 hours. After the reaction was completed, the resultant mixture was cooled to room temperature and concentrated under reduced pressure. The residue was purified by alkaline HPLC to give 4-(2-amino-[1,2,4]triazole[1,5-*a*]pyridin-7-yl)-*N*-(1-(2-(trifluoromethoxy)phenyl)ethyl)-1*H-*indazole-6-carboxamide (3.0 mg, Y: 5%) as an off-white solid. ES-API:[M+H]⁺=481.6. ¹H NMR (400 MHz, CDCl₃) δ 8.42 (d, J= 6.4 Hz, 1 H), 8.25 (s, 1 H), 8.03 (s, 1 H), 7.71 (s, 1 H), 7.63 (s, 1 H), 7.50-7.46 (m, 1 H), 7.34-7.21 (m, 4 H), 7.19-7.17 (m, 1 H), 6.59 (s, 1 H), 5.59-5.56 (m, 1 H), 4.59 (s, 1 H), 1.62-1.59 (m, 3 H).

### Example 29: Synthesis of (4-(2-amino-[1,2,4]triazolo[1,5-a]pyridin-7-yl)-1-methyl-1H-indazol-6-yl)(3-(4-fluorobenzyl)piperidin-1-yl)methanone

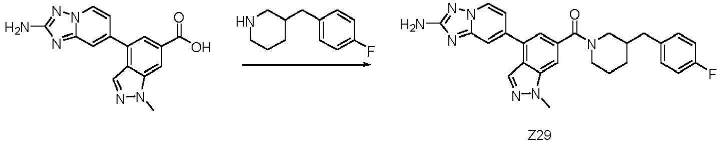

4-(2-Amino-[1,2,4]triazolo[1,5-a]pyridin-7-yl)-1-methyl-1H-indole-6-carboxylic acid (60mg, 0.195mmol) 3-(4-fluorobenzyl)piperidine (38 mg, 0.195 mmol), ((benzotriazol-1-yl)oxy)tris(dimethylamino)phosphonium hexafluorophosphate (129 mg, 0.293 mmol), triethylamine (59 mg, 0.585 mmol) and *N,N*-dimethylformamide (5ml) were added into a 50ml round-bottomed flask. The reaction was carried out at room temperature for 2 hours. 15 mL of water was added. The resultant mixture was extracted with 30 mL of ethyl acetate for 3 times. The organic phases were combined, dried over anhydrous sodium sulfate, and filtered. The crude product obtained by concentrating the organic phase was purified by alkaline HPLC to give (4-(2-amino-[1,2,4]triazolo[1,5-a]pyridin-7 -yl)-1 -methyl-1H-indazol-6 -yl)(3 -(4 -fluorobenzyl) piperidin-1-yl)methanone as an off-white powder (38.97mg, Y:44 %). ES-API:[M+H]+= 484.1. ¹H NMR (400 MHz, DMSO-d6) δ 8.65 (d, J = 7.2 Hz, 1 H), 8.25 (s, 1 H), 7.67-7.58 (m, 2 H), 7.33-7.14 (m, 4 H), 6.99-6.94 (m, 1 H), 6.70-6.65 (m, 1 H), 6.11 (s, 2 H), 4.45-4.26 (m, 1 H), 4.12 (s, 3 H), 3.59-3.36 (m, 1 H), 3.12-2.56 (m, 3 H), 2.43-2.17 (m, 1 H), 1.86-1.21 (m, 5 H).

### Example 30: Synthesis of 4-(2-amino-[1,2,4]triazolo[1,5-a]pyridin-7-yl)-N-(2-fluoro-5-(trifluoromethoxy)benzyl)-1-methyl-1H-indazole-6-carboxamide

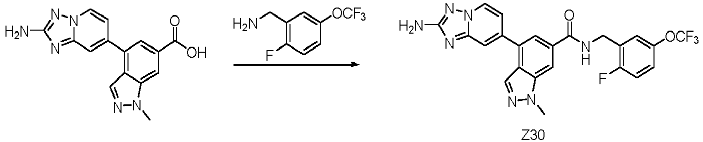

4-(2-Amino-[1,2,4]triazolo[1,5-a]pyridin-7-yl)-1-methyl-1H-indole-6-carboxylic acid (60mg, 0.195mmol), (2-fluoro-5-(trifluoromethoxy)phenyl)methylamine (41 mg, 0.195 mmol), ((benzotriazol-1-yl)oxy)tris(dimethylamino)phosphonium hexafluorophosphate (129 mg, 0.293 mmol), triethylamine (59 mg, 0.585 mmol) and *N,N*-dimethylformamide (5ml) were added to a 50ml round-bottomed flask. The reaction was carried out at room temperature for 2 hours. 15 mL of water was added. The resultant mixture was extracted with 30 mL of ethyl acetate for 3 times. The organic phases were combined, dried over anhydrous sodium sulfate, and filtered. The crude product obtained by concentrating the organic phase was purified by alkaline HPLC to give 4-(2-amino-[1,2,4]triazolo[1,5-a]pyridin-7-yl)-N-(2-fluoro-5-(trifluoromethoxy) benzyl)-1-methyl-1H-indazole-6-carboxamide as an off-white powder (36.39 mg, Y:38 %). ES-API:[M+H]+= 500.1. ¹HNMR (400 MHz, DMSO-d6) δ 9.33 (t, J = 5.6 Hz, 1 H), 8.69 (d, J = 6.8 Hz, 1 H), 8.31 (s, 1 H), 8.29 (s, 1 H), 7.91 (d, J = 1.2 Hz, 1 H), 7.73 (d, J = 1.2 Hz, 1 H), 7.44-7.41 (m, 1 H), 7.39-7.37 (m, 2 H), 7.30 (dd, J1 = 1.6 Hz, J = 6.8 Hz, 1 H), 6.12 (s, 2 H), 4.62 (d, J = 5.6 Hz, 2 H), 4.17 (s, 3 H).

### Example 31: Synthesis of 4-(2-amino-[1,2,4]triazolo[1,5-a]pyridin-7-yl)-N-(2-(cyclopentyloxy)-5-fluorobenzyl)-1-methyl-1H-indazole-6-carboxamide

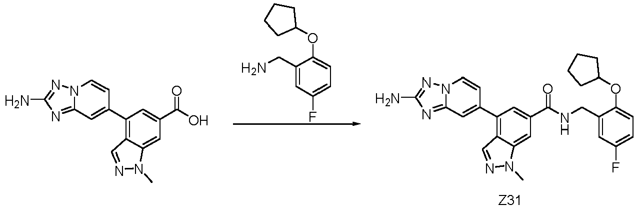

4-(2-Amino-[1,2,4]triazolo[1,5-a]pyridin-7-yl)-1-methyl-1H-indole-6-carboxylic acid (60mg, 0.195mmol), (2-(cyclopentyloxy)-5-fluorophenyl)methylamine (41 mg, 0.195 mmol), ((benzotriazol-1-yl)oxy)tris(dimethylamino)phosphonium hexafluorophosphate (129 mg, 0.293 mmol), triethylamine (59 mg, 0.585 mmol) and *N,N-*dimethylformamide (5ml) were added to a 50ml round-bottomed flask. The reaction was carried out at room temperature for 2 hours. 15 mL of water was added. The resultant mixture was extracted with 30 mL of ethyl acetate for 3 times. The organic phases were combined, dried over anhydrous sodium sulfate, and filtered. The crude product obtained by concentrating the organic phase was purified by alkaline HPLC to give 4-(2-amino-[1,2,4]triazolo[1,5-a]pyridin-7-yl)-N-(2-(cyclopentyloxy)-5-fluorobenzyl)-1-methyl-1H-indazole-6-carboxamide as an off-white powder (32.6 mg, Y:34 %). ES-API:[M+H]+= 500.2. ¹H NMR (400 MHz, DMSO-d6) δ 9.08 (t, J = 5.6 Hz, 1 H), 8.69 (d, J = 6.8 Hz, 1 H), 8.31 (s, 1 H), 8.30 (s, 1 H), 7.93 (s, 1 H), 7.75 (s, 1 H), 7.31 (dd, J1 = 1.2 Hz, J2= 6.4 Hz, 1 H), 7.05-7.01 (m,3 H), 6.11 (s, 2 H), 4.89-4.86 (m, 1 H), 4.50 (d, J = 5.2 Hz, 2 H), 4.18 (s, 3 H), 1.93-1.54 (m, 8 H).

Compounds Z32 to Z35 were prepared according to the methods as desecribed in the above Examples.

| Example | Structure | Name |
|---|---|---|
| 32 | 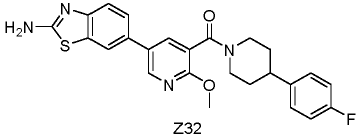 | (5-(2-aminobenzo[d]thiazol-6-yl)-2-methoxypyridin-3-yl)(4-(4-fluorophenyl)piperidin-1-yl)methanone |
| | ES-API:[M+H]⁺= 463.2 | |
| 33 | 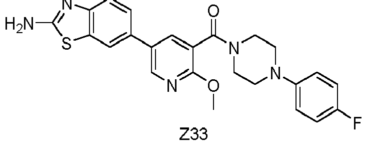 | (5-(2-aminobenzo[d]thiazol-6-yl)-2-methoxypyridin-3-yl)(4-(4-fluorophenyl)piperazin- 1 -yl)methanone |
| | ES-API:[M+H]⁺= 464.1 | |
| 34 | 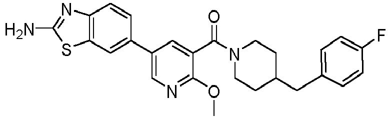 | (5-(2-aminobenzo[d]thiazol-6-yl)-2-methoxypyridin-3-yl)(4-(4-fluorobenzyl)piperidin-1 -yl)methanone |
| | Z34 ES-API:[M+H]⁺= 477.2 | |
| 35 | 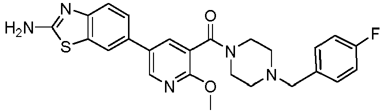 | (5-(2-aminobenzo[d]thiazol-6-yl)-2-methoxypyridin-3-yl)(4-(4-fluorobenzyl)piperazin-1 -yl)methanone |
| | Z35 ES-API:[M+H]⁺= 478.2 | |

### Example 36: Synthesis of 5-(2-amino-[1,2,4]triazolo[1,5-a]pyridin-7-yl)-3-(3-(4- fluorobenzyl)piperidin-1-carbonyl)-1- methylpyridine-2(1H)-one

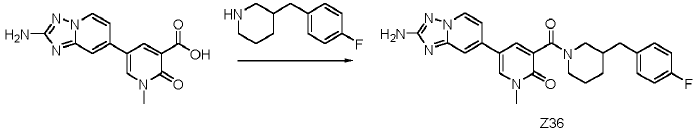

The preparation method was similar to Example 11. ES-API:[M+H]⁺=461.2. ¹H NMR (400 MHz, DMSO-d6) δ 8.52 (t, *J* = 8.2 Hz, 1H), 8.39 (dd, *J* = 22.6, 2.6 Hz, 1H), 7.91 (dd, *J* = 36.2, 2.5 Hz, 1H), 7.56 (d, *J* = 10.7 Hz, 1H), 7.27 - 7.18 (m, 1H), 7.10 (dd, *J* = 18.3, 9.5 Hz, 2H), 6.92 (d, *J* = 69.9 Hz, 2H), 5.97 (d, *J* = 5.2 Hz, 2H), 4.35 - 4.07 (m, 1H), 3.48 (d, *J* = 31.5 Hz, 3H), 3.04 (d, *J* = 62.9 Hz, 1H), 2.80 - 2.49 (m, 3H), 1.84 - 1.11 (m, 6H).

### Example 37: Synthesis of 5-(2-aminobenzo[d]thiazol-6-yl)-2-methoxy-6-methyl-N-(1-(2-(trifluoromethoxy)phenyl)ethyl)nicotinamide

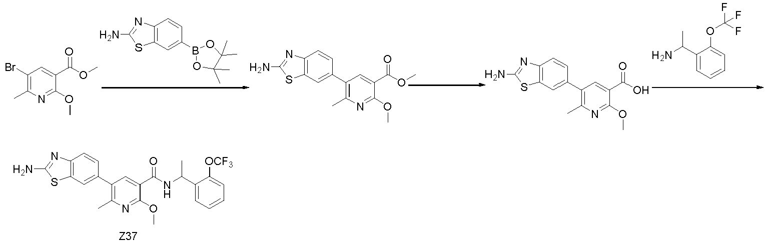

Step 1: Methyl 5-bromo-2-methoxy-6-methylnicotinate (400mg, 1.538mmol), 6-(4,4,5,5-tetramethyl-1,3,2-dioxaborolan-2-yl)benzothiazol-2-amine (510mg, 1.8478mmol), tetrakis(triphenylphosphine)palladium (100.0mg, 0.0865mmol) and sodium carbonate (500mg, 4.717mmol), and finally 40mL of dimethoxyethane and 6mL of water were added to a 100mL single-neck flask. The mixture was purged with nitrogen gas for about 1.5 minutes. The reaction was carried out in an oil bath at 105°C for 5 hours. 100mL of ethyl acetate was added to the system. The resultant mixture was washed with saturated brine for 5 times (80mL×5). The phase of ethyl acetate was dried over anhydrous sodium sulfate and filtered. The filtrate was spin-dried, and the concentrate was purified by PTLC [dichloromethane:methanol=10:1, v/v] to give methyl 5-(2-aminobenzo[d]thiazol-6-yl)-2-methoxy-6-methylnicotinate (545 mg, crude product). ES-API: [M+H]⁺=330.2.

Step 2: Methyl 5-(2-aminobenzo[d]thiazol-6-yl)-2-methoxy-6-methylnicotinate (545mg, 1.538mmol), lithium hydroxide monohydrate (323mg, 7.690mmol), 5mL of tetrahydrofuran, 5mL of methanol, and 5mL of water were added to a 50mL round-bottomed flask. The mixture was stirred for reaction overnight at room temperature. As monitored by TLC [dichloromethane:methanol=10:1, v/v] when the spot for the starting materials disappeared, the reaction was terminated. The reaction system was spin-dried under reduced pressure; thereafter, 10mL of water was added. The reaction system was cooled to 0°C in an ice-water bath. Hydrogen chloride solution (4M in dioxane) was added dropwise to adjust the pH to 5-6. 70ml of toluene was added. The resultant mixture was spin-dried under reduced pressure, to give 5-(2-aminobenzo[d]thiazol-6-yl)-2-methoxy-6-methylnicotinic acid (251 mg, Y: 52%). ES-API: [M+H]+=316.2..

Step 3: 5-(2-Aminobenzo[d]thiazol-6-yl)-2-methoxy-6-methylnicotinic acid (151mg, 0.380mmol) and 3.0mL anhydrous *N,N*-dimethylformamide were added to a 50mL round-bottomed flask. Next, 1-(2-(trifluoromethoxy)phenyl)ethan-1-amine (116.8mg, 0.570mmol), triethylamine (384.0mg, 3.80 mmol), and BOP reagent (252mg, 0.570mmol) were added sequentially. The reaction was carried out at room temperature under the protection of nitrogen gas for 12 hours. 50mL of ethyl acetate was added to the system. The resultant mixture was washed with saturated brine for 4 times (30mLx4). The phase of ethyl acetate was dried over anhydrous sodium sulfate and filtered, and the filtrate was spin-dried to give 5-(2-aminobenzo[d]thiazol-6-yl)-2-methoxy-6-methyl-*N*-(1-(2-(trifluoromethoxy)phenyl)ethyl)nicotinamide (22.0mg, Y: 9%). ES-API:[M+H]⁺=503. ¹H NMR (400 MHz, DMSO-d6) δ 8.59 (d, *J* = 7.7 Hz, 1H), 7.81 (s, 1H), 7.65 (d, *J =* 1.6 Hz, 1H), 7.63 - 7.57 (m, 1H), 7.52 (s, 2H), 7.42 - 7.28 (m, 4H), 7.16 (dd, *J =* 8.3, 1.7 Hz, 1H), 5.35 (p, *J =* 7.0 Hz, 1H), 3.99 (s, 3H), 2.40 (s, 3H), 1.41 (d, *J* = 7.0 Hz, 3H).

### Example 38: Synthesis of 5-(2-aminobenzo[d]thiazol-6-yl)-N-(2-(cyclopropylmethoxy)-3,5-difluorobenzyl)-2-methoxy-6-methylnicotinamide

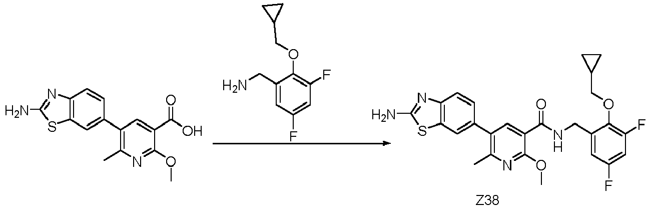

5-(2-Aminobenzo[d]thiazol-6-yl)-2-methoxy-6-methylnicotinic acid (60mg, 0.1904mmol) and 5.0mL of anhydrous *N,N*-dimethylformamide were added to a 50mL round-bottomed flask. Next, (2-(cyclopropylmethoxy)-3,5-difluorophenyl)methylamine (48.7mg, 0.2286mmol), triethylamine (192mg, 1.904mmol), and BOP reagent (101mg, 0.2286mmol) were added sequentially. The reaction was carried out at room temperature under the protection of nitrogen gas for 12 hours. 50mL of ethyl acetate was added to the system. The resultant mixture was washed with saturated brine for 6 times (30mLx6). The phase of ethyl acetate was dried over anhydrous sodium sulfate and filtered, and the filtrate was spin-dried to give 5-(2-aminobenzo[d]thiazol-6-yl)-*N*-(2-(cyclopropylmethoxy)-3,5-difluorobenzyl)-2-methoxy-6-methylnicotinamide (6.3mg, Y: 7.8%). ES-API: [M+H]+=511. ¹H NMR (400 MHz, DMSO-d6) δ 8.74 (t, *J* = 6.1 Hz, 1H), 7.93 (s, 1H), 7.66 (d, *J=* 1.4 Hz, 1H), 7.50 (s, 2H), 7.35 (d, *J=* 8.2 Hz, 1H), 7.23 - 7.10 (m, 2H), 6.91 (d, *J* = 9.3 Hz, 1H), 4.55 (d, *J* = 6.1 Hz, 2H), 4.00 (s, 3H), 3.84 (d, *J* = 7.1 Hz, 2H), 2.41 (s, 3H), 1.20 (d, *J=* 5.3 Hz, 1H), 0.55 - 0.49 (m, 2H), 0.30 - 0.22 (m, 2H).

### Example 39: Synthesis of 5-(2-aminobenzo[d]thiazol-6-yl)-1,6-dimethyl-2-oxo-N-(1-(2-(trifluoromethoxy)phenyl)ethyl)-1,2-dihydropyridine-3-carboxamide

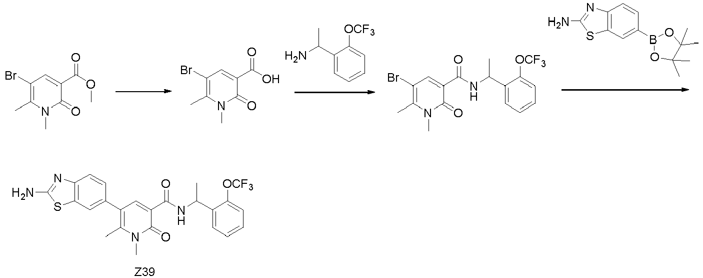

Step 1: 9.0mL of tetrahydrofuran/methanol/water (V:V:V=1:1:1) was added to a 50mL single-necked round-bottomed flask. Next, methyl 5-bromo-1,6-dimethyl-2-oxo-1,2-dihydropyridine-3-carboxylate (100mg, 0.3846mmol) was added. Finally, lithium hydroxide monohydrate (81mg, 1.923mmol) was added. The reaction was carried out at room temperature under the protection of nitrogen gas for 12 hours. After the reaction was completed, the reaction mixture was spin-dried under reduced pressure to remove the solvent. 10 mL of water was added to the system. The system was adjusted to pH 5~6 with a solution of hydrogen chloride solution (4M). The resultant mixture was spin-dried under reduced pressure to remove the solvent, to give the target compound of 5-bromo-1,6-dimethyl-2-oxo-1,2-dihydropyridine-3-carboxylic acid as a crude product (50 mg, Y: 53%). ES-API: [M+H]⁺= 246.0.

Step 2: 5mL of *N,N*-dimethylformamide/triethylamine (V:V=9:1) was added to a 50mL single-necked round-bottomed flask. Next, 5-bromo-1,6-dimethyl-2-oxo-1,2-dihydropyridine-3-carboxylic acid (60mg, 0.2597mmol) and 1-(2-(trifluoromethoxy)phenyl)ethan-1-amine (63mg, 0.3061mmol) was added. Finally, BOP reagent (136mg, 0.3061mmol) was added. The reaction was carried out at room temperature under the protection of nitrogen gas for 2 hours. After the reaction was completed, 80mL of ethyl acetate was added to the system. The resultant mixture was sequentially washed with saturated ammonium chloride and sodium chloride for 5 times (60mL×5). The phase of ethyl acetate was dried over anhydrous sodium sulfate and filtered. The filtrate was spin-dried and purified on silica gel using automatic flash chromatography (ethyl acetate/petroleum ether=0-50%). The resultant solution was spin-dried under reduced pressure to remove the solvent, to give the target compound of 5-bromo-1,6-dimethyl-2-oxo-*N*-(1-(2-(trifluoromethoxy)phenyl)ethyl)-1,2-dihydro pyridine-3-carboxamide (90mg, Y: 88%). ES-API: [M+H]⁺= 435.1.

Step 3:, 20.0 mL of dioxane and 6.0 mL of water were added to a 100 mL single-necked flask under the protection of nitrogen gas. Next, 5-bromo-1,6-dimethyl-2-oxo-*N*-(1-(2-(trifluoromethoxy)phenyl)ethyl)-1,2-dihydropyridine-3-carboxamide (70.0mg, 0.21428mmol), 6-(4,4,5,5-tetramethyl-1,3,2-dioxaborolan-2-yl)benzo[d]thiazol-2-amine (47mg, 0.1713mmol), sodium carbonate (45.4mg, 0.4284 mmol) and tetrakis(triphenylphosphine) palladium (10mg, 0.007mmol) were added. The mixture was nitrogen-purged for three times. The reaction was carried out in an oil bath at 110°C for 4 hours. After the reaction was completed, 60mL of ethyl acetate was added to the system. The resultant mixture was sequentially washed with saturated ammonium chloride and sodium chloride twice (80mL×2). The phase of ethyl acetate was dried over anhydrous sodium sulfate and filtered. The crude product of the target compound obtained by spin-drying the was purified by pre-HPLC to give the target compound of 5-(2-aminobenzo[d]thiazol-6-yl)-1,6-dimethyl-2-oxo-*N*-(1-(2-(trifluoromethoxy)phenyl)ethyl)-1,2-dihydropyridine-3-carboxamide (6.4 mg, Y: 6.2%). ES-API:[M+H]⁺=503.3. ¹H NMR (400 MHz, DMSO-d6) δ 10.35 (d, *J=* 7.6 Hz, 1H), 8.09 (s, 1H), 7.61 - 7.48 (m, 4H), 7.42 - 7.29 (m, 4H), 7.08 (dd, *J* = 8.2, 1.8 Hz, 1H), 5.35 (p, *J=* 6.9 Hz, 1H), 3.62 (s, 3H), 2.39 (s, 3H), 1.43 (d, *J* = 6.9 Hz, 3H).

### Example 40: Synthesis of 5-(2-aminobenzo[d]thiazol-6-yl)-2-methoxy-6-methyl-N-(2-((tetrahydrofuran-3-yl)oxy)benzyl)nicotinamide

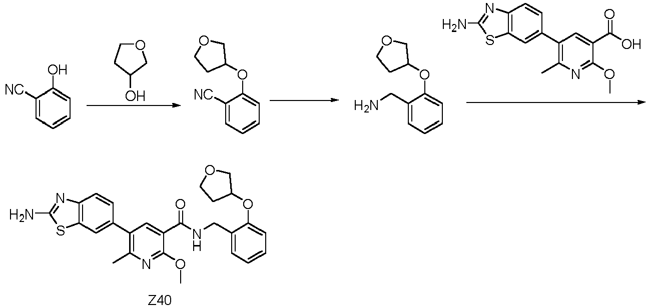

Step 1: 50 mL of anhydrous toluene was added to a 100 mL three-necked round-bottomed flask at room temperature under the protection of nitrogen gas. Next, 2-hydroxybenzonitrile (2.0g, 16.80mmol) and tetrahydrofuran-3-ol (2.22g, 25.22mmol) were added. Finally, triphenyl phosphate (5.72g, 21.81 mmol) was added. The reaction was carried out at room temperature for 5 minutes. Thereafter, diisopropyl azodicarboxylate (4.65 mL, 23.01 mmol) was added. The reaction was carried out at room temperature for another 12 hours. After the reaction was completed, 80 mL of dichloromethane was added to the system. The resultant mixture was sequentially washed with saturated ammonium chloride and sodium chloride twice (60 mL×2). The phase of ethyl acetate was dried over anhydrous sodium sulfate and filtered. The filtrate was spin-dried and purified by PTLC [petroleum ether:ethyl acetate=100:0 ~ 85:15, v/v] to give the target compound of 2-((tetrahydrofuran-3-yl)oxy)benzonitrile (4.4g, crude product). ES-API: [M+H]⁺=190.1.

Step 2: 20mL of tetrahydrofuran and 2-((tetrahydrofuran-3-yl)oxy)benzonitrile (1.4g, 4.2mmol) were added to a 500mL single-necked round-bottomed flask under the protection of nitrogen gas. Finally, borane-tetrahydrofuran complex (20mL, 1M, 20.0mmol) was added. The mixture was warmed up slowly from room temperature to be boiling, and the reaction was carried out overnight. After the reaction was completed, the resultant mixture was cooled to room temperature. Methanol was carefully added dropwise until no more bubbles were generated. 80mL of ethyl acetate was added to the system. The resultant mixture was sequentially washed with saturated ammonium chloride and sodium chloride twice (60mL×2). The phase of ethyl acetate was dried over anhydrous sodium sulfate and filtered. The filtrate was spin-dried to give the target compound of (2-((tetrahydrofuran-3-yl)oxy)phenyl)methylamine (76mg, Y: 10%). ES-API: [M+H]⁺=194.3.

Step 3: 5-(2-Aminobenzo[d]thiazol-6-yl)-2-methoxy-6-methylnicotinic acid (109mg, 0.3460mmol) and 3.0mL of anyhydrous N,N-dimethylformamide were added to a 50mL round-bottomed flask. Next, (2-((tetrahydrofuran-3-yl)oxy)phenyl)methylamine (76.0mg, 0.3937mmol), triethylamine (350mg, 3.460mmol), and BOP reagent (184mg, 0.4163mmol) were added. The reaction was carried out at room temperature under the protection of nitrogen gas for 2 hours. 50mL of ethyl acetate was added to the system. The resultant mixture was washed with saturated brine for 4 times (30mLx4). The phase of ethyl acetate was dried over anhydrous sodium sulfate and filtered. The filtrate was spin-dried to give 5-(2-aminobenzo[d]thiazol-6-yl)-2-methoxy-6-methyl-*N*-(2-((tetrahydrofuran-3-yl)oxy)benzyl)nicotinamide (7.5 mg, Y: 8%). ES-API:[M+H]⁺=491.5. ¹H NMR (400 MHz, DMSO-d6) δ 8.68 (t, *J=* 5.9 Hz, 1H), 8.58 (d, *J* = 2.6 Hz, 1H), 8.33 *(d, J =* 2.6 Hz, 1H), 8.07 - 7.94 (m, 2H), 7.51 (dt, *J=* 8.4, 4.8 Hz, 4H), 7.37 (d, *J=* 8.3 Hz, 1H), 6.90 (dd, *J* = 7.2, 5.0 Hz, 1H), 5.43 (dd, *J* = 6.9, 4.5 Hz, 1H), 4.38 (d, *J* = 5.9 Hz, 2H), 4.00 (s, 3H), 2.04 (s, 1H), 1.98 - 1.83 (m, 2H), 1.78 - 1.66 (m, 4H), 1.57 (dd, *J* = 13.0, 6.4 Hz, 2H).

### Example 41: Synthesis of 5-(2-aminobenzo[d]thiazol-6-yl)-N-((2-(cyclopentyloxy)pyridin-3-yl)methyl)-2-methoxy-6-methylnicotinamide

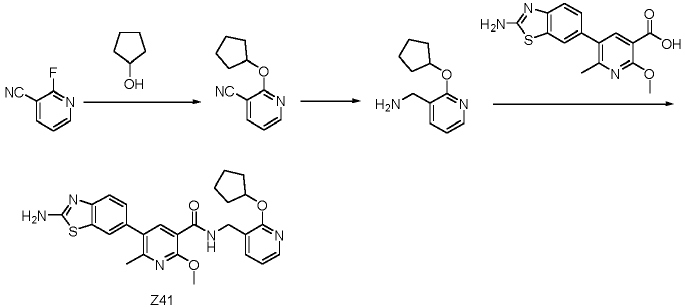

Step 1: 50 mL of anhydrous *N,N*-dimethylformamide and cyclopentanol (1.40g, 16.25mmol) were added to a 100 mL three-necked round-bottomed flask at 0°C under the protection of nitrogen gas. Next, sodium hydride (409mg, 10.23mmol) was added. Finally, 2-fluoronicotinonitrile (1.0g, 8.190mmol) was added. The resultant mixture was slowly warmed up to 55°C, and the reaction was carried out for 12 hours. After the reaction was completed, the reaction system was cooled to 0°C, and 80 mL of ethyl acetate was added to the system. The resultant mixture was sequentially washed with saturated ammonium chloride and sodium chloride twice (60 mL×2). The phase of ethyl acetate was dried over anhydrous sodium sulfate and filtered. The filtrate was spin-dried to give the target compound of 2-(cyclopentyloxy)nicotinonitrile (2.0g, crude product). ES-API: [M+H]⁺=189.1.

Step 2: 20mL of tetrahydrofuran and 2-(cyclopentyloxy)nicotinonitrile (1.0g, 4.1mmol) were added to a 500mL single-necked round-bottomed flask under the protection of nitrogen gas. Finally, borane-tetrahydrofuran complex (20mL, 1M, 20.0mmol) was added. The mixture was warmed up slowly from room temperature to be boiling, and the reaction was carried out overnight. After the reaction was completed, the resultant mixture was cooled to room temperature. Methanol was carefully added dropwise until no more bubbles were generated. 80mL of ethyl acetate was added to the system. The resultant mixture was sequentially washed with saturated ammonium chloride and sodium chloride twice (60mL×2). The phase of ethyl acetate was dried over anhydrous sodium sulfate and filtered. The filtrate was spin-dried to give the target compound of (2-(cyclopentyloxy)pyridin-3-yl)methylamine (130mg, Y: 16.5%). ES-API: [M+H]⁺=193.4.

Step 3: 5-(2-Aminobenzo[d]thiazol-6-yl)-2-methoxy-6-methylnicotinic acid (66mg, 0.2095mmol) and 3.0mL of anyhydrous *N,N*-dimethylformamide were added to a 50mL round-bottomed flask. Next, ((2-(cyclopentyloxy)pyridin-3-yl)methylamine (40.0mg, 0.2083mmol), triethylamine (211mg, 2.095mmol), and BOP reagent (110mg, 0.2488mmol) were added. The reaction was carried out at room temperature under the protection of nitrogen gas for 2 hours. 50mL of ethyl acetate was added to the system. The resultant mixture was washed with saturated brine for 6 times (30mL×6). The phase of ethyl acetate was dried over anhydrous sodium sulfate and filtered. The filtrate was spin-dried to give 5-(2-aminobenzo[d]thiazol-6-yl)-*N-*((2-(cyclopentyloxy)pyridin-3-yl)methyl)-2-methoxy-6-methylnicotinamide (18mg, Y: 22.5%). ES-API: [M+H]⁺=490.5. ¹H NMR (400 MHz, DMSO-d6) δ 8.58 (t, *J* = 6.0 Hz, 1H), 8.01 (dd, *J* = 5.0, 1.7 Hz, 1H), 7.96 (s, 1H), 7.66 (d, *J* = 1.7 Hz, 1H), 7.51 (d, *J* = 7.0Hz, 3H), 7.35 (d, *J* = 8.2 Hz, 1H), 7.17 (dd, *J* = 8.2, 1.8 Hz, 1H), 6.88 (dd, *J* = 7.2, 5.0 Hz, 1H), 5.42 (dd, *J* = 6.9, 4.4 Hz, 1H), 4.36 (d, *J* = 5.9 Hz, 2H), 3.99 (s, 3H), 2.41 (s, 3H), 1.97 - 1.84 (m, 2H), 1.71 (d, *J* =4.7 Hz, 4H), 1.61 - 1.52 (m, 2H).

### Example 42: Synthesis of 5-(2-aminobenzo[d]thiazol-6-yl)-N-(2-isopropoxybenzyl)-2-methoxy-6-methylnicotinamide

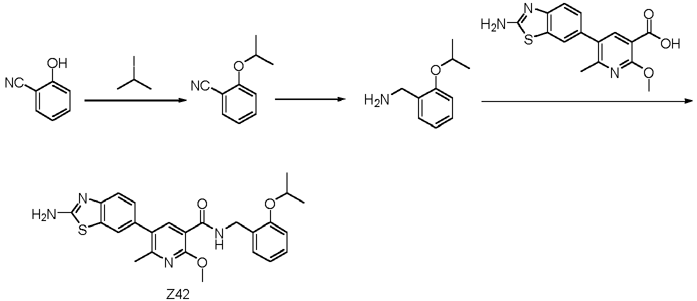

Step 1: 20mL of Acetone was added to a 100 mL three-necked round-bottomed flask at room temperature under the protection of nitrogen gas. Next, 2-hydroxybenzonitrile (0.77g, 6.458mmol) and 2-iodopropane (2.0g, 11.76mmol) were added. Finally, cesium carbonate (4.2g, 13.0mmol) was added. The resultant mixture was heated to 60°C, and the reaction was carried out for 45 minutes. After the reaction was completed, 80 mL of ethyl acetate was added to the system. The resultant mixture was sequentially washed with saturated ammonium chloride and sodium chloride twice (60 mL×2). The phase of ethyl acetate was dried over anhydrous sodium sulfate and filtered. The filtrate was spin-dried to give the target compound of 2-isopropylbenzonitrile (1.0 g, crude product).

Step 2: 30mL of tetrahydrofuran and 2-isopropylbenzonitrile (1.0g, 6.211mmol) were added to a 500mL single-necked round-bottomed flask under the protection of nitrogen gas. Finally, borane-tetrahydrofuran complex (30mL, 1M, 30.0mmol) was added. The mixture was warmed up slowly from room temperature to be boiling, and the reaction was carried out overnight. After the reaction was completed, the resultant mixture was cooled to room temperature. Methanol was carefully added dropwise until no more bubbles were generated. 80mL of ethyl acetate was added to the system. The resultant mixture was sequentially washed with saturated ammonium chloride and sodium chloride twice (60mL×2). The phase of ethyl acetate was dried over anhydrous sodium sulfate and filtered. The filtrate was spin-dried to give the target compound of (2-isopropoxyphenyl) methylamine (150 mg, Y: 15%). ES-API: [M+H]⁺=166.2.

Step 3: 5-(2-Aminobenzo[d]thiazol-6-yl)-2-methoxy-6-methylnicotinic acid (62mg, 0.1968mmol) and 3.0mL of anyhydrous *N,N*-dimethylformamide were added to a 50mL round-bottomed flask. Next, (2-isopropoxyphenyl) methylamine (49.0mg, 0.2969mmol), triethylamine (200mg, 1.968mmol), and BOP reagent (125mg, 0.2829mmol) were added seuentially. The reaction was carried out at room temperature under the protection of nitrogen gas for 2 hours. 50mL of ethyl acetate was added to the system. The resultant mixture was washed with saturated brine for 4 times (30mL×4). The phase of ethyl acetate was dried over anhydrous sodium sulfate and filtered. The filtrate was spin-dried to give 5-(2-aminobenzo[d]thiazol-6-yl)-*N*-(2-isopropoxybenzyl)-2-methoxy-6-methylnicotinamide (10.7mg, Y:16.8%). ES-API:[M+H]⁺=463.3. ¹H NMR (400 MHz, DMSO-d6) δ 8.52 (t, *J* = 6.0 Hz, 1H), 7.98 (s, 1H), 7.66 (d, *J* = 1.6 Hz, 1H), 7.53 (s, 2H), 7.35 (d, *J* = 8.2 Hz, 1H), 7.17 (dd, *J=* 12.0, 4.1 Hz, 3H), 7.03 - 6.94 (m, 1H), 6.84 (t, *J* = 7.3 Hz, 1H), 4.64 (dt, *J* = 12.1, 6.1 Hz, 1H), 4.42 (d, *J=* 6.1 Hz, 2H), 4.00 (s, 3H), 2.41 (s, 3H), 1.28 (d, *J* = 6.0 Hz, 6H).

### Example 43: Synthesis of 5-(2-aminobenzo[d]thiazol-6-yl)-N-(1-(2-fluoro-5-(trifluoromethoxy)phenyl)ethyl)-2-methylnicotinamide

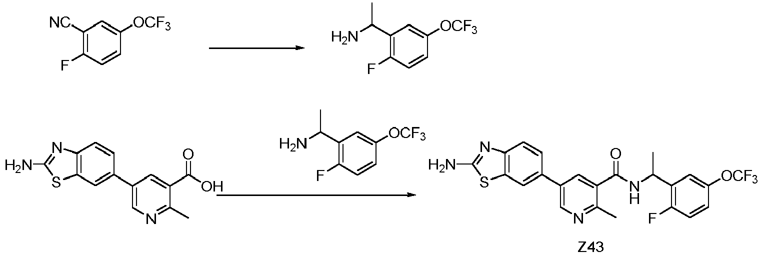

Step 1:, 20mL of anhydrous tetrahydrofuran, and then 2-fluoro-5-(trifluoro methoxy)benzonitrile (0.50g, 2.475mmol) and methyllithium (2.96 mL, 4.950 mmol) were added to a 100mL three-necked round-bottomed flask at -78°C under the protection of nitrogen gas. The reaction was carried out at this temperature for 2 hours. Thereafter, sodium borohydride (188 mg, 4.950 mmol) was added. The mixtue was slowly warmed up to 0°C, and the reaction was carried out for another 2 hours. After the reaction was completed, 50mL of 1M hydrochloric acid solution was added to the system. The resultant mixture was spin-dried under reduced pressure and purified by PTLC [dichloromethane:methanol=10:1, v/v] to give the target compound of 1-(2-fluoro-5-(trifluoromethoxy)phenyl)ethan-1-amine (168mg, Y: 30%). ES-API: [M+H]+=224.0.

Step 2: 5-(2-Aminobenzo[d]thiazol-6-yl)-2-methylnicotinic acid (50.0mg, 0.1754mmol) and 4.0mL of anyhydrous *N,N*-dimethylformamide were added to a 50mL round-bottomed flask. Next, 1-(2-fluoro-5-(trifluoromethoxy)phenyl)ethan-1-amine (58.7mg, 0.2637mmol), triethylamine (177mg, 1.754mmol), and BOP reagent (116.3mg, 0.2637mmol) were added sequentially. The reaction was carried out at room temperature under the protection of nitrogen gas for 12 hours. 50mL of ethyl acetate was added to the system. The resultant mixture was washed with saturated brine for 4 times (30mLx4). The phase of ethyl acetate was dried over anhydrous sodium sulfate and filtered. The filtrate was spin-dried to give 5-(2-aminobenzo[d]thiazol-6-yl)-*N*-(1-(2-fluoro-5-(trifluoromethoxy)phenyl)ethyl)-2-methylnicotinamide (12.2 mg, Y: 14.0%). ES-API:[M+H]⁺=491.3. ¹H NMR (400 MHz, DMSO-d6) δ 9.06 (d, *J* = 7.7 Hz, 1H), 8.80 (d, *J* = 2.3 Hz, 1H), 8.06 *(d, J =* 1.8 Hz, 1H), 7.95 (d, *J* = 2.3 Hz, 1H), 7.67 - 7.54 (m, 3H), 7.45 (d, *J* = 3.5 Hz, 1H), 7.44 - 7.28 (m, 3H), 5.31 (dd, *J* = 14.5, 7.2 Hz, 1H), 2.42 (s, 3H), 1.43 (d, *J* = 7.0 Hz, 3H).

### Example 44: Synthesis of 6-amino-5-(2-aminobenzo[d]thiazol-6-yl)-N-(1-(2-(trifluoromethoxy)phenyl)ethyl)nicotinamide

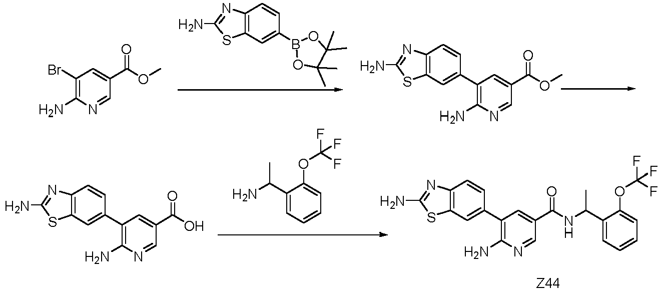

Step 1: Methyl 6-amino-5-bromonicotinate (200mg, 0.8656mmol), 6-(4,4,5,5-tetramethyl-1,3,2-dioxaborolan-2-yl)benzothiazol-2-amine (270mg, 0.9783mmol), tetrakis(triphenylphosphine) palladium (60mg, 0.052mmol), and sodium carbonate (200mg, 1.886mmol), and finally 20mL of dimethoxyethane and 4mL of water were added to a 100mL single-necked flask. The mixture was purged with nitrogen gas for about 1.5 minutes. The reaction was carried out in an oil bath at 105°C for 5 hours. 100mL of ethyl acetate was added to the system. The resultant mixture was washed with saturated brine for 5 times (80mL×5). The phase of ethyl acetate was dried over anhydrous sodium sulfate and filtered. The filtrate was spin-dried, and the concentrate was purified by PTLC [dichloromethane:methanol=10:1, v/v] to give methyl 6-amino-5-(2-aminobenzo[d]thiazol-6-yl)nicotinate (120mg, Y:46%). ES-API:[M+H]⁺=301.

Step 2: Methyl 6-amino-5-(2-aminobenzo[d]thiazol-6-yl)nicotinate (120mg, 0.400mmol), lithium hydroxide monohydrate (80mg, 2.000mmol), 3mL of tetrahydrofuran, 3mL of methanol and 3mL of water were added to a 50mL round-bottomed flask. The reaction was carried out overnight at room temperature. As monitored by TLC [dichloromethane:methanol=10:1, v/v] when the spot for the starting materials disappeared, the reaction was terminated. The reaction system was spin-dried under reduced pressure; thereafter, 10mL of water was added. The reaction system was cooled to 0°C in an ice-water bath. Hydrogen chloride solution (4M in dioxane) was added dropwise to adjust the pH to 5-6. 70ml of toluene was added. The resultant mixture was spin-dried under reduced pressure, to give 6-amino-5-(2-aminobenzo[d]thiazol-6-yl) nicotinic acid (132mg, crude product). ES-API: [M+H]⁺= 287.

Step 3: 6-amino-5-(2-aminobenzo[d]thiazol-6-yl) nicotinic acid (66mg, 0.2000mmol) and 4.0mL of anhydrous *N,N*-dimethylformamide were added to a 50mL round-bottomed flask. Next, 1-(2-(trifluoromethoxy)phenyl)ethan-1-amine (61.5mg, 0.3000mmol), triethylamine (202.0mg, 2.000mmol) and BOP reagent (133.0mg, 0.3000mmol) were added sequentially. The reaction was carried out at room temperature under the protection of nitrogen gas for 12 hours. 50mL of ethyl acetate was added to the system. The resultant mixture was washed with saturated brine for 4 times (30mL×4). The phase of ethyl acetate was dried over anhydrous sodium sulfate and filtered, and the filtrate was spin-dried to give 6-amino-5-(2-aminobenzo[d]thiazol-6-yl)-N-(1-(2-(trifluoromethoxy)phenyl)ethyl)nicotinamide (23.0mg, Y: 25.3%). ES-API: [M+H]⁺=474.0. ¹H NMR (400 MHz, DMSO-d6) δ 8.58 (d, *J* = 7.5 Hz, 1H), 8.44 (d, *J=* 2.3 Hz, 1H), 7.83 (d, *J* = 2.3 Hz, 1H), 7.70 (d, *J* = 1.6 Hz, 1H), 7.63 - 7.48 (m, 3H), 7.43 - 7.22 (m, 5H), 6.17 (s, 2H), 5.38 (p, *J* = 7.0 Hz, 1H), 1.38 (d, *J* = 7.0 Hz, 3H).

### Example 46: Synthesis of 2-amino-5-(2-aminobenzo[d]thiazol-6-yl)-N-(1-(2-(trifluoromethoxy)phenyl)ethyl)nicotinamide

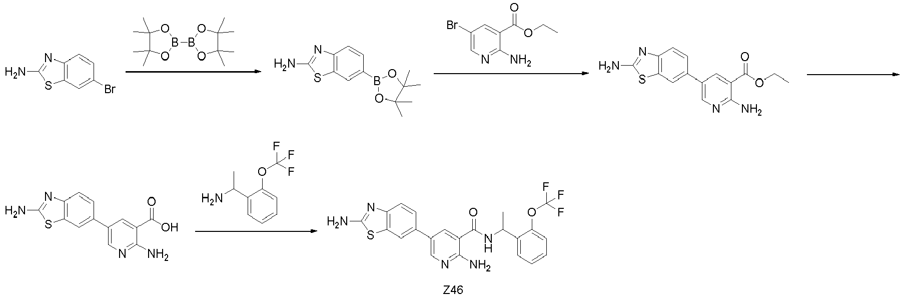

Step 1: 2-Amino-6-bromobenzothiazole (3.0 g, 13.1 mmol), bis(pinacolato)diboron (6.65 g, 26.2 mmol), potassium acetate (3.21 g, 32.74mmol), [1,1'-bis(diphenylphosphino)ferrocene]dichloropalladium-dichlorom ethane complex (1g, 1.31 mmol), and 1,4-dioxane (60ml) were added to a 250mL round-bottomed flask. The mixture was nitrogen-purged for three times. The reaction was carried out at 100°C for 8 hours. Then, the reaction mixture was cooled to room temperature, and was suction filtered. The filtrate obtained by washing the filter cake with ethyl acetate for three times was concentrated to give a crude product of 6-(4,4,5,5-tetramethyl-1,3,2-dioxaborolan-2-yl)benzothiazol-2-amine (3.8 g, Y: 100%). ES-API: [M+H]⁺= 277.2.

Step 2: Ethyl 2-amino-5-bromonicotinate (300 mg, 1.230 mmol), 6-(4,4,5,5-tetramethyl-1,3,2-dioxaborolan-2-yl)benzothiazol-2-amine (408 mg, 1.475 mmol), sodium carbonate (260 mg, 2.460 mmol), tetrakis(triphenylphosphine)palladium (72 mg, 0.0615 mmol), dimethoxyethane (10 ml), and water (2 ml) were added to a 50 mL round-bottomed flask. The mixture was nitrogen-purged for three times. The reaction was carried out at 105°C for 3 hours. Then, the reaction mixture was cooled to room temperature, and was suction filtered. 50mL of water was added to a filtrate obtained by washing the filter cake with ethyl acetate for three times. The resultant mixture was exracted with 30 mL of ethyl acetate for 3 times. The organic phase was dried, concentrated, and subjected to thin layer chromatography (methanol/ dichloromethane =0~10%) to give ethyl 2-amino-5-(2-aminobenzo[d]thiazol-6-yl) nicotinate (255 mg, Y: 66%). ES-API: [M+H]⁺= 315.1.

Step 3: Ethyl 2-amino-5-(2-aminobenzo[d]thiazol-6-yl) nicotinate (200 mg, 0.636 mmol), lithium hydroxide monohydrate(134 mg, 3.181 mmol), tetrahydrofuran (5 ml), methanol (5 ml) and water (5 ml) were added to a 100mL round-bottomed flask. The reaction was carried out overnight at room temperature. After concentrating the solvent, 10 ml of water was added to the flask. Next, the reaction was adjusted to pH=5 by adding 1M diluted hydrochloric acid. The resultant mixture was concentrated. The concentrate was vacuum-dried to give a crude product of 2-amino-5-(2-aminobenzo[d]thiazol-6-yl)nicotinic acid (182 mg, Y: 99%), ES-API:[M+H]⁺= 287.1.

Step 4: 2-amino-5-(2-aminobenzo[d]thiazol-6-yl)nicotinic acid (50 mg, 0.175 mmol), 1-(2-(trifluoromethoxy)phenyl)ethan-1-amine (36 mg, 0.175 mmol), ((benzotriazol-1-yl)oxy)tris(dimethylamino)phosphonium hexafluorophosphate (116 mg, 0.263 mmol), triethylamine (53 mg, 0.252 mmol) and *N,N*-dimethylformamide (5 mL) were added to a 50mL round-bottomed flask. The reaction was carried out at room temperature for 2 hours. 15mL of water was added to the reaction mixture. The resultant mixture was extracted with 30mL of ethyl acetate for 3 times. The organic phases were combined, dried over anhydrous sodium sulfate, and filtered. The crude product obtained by concentrating the organic phase was purified by alkaline HPLC to give 2-amino-5-(2-aminobenzo[d]thiazol-6-yl)-*N*-(1-(2-(trifluoro methoxy)phenyl)ethyl)nicotinamide as an off-white powder (34.05 mg, Y: 42%). ES-API:[M+H]+= 474.0. ¹H NMR (400 MHz, DMSO-d6) δ 8.94 (d, J = 7.2 Hz, 1 H), 8.42 (d, J = 2.4 Hz, 1 H), 8.31 (d, J = 2.0 Hz, 1H), 7.99 (d, J =1.6 Hz, 1 H), 7.65-7.62 (m, 1 H), 7.56-7.52 (m, 3 H), 7.42-7.34 (m, 4 H), 7.05 (s, 2 H), 5.45-5.41 (m, 1 H), 1.48 (d, J = 7.2 Hz, 3 H).

### Example 47: Synthesis of 4-(2-(cyclopropylformamido)-[1,2,4]triazolo[1,5-a]pyridin-7-yl)-N-(1-(2-(trifluoromethoxy)phenyl)ethyl)-1H-indazole-6-carboxamide

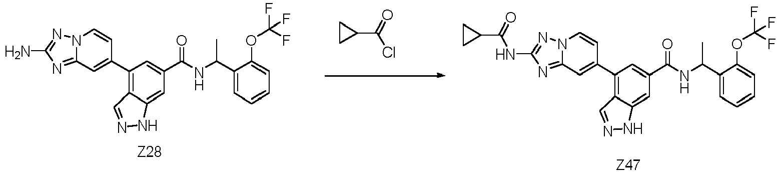

The compound of 4-(2-amino-[1,2,4]triazolo[1,5-*a*]pyridin-7-yl-*N*-(1-(2-(tris(trifluoromethoxy)phenyl)ethyl)-1*H*-indazole-6-carboxamide (18mg, 0.37mmol) and triethylamine (0.1ml) was disolved in dichloromethane (5ml). Cyclopropylformal chloride (4mg, 0.041 mmol) was added dropwise in an ice-water bath. The reaction was carried out in an ice-water bath for 5 minutes. 10ml of water was added to the mixture. The resultant mixture was extracted with 10ml of dichloromethane twice. The organic phases were combined, washed with 15 ml of saturated saline, dried over anhydrous sodium sulfate, and concentrated to remove the solvent. The crude product was purified by alkaline HPLC to give the compound of 4-(2-(cyclopropylformamido)-[1,2,4]triazolo[1,5-a]pyridin-7-yl)-N-(1-(2-(trifluoro methoxy)phenyl)ethyl)-1H-indazole-6-carboxamide (1.2mg, Y: 5.9%). ES-API: [M+H]+=550.7.

### Example 48: Synthesis of 5-(2-aminobenzo[d]thiazol-6-yl)-N-(1-(2-fluoro-6-(trifluoromethoxy)phenyl)ethyl)-2-methoxynicotinamide

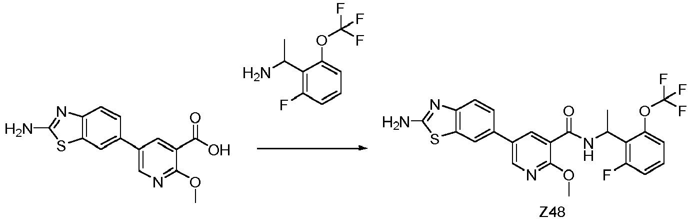

The compound of 5-(2-aminobenzo[d]thiazol-6-yl)-2-methoxynicotinic acid (50 mg, 0.166 mmol) was added to a 50 ml round-bottomed flask. 1-(2-fluoro-6-(trifluoromethoxy)phenyl)ethan-1-amine (37 mg, 0.166 mmol), ((benzotriazol-1-yl)oxy)tris(dimethylamino)phosphonium hexafluorophosphate (105 mg, 0.249 mmol), triethylamine (0.1ml) and *N,N*-dimethylformamide (5 mL) were added sequentially. The reaction was carried out at room temperature for 2 hours. 15mL of water was added to the reaction mixture. The resultant mixture was extracted with 30mL of ethyl acetate for 3 times. The organic phases were combined, dried over anhydrous sodium sulfate, and filtered. The crude product obtained by concentrating the organic phase was purified by alkaline HPLC to give the compound of 5-(2-aminobenzo[d]thiazol-6-yl)-*N*-(1-(2-fluoro-6-(trifluoromethoxy)phenyl)ethyl)-2-methoxynicotinamide (11mg, Y:13%). ES-API:[M+H]⁺=507.0. ¹H NMR (400 MHz, DMSO), δ 8.83 (d, J = 7.8 Hz, 1H), 8.56 (d, J = 2.6 Hz, 1H), 8.13 (d, J = 2.6 Hz, 1H), 7.99 (d, J = 2.0 Hz, 1H), 7.58-7.47 (m, 4H), 7.35 (dd, J = 12.0, 8.6 Hz, 3H), 5.38-5.25 (m, 1H), 3.97 (s, 3H), 1.43 (d, J = 8.0 Hz, 3H).

### Example 49: Synthesis of 5-(2-aminobenzo[d]thiazol-6-yl)-N-(3,5-difluoro-2-isobutoxybenzyl)-2-methoxynicotinamide

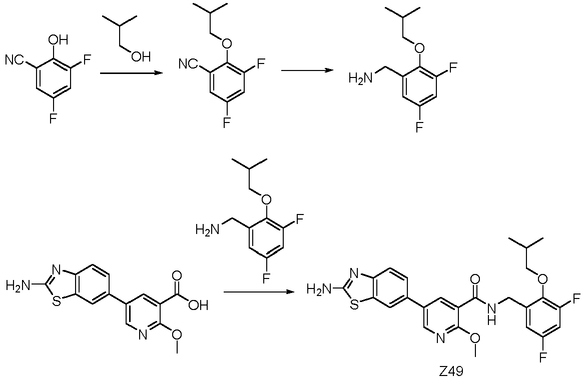

Step 1: A compound of 3,5-difluoro-2-hydroxybenzonitrile (100mg, 0.645mmol) was added to a 50ml round-bottomed flask. Next, triphenylphosphine (57mg, 0.774mmol), diisopropyl azodicarboxylate (253mg, 0.97mmol) and tetrahydrofuran (10ml) were added sequentially. Thereafter, the reaction was carried out overnight at room temperature under the protection of nitrogen gas. 15 mL water was added to the mixture. The resultant mixture was extracted with 30 mL of ethyl acetate twice. The organic phases were combined, washed with 15 mL of saturated brine, dried over anhydrous sodium sulfate, and concentrated under reduced pressure to remove the solvent. The residue was purified on silica gel by column chromatography (petroleum ether:ethyl acetate=100:1-20:1) to give the product of 3,5-difluoro-2-isobutoxybenzonitrile (101mg, Y:74%). ES-API: [M+H]⁺=212.0.

Step 2: The compound of 3,5-difluoro-2-isobutoxybenzonitrile (101mg, 0.48mmol) was dissolved in tetrahydrofuran (5ml). Next, a solution of borane in tetrahydrofuran (2.4ml, 2.4mmol) was added under the protection of nitrogen gas. The reaction was carried out overnight at 80°C. After the reacton system was cooled to room temperature, 0.5ml of methanol was added in an ice-water bath to quench the reaction. Thereafter, 15 mL of water was added to the mixture. The resultant mixture was extracted with 30 mL of ethyl acetate twice. The organic phases were combined, washed with 30ml of saturated brine, dried over anhydrous sodium sulfate, and concentrated under reduced pressure to remove the solvent. The residue was purified on silica gel by column chromatography (dichloromethane:methanol = 100:1-20:1) to give the product of (3,5-difluoro-2-isobutoxyphenyl)methylamine (50 mg, Y: 48%). ES-API: [M+H]⁺=216.2.

Step 3: The compound of 5-(2-aminobenzo[d]thiazol-6-yl)-2-methoxynicotinic acid (47mg, 0.155mmol) was added to a 50ml round-bottomed flask. (3,5-difluoro-2-isobutoxyphenyl)methylamine (50mg, 0.232mmol), ((benzo-triazol-1-yl)oxy) tris(dimethylamino)phosphonium hexafluorophosphate (98mg, 0.232mmol), triethylamine (0.1 ml) and *N,N*-dimethylformamide (5ml) were added sequentially. The reaction was carried out at room temperature for 2 hours. 15 mL of water was added. The resultant mixture was extracted with 30 mL of ethyl acetate for 3 times. The organic phases were combined, dried over anhydrous sodium sulfate, and filtered. The crude product obtained by concentrating the organic phase was purified by alkaline HPLC to give the compound of 5-(2-aminobenzo[d]thiazol-6-yl)-*N*-(3,5-difluoro-2-isobutoxybenzyl)-2-methoxynicotinamide (22 mg, Y: 29%). ES-API: [M+H]⁺=499.1. ¹H NMR (400 MHz, DMSO), δ 8.88 (s, 1H), 8.62 (d, *J* = 2.6 Hz, 1H), 8.35 (d, *J* = 2.6 Hz, 1H), 8.05 (s, 1H), 7.63 (s, 1H), 7.55 (d, *J=* 8.2 Hz, 1H), 7.42 (s, 1H), 7.24 (s, 1H), 7.00 (d, *J=* 9.6 Hz, 1H), 4.57 (d, *J=* 6.0Hz, 2H), 4.03 (s, 3H), 3.82 (d, *J* = 6.0 Hz, 2H), 2.17-1.94 (m, 1H), 1.03 (d, *J* = 6.8 Hz, 6H).

### Example 50: Synthesis of 5-(2-aminobenzo[d]thiazol-6-yl)-N-(2-isopropoxybenzyl)-2-methoxynicotinamide

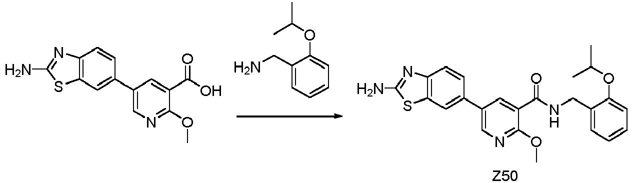

The compound of 5-(2-aminobenzo[d]thiazol-6-yl)-2-methoxynicotinic acid (50mg, 0.166mmol) was added to a 50ml round-bottomed flask. (2-isopropoxyphenyl)methylamine (27mg, 0.166 mmol), ((benzo-triazol-1-yl)oxy) tris(dimethylamino)phosphonium hexafluorophosphate (105 mg, 0.249mmol), triethylamine (0.1 ml) and *N,N*-dimethylformamide (5ml) were added sequentially. The reaction was carried out at room temperature for 2 hours. 15 mL of water was added. The resultant mixture was extracted with 30 mL of ethyl acetate for 3 times. The organic phases were combined, dried over anhydrous sodium sulfate, and filtered. The crude product obtained by concentrating the organic phase was purified by alkaline HPLC to give the compound of 5-(2-aminobenzo[d]thiazol-6-yl)-*N*-(2-isopropoxybenzyl)-2-methoxynicotinamide (10mg, Y: 13%). ES-API: [M+H]⁺=499.2. ¹H NMR (400 MHz, DMSO) , δ 8.57 (d, J = 2.4 Hz, 2H), 8.35 (d, J = 2.6 Hz, 1H), 7.99 (s, 1H), 7.55 (s, 2H), 7.51-7.44 (m, 1H), 7.38 (s, 1H), 7.20 (dd, J = 17.8, 7.6 Hz, 2H), 6.98 (d, J = 8.0 Hz, 1H), 6.86 (t, J = 7.4 Hz, 1H), 4.64 (dt, J = 11.8, 5.8 Hz, 1H), 4.44 (d, J = 6.0 Hz, 2H), 4.00 (s, 3H), 1.28 (d, J = 6.0 Hz, 6H).

### Example 51: Synthesis of 5-(2-aminobenzo[d]thiazol-6-yl)-N-(2-(cyclopentylmethoxy)-6-fluorobenzyl)-2-methoxynicotinamide

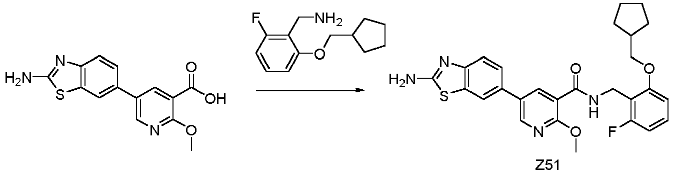

The compound of 5-(2-aminobenzo[d]thiazol-6-yl)-2-methoxynicotinic acid (50mg, 0.166mmol) was added to a 50ml round-bottomed flask. (2-(cyclopentylmethoxy)-6-fluorophenyl)methylamine (37mg, 0.166mmol), ((benzotriazol-1-yl)oxy)tris(dimethylamino)phosphonium hexafluorophosphate (105 mg, 0.249mmol), triethylamine (0.1 ml) and *N,N*-dimethylformamide (5ml) were added sequentially. The reaction was carried out at room temperature for 2 hours. 15 mL of water was added. The resultant mixture was extracted with 30 mL of ethyl acetate for 3 times. The organic phases were combined, dried over anhydrous sodium sulfate, and filtered. The crude product obtained by concentrating the organic phase was purified by alkaline HPLC to give the compound of 5-(2-aminobenzo[d]thiazol-6-yl)-*N*-(2-(cyclopentylmethoxy-6-fluorobenzyl)-2-methoxynicotinamide. (9mg, Y:11%). ES-API: [M+H]⁺=507.4. ¹H NMR (400 MHz, DMSO) , δ 8.55 (d, *J* = 2.5 Hz, 1H), 8.32 (d, *J* = 2.5 Hz, 2H), 7.97 (s, 1H), 7.54 (s, 2H), 7.48 (d, *J* = 8.4 Hz, 1H), 7.37 (s, 1H), 7.26 (dd, *J* = 15.6, 8.0Hz, 1H), 6.86 (d, *J* = 8.2 Hz, 1H), 6.77 (t, *J* = 8.8 Hz, 1H), 4.54 (d, *J* = 5.2 Hz, 2H), 3.95 (s, 3H), 3.92 (d, *J=* 7.0 Hz, 2H), 2.30 (d, *J* = 4.8 Hz, 1H), 1.77 (d, *J* = 7.4 Hz, 2H), 1.63-1.44 (m, 5H), 1.34 (dd, *J* = 12.0, 8.0 Hz, 2H).

### Example 52: Synthesis of 5-(2-aminobenzo[d]thiazol-6-yl)-N-((2-(cyclopentyloxy)pyridin-3-yl)methyl)-2-methoxynicotinamide

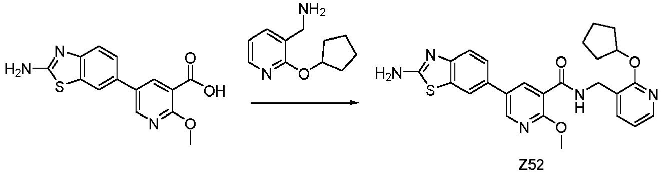

The compound of 5-(2-aminobenzo[d]thiazol-6-yl)-2-methoxynicotinic acid (50mg, 0.166mmol) was added to a 50ml round-bottomed flask. (2-(cyclopentyloxy)pyridin-3-yl)methylamine (32mg, 0.166mmol), ((benzotriazol-1-yl)oxy)tris(dimethylamino)phosphonium hexafluorophosphate (105mg, 0.249mmol), triethylamine (0.1ml), and *N,N*-dimethylformamide (5ml) were added sequentially. The reaction was carried out at room temperature for 2 hours. 15 mL of water was added. The resultant mixture was extracted with 30 mL of ethyl acetate for 3 times. The organic phases were combined, dried over anhydrous sodium sulfate, and filtered. The crude product obtained by concentrating the organic phase was purified by alkaline HPLC to give 5-(2-aminobenzo[d]thiazol-6-yl)-*N*-((2-(cyclopentyloxy)pyridin-3-yl)methyl)-2-methoxynicotinamide (18 mg, Y: 23%). ES-API:[M+H]⁺=476.4. ¹H NMR (400 MHz, DMSO), δ 8.68 (s, 1H), 8.58 (d, *J =* 2.6 Hz, 1H), 8.33 (d, *J=* 2.6 Hz, 1H), 8.04-7.97 (m, 2H), 7.55 (d, *J=* 7.8 Hz, 3H), 7.50 (dd, *J* = 8.4, 2.0 Hz, 1H), 7.37 (d, *J* = 8.4 Hz, 1H), 6.90 (dd, *J* = 7.2, 5.0 Hz, 1H), 5.43 (s, 1H), 4.38 (d, *J=* 6.0Hz, 2H), 4.00 (s, 3H), 2.04 (s, 1H), 1.90 (d, *J=* 4.0 Hz, 2H), 1.79-1.65 (m, 4H), 1.57 (s, 2H).

### Example 53: Synthesis of 5-(2-aminobenzo[d]thiazol-6-yl)-2-methoxy-N-(2-((tetrahydrofuran-3-yl)oxy)benzyl)nicotinamide

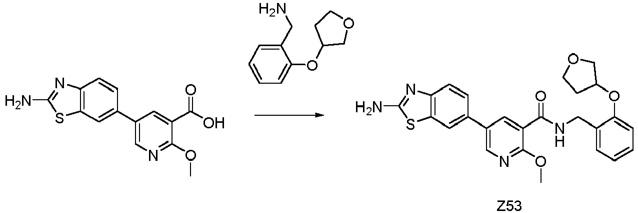

The compound of 5-(2-aminobenzo[d]thiazol-6-yl)-2-methoxynicotinic acid (50mg, 0.166mmol) was added to a 50ml round-bottomed flask. (2-((tetrahydrofuran-3-yl)oxy)phenyl)methylamine (37mg, 0.166mmol), ((benzotriazol-1-yl)oxy)tris(dimethylamino)phosphonium hexafluorophosphate (105mg, 0.249mmol), triethylamine (0.1ml), and *N,N*-dimethylformamide (5ml) were added sequentially. The reaction was carried out at room temperature for 2 hours. 15 mL of water was added. The resultant mixture was extracted with 30 mL of ethyl acetate for 3 times. The organic phases were combined, dried over anhydrous sodium sulfate, and filtered. The crude product obtained by concentrating the organic phase was purified by alkaline HPLC to give the compound of 5-(2-aminobenzo[d]thiazol-6-yl)-2-methoxy-*N*-(2-((tetrahydrofuran-3-yl)oxy)benzyl)nicotinamide (11mg, Y:14%). ES-API:[M+H]⁺=477.6. ¹H NMR (400 MHz, DMSO), δ 8.57 (d, *J* = 2.6 Hz, 2H), 8.34 (d, *J* = 2.6 Hz, 1H), 8.03 (s, 1H), 7.82 (s, 2H), 7.53 (d, *J*= 8.4 Hz, 1H), 7.39 (d, *J=* 8.4 Hz, 1H), 7.22 (dd, *J* = 18.0, 7.6 Hz, 2H), 6.96 *(d, J =* 8.0 Hz, 1H), 6.90 (t, *J* = 7.0 Hz, 1H), 5.07 (s, 1H), 4.44 (d, *J=* 6.0 Hz, 2H), 3.92 (dd, *J=* 10.0, 4.6 Hz, 1H), 3.84 (dd, *J* = 15.0, 8.0 Hz, 2H), 3.76 (td, *J* = 8.0, 4.0 Hz, 2H), 2.20 (dt, *J* = 14.0, 7.0 Hz, 1H), 2.07-1.91 (m, 1H), 1.20 (s, 2H).

### Example 54: Preparation of 3-(2-aminobenzo[d]thiazol-6-yl)-6-(1-(2-(trifluoromethoxy)phenyl)ethyl)-7,8-dihydro-1,6-naphthyridin-5(6H)-one (Z54)

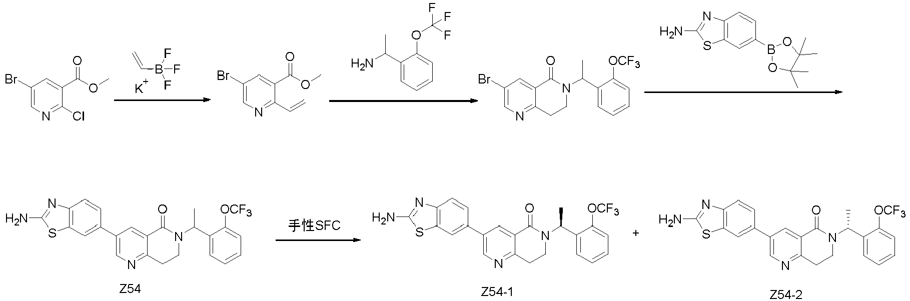

Step 1: Methyl 5-bromo-2-chloronicotinate (10 g, 39.923 mmol), potassium vinyltrifluoroborate (5.348 g, 39.923 mmol), triethylamine (5.56 mL, 39.923 mmol), and [1,1-bis(diphenylphosphino)ferrocene]dichloropalladium (585 mg, 0.798 mmol) was dissolved in 200 mL of EtOH under the protection of nitrogen gas. The mixture was purged with nigrogen gas for three times. The reaction was carried out at 80°C for 1 hour. Then, the reaction mixture was cooled to room temperature, and was filtered. The filtrate was spin-dried. The residue was dissloved in 300 mL of ethyl acetate and 300 mL of water, then separated, and the organic phase was concentrated. The resultant residue was purified on silicon gel by automatic flash chromatography (ethyl acetate/petroleum ether=0-20%) to give the compound of methyl 5-bromo-2-vinylnicotinate (5.186 g, Y: 54 %). ES-API:[M+H]⁺= 242.1.

Step 2: Methyl 5-bromo-2-vinylnicotinate(780 mg, 3.222 mmol) and 1-(2-(trifluoromethoxy)phenyl)ethan-1-amine (1.653 g, 8.055 mmol) were mixed in DMA (10 mL) in a microwave bottle. The reaction was irradiated with microwave at 150°C for 8 hours. The reaction was cooled to room temperature. 50 mL of ethyl acetate was added to the reaction solution. The resulatant mixture was washed with water (25 mL^{∗}3) and saturated saline (25 mL^{∗}3). The organic layers were combined and concentrated. The concentrate was purified on silicon gel by automatic flash chromatography to give the compound of 3-bromo-6-(1-(2-(trifluoromethoxy) phenyl)ethyl)-7,8-dihydro-1,6-naphthyridin-5(6*H*)-one (769 mg, Y: 57 %). ES-API: [M+H]⁺= 415.0.

Step 3: 3-bromo-6-(1-(2-(trifluoromethoxy)phenyl)ethyl)-7,8-dihydro-1,6-naphthyridin-5(6*H*)-one (50 mg, 0.120 mmol), 6-(4,4,5,5-tetramethyl-1,3,2-dioxaborolan-2-yl)benzo[d]thiazole-2-amine (67 mg, 0.240 mmol), sodium carbonate (38 mg, 0.360 mmol), and [1,1-bis(diphenylphosphino)ferrocene]dichloropalladium (5 mg, 0.006 mmol) was dissolved in 5 mL of dioxane and 1 mL of H₂O under the protection of nitrogen gas. The mixture was purged with nigrogen gas for three times. The reaction was carried out at 95°C for 3 hours. The reaction was cooled to room temperature. 20 mL of EtOAc was added to the reaction solution. The resultant mixture was washed with water (15 mL^{∗}3) and saturated saline (15 mL^{∗}3). The organic layers were combined and concentrated. The residue was purified by alkaline HPLC to give a white compound of 3-(2-aminobenzo[d]thiazol-6-yl)-6-(1-(2-(trifluoromethoxy)phenyl)ethyl)-7,8-dihydro-1,6-naphthyridinyl-5(6*H*)-one (25.67 mg, Y: 44 %). ES-API:[M+H]⁺= 485.0. ¹H NMR (400 MHz, DMSO-d6) δ 8.85 (d, J = 1.6 Hz, 1 H), 8.33 (d, J = 2.4 Hz, 1 H), 8.05 (d, J = 1.2 Hz, 1 H), 7.64 (dd, J1 = 1.6 Hz, J2 = 6.0 Hz, 1 H), 7.55-7.51 (m, 3 H), 7.45-7.30 (m, 4 H), 6.12-6.08 (m, 1 H), 3.53-3.47 (m, 1 H), 3.08-2.96 (m, 2 H), 2.87-2.81 (m, 1 H), 1.50 (d, J = 5.6 Hz, 3 H).

Step 4: The compound of 3-(2-aminobenzo[d]thiazol-6-yl)-6-(1-(2-(trifluoromethoxy)phenyl)ethyl)-7,8-dihydro-1,6-naphthyridinyl-5(6*H*)-one was separated by SFC chiral resolution (Co-solvent: methanol (ammonia containing 0.2% methanol); column type: AD-H (4.6^{∗}100mm ^{∗}5um)) to give the compound Z54-1 (peak 1, retention time: 1.25 min): (S)-3-(2-aminobenzo[d]thiazol-6-yl)-6-(1-(2-(trifluoromethoxy)phenyl)ethyl)-7,8-dihydro-1,6-naphthyridin-5(6*H*)-one (an arbitrarily assigned absolute configuration, 10.62 mg, Y: 41%), ES-API: [M+H]⁺= 485.1, and the compound Z54-2 (peak 2, retention time: 1.67 min): (R)-3-(2-aminobenzo[d]thiazol-6-yl)-6-(1-(2-(trifluoromethoxy)phenyl)ethyl)-7 ,8-dihydro-1,6-naphthyridin-5(6*H*)-one (an arbitrarily assigned absolute configuration, 9.50 mg, Y: 37 %), ES-API: [M+H]⁺= 485.1.

### Example 55: Preparation of 3-(2-aminobenzo[d]thiazol-6-yl)-6-(2-fluoro-5-(trifluoromethoxy)benzyl)-7,8-dihydro-1,6-naphthyridin-5(6H)-one (Z55)

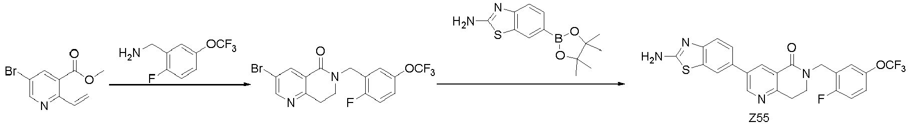

Step 1: Methyl 5-bromo-2-vinyl nicotinate (2.5 g, 10.328 mmol) and (2-fluoro-5-(trifluoromethoxy)phenyl)methylamine (4.32 g, 20.656 mmol) were mixed in DMA (75 mL) in a microwave bottle. The reaction was irradiated with microwave at 150°C for 3 hours. The reaction was cooled to room temperature. 100 mL of ethyl acetate was added to the reaction solution. The resulatant mixture was washed with water (30 mL^{∗}3) and saturated saline (30 mL^{∗}3). The organic layers were combined and concentrated. The concentrate was purified on silicon gel by automatic flash chromatography (ethyl acetate/petroleum ether=0-20%) to give the compound of 3-bromo-6-(2-fluoro-5-(trifluoromethoxy)benzyl)-7,8-dihydro-1,6-naphthyridin-5(6*H*)-one (2.356 g, Y: 62 %). ES-API: [M+H]⁺= 419.1.

Step 2: 3-bromo-6-(2-fluoro-5-(trifluoromethoxy)benzyl)-7,8-dihydro-1,6-naphthyridin-5(6*H*)-one (80 mg, 0.191 mmol), 6-(4,4,5,5-tetramethyl-1,3,2-dioxa borolan-2-yl)benzo[d]thiazole-2-amine (105 mg, 0.382 mmol), sodium carbonate (51 mg, 0.478 mmol), and [1,1-bis(diphenylphosphino)ferrocene]dichloropalladium-dichloromethane complex (8 mg, 0.0096 mmol) was dissolved in 5 mL of dioxane and 1 mL of H₂O under the protection of nitrogen gas. The mixture was purged with nigrogen gas for three times. The reaction was carried out at 95°C for 1.5 hours. The reaction was cooled to room temperature. 30 mL of EtOAc was added to the reaction solution. The resultant mixture was washed with water (15 mL^{∗}3) and saturated saline (15 mL^{∗}3). The organic layers were combined and concentrated. The residue was purified by alkaline HPLC to give 3-(2-aminobenzo[d]thiazol-6-yl)-6-(2-fluoro-5-(trifluoromethoxy)benzyl)-7,8-dihydro-1,6-naphthyridin-5(6H)-one as a white solid (20.1 mg, Y: 22 %). ES-API: [M+H]⁺= 489.0. ¹H NMR (400 MHz, DMSO-d6) δ 8.95 (d, J = 2.0 Hz, 1 H), 8.40 (d, J = 2.0 Hz, 1 H), 8.12 (d, J = 1.2 Hz, 1 H), 7.61-7.59 (m, 3 H), 7.44-7.42 (m, 2 H), 7.40-7.39 (m, 2 H), 4.81 (s, 2 H), 3.71 (t, J1 = 5.6 Hz, J2 = 10.8 Hz, 2 H), 3.17 (t, J1 = 5.6 Hz, J2 = 10.8 Hz, 2 H).

### Example 56: Synthesis of 5-(2-(cyclopropylformamido)benzo[d]thiazol-6-yl)-2-methoxy-N-(I-(2-(trifluoromethoxy)phenyl)ethyl)nicotinamide

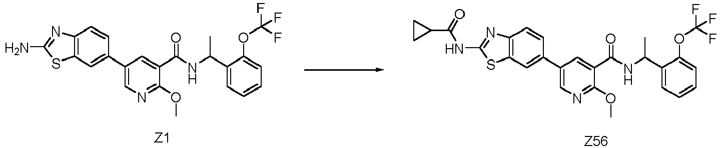

5-(2-Aminobenzothiazol-6-yl)-2-methoxy-*N*-(1-(2-(trifluoromethoxy)phenyl) ethyl)nicotinamide (50mg, 0.1 mmol), triethylamine (30mg, 0.3mmol) and dichloromethane (5ml) was added to a 50mL round-bottomed flask. The reaction was carried out in an ice-water bath for 5 minutes. Thereafter, a solution of cyclopropylformal chloride (16mg, 0.15mmol) in dichloromethane (1ml) was added. The reaction was carried out in the ice-water bath for another 10 minutes. 10ml of water was added to the mixture. The resultant mixture was extracted with 20ml of dichloromethane for three times. The organic phases were combined, and dried over anhydrous sodium sulfate. The crude product obtained by concentrating the organic phase was purified by alkaline HPLC to give the compound of 5-(2-(cyclopropylformamido)benzo[d]thiazol-6-yl)-2-methoxy-N-(1-(2-(trifluoromethoxy) phenyl)ethyl)nicotinamide (8mg, Y: 14%). ES-API: [M+H]⁺=557.2.

### Example 57: Synthesis of 4-(2-amino-[1,2,4]triazolo[1,5-a]pyridin-7-yl)-N-((2-(cyclopentyloxy)pyridin-3-yl)methyl)-1-methyl-1H-indazole-6-carboxamide

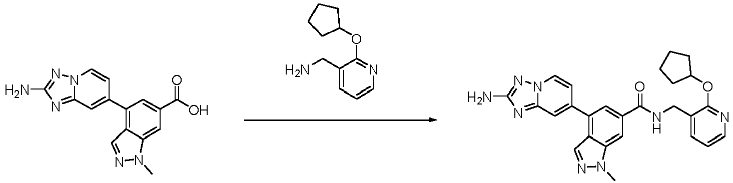

4-(2-Amino-[1,2,4]triazolo[1,5-*a*]pyridin-7-yl)-1-methyl-1*H*-indazole-6-carboxylic acid (46 mg, 0.149 mmol), (2-(cyclopentyloxy)pyridin-3-yl)methylamine (51 mg, 0.224 mmol), ((benzotriazol-1-yl)oxy)tris(dimethylamino)phosphonium hexafluoro-phosphate (99 mg, 0.224 mmol), triethylamine (45 mg, 0.448 mmol), and *N,N*-dimethylformamide (5 ml) were added to a 50 mL round-bottomed flask. The reaction was carried out at room temperature for 2 hours. 15 mL of water was added. The resultant mixture was extracted with 30 mL of ethyl acetate for 3 times. The organic phases were combined, dried over anhydrous sodium sulfate, and filtered. The crude product obtained by concentrating the organic phase was purified by alkaline HPLC to give 4-(2-amino-[1,2,4]triazolo[1,5-a]pyridin-7-yl)-N-((2-(cyclopentyloxy)pyridin-3-yl)methyl)-1-methyl-1H-indazole-6-carboxamide as an off-white powder (4.0 mg, Y: 6 %). ES-API:[M+H]⁺= 483.2.

### Example 58: Synthesis of 4-(2-amino-[1,2,4]triazolo[1,5-a]pyridin-7-yl)-N-((2-(cyclopropylmethoxy)pyridin-3-yl)methyl)-1-methyl-1H-indazole-6-carboxamide

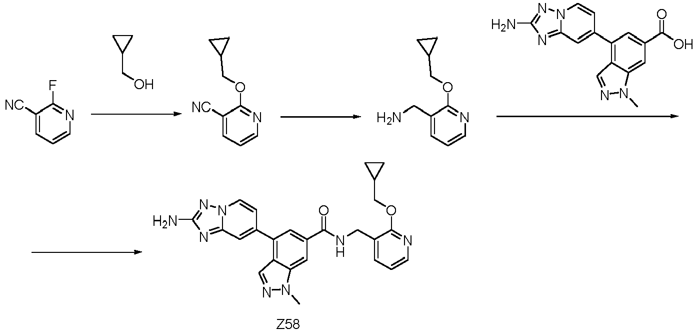

Step 1: 50mL of tetrahydrofuran and cyclopropyl methanol (1.08g, 15.0mmol) were added to a 100mL three-necked round-bottomed flask at 0°C under the protection of nitrogen gas. Next, sodium hydride (600mg, 15.0mmol) was added. Finally, 2-fluoronicotinonitrile (1.22g, 10.0mmol) was added. The mixture was slowly warmed up to 55°C and reacted for 12 hours. After the reaction was completed, the mixture was cooled to 0°C. 80 mL of ethyl acetate was added to the system. The resultant mixture was sequentially washed with saturated ammonium chloride and sodium chloride twice (60 mL×2). The phase of ethyl acetate was dried over anhydrous sodium sulfate and filtered. The filtrate was spin-dried to give 2-(cyclopropylmethoxy) nicotinonitrile (1.58 g, Y: 91%). ES-API: [M+H]⁺=175.1.

Step 2: 50mL of tetrahydrofuran and 2-(cyclopropylmethoxy) nicotinonitrile (0.50g, 2.87mmol) were added to a 500mL single-necked round-bottomed flask under the the protection of nitrogen gas. Finally, borane-tetrahydrofuran complex (14mL, 1M, 14.0mmol) was added. The mixture was warmed up slowly from room temperature to be boiling, and the reaction was carried out overnight. After the reaction was completed, the resultant mixture was cooled to room temperature. Methanol was carefully added dropwise until no more bubbles were generated. 80mL of ethyl acetate was added to the system. The resultant mixture was sequentially washed with saturated ammonium chloride and sodium chloride twice (60mL×2). The phase of ethyl acetate was dried over anhydrous sodium sulfate and filtered. The filtrate was spin-dried to give (2-(cyclopropylmethoxy)pyridin-3-yl)methylamine (0.20 g, Y: 40%). ES-API: [M+H]⁺=179.1.

Step 3: 3.0mL of N,N-dimethylformamide and triethylamine (164mg, 1.628mmol) were added to a 50mL round-bottomed flask. Next, 4-(2-amino-[1,2,4]triazolo[1,5-*a*]pyridin-7-yl)-1-methyl-1*H*-indazole-6-carboxylic acid (50mg, 0.1623mmol) and (2-(cyclopropylmethoxy)pyridin-3-yl) methylamine (35mg, 0.1948mmol) were added. Finally, BOP reagent (87mg, 0.1948mmol) was added. The reaction was carried out at room temperature under the protection of nitrogen gas for 2 hours. After the reaction was completed, 50mL of ethyl acetate was added to the system. The resultant mixture was sequentially washed with saturated ammonium chloride and sodium chloride for 3 times (40mL×3). The phase of ethyl acetate was dried over anhydrous sodium sulfate and filtered. The filtrate was spin-dried, and then was purified by pre-HPLC to give the target compound of 4-(2-amino-[1,2,4]triazolo[1,5-a]pyridin-7-yl)-N-((2-(cyclopropylmethoxy)pyridin-3-yl) methyl)-1-methyl-1H-indazole-6-carboxamide (11.0mg, Y:21%). ES-API:[M+H]⁺= 469.1. ¹H NMR (400 MHz, DMSO-d6) δ 9.14 (t, *J* = 5.4 Hz, 1H), 8.60 (d, *J* = 6.9 Hz, 1H), 8.23 (d, *J* = 14.7 Hz, 2H), 7.98 (d, *J* = 3.7 Hz, 1H), 7.85 (s, 1H), 7.68 (s, 1H), 7.56 (d, *J* = 6.7 Hz, 1H), 7.34 - 7.24 (m, 1H), 6.91 (dd, *J* = 7.1, 5.2 Hz, 1H), 6.03 (s, 2H), 4.45 (d*, J* = 5.4 Hz, 2H), 4.15 - 4.09 (m, 5H), 1.21 (dd, *J* = 13.3, 6.0 Hz, 1H), 0.46 (q, *J* = 5.5 Hz, 2H), 0.27 (t, *J* = 4.7 Hz, 2H).

### Example 59: Synthesis of 4-(2-amino-[1,2,4]triazolo[1,5-a]pyridin-7-yl)-N-((2-(cyclopropylmethoxy)-5-fluorobenzyl)-1-methyl-1H-indazole-6-carboxamide

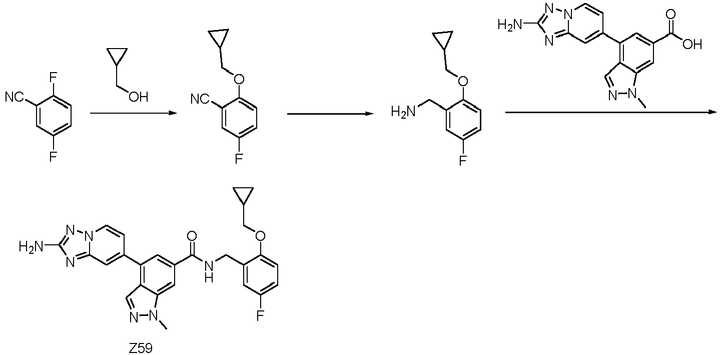

Step 1: 50mL of tetrahydrofuran and cyclopropyl methanol (1.08g, 15.0mmol) were added to a 100mL three-necked round-bottomed flask at 0°C under the protection of nitrogen gas. Next, sodium hydride (600mg, 15.0mmol) was added. Finally, 2,5-difluorobenzonitrile (1.39g, 10.0mmol) was added. The mixture was slowly warmed up to 55°C and reacted for 12 hours. After the reaction was completed, the mixture was cooled to 0°C. 80 mL of ethyl acetate was added to the system. The resultant mixture was sequentially washed with saturated ammonium chloride and sodium chloride twice (60 mL×2). The phase of ethyl acetate was dried over anhydrous sodium sulfate and filtered. The filtrate was spin-dried to give 2-(cyclopropylmethoxy)-5-fluorobenzonitrile (1.757g, Y: 92%). ES-API: [M+H]⁺=192.1.

Step 2: 50mL of tetrahydrofuran and 2-(cyclopropylmethoxy)-5-fluorobenzonitrile ((1.30g, 6.80mmol) were added to a 500mL single-necked round-bottomed flask under the protection of nitrogen gas. Finally, borane-tetrahydrofuran complex (34mL, 1M, 34.0mmol) was added. The mixture was warmed up slowly from room temperature to be boiling, and the reaction was carried out overnight. After the reaction was completed, the resultant mixture was cooled to room temperature. Methanol was carefully added dropwise until no more bubbles were generated. 80mL of ethyl acetate was added to the system. The resultant mixture was sequentially washed with saturated ammonium chloride and sodium chloride twice (60mL×2). The phase of ethyl acetate was dried over anhydrous sodium sulfate and filtered. The filtrate was spin-dried to give (2-(cyclopropylmethoxy)-5-fluorophenyl)methylamine (0.50 g, Y: 37%). ES-API: [M+H]⁺=196.0.

Step 3: 10mL of N,N-dimethylformamide and triethylamine (164mg, 1.628mmol) were added to a 50mL round-bottomed flask. Next, 4-(2-amino-[1,2,4]triazolo[1,5-*a*]pyridin-7-yl)-1-methyl-1*H*-indazole-6-carboxylic acid (50mg, 0.1623mmol) and (2-(cyclopropylmethoxy)-5-fluorophenyl)methylamine (40mg, 0.1948mmol) were added. Finally, BOP reagent (87mg, 0.1948mmol) was added. The reaction was carried out at room temperature under the protection of nitrogen gas for 2 hours. After the reaction was completed, 50mL of ethyl acetate was added to the system. The resultant mixture was sequentially washed with saturated ammonium chloride and sodium chloride for 3 times (40mL×3). The phase of ethyl acetate was dried over anhydrous sodium sulfate and filtered. The filtrate was spin-dried, and then was purified by pre-HPLC to give the compound of 4-(2-amino-[1,2,4]triazolo[1,5-a]pyridin-7-yl)-N-(2-(cyclopropylmethoxy)-5-fluorobenzyl)-1-methyl-1H-indazole-6-carboxamide (13.0mg, Y:22%). ES-API:[M+H]⁺= 486.1. ¹H NMR (400 MHz, DMSO-d6) δ 9.10 (d, *J* = 5.5 Hz, 1H), 8.64 (d, *J* = 6.9 Hz, 1H), 8.27 (s, 2H), 7.91 (s, 1H), 7.71 (s, 1H), 7.28 (d, *J* = 6.8 Hz, 1H), 6.99 (dd, *J* = 13.8, 6.9 Hz, 3H), 6.06 (s, 2H), 4.52 (d, *J* = 5.4 Hz, 2H), 4.14 (s, 3H), 3.86 (d, *J* = 6.9 Hz, 2H), 1.22 (s, 1H), 0.53 (d, *J* = 7.0 Hz, 2H), 0.32 (d, *J* = 5.0 Hz, 2H).

### Example 60: Synthesis of 4-(2-amino-[1,2,4]triazolo[1,5-a]pyridin-7-yl)-N-(1-(1-(4-fluorophenyl)ethyl)-1H-pyrazol-4-yl)-l-methyl-1H-indazole-6-carboxamide

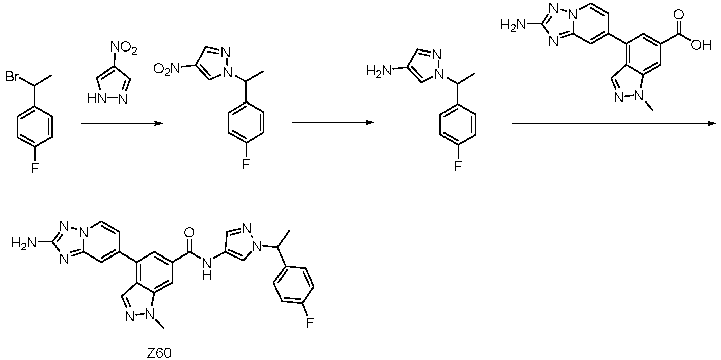

Step 1: 20mL of *N,N*-dimethylformamide and 4-nitro-1*H*-pyrazole (500mg, 4.425mmol) were added to a 50mL three-necked round-bottomed flask at room temperature under the protection of nitrogen gas. Next, potassium carbonate (733mg, 5.311mmol) was added. Finally, 1-(1-bromoethyl)-4-fluorobenzene (0.9g, 4.433mmol) was added. The reaction was carried out at room temperature for 2 hours. After the reaction was completed, 80 mL of ethyl acetate was added to the system. The resultant mixture was sequentially washed with saturated ammonium chloride and sodium chloride twice (60 mL×2). The phase of ethyl acetate was dried over anhydrous sodium sulfate and filtered. The filtrate was spin-dried, and then purified on solica gel by automatic flash chromatography (ethyl acetate/petroleum ether=0-70%) to give the target compound of 1-(1-(4-fluorophenyl)ethyl)-4-nitro-1*H-*pyrazole (750 mg, Y: 72%). ES-API: [M+H]⁺=236.1.

Step 2: 50mL of methanol, 1-(1-(4-fluorophenyl)ethyl)-4-nitro-1*H*-pyrazole (750mg, 3.19mmol), and finally 0.5g of palladium on carbon were added to a 100mL single-necked round-bottomed flask. The reaction was carried out at room temperature under the protection of hydrogen gas for 12 hours. After the reaction was completed, the resultant mixture was filtered. The filtrate was spin-dried to give the target compound of 1-(1-(4-fluorophenyl)ethyl)-1*H*-pyrazole-4-amine (0.61g, Y: 93%). ES-API: [M+H]+=206.1.

Step 3: 3mL of N,N-dimethylformamide and triethylamine (164mg, 1.628mmol) were added to a 50mL single-necked round-bottomed flask. Next, 4-(2-amino-[1,2,4]triazolo[1,5-*a*]pyridin-7-yl)-1-methyl-1*H*-indazole-6-carboxylic acid (50mg, 0.1628mmol) and 1-(1-(4-fluorophenyl)ethyl)-1*H*-pyrazol-4-amine (50mg, 0.2440mmol) were added. Finally, BOP reagent (107mg, 0.2440mmol) was added. The reaction was carried out at room temperature under the protection of nitrogen gas for 2 hours. After the reaction was completed, 80mL of ethyl acetate was added to the system. The resultant mixture was sequentially washed with saturated ammonium chloride and sodium chloride for 5 times (60mL×5). The phase of ethyl acetate was dried over anhydrous sodium sulfate and filtered. The filtrate was spin-dried, and then was purified by pre-HPLC to give the target compound of 4-(2-amino-[1,2,4]triazolo[1,5-*a*]pyridin-7-yl)-*N*-(1-(1-(4-fluorophenyl)ethyl)-1*H*-pyrazol-4-yl)-1-methyl-1*H*-indazole-6-carboxamide (20.7mg, Y: 25.6%). ES-API: [M+H]⁺= 496.41. ¹H NMR (400 MHz, DMSO-d6) δ 10.57 (s, 1H), 8.63 (d, *J* = 6.9 Hz, 1H), 8.24 (d, *J* = 4.1 Hz, 2H), 8.10 (s, 1H), 7.86 (d, *J* = 1.1 Hz, 1H), 7.74 - 7.61 (m, 2H), 7.39 - 7.20 (m, 3H), 7.21 - 7.04 (m, 2H), 6.06 (s, 2H), 5.60 (q, *J* = 7.0 Hz, 1H), 4.12 (s, 3H), 1.75 (d, *J* = 7.1 Hz, 3H).

### Example 61: Synthesis of 4-(2-amino-[1,2,4]triazolo[1,5-a]pyridin-7-yl)-N-(5-fluoro-2-((tetrahydro-2H-pyran-4-yl)oxy)benzyl)-1-methyl-1H-indazole-6-carboxamide

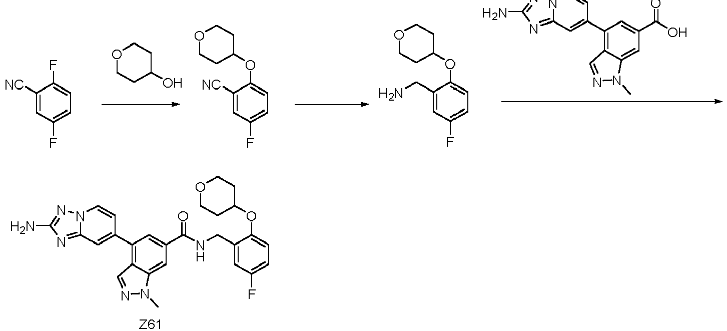

Step 1: 50mL of tetrahydrofuran and tetrahydro-2*H*-pyran-4-ol (2.04g, 20.0mmol) were added to a 100mL three-necked round-bottomed flask at 0°C under the protection of nitrogen gas. Next, sodium hydride (600mg, 15.0mmol) was added. Finally, 2,5-difluorobenzonitrile (1.39g, 10.0mmol) was added. The mixture was slowly warmed up to 55°C and reacted for 12 hours. After the reaction was completed, the mixture was cooled to 0°C. 80 mL of ethyl acetate was added to the system. The resultant mixture was sequentially washed with saturated ammonium chloride and sodium chloride twice (60 mL×2). The phase of ethyl acetate was dried over anhydrous sodium sulfate and filtered. The filtrate was spin-dried to give the target compound of 5-fluoro-2-((tetrahydro-2*H*-pyran-4-yl)oxy)benzonitrile (2.63g, crude product). ES-API: [M+H]⁺=222.1.

Step 2: 50mL of tetrahydrofuran and 5-fluoro-2-((tetrahydro-2*H*-pyran-4-yl)oxy)benzonitrile (2.53g, 10.0mmol) were added to a 500mL single-necked round-bottomed flask under the protection of nitrogen gas. Finally, borane-tetrahydrofuran complex (50mL, 1M, 50mmol) was added. The mixture was warmed up slowly from room temperature to be boiling, and the reaction was carried out overnight. After the reaction was completed, the resultant mixture was cooled to room temperature. Methanol was carefully added dropwise until no more bubbles were generated. 80mL of ethyl acetate was added to the system. The resultant mixture was sequentially washed with saturated ammonium chloride and sodium chloride twice (60mL×2). The phase of ethyl acetate was dried over anhydrous sodium sulfate and filtered. The filtrate was spin-dried to give the target compound of (5-fluoro-2-(((tetrahydro-2H-pyran-4-yl)oxy)phenyl)methylamine (1.71g, Y: 76%). ES-API: [M+H]⁺=226.1.

Step 3: 3mL of N,N-dimethylformamide and triethylamine (164mg, 1.628mmol) were added to a 50mL round-bottomed flask. Next, 4-(2-amino-[1,2,4]triazolo[1,5-*a*]pyridin-7-yl)-1-methyl-1*H*-indazole-6-carboxylic acid (50mg, 0.1623mmol) and (5-fluoro-2-(((tetrahydro-2*H*-pyran-4-yl)oxy)phenyl)methylamine (55mg, 0.2444mmol) were added. Finally, BOP reagent (108mg, 0.2444mmol) was added. The reaction was carried out at room temperature under the protection of nitrogen gas for 2 hours. After the reaction was completed, 50mL of ethyl acetate was added to the system. The resultant mixture was sequentially washed with saturated ammonium chloride and sodium chloride for 3 times (40mL×3). The phase of ethyl acetate was dried over anhydrous sodium sulfate and filtered. The filtrate was spin-dried, and then was purified by pre-HPLC to give the target compound of 4-(2-amino-[1,2,4]triazolo[1,5-*a*]pyridin-7-yl)-*N*-(5-fluoro-2-((tetrahydro-2*H*-pyran-4-yl)oxy)benzyl)-1-methyl-1*H-*indazole-6-carboxamide (23.6mg, Y:28%). ES-API:[M+H]⁺= 516.1. ¹H NMR (500 MHz, DMSO-d6) δ 9.06 (t, *J* = 5.8 Hz, 1H), 8.62 (dd, *J* = 6.9, 0.6 Hz, 1H), 8.30 - 8.17 (m, 2H), 7.87 (d*, J* = 1.1 Hz, 1H), 7.68 (d*, J* = 1.1 Hz, 1H), 7.24 (dd, *J* = 6.9, 1.9 Hz, 1H), 7.00 (dtd, *J* = 11.8, 8.8, 3.9 Hz, 3H), 6.04 (s, 2H), 4.60 - 4.43 (m, 3H), 4.11 (s, 3H), 3.84 - 3.77 (m, 2H), 3.43 (ddd, *J* = 11.5, 8.5, 3.0 Hz, 2H), 1.95 - 1.85 (m, 2H), 1.59 (dtd, *J* = 12.5, 8.3, 3.9 Hz, 2H).

### Example 62: Synthesis of 4-(2-amino-[1,2,4]triazolo[1,5-a]pyridin-7-yl)-N-(5-fluoro-2-((tetrahydrofuran-3-yl)oxy)benzyl)-1-methyl-1H-indazole-6-carboxamide

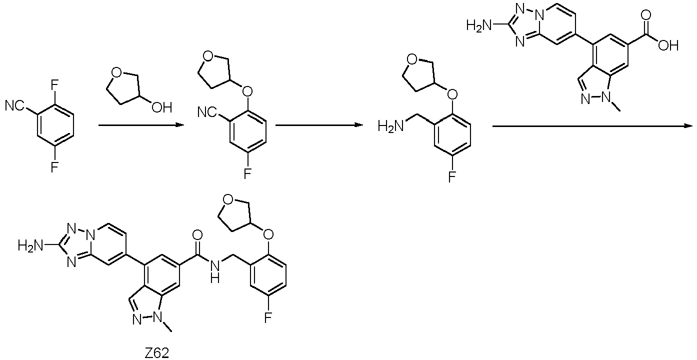

Step 1: 50mL of tetrahydrofuran and tetrahydrofuran-3-ol (1.76g, 20.0mmol) were added to a 100mL three-necked round-bottomed flask at 0°C under the protection of nitrogen gas. Next, sodium hydride (600mg, 15.0mmol) was added. Finally, 2,5-difluorobenzonitrile (1.39g, 10.0mmol) was added. The mixture was slowly warmed up to 55°C and reacted for 12 hours. After the reaction was completed, the mixture was cooled to 0°C. 80 mL of ethyl acetate was added to the system. The resultant mixture was sequentially washed with saturated ammonium chloride and sodium chloride twice (60 mL×2). The phase of ethyl acetate was dried over anhydrous sodium sulfate and filtered. The filtrate was spin-dried to give the target compound of 5-fluoro-2-((tetrahydrofuran-3-yl)oxy)benzonitrile (2.35 g, crude product). ES-API: [M+H]⁺=208.0.

Step 2: 50mL of tetrahydrofuran and 5-fluoro-2-((tetrahydrofuran-3-yl)oxy)benzonitrile (2.35g, 10.0mmol) were added to a 500mL single-necked round-bottomed flask under the protection of nitrogen gas. Finally, borane-tetrahydrofuran complex (50mL, 1M, 50mmol) was added. The mixture was warmed up slowly from room temperature to be boiling, and the reaction was carried out overnight. After the reaction was completed, the resultant mixture was cooled to room temperature. Methanol was carefully added dropwise until no more bubbles were generated. 80mL of ethyl acetate was added to the system. The resultant mixture was sequentially washed with saturated ammonium chloride and sodium chloride twice (60mL×2). The phase of ethyl acetate was dried over anhydrous sodium sulfate and filtered. The filtrate was spin-dried to give the target compound of (5-fluoro-2-(((tetrahydrofuran-3-yl)oxy)phenyl)methylamine (1.51 g, Y: 71%). ES-API: [M+H]⁺ =212.1.

Step 3: 3mL of N,N-dimethylformamide and triethylamine (164mg, 1.628mmol) were added to a 50mL single-necked round-bottomed flask. Next, 4-(2-amino-[1,2,4]triazolo[1,5-*a*]pyridin-7-yl)-1-methyl-1*H*-indazole-6-carboxylic acid (50mg, 0.1623mmol) and (5-fluoro-2-(((tetrahydrofuran-3-yl)oxy)phenyl)methylamine (50mg, 0.2370mmol) were added. Finally, BOP reagent (107mg, 0.2434mmol) was added. The reaction was carried out at room temperature under the protection of nitrogen gas for 2 hours. After the reaction was completed, 50mL of ethyl acetate was added to the system. The resultant mixture was sequentially washed with saturated ammonium chloride and sodium chloride for 3 times (40mL×3). The phase of ethyl acetate was dried over anhydrous sodium sulfate and filtered. The filtrate was spin-dried, and then was purified by pre-HPLC to give the target compound of 4-(2-amino-[1,2,4]triazolo[1,5-*a*]pyridin-7-yl)-*N*-(5-fluoro-2-((tetrahydrofuran-3-yl)oxy) benzyl)-1-methyl-1*H*-indazole-6-carboxamide (27.4mg, Y: 34%). ES-API: [M+H]⁺= 502.1. ¹H NMR (500 MHz, DMSO-d6) δ 9.03 (t, *J* = 5.8 Hz, 1H), 8.63 (d, *J* = 6.9 Hz, 1H), 8.23 (d*, J* = 5.8 Hz, 2H), 7.86 (d, *J* = 1.0 Hz, 1H), 7.69 (d, *J* = 1.2 Hz, 1H), 7.26 (dd, *J* = 6.9, 1.9 Hz, 1H), 6.97 (ddd, *J* = 14.5, 12.5, 6.7 Hz, 3H), 6.09 (s, 2H), 5.01 (dd, *J* = 5.9, 4.5 Hz, 1H), 4.51 - 4.37 (m, 2H), 4.11 (s, 3H), 3.82 (ddd, *J* = 24.3, 13.7, 6.9 Hz, 3H), 3.71 (td, *J* = 8.3, 4.3 Hz, 1H), 2.14 (dtd, *J* = 14.3, 8.3, 6.1 Hz, 1H), 2.01 - 1.92 (m, 1H).

### Example 63: Synthesis of 5-(2-aminobenzo[d]thiazol-6-yl)-N-(1-(1-(4-fluorophenyl)ethyl)-1H-pyrazol-4-yl)-2-methoxynicotinamide

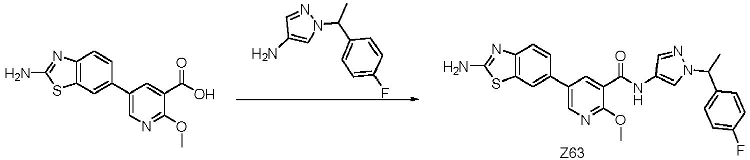

5-(2-Aminobenzo[d]thiazol-6-yl)-2-methoxynicotinic acid (80 mg, 0.253 mmol), 1-(1-(4-fluorophenyl)ethyl)-1*H*-pyrazole-4-amine (62 mg, 0.304 mmol), ((benzotriazol-1-yl)oxy)tris(dimethylamino)phosphonium hexafluorophosphate (134 mg, 0.304 mmol), triethylamine (77 mg, 0.759 mmol), and *N*,*N*-dimethylformamide (5 ml) were added to a 100mL round-bottom flask. The mixture was reacted at room temperature for 2 hours. 15 mL of water was added. The resultant mixture was extracted with 30 mL of ethyl acetate for 3 times. The organic phases were combined, dried over anhydrous sodium sulfate, and filtered. The crude product obtaind by concentrating the organic phase was purified by alkaline HPLC to give 5-(2-aminobenzo[ d]thiazol-6-yl)-*N*-(1-(1-( 4-fluorophenyl)ethyl)-1*H*-pyrazol-4-yl)-2-methoxynicotinamide as an off-white powder (29.96 mg, Y: 24%). ES-API: [M+H]⁺= 489.1. ¹H NMR (400 MHz, DMSO-d6) δ 10.27 (s, 1 H), 8.61 (d, J = 2.0 Hz, 1 H), 8.28 (d, J = 2.0 Hz, 1 H), 8.13 (s, 1 H), 8.04 (d, J = 1.6 Hz, 1 H), 7.64 (s, 1 H), 7.58 (s, 2 H), 7.55 (dd, J1 = 1.2 Hz, J2 = 6.4 Hz, 1 H), 7.40 (d, J = 6.8 Hz, 1 H), 7.34-7.31 (m, 2 H), 7.19-7.16 (m, 2 H), 5.66-5.62 (m, 1 H), 4.01 (s, 3 H), 1.80 (d, J = 6.0 Hz, 3 H).

### Example 64: Synthesis of 3-(2-aminobenzo[d]thiazol-6-yl)-6-(2-cyclopropoxy-3,5-difluorobenzyl)-7,8-dihydro-1,6-naphthyridin-5(6H)-one

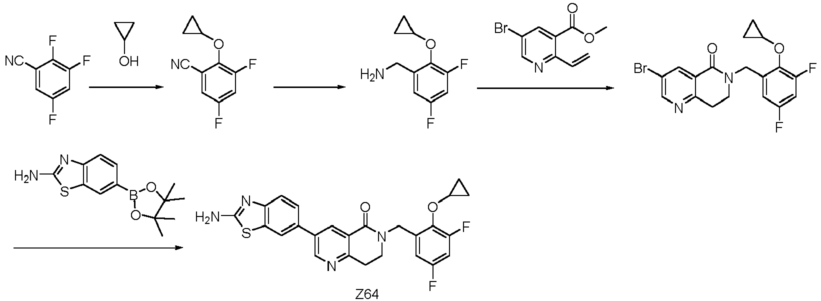

Step 1: 25mL of anhydrous *N*,*N-*dimethylformamide and cyclopropanol (554mg, 9.552mmol) were added to a 100mL three-necked round-bottomed flask at room temperature under the protection of nitrogen gas. Next, cesium carbonate (6.21g, 19.06mmol) was added. Finally, 2,3,5-trifluorobenzonitrile (1.0g, 6.370mmol) was added. The mixture was slowly warmed up to 75°C and reacted for 7 hours. After the reaction was completed, the mixture was cooled to room temperature. 80 mL of ethyl acetate was added to the system. The resultant mixture was sequentially washed with saturated ammonium chloride and sodium chloride twice (60 mL×2). The phase of ethyl acetate was dried over anhydrous sodium sulfate and filtered. The filtrate was spin-dried to give the target compound of 2-cyclopropoxy-3,5-difluorobenzonitrile (1.52g, crude product). ES-API: [M+H]⁺=196.05.

Step 2: 50mL of tetrahydrofuran and 2-cyclopropoxy-3,5-difluorobenzonitrile (1.52g, 6.37mmol) were added to a 500mL single-necked round-bottomed flask under the protection of nitrogen gas. Finally, borane-tetrahydrofuran complex (30mL, 1M, 30mmol) was added. The mixture was warmed up slowly from room temperature to be boiling, and the reaction was carried out overnight. After the reaction was completed, the resultant mixture was cooled to room temperature. Methanol was carefully added dropwise until no more bubbles were generated. 80mL of ethyl acetate was added to the system. The resultant mixture was sequentially washed with saturated ammonium chloride and sodium chloride twice (70mL×2). The phase of ethyl acetate was dried over anhydrous sodium sulfate and filtered. The filtrate was spin-dried to give the target compound of (2-cyclopropoxy-3,5-difluorophenyl)methylamine (0.80 g, Y: 64%). ES-API: [M+H]⁺=200.1.

Step 3: Methyl 5-bromo-2-vinylnicotinate (150mg, 0.625 mmol) and (2-cyclopropoxy-3,5-difluorophenyl) methylamine (400mg, 2.010 mmol) were mixed in DMA (2 mL) in a microwave bottle. The reaction was irradiated with microwave at 180°C for 1 hour. The reaction was cooled to room temperature. 50 mL of ethyl acetate was added to the reacton solution. The resulatant mixture was washed with water (45 mL^{∗}3) and saturated saline (45 mL^{∗}3). The organic layers were combined and concentrated. The concentrate was purified on silicon gel by automatic flash chromatography (ethyl acetate/petroleum ether=0-50%) to give the compound of 3-bromo-6-(2-cyclopropoxy-3,5-difluorobenzyl)-7,8-dihydro-1,6-naphthyridin-5(6*H*)-1-one (95 mg, Y: 55%). ES-API:[M+H]⁺= 409.

Step 4: 3-Bromo-6-(2-cyclopropoxy-3,5-difluorobenzy1)-7,8-dihydro-1,6-naphthyridin-5(6*H*)-1-one (135.0 mg, 0.3300mmol), 6-(4,4,5,5-tetramethyl-1,3,2-dioxaborolan-2-yl)benzo[d]thiazole-2-amine (109.3 mg, 0.4644mmol), sodium carbonate (105mg, 0.990 mmol), and [1,1'-bis(diphenylphosphino)ferrocene] dichloropalladium-dichloromethan complex (36.0mg , 0.050 mmol) were dissolved in 9 mL of dioxane and 1.5 mL of H₂O under the protection of nitrogen gas. The mixture was purged with nigrogen gas for three times. The reaction was carried out at 90°C at a condition of microwave for 35 minutes. The reaction mixture was cooled to room temperature, and concentrated. The crude product was purified by alkaline HPLC to give 3-(2-aminobenzo[d]thiazol-6-yl)-6-(2-cyclopropoxy-3,5-difluorobenzyl)-7,8-dihydro-1,6-naphthyridin-5(6*H*)-one as a white solid (51mg, Y: 22%). ES-API: [M+H]⁺=479.1. ¹H NMR (500 MHz, DMSO-d6) δ 8.95 (d, *J* = 2.4 Hz, 1H), 8.40 (d, *J* = 2.4 Hz, 1H), 8.13 (d, *J* = 1.9 Hz, 1H), 7.69 - 7.58 (m, 3H), 7.44 (d, *J* = 8.3 Hz, 1H), 7.29 (ddd, *J* = 11.6, 8.7, 3.0 Hz, 1H), 6.97 (d, *J* = 9.0 Hz, 1H), 4.69 (s, 2H), 4.21 (dt, *J* = 9.0, 3.0 Hz, 1H), 3.66 (t, *J* = 6.8 Hz, 2H), 3.18 (t, *J* = 6.7 Hz, 2H), 0.88 - 0.80 (m, 2H), 0.65 - 0.59 (m, 2H).

### Example 65: Synthesis of 3-(2-aminobenzo[d]thiazol-6-yl)-6-(2-fluoro-5-(trifluoromethyl)benzyl)-7,8-dihydro-1,6-naphthyridin-5(6H)-one

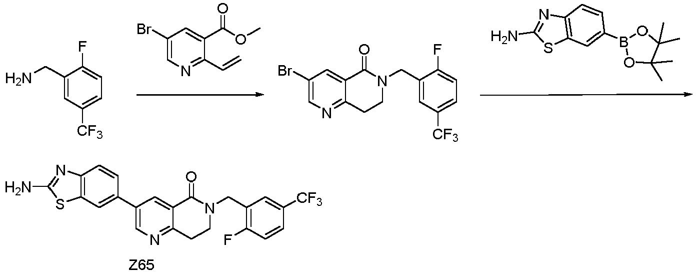

Step 1: Methyl 5-bromo-2-vinylnicotinate (300mg, 1.250 mmol) and (2-fluoro-5-(trifluoromethyl)phenyl)methylamine (600mg, 3.108 mmol) were mixed in dimethylacetamide (2.5mL) in a microwave bottle. The reaction was irradiated with microwave at 150°C for 2 hours. The reaction was cooled to room temperature. 50 mL of ethyl acetate was added to the reaction solution. The resulatant mixture was washed with water (45 mL^{∗}3) and saturated saline (45 mL^{∗}3). The organic layers were combined and concentrated. The concentrate was purified on silicon gel by automatic flash chromatography (ethyl acetate/petroleum ether=0-50%) to give the compound of 3-bromo-6-(2-fluoro-5-(trifluoromethyl)benzyl)-7,8-dihydro-1 ,6-naphthyridin-5(6*H*)-one (320 mg, Y: 63.7%). ES-API:[M+H]⁺= 401.

Step 2: 3-Bromo-6-(2-fluoro-5-(trifluoromethyl)benzyl)-7,8-dihydro-1 ,6-naphthyridin-5(6H)-one (160mg, 0.3960 mmol), 6-(4,4,5,5-tetramethyl-1,3,2-dioxaborolan-2-yl)benzo[d]thiazole-2-amine (131.0 mg, 0.4746mmol), sodium carbonate (125mg, 1.180 mmol), and [1,1'-bis(diphenylphosphino)ferrocene] dichloropalladium-dichloromethan complex (40.0mg, 0.05451 mmol) were dissolved in 9 mL of dioxane and 2 mL of H₂O under the protection of nitrogen gas. The mixture was purged with nigrogen gas for three times. The reaction was carried out at 90°C at a condition of microwave for 35 minutes. The reaction mixture was cooled to room temperature, and concentrated. The crude product was purified by alkaline HPLC to give 3-(2-aminobenzo[d]thiazol-6-yl)-6-(2-fluoro-5-(trifluoro methyl)benzyl)-7,8-dihydro-1,6-naphthyridin-5(6*H*)-one as a white solid (58.0mg, Y: 32%). ES-API:[M+H]⁺=473.0. ¹H NMR ¹H NMR (400MHz, DMSO-d6) δ 8.96 (d, *J* = 2.4 Hz, 1H), 8.41 (d, *J* = 2.3 Hz, 1H), 8.13 (d, *J* = 1.8 Hz, 1H), 7.86 - 7.74 (m, 2H), 7.70 - 7.58 (m, 3H), 7.50 (t, *J* = 9.2 Hz, 1H), 7.43 (d, *J* = 8.3 Hz, 1H), 4.86 (s, 2H), 3.73 (t, *J* = 6.7 Hz, 2H), 3.17 (t, *J* = 6.7 Hz, 2H).

### Biological Assays

The U937 cell strain which was used in the following Testing Examples was derived from ATCC, No.: CRL-1593.2, batch No.: 63479999, culture medium: RPMI-1640 + 10%FBS.

The agents and their suppliers and product Nos are as follows:
RPMI-1640, Gibco, 11875-093;
FBS, Gibco, 10099-141;
Trypsin-EDTA, Gibco, 25200-072;
PS, Gibco, 15140-122;
CellTiter Glo, Progema, G7573;
DMSO, VWR AMRESCO, 0231-500ML;
TNF-α protein (human, recombinant), Peprotech, 300-01A;
Q-VD-Oph, MCE, HY-12305;
V type plate, Corning, 3894;
384-cell low-flange white flat-bottom microplate, Corning, 3570;
RIPK1, Eurofins, 16-022;
MOPS, BDH, 441644J;
EDTA, Sigma, E5134;
Myelin basic protein, Sigma, M1891-25.00 MG;
Magnesium acetate, Merck, DU008026;
ATP (non-radioactive labeled), Sigma, A-7699;
ATP (radioactive labelled), Hartmann Analytic, DU008054;
Phosphoris acid, Metlab, DU003000.

### Testing Exmple 1: Inhibitory activity of the compounds against RIPK1 enzyme

The test compound was dissolved in DMSO to prepare a 10 mM stock solution. The stock solution was diluted in 3.16 folds with DMSO to a series of gradient concentrations. Then, the resultant solution was diluted in 50 folds with MOPS buffer pH 7.0 to prepare a working solution. The working solution was mixed with 36nM RIPK1 (final concentration) and 0.33 mg/ml substrate MBP homogeneously. Thereafter, 10mM magnesium ion and 155µM P³³ isotopically labeled ATP were added for the reaction. The final concentration of DMSO was 2%. After the reaction was carried out at room temperatue for 2 hours phosphoric acid was added to terminate the reaction. The final reaction system was tested using a liquid scintillation counter after treatment. The result obtained by subtracting a blank control from the tested value was converted to activity percentage as compared to the readout value of control group. The activity percentage was plotted against the final concentration of respective compound using a 4-parameter fitting to obtain the inhibitory IC₅₀ of the compound against RIPK1 enzyme. The testing results are shown in Table 1.

**Table 1. Inhibitory activity of the compounds against RIPK1 enzyme**

| Compound No. | RIPK1 enzyme IC₅₀(nM) | Compound No. | RIPK1 enzyme IC₅₀(nM) |
|---|---|---|---|
| Z1-2 | 14 | Z7 | 84 |
| Z2 | 23 | Z9 | 60 |
| Z3 | 32 | Z10 | 64 |
| Z4 | 17 | Z54-2 | 51 |
| Z5 | 29 | Z55 | 39 |
| Z5-1 | 28 | Z56 | 36 |
| Z6 | 36 | Z64 | 34 |
| Z6-1 | 60 | Z65 | 43 |

### Testing Example 2: Inhibitory activity of the compounds against programmed necroptosis of U937 cells induced by TNF-a

The compound was dissolved in DMSO to prepare a 10 mM stock solution. The stock solution was diluted in 3.16 folds with DMSO to a series of gradient concentrations. Then, the resultant solution was diluted in 250 folds with a culture medium to prepare a working solution. The U937 cells were inoculated in a 384-well plate at 5000/well. The compound at respective concentration was added to each of the cells, and was mixed with the cells homogeneously. 100 ng/ml human TNF-α and 25µM Q-VD-Oph were added simultaneously to induce programmed necroptosis of the cells. The final concentration of DMSO was 2%. The cells were placed in the incubator at 37°C and 5% CO₂ for further culture of 48 hours. Celltiter-Glo reagent was used for detection. After adequate pyrolysis reaction, the chemiluminescence readout value was recorded by a microplate reader. The survival rate was calculated from the detection results using the equation as follows: SR (%) = (RLU compound - RLU blank) / (RLU high control - RLU blank) ×100%. The survival rate was plotted against the final concentration of respective compound using a 4-parameter fitting to calculate the inhibitory IC₅₀ of the compound against programmed necroptosis of cells induced by TNF-a. The testing results of exemplary compounds are shown in Table 2.

**Table 2. Inhibitory activity of the compounds against U937 cells**

| Compound No. | U937 IC₅₀(µM) | Compound No. | U937 IC₅₀(µM) | Compound No. | U937 IC₅₀(µM) |
|---|---|---|---|---|---|
| Z1 | 0.052 | Z19 | 0.007 | Z49 | 0.006 |
| Z1-2 | 0.028 | Z20 | 0.018 | Z50 | 0.049 |
| Z2 | 0.038 | Z22 | 0.062 | Z52 | 0.019 |
| Z5 | 0.015 | Z24 | 0.151 | Z53 | 0.042 |
| Z5-1 | 0.005 | Z25 | 0.140 | Z54-2 | 0.001 |
| Z6 | 0.008 | Z29 | 0.007 | Z55 | 0.008 |
| Z6-1 | 0.003 | Z31 | 0.002 | Z56 | 0.008 |
| Z8 | 0.088 | Z37 | 0.045 | Z57 | 0.011 |
| Z12 | 0.010 | Z38 | 0.014 | Z59 | 0.024 |
| Z13 | 0.017 | Z40 | 0.068 | Z60 | 0.213 |
| Z14 | 0.006 | Z41 | 0.014 | Z61 | 0.002 |
| Z16 | 0.026 | Z42 | 0.043 | Z62 | 0.030 |
| Z17 | 0.004 | Z43 | 0.003 | Z63 | 0.007 |
| Z17-1 | 0.009 | Z46 | 0.076 | Z64 | 0.004 |
| Z18 | 0.004 | Z48 | 0.088 | Z65 | 0.048 |

### Testing Example 3: Inhibitory activity of the compounds against programmed necroptosis of L929 cells induced by TNF-a

The compound was dissolve in DMSO to prepare a 10 mM stock solution. The stock solution was diluted in 3.16 folds with DMSO to a series of gradient concentrations. Then, the resultant solution was diluted in 100 folds with a culture medium to prepare a working solution. The L929 cells were inoculated in a 384-well plate at 10000/well. The compound at respective concentration was added to each of the wells, and was mixed with the cells homogeneously. 30 ng/ml murine TNF-α and 15µM Z-VAD were added simultaneously to induce programmed necroptosis of the cells. The final concentration of DMSO was 2%. The cells were placed in an incubator at 37°C and 5% CO₂ for further culture of 6 hours. CellTiter-Glo reagent was used for detection. After adequate pyrolysis reaction, the chemiluminescence readout value was recorded by a microplate reader. The survival rate was calculated from the detection results using the equation as follows: SR (%) = (RLU compound - RLU blank) / (RLU high control - RLU blank) × 100%. The survival rate was plotted against the final concentration of respective compound using a 4-parameter fitting to calculate the inhibitory IC₅₀ of the compound against programmed necroptosis of cells induced by TNF-a. The testing results of exemplary compounds are shown in Table 3.

**Table 3. Inhibitory activity of the compounds against L929 cells**

| Compound No. | L929 AbIC₅₀(µM) |
|---|---|
| Z1-2 | 0.006 |
| Z17-1 | 0.010 |
| Z29 | 0.012 |
| Z31 | 0.041 |
| Z49 | 0.037 |
| Z54-2 | <0.003 |
| Z64 | 0.003 |

Although specific embodiments of the invention have been described in details, from all the public teachings, a person skilled in the art may make various modifications and alternatives, which are within the protection scope of the present invention. The protection scope of the present invention is provided by the attached claims and any equivalents thereof.

## Claims

1. A compound or a pharmaceutically acceptable salt, stereoisomer, solvate or prodrug thereof, the compound has a structure as represented by formula (I): 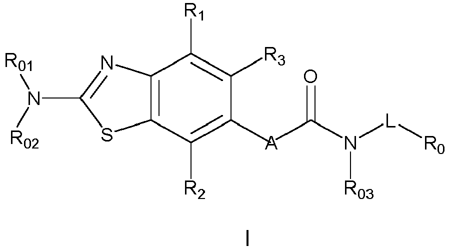 wherein,
R₀ is a substituted or unsubstituted C₆₋₁₄ aryl or a substituted or unsubstituted C₅₋₁₄ heteroaryl;
R₁, R₂, R₃ are each independently hydrogen, hydroxy, halo, nitro, substituted or unsubstituted C₁₋₁₀ alkyl, substituted or unsubstituted C₁₋₁₀ alkoxy, substituted or unsubstituted C₃₋₂₀ cycloalkyl, -(CH₂)ᵤ-C₃₋₆ monocyclic heterocyclyl, -(CH₂)ᵤ-phenyl, -(CH₂)ᵤ-5- or 6-membered monoheteroaryl, -(CH₂)ᵤ-C₃₋₈ monocyclic cycloalkyl, - SO₂C₁₋₆ alkyl, -(CH₂)ᵤ-NRₐ₀R_{b0}, -(CH₂)ᵤ-C(O)NRₐ₀R_{b0}, -C(O)C₁₋₆ alkyl, -C(O)OC₁₋₆ alkyl; wherein the phenyl, C₃₋₈ monocyclic cycloalkyl, C₃₋₆ monocyclic heterocyclyl, 5- or 6-membered monoheteroaryl is optionally substituted by 1, 2 or 3 substituent(s) selected from the group consisting of halo, cyano, C₁₋₃ alkyl, C₁₋₃ alkoxy and C₃₋₆ monocyclic cycloalkyl; u is 0, 1, 2, 3 or 4; Rₐ₀, R_{b0} are each independently hydrogen or C₁₋₃ alkyl, or Rₐ₀ and R_{b0} together with the nitrogen atom attached thereto form a C₃₋₈ monocyclic heterocyclyl, the C₃₋₈ monocyclic heterocyclyl is optionally substituted by 1, 2 or 3 substituent(s) selected from the group consisting of halo or C₁₋₃ alkyl;
A has a structure as represented by formula (a) or formula (b) 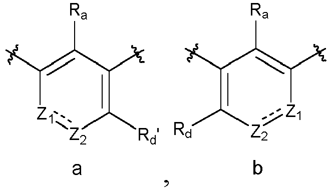 wherein Z1, Z2 and " 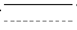 " are selected from the group consisting of:
(a1) Z₁ is CR_{b}, Z₂ is N, and " 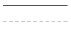 " is a double bond; wherein R_{b} is hydrogen, halo, substituted or unsubstituted C₁₋₁₀ alkyl, substituted or unsubstituted C₁₋₁₀ alkoxy or NRₐ₀R_{b0};
(b1) Z₁ is C(O), Z₂ is NR_{c}, and " 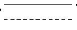 " is a single bond; wherein R_{c} is hydrogen or substituted or unsubstituted C₁₋₁₀ alkyl;
(c1) Z₁ is CR_{b}, Z₂ is CR_{c}, and " 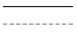 " is a single bond or a double bond; wherein R_{b}, R_{c} together with the ring attached thereto form a substituted or unsubstituted 9- or 10-membered biheteroaryl fused ring;
(d1) Z₁ is NR_{b}, Z₂ is CR_{c}, and " 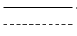 " is a single bond; wherein R_{b}, R_{c} together with the ring attached thereto form a substituted or unsubstituted 9- or 10-membered biheteroaryl fused ring;
(e1) Z₁ is CR_{b}, Z₂ is NR_{c}, and " 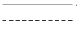 " is a single bond; wherein R_{b}, R_{c} together with the ring attached thereto form a substituted or unsubstituted 9- or 10-membered biheteroaryl fused ring;
Rₐ, R_{d} are each independently hydrogen, halo, substituted or unsubstituted C₁₋₁₀ alkyl, substituted or unsubstituted C₁₋₁₀ alkoxy or NRₐ₀R_{b0};
R₀₁, R₀₂ are each independently hydrogen, -C(O)C₁₋₆ alkyl or -C(O)C₃₋₈ monocyclic cycloalkyl;
L is a bond, -(CR₁₁R₁₂)ₜ₁-(CR₂₁R₂₂)ₜ₂-(CR₃₁R₃₂)ₜ₃-(CR₄₁R₄₂)ₜ₄-(O)ₜ₅ or - (CR₁₁R₁₂)ₜ₁-(CR₂₁R₂₂)ₜ₂-(NR₃₁)ₜ₃-(CR₄₁R₄₂)ₜ₄-(O)ₜ₅; wherein t1, t2, t3, t4, t5 are each independently 0 or 1;
R_{d}', R₀₃, R₁₁, R₁₂, R₂₁, R₂₂, R₃₁, R₃₂, R₄₁, R₄₂ are selected from the combination selected from the group consisting of:
(a2) R_{d}' are each independently hydrogen, halo, substituted or unsubstituted C₁₋₁₀ alkyl, substituted or unsubstituted C₁₋₁₀ alkoxy or NRₐ₀R_{b0};
R₀₃ is hydrogen or substituted or unsubstituted C₁₋₁₀ alkyl;
R₁₁, R₁₂, R₄₁, R₄₂ are each independently hydrogen, halo, hydroxy, hydroxymethyl, hydroxyethyl or C₁₋₃ alkyl;
R₂₁, R₂₂, R₃₁, R₃₂ are each independently hydrogen, halo, hydroxy, hydroxymethyl, hydroxyethyl or C₁₋₃ alkyl;
(b2) R_{d}' are each independently hydrogen, halo, substituted or unsubstituted C₁₋₁₀ alkyl, substituted or unsubstituted C₁₋₁₀ alkoxy or NRₐ₀R_{b0};
R₀₃ is hydrogen or substituted or unsubstituted C₁₋₁₀ alkyl;
R₁₁, R₁₂, R₄₁, R₄₂ are each independently hydrogen, halo, hydroxy, hydroxymethyl, hydroxyethyl or C₁₋₃ alkyl;
R₃₁, R₃₂ are each independently hydrogen, halo, hydroxy, hydroxymethyl, hydroxyethyl or C₁₋₃ alkyl;
R₂₁, R₂₂ together with the carbon atom attached thereto form a substituted or unsubstituted C₃₋₈ monocyclic cycloalkyl or substituted or unsubstituted C₃₋₆ monocyclic heterocyclyl;
(c2) R_{d}' are each independently hydrogen, halo, substituted or unsubstituted C₁₋₁₀ alkyl, substituted or unsubstituted C₁₋₁₀ alkoxy or NRₐ₀R_{b0};
R₀₃ is hydrogen, or substituted or unsubstituted C₁₋₁₀ alkyl;
R₁₁, R₁₂, R₄₁, R₄₂ are each independently hydrogen, halo, hydroxy, hydroxymethyl, hydroxyethyl or C₁₋₃ alkyl;
R₂₁, R₂₂ are each independently hydrogen, halo, hydroxy, hydroxymethyl, hydroxyethyl or C₁₋₃ alkyl;
R₃₁, R₃₂ together with the carbon atom attached thereto form a substituted or unsubstituted C₃₋₈ monocyclic cycloalkyl or substituted or unsubstituted C₃₋₆ monocyclic heterocyclyl;
(d2) R_{d}' are each independently hydrogen, halo, substituted or unsubstituted C₁₋₁₀ alkyl, substituted or unsubstituted C₁₋₁₀ alkoxy or NRₐ₀R_{b0};
R₀₃ is hydrogen, or substituted or unsubstituted C₁₋₁₀ alkyl;
R₁₁, R₁₂, R₄₁, R₄₂ are each independently hydrogen, halo, hydroxy, hydroxymethyl, hydorxyethyl or C₁₋₃ alkyl;
R₂₂, R₃₂ are each independently hydrogen, halo, hydroxy, hydroxymethyl, hydorxyethyl or C₁₋₃ alkyl;
R₂₁, R₃₁ together with the carbon atom attached thereto form a substituted or unsubstituted C₃₋₈ monocyclic cycloalkyl or substituted or unsubstituted C₃₋₆ monocyclic heterocyclyl;
(e2) R_{d}' are each independently hydrogen, halo, substituted or unsubstituted C₁₋₁₀ alkyl, substituted or unsubstituted C₁₋₁₀ alkoxy or NRₐ₀R_{b0};
R₁₁, R₁₂, R₄₁, R₄₂ are each independently hydrogen, halo, hydroxy, hydroxymethyl, hydroxyethyl or C₁₋₃ alkyl;
R₂₁, R₂₂, R₃₂ are each independently hydrogen, halo, hydroxy, hydroxymethyl, hydroxyethyl or C₁₋₃ alkyl;
R₀₃ and R₃₁ are linked together to form a substituted or unsubstituted C₃₋₈ monocyclic heterocyclyl;
(f2) R_{d}' are each independently hydrogen, halo, substituted or unsubstituted C₁₋₁₀ alkyl, substituted or unsubstituted C₁₋₁₀ alkoxy or NRₐ₀R_{b0};
R₁₁, R₁₂, R₄₁, R₄₂ are each independently hydrogen, halo, hydroxy, hydroxymethyl, hydroxyethyl or C₁₋₃ alkyl;
R₂₂, R₃₁, R₃₂ are each independently hydrogen, halo, hydroxy, hydroxymethyl, hydroxyethyl or C₁₋₃ alkyl;
R₀₃ and R₂₁ are linked together to form a substituted or unsubstituted C₃₋₈ monocyclic heterocyclyl;
(g2) R₁₁, R₁₂, R₄₁, R₄₂ are each independently hydrogen, halo, hydroxy, hydroxymethyl, hydroxyethyl or C₁₋₃ alkyl;
R₂₁, R₂₂, R₃₁, R₃₂ are each independently hydrogen, halo, hydroxy, hydroxymethyl, hydroxyethyl or C₁₋₃ alkyl;
R_{d}' and R₀₃ are linked together to form a substituted or unsubstituted 4- to 7-membered oxo-substituted heterocyclyl;
(h2) R_{d}' are each independently hydrogen, halo, substituted or unsubstituted C₁₋₁₀ alkyl, substituted or unsubstituted C₁₋₁₀ alkoxy or NRₐ₀R_{b0};
R₀₃ is hydrogen or substituted or unsubstituted C₁₋₁₀ alkyl;
R₁₂, R₄₁, R₄₂ are each independently hydrogen, halo, hydroxy, hydroxymethyl, hydroxyethyl or C₁₋₃ alkyl;
R₂₁, R₂₂, R₃₂ are each independently hydrogen, halo, hydroxy, hydroxymethyl, hydroxyethyl or C₁₋₃ alkyl;
R₁₁ and R₃₁ are linked together to form a substituted or unsubstituted C₃₋₈ monocyclic cycloalkyl, or substituted or unsubstituted C₃₋₆ monocyclic heterocyclyl;
in the above groups, the "substituted" means that 1, 2, 3 or 4 hydrogen atom(s) in the group are substituted by a substituent independently selected from the group S consisting of: hydroxy, halo, nitro, oxo, C₁₋₆ alkyl, hydroxy-substituted C₁₋₆ alkyl, benzyl, -S-C₁₋₆ alkyl, -S-halo C₁₋₆ alkyl, -(CRₐ₁R_{b1})ᵤ-cyano, -(CRₐ₁R_{b1})ᵤ-C₁₋₆ alkoxy, - (CRₐ₁R_{b1})ᵤ-halo C₁₋₆ alkoxy, -(CRₐ₁R_{b1})ᵤ-halo C₁₋₆ alkyl, -(CRₐ₁R_{b1})ᵤ-C₃₋₆ monocyclic heterocyclyl, -(CRₐ₁R_{b1})ᵤ-C₃₋₈ monocyclic cycloalkyl, -(CRₐ₁R_{b1})ᵤ-phenyl, - (CRₐ₁R_{b1})ᵤ-5- or 6-membered monoheteroaryl, -(CRₐ₁R_{b1})ᵤ-O-(CRₐ₂R_{b2})ᵥ-halo C₁₋₆ alkyl, -(CRₐ₁R_{b1})ᵤ-O-(CRₐ₂R_{b2})ᵥ-C₃₋₈ monocyclic cycloalkyl, -(CRₐ₁R_{b1})ᵤ-O-(CRₐ₂R_{b2})ᵥ-C₃₋₆ monocyclic heterocyclyl, -(CRₐ₁R_{b1})ᵤ-O-(CRₐ₂R_{b2})ᵥ-phenyl, - (CRₐ₁R_{b1})ᵤ-O-(CRₐ₂R_{b2})ᵥ-5- or 6-membered monoheteroaryl, -(CRₐ₁R_{b1})ᵤ-S-(CRₐ₂R_{b2})ᵥ-phenyl, -(CRₐ₁R_{b1})ᵤ-SO₂-(CRₐ₂R_{b2})ᵥ-phenyl, -(CRₐ₁R_{b1})ᵤ-O-C(O)NRₐ₀R_{b0}, -(CRₐ₁R_{b1})ᵤ-O-(CRₐ₂R_{b2})ᵥ-C₁₋₆ alkoxy, -(CRₐ₁R_{b1})ᵤ-O-(CRₐ₂R_{b2})ᵥOH, -(CRₐ₁R_{b1})ᵤ-SO₂C₁₋₆alkyl, -(CRₐ₁R_{b1})ᵤ-SO₂NRₐ₀R_{b0}, -(CRₐ₁R_{b1})ᵤ-C(O)NRₐ₀R_{b0}, -(CRₐ₁R_{b1})ᵤ-NRₐ₀R_{b0}, -(CRₐ₁R_{b1})ᵤ-C(O)C₁₋₆ alkyl, -C(O)OC₁₋₆alkyl, NRₐ₀C(O)-(CRₐ₁R_{b1})ᵤ-NRₐ₀R_{b0}, NRₐ₀C(O)-(CRₐ₁R_{b1})ᵤOH, NRₐ₀C(O)-halo C₁₋₆alkyl; wherein the C₃₋₈ monocyclic cycloalkyl, C₃₋₆ monocyclic heterocyclyl, phenyl, 5-or 6-membered monoheteroaryl is optionally substituted by 1, 2 or 3 substituent(s) selected from the group consisting of hydroxy, hydroxymethyl, hydroxyethyl, halo, cyano, cyanomethyl, cyanoethyl, C₁₋₃ alkyl, C₁₋₃ alkoxy and C₃₋₆ monocyclic cycloalkyl;
u, v are each independently 0, 1, 2, 3 or 4;
Rₐ₀, R_{b0} are each independently hydrogen or C₁₋₃ alkyl; or Rₐ₀, R_{b0} together with the nitrogen atom attached thereto form a C₃₋₈ monocyclic heterocyclyl, the C₃₋₈ monocyclic heterocyclyl is optionally substituted by 1, 2 or 3 halo or C₁₋₃ alkyl;
Rₐ₁, R_{b1}, Rₐ₂, R_{b2} are the same or different, and are each independently hydrogen, hydroxyl or C₁₋₃-alkyl.

2. A compound or a pharmaceutically acceptable salt, stereoisomer, solvate or prodrug thereof, the compound has a structure as represented by formula (II): 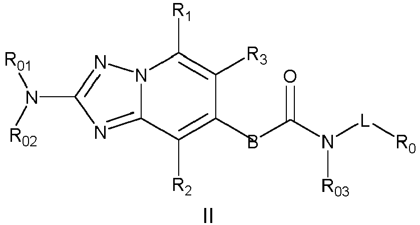 wherein,
R₀ is a substituted or unsubstituted C₆₋₁₄ aryl, or a substituted or unsubstituted C₅₋₁₄ heteroaryl;
R₁, R₂, R₃ are each independently hydrogen, hydroxy, halo, nitro, substituted or unsubstituted C₁₋₁₀ alkyl, substituted or unsubstituted C₁₋₁₀ alkoxy, substituted or unsubstituted C₃₋₂₀ cycloalkyl, -(CH₂)ᵤ-C₃₋₆ monocyclic heterocyclyl, -(CH₂)ᵤ-phenyl, -(CH₂)ᵤ-5- or 6-membered monoheteroaryl, -(CH₂)ᵤ-C₃₋₈ monocyclic cycloalkyl, - SO₂C₁₋₆ alkyl, -(CH₂)ᵤ-NRₐ₀R_{b0}, -(CH₂)ᵤ-C(O)NRₐ₀R_{b0}, -C(O)C₁₋₆ alkyl, -C(O)OC₁₋₆ alkyl; wherein the phenyl, C₃₋₈ monocyclic cycloalkyl, C₃₋₆ monocyclic heterocyclyl, 5- or 6-membered monoheteroaryl is optionally substituted by 1, 2 or 3 substituent(s) selected from the group consisting of halo, cyano, C₁₋₃ alkyl, C₁₋₃ alkoxy and C₃₋₆ monocyclic cycloalkyl; u is 0, 1, 2, 3 or 4; Rₐ₀, R_{b0} are each independently hydrogen or C₁₋₃ alkyl, or Rₐ₀ and R_{b0} together with the nitrogen atom attached thereto form a C₃₋₈ monocyclic heterocyclyl, the C₃₋₈ monocyclic heterocyclyl is optionally substituted by 1, 2 or 3 substituent(s) selected from the group consisting of halo or C₁₋₃ alkyl;
B has a structure as represented by formula (II-a) or formula (II-b): 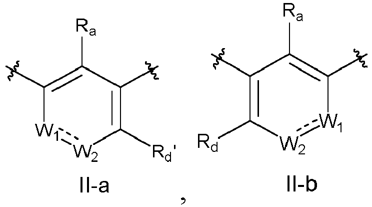 wherein W₁, W₂ and " 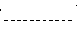 " are selected from the group consisting of:
(a1) W₁ is C(O), W₂ is NR_{c}, and " 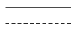 " is a single bond; wherein R_{c} is hydrogen or substituted or unsubstituted C₁₋₁₀ alkyl;
(b1) W₁ is CR_{b}, W₂ is CR_{c}, and " 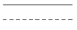 " is a single bond or a double bond; wherein R_{b}, R_{c} together with the ring attached thereto form a substituted or unsubstituted 9- or 10-membered biheteroaryl fused ring;
(c1) W₁ is NR_{b}, W₂ is CR_{c}, and " 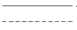 " is a single bond; wherein R_{b}, R_{c} together with the ring attached thereto form a substituted or unsubstituted 9- or 10-membered biheteroaryl fused ring;
(d1) W₁ is CR_{b}, W₂ is NR_{c}, and " 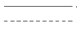 " is a single bond; wherein R_{b}, R_{c} together with the ring attached thereto form a substituted or unsubstituted 9- or 10-membered biheteroaryl fused ring;
Rₐ, R_{d} are each independently hydrogen, halo, substituted or unsubstituted C₁₋₁₀ alkyl, substituted or unsubstituted C₁₋₁₀ alkoxy or NRₐ₀R_{b0};
R₀₁, R₀₂ are each independently hydrogen, -C(O)C₁₋₆ alkyl or -C(O)C₃₋₈ monocyclic cycloalkyl;
L is a bond, -(CR₁₁R₁₂)ₜ₁-(CR₂₁R₂₂)ₜ₂-(CR₃₁R₃₂)ₜ₃-(CR₄₁R₄₂)ₜ₄-(O)ₜ₅ or - (CR₁₁R₁₂)ₜ₁-(CR₂₁R₂₂)ₜ₂-(NR₃₁)ₜ₃-(CR₄₁R₄₂)ₜ₄-(O)ₜ₅; wherein t1, t2, t3, t4 and t5 are each independently 0 or 1;
R_{d}', R₀₃, R₁₁, R₁₂, R₂₁, R₂₂, R₃₁, R₃₂, R₄₁, R₄₂ are selected from the combination selected from the group consisting of:
(a2) R_{d}' are each independently hydrogen, halo, substituted or unsubstituted C₁₋₁₀ alkyl, substituted or unsubstituted C₁₋₁₀ alkoxy or NRₐ₀R_{b0};
R₀₃ is hydrogen or substituted or unsubstituted C₁₋₁₀ alkyl;
R₁₁, R₁₂, R₄₁, R₄₂ are each independently hydrogen, halo, hydroxy, hydroxymethyl, hydroxyethyl or C₁₋₃ alkyl;
R₂₁, R₂₂, R₃₁, R₃₂ are each independently hydrogen, halo, hydroxy, hydroxymethyl, hydroxyethyl or C₁₋₃ alkyl;
(b2) R_{d}' are each independently hydrogen, halo, substituted or unsubstituted C₁₋₁₀ alkyl, substituted or unsubstituted C₁₋₁₀ alkoxy or NRₐ₀R_{b0};
R₀₃ is hydrogen or substituted or unsubstituted C₁₋₁₀ alkyl;
R₁₁, R₁₂, R₄₁, R₄₂ are each independently hydrogen, halo, hydroxy, hydroxymethyl, hydroxyethyl or C₁₋₃ alkyl;
R₃₁, R₃₂ are each independently hydrogen, halo, hydroxy, hydroxymethyl, hydroxyethyl or C₁₋₃ alkyl;
R₂₁, R₂₂ together with the carbon atom attached thereto form a substituted or unsubstituted C₃₋₈ monocyclic cycloalkyl, or substituted or unsubstituted C₃₋₆ monocyclic heterocyclyl;
(c2) R_{d}' are each independently hydrogen, halo, substituted or unsubstituted C₁₋₁₀ alkyl, substituted or unsubstituted C₁₋₁₀ alkoxy or NRₐ₀R_{b0};
R₀₃ is hydrogen or substituted or unsubstituted C₁₋₁₀ alkyl;
R₁₁, R₁₂, R₄₁, R₄₂ are each independently hydrogen, halo, hydroxy, hydroxymethyl, hydroxyethyl or C₁₋₃ alkyl;
R₂₁, R₂₂ are each independently hydrogen, halo, hydroxy, hydroxymethyl, hydroxyethyl or C₁₋₃ alkyl;
R₃₁, R₃₂ together with the carbon atom attached thereto form a substituted or unsubstituted C₃₋₈ monocyclic cycloalkyl, or substituted or unsubstituted C₃₋₆ monocyclic heterocyclyl;
(d2) R_{d}' are each independently hydrogen, halo, substituted or unsubstituted C₁₋₁₀ alkyl, substituted or unsubstituted C₁₋₁₀ alkoxy or NRₐ₀R_{b0};
R₀₃ is hydrogen, or substituted or unsubstituted C₁₋₁₀ alkyl;
R₁₁, R₁₂, R₄₁, R₄₂ are each independently hydrogen, halo, hydroxy, hydroxymethyl, hydroxyethyl or C₁₋₃ alkyl;
R₂₂, R₃₂ are each independently hydrogen, halo, hydroxy, hydroxymethyl, hydorxyethyl or C₁₋₃ alkyl;
R₂₁, R₃₁ together with the carbon atom attached thereto form a substituted or unsubstituted C₃₋₈ monocyclic cycloalkyl, or substituted or unsubstituted C₃₋₆ monocyclic heterocyclyl;
(e2) R_{d}' are each independently hydrogen, halo, substituted or unsubstituted C₁₋₁₀ alkyl, substituted or unsubstituted C₁₋₁₀ alkoxy or NRₐ₀R_{b0};
R₁₁, R₁₂, R₄₁, R₄₂ are each independently hydrogen, halo, hydroxy, hydroxymethyl, hydroxyethyl or C₁₋₃ alkyl;
R₂₁, R₂₂, R₃₂ are each independently hydrogen, halo, hydroxy, hydroxymethyl, hydroxyethyl or C₁₋₃ alkyl;
R₀₃ and R₃₁ are linked together to form a substituted or unsubstituted C₃₋₈ monocyclic heterocyclyl;
(f2) R_{d}' are each independently hydrogen, halo, substituted or unsubstituted C₁₋₁₀ alkyl, substituted or unsubstituted C₁₋₁₀ alkoxy or NRₐ₀R_{b0};
R₁₁, R₁₂, R₄₁, R₄₂ are each independently hydrogen, halo, hydroxy, hydroxymethyl, hydroxyethyl or C₁₋₃ alkyl;
R₂₂, R₃₁, R₃₂ are each independently hydrogen, halo, hydroxy, hydroxymethyl, hydroxyethyl or C₁₋₃ alkyl;
R₀₃ and R₂₁ are linked together to form a substituted or unsubstituted C₃₋₈ monocyclic heterocyclyl;
(g2) R₁₁, R₁₂, R₄₁, R₄₂ are each independently hydrogen, halo, hydroxy, hydroxymethyl, hydroxyethyl or C₁₋₃ alkyl;
R₂₁, R₂₂, R₃₁, R₃₂ are each independently hydrogen, halo, hydroxy, hydroxymethyl, hydroxyethyl or C₁₋₃ alkyl;
R_{d}' and R₀₃ are linked together to form a substituted or unsubstituted 4- to 7-membered oxo-substituted heterocyclyl;
(h2) R_{d}' are each independently hydrogen, halo, substituted or unsubstituted C₁₋₁₀ alkyl, substituted or unsubstituted C₁₋₁₀ alkoxy or NRₐ₀R_{b0};
R₀₃ is hydrogen or substituted or unsubstituted C₁₋₁₀ alkyl;
R₁₂, R₄₁, R₄₂ are each independently hydrogen, halo, hydroxy, hydroxymethyl, hydroxyethyl or C₁₋₃ alkyl;
R₂₁, R₂₂, R₃₂ are each independently hydrogen, halo, hydroxy, hydroxymethyl, hydroxyethyl or C₁₋₃ alkyl;
R₁₁ and R₃₁ are linked together to form a substituted or unsubstituted C₃₋₈ monocyclic cycloalkyl or substituted or unsubstituted C₃₋₆ monocyclic heterocyclyl;
in the above groups, the "substituted" means that 1, 2, 3 or 4 hydrogen atom(s) in the group are substituted by a substituent independently selected from the group S consisting of: hydroxy, halo, nitro, oxo, C₁₋₆ alkyl, hydroxy-substituted C₁₋₆ alkyl, benzyl, -S-C₁₋₆ alkyl, -S-halo C₁₋₆ alkyl, -(CRₐ₁R_{b1})ᵤ-cyano, -(CRₐ₁R_{b1})ᵤ-C₁₋₆ alkoxy,-(CRₐ₁R_{b1})ᵤ-halo C₁₋₆ alkoxy, -(CRₐ₁R_{b1})ᵤ-halo C₁₋₆ alkyl, -(CRₐ₁R_{b1})ᵤ-C₃₋₆ monocyclic heterocyclyl, -(CRₐ₁R_{b1})ᵤ-C₃₋₈ monocyclic cycloalkyl, -(CRₐ₁R_{b1})ᵤ-phenyl, - (CRₐ₁R_{b1})ᵤ-5- or 6-membered monoheteroaryl, -(CRₐ₁R_{b1})ᵤ-O-(CRₐ₂R_{b2})ᵥ-halo C₁₋₆ alkyl, -(CRₐ₁R_{b1})ᵤ-O-(CRₐ₂R_{b2})ᵥ-C₃₋₈ monocyclic cycloalkyl, -(CRₐ₁R_{b1})ᵤ-O-(CRₐ₂R_{b2})ᵥ-C₃₋₆ monocyclic heterocyclyl, -(CRₐ₁R_{b1})ᵤ-O-(CRₐ₂R_{b2})ᵥ-phenyl, - (CRₐ₁R_{b1})ᵤ-O-(CRₐ₂R_{b2})ᵥ-5- or 6-membered monoheteroaryl, -(CRₐ₁R_{b1})ᵤ-S-(CRₐ₂R_{b2})ᵥ-phenyl, -(CRₐ₁R_{b1})ᵤ-SO₂-(CRₐ₂R_{b2})ᵥ-phenyl, -(CRₐ₁R_{b1})ᵤ-O-C(O)NRₐ₀R_{b0}, -(CRₐ₁R_{b1})ᵤ-O-(CRₐ₂R_{b2})ᵥ-C₁₋₆ alkoxy, -(CRₐ₁R_{b1})ᵤ-O-(CRₐ₂R_{b2})ᵥOH, -(CRₐ₁R_{b1})ᵤ-SO₂C₁₋₆ alkyl, -(CRₐ₁R_{b1})ᵤ-SO₂NRₐ₀R_{b0}, -(CRₐ₁R_{b1})ᵤ-C(O)NRₐ₀R_{b0}, -(CRₐ₁R_{b1})ᵤ-NRₐ₀R_{b0}, -(CRₐ₁R_{b1})ᵤ-C(O)C₁₋₆ alkyl, -C(O)OC₁₋₆ alkyl, NRₐ₀C(O)-(CRₐ₁R_{b1})ᵤ-NRₐ₀R_{b0}, NRₐ₀C(O)-(CRₐ₁R_{b1})ᵤOH, NRₐ₀C(O)-halo C₁₋₆ alkyl; wherein the C₃₋₈ monocyclic cycloalkyl, C₃₋₆ monocyclic heterocyclyl, phenyl, 5-or 6-membered monoheteroaryl is optionally substituted by 1, 2 or 3 substituent(s) selected from the group consisting of hydroxy, hydroxymethyl, hydroxyethyl, halo, cyano, cyanomethyl, cyanoethyl, C₁₋₃ alkyl, C₁₋₃ alkoxy and C₃₋₆ monocyclic cycloalkyl;
u, v are each independently 0, 1, 2, 3 or 4;
Rₐ₀, R_{b0} are each independently hydrogen or C₁₋₃ alkyl; or Rₐ₀, R_{b0} together with the nitrogen atom attached thereto form a C₃₋₈ monocyclic heterocyclyl, the C₃₋₈ monocyclic heterocyclyl is optionally substituted by 1, 2 or 3 halo or C₁₋₃ alkyl;
Rₐ₁, R_{b1}, Rₐ₂, R_{b2} are the same or different, and are each independently hydrogen, hydroxyl or C₁₋₃-alkyl.

3. The compound or a pharmaceutically acceptable salt, stereoisomer, solvate or prodrug thereof according to claim 1 or 2, **characterized in that**, -N(R₀₃)-L- has a structure as represented by formula (c), formula (d), formula (e) or fomula (f): 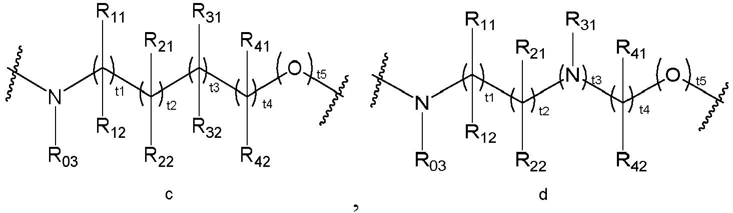
in the formula (c) or formula (d), t1, t2, t3, t4, t5 are each independently 0 or 1; R₀₃ is hydrogen or a substituted or unsubstituted C₁₋₁₀ alkyl;
R₁₁, R₁₂, R₂₁, R₂₂, R₃₁, R₃₂, R₄₁, R₄₂ are selected from the combination selected from the group consisting of:
(1) R₁₁, R₁₂, R₂₁, R₂₂, R₃₁, R₃₂, R₄₁, R₄₂ are each independently hydrogen, halo, hydroxy, hydroxymethyl, hydroxyethyl or C₁₋₃ alkyl;
(2) R₁₁, R₁₂, R₃₁, R₃₂, R₄₁, R₄₂ are each independently hydrogen, halo, hydroxy, hydroxymethyl, hydroxyethyl or C₁₋₃ alkyl;
R₂₁, R₂₂ together with the carbon atom attached thereto form a substituted or unsubstituted C₃₋₈ monocyclic cycloalkyl, or substituted or unsubstituted C₃₋₆ monocyclic heterocyclyl;
(3) R₁₁, R₁₂, R₂₁, R₂₂, R₄₁, R₄₂ are each independently hydrogen, halo, hydroxy, hydroxymethyl, hydroxyethyl or C₁₋₃ alkyl;
R₃₁, R₃₂ together with the carbon atom attached thereto form a substituted or unsubstituted C₃₋₈ monocyclic cycloalkyl, or substituted or unsubstituted C₃₋₆ monocyclic heterocyclyl;
(4) R₁₁, R₁₂, R₂₂, R₃₂, R₄₁, R₄₂ are each independently hydrogen, halo, hydroxy, hydroxymethyl, hydroxyethyl or C₁₋₃ alkyl;
R₂₁, R₃₁ together with the carbon atom attached thereto form a substituted or unsubstituted C₃₋₈ monocyclic cycloalkyl, or substituted or unsubstituted C₃₋₆ monocyclic heterocyclyl;
(5) R₁₂, R₂₁, R₂₂, R₃₂, R₄₁, R₄₂ are each independently hydrogen, halo, hydroxy, hydroxymethyl, hydroxyethyl or C₁₋₃ alkyl;
R₁₁, R₃₁ are linked together to form a substituted or unsubstituted C₃₋₈ monocyclic cycloalkyl, or substituted or unsubstituted C₃₋₆ monocyclic heterocyclyl; 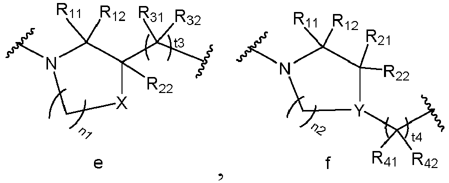 wherein in the formula (e) or formula (f), t3, t4 are each independently 0 or 1; R₁₁, R₁₂, R₂₁, R₂₂, R₃₁, R₃₂, R₄₁, R₄₂ are each independently hydrogen, halo, hydroxy, hydroxymethyl, hydroxyethyl or C₁₋₃ alkyl;
X is NRₓ₁, O or CRₓ₂Rₓ₃;
Y is N or CR_{y};
Rₓ₁, Rₓ₂, Rₓ₃, R_{y} are each independently hydrogen or C₁₋₃ alkyl;
n1, n2 are each independently 1, 2 or 3.

4. The compound or a pharmaceutically acceptable salt, stereoisomer, solvate or prodrug thereof according to claim 1, **characterized in that**, the formula (a) has a structure selected from the following: 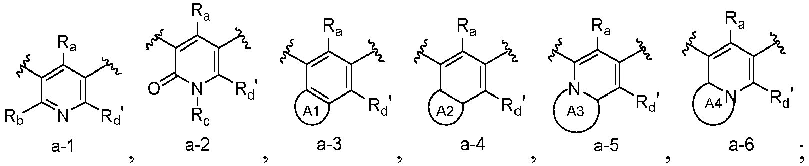
wherein the A1, A2 rings are each independently 5- or 6-membered monoheteroaryl selected from the group consisting of: thiophene, furan, thiazole, isothiazole, imidazole, oxazole, pyrrole, pyrazole, triazole, 1,2,3-triazole, 1,2,4-triazole, 1,2,5-triazole, 1,3,4-triazole, tetrazole, isoxazole, oxadiazole, 1,2,3-oxadiazole, 1,2,4-oxadiazole, 1,2,5-oxadiazole, 1,3,4-oxadiazole, thiadiazole, pyridine, pyridazine, pyrimidine or pyrazine; the 5- or 6-membered monoheteroaryl is unsubstituted or substituted by 1, 2 or 3 substituent(s) each independently selected from the group S;
the A3, A4 rings are each independently 5- or 6-membered monoheteroaryl selected from the group consisting of: imidazole, pyrrole, 2,3-dihydrothiazole, 2,3-dihydrooxazole, 2,3-dihydro-1H-imidazole, 2,5-dihydro-1H-imidazole, 1,2,4-triazole; the 5- or 6-membered monoheteroaryl is unsubstituted or substituted by 1, 2 or 3 substituent(s) each independently selected from the group S.

5. The compound or a pharmaceutically acceptable salt, stereoisomer, solvate or prodrug thereof according to claim 1, **characterized in that**, the formula (b) has a structure selected from the following: 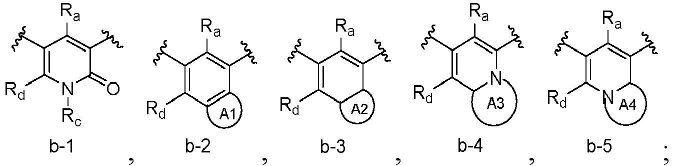
wherein the A1, A2 rings are each independently 5- or 6-membered monoheteroaryl selected from the group consisting of: thiophene, furan, thiazole, isothiazole, imidazole, oxazole, pyrrole, pyrazole, triazole, 1,2,3-triazole, 1,2,4-triazole, 1,2,5-triazole, 1,3,4-triazole, tetrazole, isoxazole, oxadiazole, 1,2,3-oxadiazole, 1,2,4-oxadiazole, 1,2,5-oxadiazole, 1,3,4-oxadiazole, thiadiazole, pyridine, pyridazine, pyrimidine or pyrazine; the 5- or 6-membered monoheteroaryl is unsubstituted or substituted by 1, 2 or 3 substituent(s) each independently selected from the group S;
the A3, A4 rings are each independently 5- or 6-membered monoheteroaryl selected from the group consisting of: imidazole, pyrrole, 2,3-dihydrothiazole, 2,3-dihydrooxazole, 2,3-dihydro-1H-imidazole, 2,5-dihydro-1H-imidazole, 1,2,4-triazole; the 5- or 6-membered monoheteroaryl is unsubstituted or substituted by 1, 2 or 3 substituent(s) each independenly selected from the group S.

6. The compound or a pharmaceutically acceptable salt, stereoisomer, solvate or prodrug thereof according to claim 1 or 2, **characterized in that**, -N(R₀₃)-L- has a structure selected from the group consisting of:

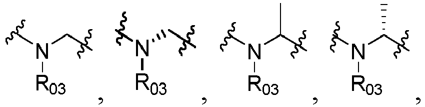

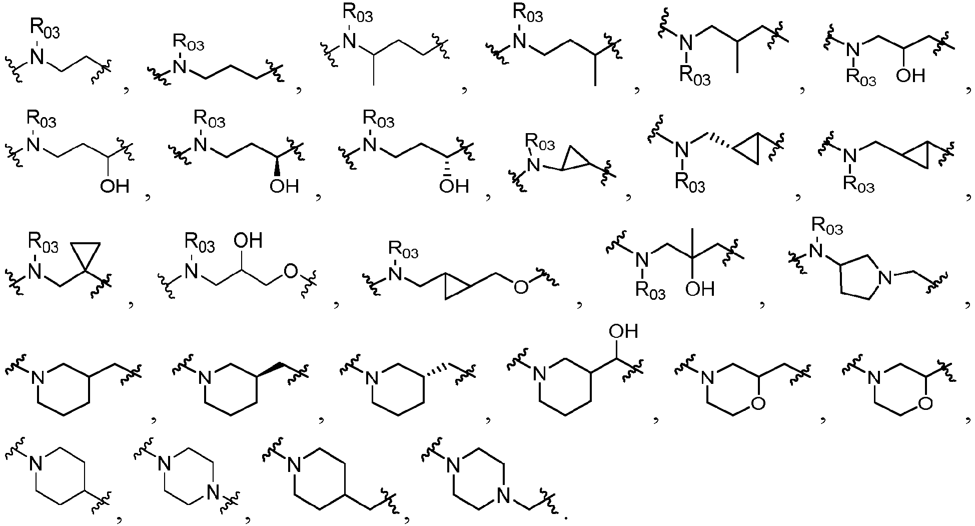

7. The compound or a pharmaceutically acceptable salt, stereoisomer, solvate or prodrug thereof according to claim 1, **characterized in that**, -A-C(O)-N(R₀₃)- has a structure represented by formula (g): 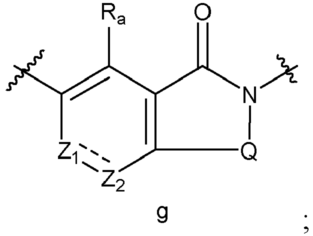
Q is -(CR_{q1}R_{q2})ₛ₁, -N(R_{q3})-(CR_{q1}R_{q2})ₛ₂, -O-(CR_{q1}R_{q2})ₛ₂, -(CR_{q1}R_{q2})-N(R_{q3})-(CR_{q3}R_{q4}), -(CR_{q1}R_{q2})-O-(CR_{q3}R_{q4}), -N=CR_{q5}-(CR_{q1}R_{q2})ₛ₃, -CR_{q5}=CR_{q6}-(CR_{q1}R_{q2})ₛ₃, - CR_{q5}=N-(CR_{q1}R_{q2})ₛ₃; wherein s1 is 0, 1, 2 or 3; s2 is 1 or 2; s3 is 0 or 1;
R_{q1}, R_{q2}, R_{q3}, R_{q4}, R_{q5}, R_{q6} are each independently hydrogen or C₁₋₃ alkyl;
Rₐ, Z₁, Z₂, " 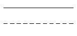 " are defined as in claim 1;
L is -(CR₁₁R₁₂)ₜ₁-(CR₂₁R₂₂)ₜ₂-(CR₃₁R₃₂)ₜ₃-(CR₄₁R₄₂)ₜ₄-(O)ₜ₅ or -(CR₁₁R₁₂)ₜ₁-(CR₂₁R₂₂)ₜ₂-(NR₃₁)ₜ₃-(CR₄₁R₄₂)ₜ₄-(O)ₜ₅; wherein t1, t2, t3, t4, t5 are each independently 0 or 1;
R₁₁, R₁₂, R₂₁, R₂₂, R₃₁, R₃₂, R₄₁, R₄₂ are selected from the combination selected from the group consisting of:
(1) R₁₁, R₁₂, R₂₁, R₂₂, R₃₁, R₃₂, R₄₁, R₄₂ are each independently hydrogen, halo, hydroxy, hydroxymethyl, hydroxyethyl or C₁₋₃ alkyl;
(2) R₁₁, R₁₂, R₃₁, R₃₂, R₄₁, R₄₂ are each independently hydrogen, halo, hydroxy, hydroxymethyl, hydroxyethyl or C₁₋₃ alkyl;
R₂₁, R₂₂ together with the carbon atom attached thereto form a substituted or unsubstituted C₃₋₈ monocyclic cycloalkyl, or substituted or unsubstituted C₃₋₆ monocyclic heterocyclyl;
(3) R₁₁, R₁₂, R₂₁, R₂₂, R₄₁, R₄₂ are each independently hydrogen, halo, hydroxy, hydroxymethyl, hydroxyethyl or C₁₋₃ alkyl;
R₃₁, R₃₂ together with the carbon atom attached thereto form a substituted or unsubstituted C₃₋₈ monocyclic cycloalkyl, or substituted or unsubstituted C₃₋₆ monocyclic heterocyclyl;
(4) R₁₁, R₁₂, R₂₂, R₃₂, R₄₁, R₄₂ are each independently hydrogen, halo, hydroxy, hydroxymethyl, hydroxyethyl or C₁₋₃ alkyl;
R₂₁, R₃₁ together with the carbon atom attached thereto form a substituted or unsubstituted C₃₋₈ monocyclic cycloalkyl, or substituted or unsubstituted C₃₋₆ monocyclic heterocyclyl;
(5) R₁₂, R₂₁, R₂₂, R₃₂, R₄₁, R₄₂ are each independently hydrogen, halo, hydroxy, hydroxymethyl, hydroxyethyl or C₁₋₃ alkyl;
R₁₁, R₃₁ are linked together to form a substituted or unsubstituted C₃₋₈ monocyclic cycloalkyl, or substituted or unsubstituted C₃₋₆ monocyclic heterocyclyl.

8. The compound or a pharmaceutically acceptable salt, stereoisomer, solvate or prodrug thereof according to claim 2, **characterized in that**, the fomula (II-a) has a structure selected from the following: 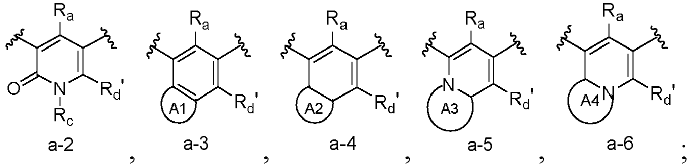
wherein the A1, A2 rings are each independently 5- or 6-membered monoheteroaryl selected from the group consisting of: thiophene, furan, thiazole, isothiazole, imidazole, oxazole, pyrrole, pyrazole, triazole, 1,2,3-triazole, 1,2,4-triazole, 1,2,5-triazole, 1,3,4-triazole, tetrazole, isoxazole, oxadiazole, 1,2,3-oxadiazole, 1,2,4-oxadiazole, 1,2,5-oxadiazole, 1,3,4-oxadiazole, thiadiazole, pyridine, pyridazine, pyrimidine or pyrazine; the 5- or 6-membered monoheteroaryl is unsubstituted or substituted by 1, 2 or 3 substituent(s) each independently selected from the group S;
the A3, A4 rings are each independently 5- or 6-membered monoheteroaryl selected from the group consisting of: imidazole, pyrrole, 2,3-dihydrothiazole, 2,3-dihydrooxazole, 2,3-dihydro-1H-imidazole, 2,5-dihydro-1H-imidazole, 1,2,4-triazole; the 5- or 6-membered monoheteroaryl is unsubstituted or substituted by 1, 2 or 3 substituent(s) each independently selected from the group S.

9. The compound or a pharmaceutically acceptable salt, stereoisomer, solvate or prodrug thereof according to claim 2, **characterized in that**, the fomula (II-b) has a structure selected from the following: 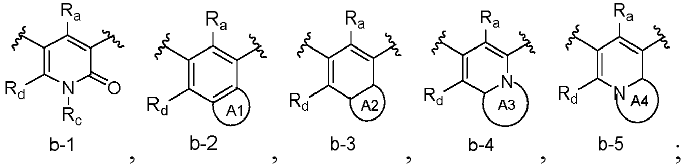
wherein the A1, A2 rings are each independently 5- or 6-membered monoheteroaryl selected from the group consisting of: thiophene, furan, thiazole, isothiazole, imidazole, oxazole, pyrrole, pyrazole, triazole, 1,2,3-triazole, 1,2,4-triazole, 1,2,5-triazole, 1,3,4-triazole, tetrazole, isoxazole, oxadiazole, 1,2,3-oxadiazole, 1,2,4-oxadiazole, 1,2,5-oxadiazole, 1,3,4-oxadiazole, thiadiazole, pyridine, pyridazine, pyrimidine or pyrazine; the 5- or 6-membered monoheteroaryl is unsubstituted or substituted by 1, 2 or 3 substituent(s) each independently selected from the group S;
the A3, A4 rings are each independently 5- or 6-membered monoheteroaryl selected from the group consisting of: imidazole, pyrrole, 2,3-dihydrothiazole, 2,3-dihydrooxazole, 2,3-dihydro-1H-imidazole, 2,5-dihydro-1H-imidazole, 1,2,4-triazole; the 5- or 6-membered monoheteroaryl is unsubstituted or substituted by 1, 2 or 3 substituent(s) each independently selected from the group S.

10. The compound or a pharmaceutically acceptable salt, stereoisomer, solvate or prodrug thereof according to claim 2, **characterized in that**, -B-C(O)-N(R₀₃)- has a structure represented by formula (II-g): 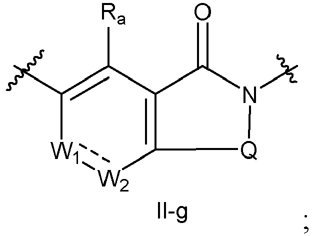
Q is -(CR_{q1}R_{q2})ₛ₁, -N(R_{q3})-(CR_{q1}R_{q2})ₛ₂, -O-(CR_{q1}R_{q2})ₛ₂, -(CR_{q1}R_{q2})-N(R_{q3})-(CR_{q3}R_{q4}), -(CR_{q1}R_{q2})-O-(CR_{q3}R_{q4}), -N=CR_{q5}-(CR_{q1}R_{q2})ₛ₃, -CR_{q5}=CR_{q6}-(CR_{q1}R_{q2})ₛ₃, - CR_{q5}=N-(CR_{q1}R_{q2})ₛ₃; wherein s1 is 0, 1, 2 or 3; s2 is 1 or 2; s3 is 0 or 1;
R_{q1}, R_{q2}, R_{q3}, R_{q4}, R_{q5}, R_{q6} are each independently hydrogen or C₁₋₃ alkyl;
Rₐ, W₁, W₂, " 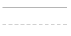 " are defined as in claim 2;
L is -(CR₁₁R₁₂)ₜ₁-(CR₂₁R₂₂)ₜ₂-(CR₃₁R₃₂)ₜ₃-(CR₄₁R₄₂)ₜ₄-(O)ₜ₅ or -(CR₁₁R₁₂)ₜ₁-(CR₂₁R₂₂)ₜ₂-(NR₃₁)ₜ₃-(CR₄₁R₄₂)ₜ₄-(O)ₜ₅; wherein t1, t2, t3, t4, t5 are each independently 0 or 1;
R₁₁, R₁₂, R₂₁, R₂₂, R₃₁, R₃₂, R₄₁, R₄₂ are selected from the combination selected from the group consisting of:
(1) R₁₁, R₁₂, R₂₁, R₂₂, R₃₁, R₃₂, R₄₁, R₄₂ are each independently hydrogen, halo, hydroxy, hydroxymethyl, hydroxyethyl or C₁₋₃ alkyl;
(2) R₁₁, R₁₂, R₃₁, R₃₂, R₄₁, R₄₂ are each independently hydrogen, halo, hydroxy, hydroxymethyl, hydroxyethyl or C₁₋₃ alkyl;
R₂₁, R₂₂ together with the carbon atom attached thereto from a substituted or unsubstituted C₃₋₈ monocyclic cycloalkyl, or substituted or unsubstituted C₃₋₆ monocyclic heterocyclyl;
(3) R₁₁, R₁₂, R₂₁, R₂₂, R₄₁, R₄₂ are each independently hydrogen, halo, hydroxy, hydroxymethyl, hydroxyethyl or C₁₋₃ alkyl;
R₃₁, R₃₂ together with the carbon atom attached thereto form a substituted or unsubstituted C₃₋₈ monocyclic cycloalkyl, or substituted or unsubstituted C₃₋₆ monocyclic heterocyclyl;
(4) R₁₁, R₁₂, R₂₂, R₃₂, R₄₁, R₄₂ are each independently hydrogen, halo, hydroxy, hydroxymethyl, hydroxyethyl or C₁₋₃ alkyl;
R₂₁, R₃₁ together with the carbon atom attached thereto form a substituted or unsubstituted C₃₋₈ monocyclic cycloalkyl, or substituted or unsubstituted C₃₋₆ monocyclic heterocyclyl;
(5) R₁₂, R₂₁, R₂₂, R₃₂, R₄₁, R₄₂ are each independently hydrogen, halo, hydroxy, hydroxymethyl, hydroxyethyl or C₁₋₃ alkyl;
R₁₁, R₃₁ are linked together to form a substituted or unsubstituted C₃₋₈ monocyclic cycloalkyl, or substituted or unsubstituted C₃₋₆ monocyclic heterocyclyl.

11. The compound or a pharmaceutically acceptable salt, stereoisomer, solvate or prodrug thereof according to claim 1 or 2, **characterized in that**, the C₆₋₁₄ aryl in R₀ is phenyl, naphthyl, or a 9- or 10-membered aromatic fused bicyclic ring formed by fusing a phenyl to one non-aromatic ring, the non-aromatic ring is 3- to 6-membered saturated or partially unsaturated monocyclic heterocyclyl, or 3- to 6-membered saturated or partially unsaturated monocyclic cycloalkyl, wherein
the 3- to 6-membered saturated or partially unsaturated monocyclic heterocyclyl is selected from the group consisting of: aziridine, oxirane, azetidine, azetidin-2-one, oxetane, oxetan-2-one, oxazolidine, pyrrolidin-2-one, pyrrolidin-2,5-dione, 1,3-dioxolane, dihydrofuran-2(3H)-one, dihydrofuran-2,5-dione, piperidin-2-one, piperidin-2,6-dione, tetrahyro-2H-pyran-2-one, imidazolidine, tetrahydrofuran, tetrahydrothiophene, tetrahydropyrrole, 1,3-dioxolan-2-one, oxazolidin-2-one, imidazolidine-2-one, piperidine, piperazine, piperazin-2-one, morpholine, morpholin-3-one, morpholin-2-one, thiomorpholin-3-one 1,1-dioxide, thiomorpholine, thiomorpholine-1,1-dioxide, tetrahydropyran, 1,2-dihydroazacyclobutadiene, 1,2-dihydrooxetadiene, 2,5-dihydro-1H-pyrrole, 2,5-dihydrofuran, 2,3-dihydrofuran, 2,3-dihydro-1H-pyrrole, 3,4-dihydro-2H-pyran, 1,2,3,4-tetrahydropyridine, 3,6-dihydron-2H-pyran, 1,2,3,6-tetrahydropyridine, 1,3-oxazinane, hexahydropyrimidine, 1,4-dioxane, tetrahydropyrimidin-2(1H)-one, 1,4-dioxan-2-one, 5,6-dihydro-2H-pyran-2-one;
the 3- to 6-membered saturated or partially unsaturated monocyclic cycloalkyl is selected from the group consisting of: cyclopropyl, cyclobutyl, cyclopentyl, cyclopentenyl, cyclohexyl, cyclohexenyl, cyclohexdienyl, cyclobutanone, cyclobutan-1,2-dione, cyclopentanone, cyclopentan-1,3-dione, cyclohexanone, cyclohexan-1,3-dione;
the 9- or 10-membered aromatic fused bicyclic ring is unsubstituted or substituted by 1, 2, 3 or 4 substituent(s) each independently selected from the group S.

12. The compound or a pharmaceutically acceptable salt, stereoisomer, solvate or prodrug thereof according to claim 1 or 2, **characterized in that**, the C₅₋₁₄ heteroaryl in R₀ is a 5- or 6-membered monoheteroaryl, wherein the 5- or 6-membered monoheteroaryl is selected from the group consisting of: thiophene, furan, thiazole, isothiazole, imidazole, oxazole, pyrrole, pyrazole, triazole, 1,2,3-triazole, 1,2,4-triazole, 1,2,5-triazole, 1,3,4-triazole, tetrazole, isoxazole, oxadiazole, 1,2,3-oxadiazole, 1,2,4-oxadiazole, 1,2,5-oxadiazole, 1,3,4-oxadiazole, thiadiazole, pyridine, pyridazine, pyrimidine or pyrazine; the 5- or 6-membered monoheteroaryl is unsubstituted or substituted by 1, 2, 3 or 4 substituent(s) each independently selected from the group S.

13. The compound or a pharmaceutically acceptable salt, stereoisomer, solvate or prodrug thereof according to claim 1 or 2, **characterized in that**, the C₅₋₁₄ heteroaryl in R₀ is a 9- or 10-membered biheteroaryl formed by fusing a phenyl to a 5- or 6-membered monoheteroaryl, wherein the 5- or 6-membered monoheteroaryl is selected from the group consisting of: thiophene, furan, thiazole, isothiazole, imidazole, oxazole, pyrrole, pyrazole, triazole, 1,2,3-triazole, 1,2,4-triazole, 1,2,5-triazole, 1,3,4-triazole, tetrazole, isoxazole, oxadiazole, 1,2,3-oxadiazole, 1,2,4-oxadiazole, 1,2,5-oxadiazole, 1,3,4-oxadiazole, thiadiazole, pyridine, pyridazine, pyrimidine or pyrazine; the 9- or 10-membered biheteroaryl is unsubstituted or substituted by 1, 2, 3 or 4 substituent(s) each independently selected from the group S.

14. The compound or a pharmaceutically acceptable salt, stereoisomer, solvate or prodrug thereof according to claim 1 or 2, **characterized in that**, the C₅₋₁₄ heteroaryl in R₀ is a 8- to 10-membered biheteroaryl formed by fusing a 5- or 6-membered monoheteroaryl to a 5- or 6-membered monoheteroaryl, wherein the 5- or 6-membered monoheteroaryl is selected from the group consisting of: thiophene, furan, thiazole, isothiazole, imidazole, oxazole, pyrrole, pyrazole, triazole, 1,2,3-triazole, 1,2,4-triazole, 1,2,5-triazole, 1,3,4-triazole, tetrazole, isoxazole, oxadiazole, 1,2,3-oxadiazole, 1,2,4-oxadiazole, 1,2,5-oxadiazole, 1,3,4-oxadiazole, thiadiazole, pyridine, pyridazine, pyrimidine or pyrazine; the 8- to 10-membered biheteroaryl is unsubstituted or substituted by 1, 2, 3 or 4 substituent(s) each independently selected from the group S.

15. The compound or a pharmaceutically acceptable salt, stereoisomer, solvate or prodrug thereof according to claim 1 or 2, **characterized in that**, the R₀ is a substituted or unsubstituted phenyl or a substituted or unsubstituted pyridyl, the "substituted" means that 1, 2, 3 or 4 hydrogen atom(s) in the group are substituted by a substituent each independently selected from the group S.

16. The compound or a pharmaceutically acceptable salt, stereoisomer, solvate or prodrug thereof according to claim 1 or 2, **characterized in that**, the R₀ has a structure selected from the following:

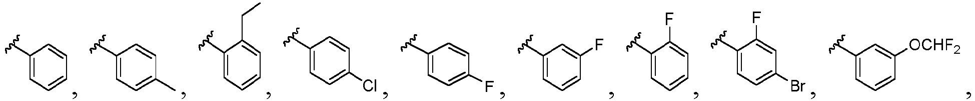

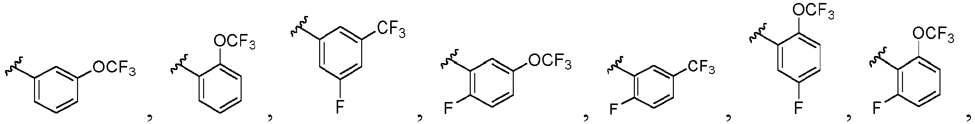

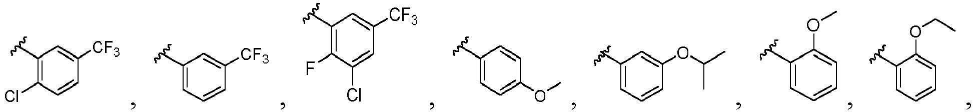

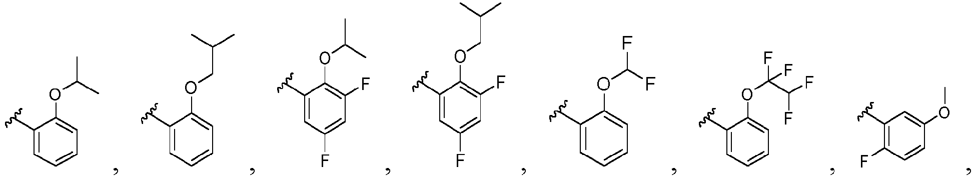

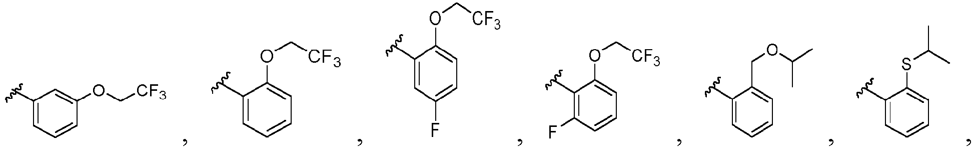

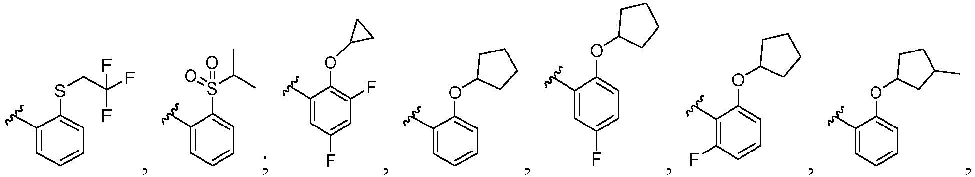

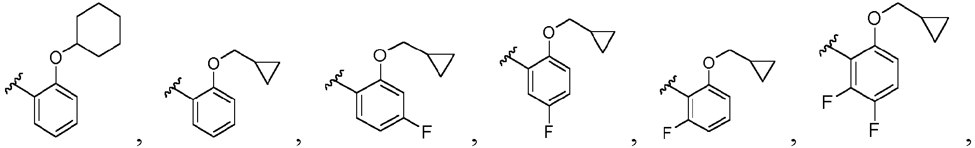

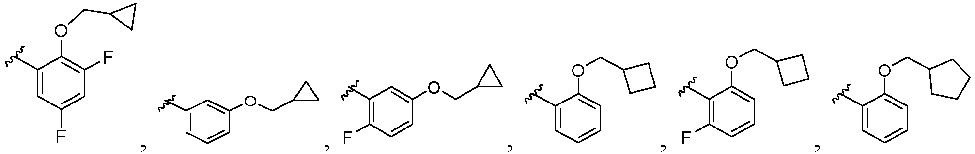

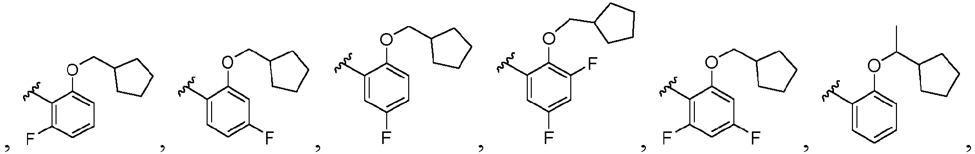

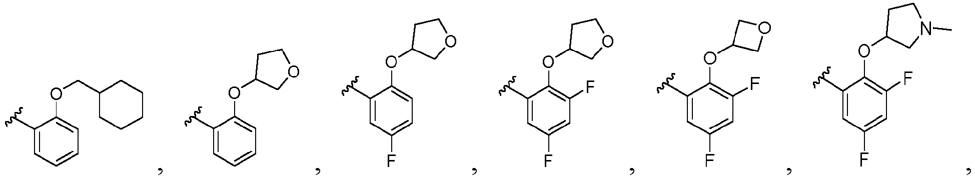

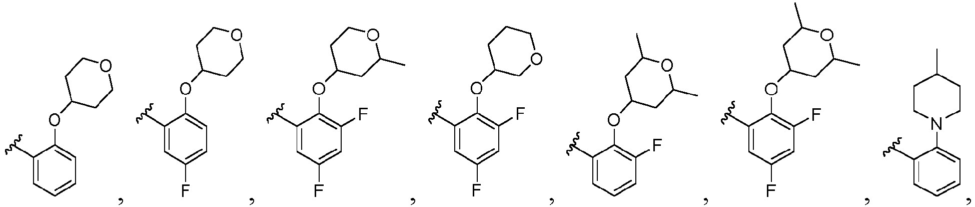

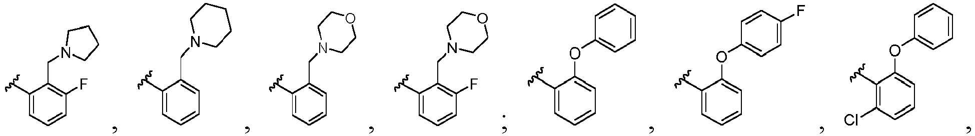

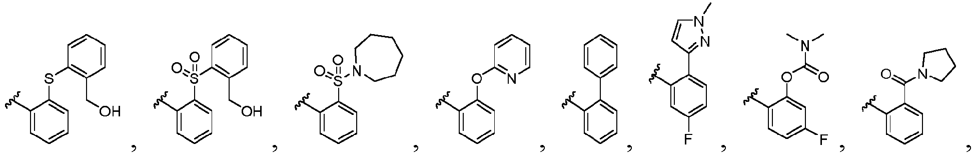

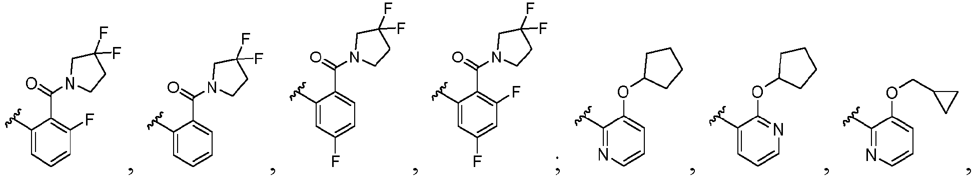

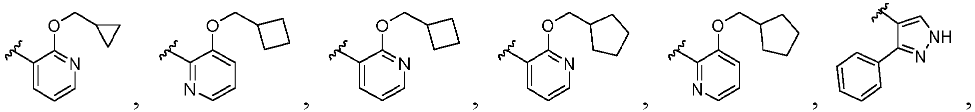

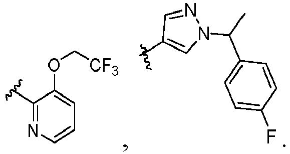

17. The compound or a pharmaceutically acceptable salt, stereoisomer, solvate or prodrug thereof according to claim 1, **characterized in that**, the compound of formula (I) is selected from the group consisting of:

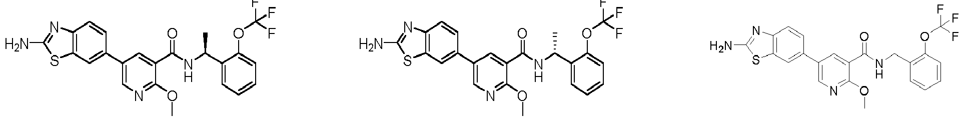

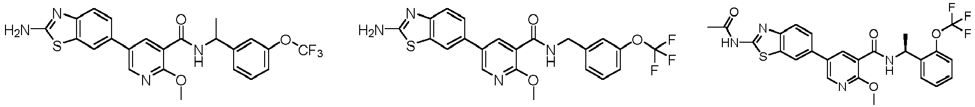

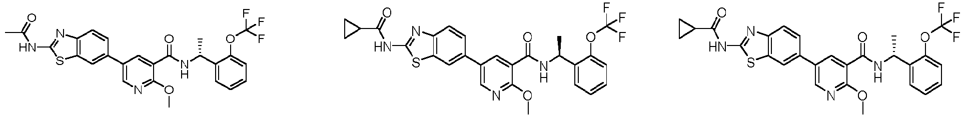

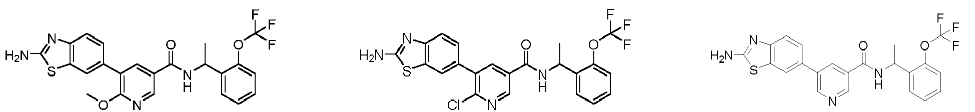

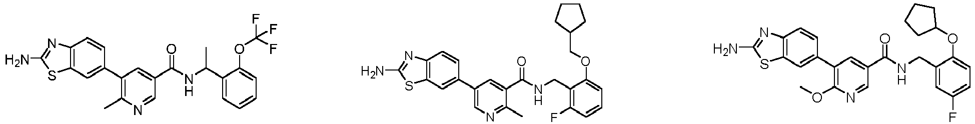

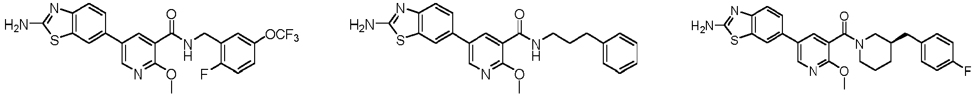

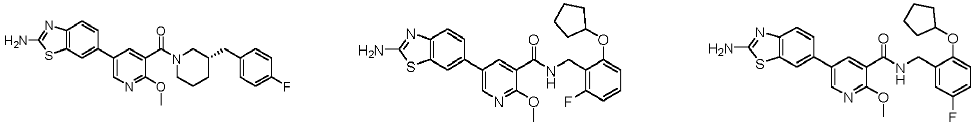

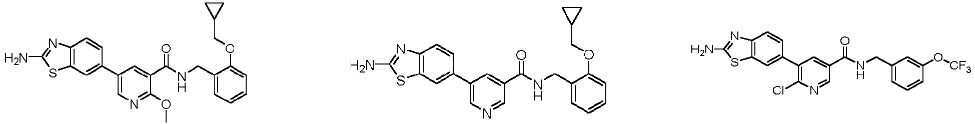

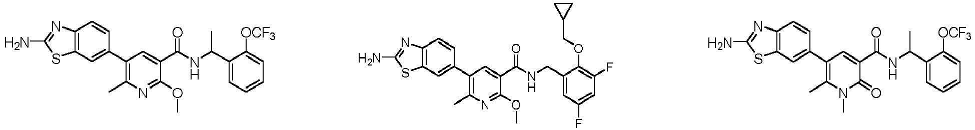

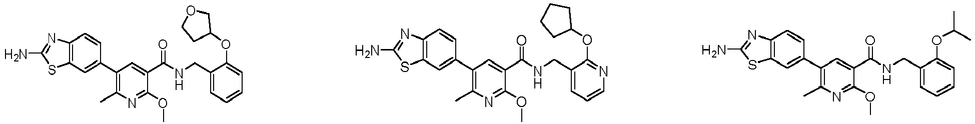

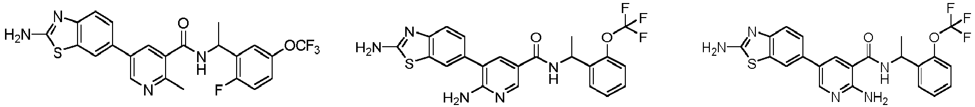

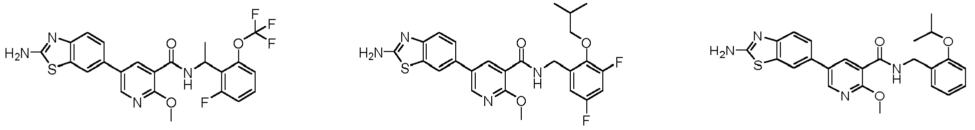

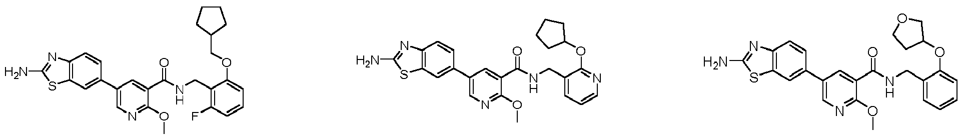

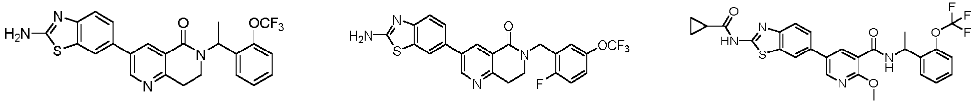

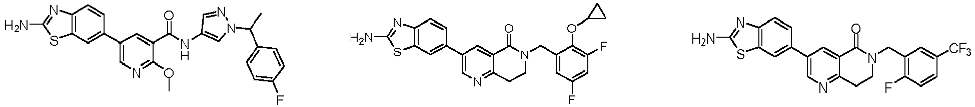

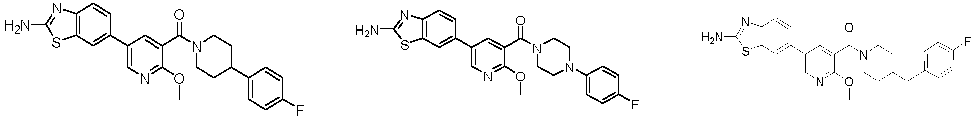

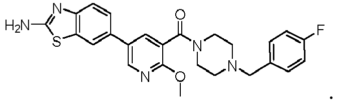

18. The compound or a pharmaceutically acceptable salt, stereoisomer, solvate or prodrug thereof according to claim 2, **characterized in that**, the compound of formula (II) is selected from the group consisting of:

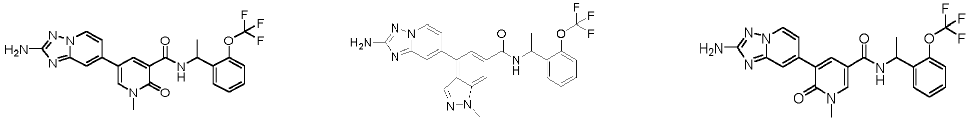

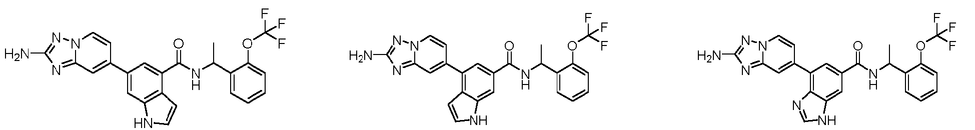

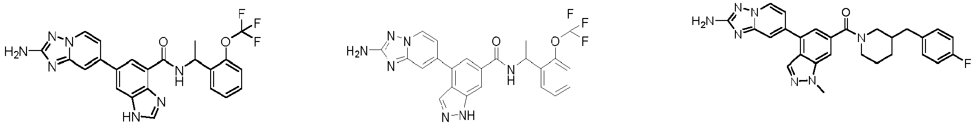

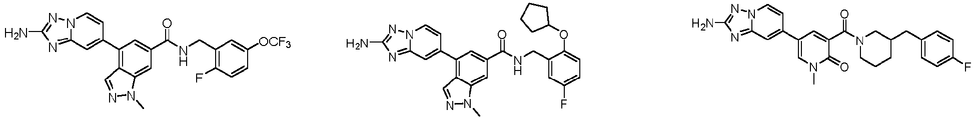

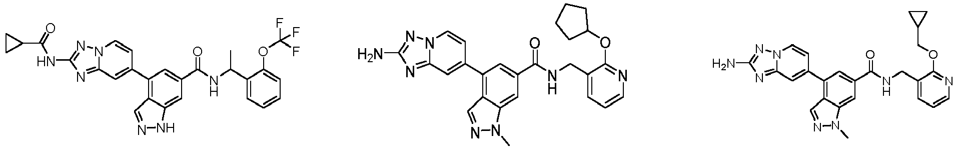

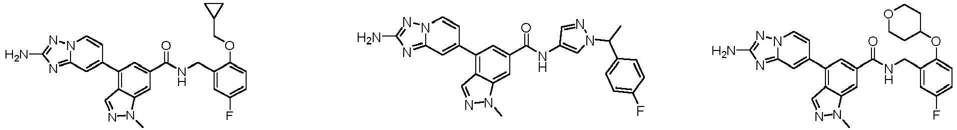

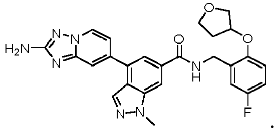

19. A pharmaceutical composition, comprising the compound or a pharmaceutically acceptable salt, stereoisomer, solvate or prodrug thereof according to any one of claims 1-18; and a pharmaceutically acceptable carrier.

20. Use of the compound or a pharmaceutically acceptable salt, stereoisomer, solvate or prodrug thereof according to any one of claims 1-18, or the pharmaceutical composition according to claim 19 in the preparation of a medicament for preventing and/or treating a disease selected from the group consisting of: inflammatory bowel disease, ulcerative colitis, Crohn's disease, psoriasis, rheumatoid arthritis, NASH and heart failure.

21. Use of the compound or a pharmaceutically acceptable salt, stereoisomer, solvate or prodrug thereof according to any one of claims 1-18, or the pharmaceutical composition according to claim 19 in the preparation of a selective RIPK1 inhibitor, the selective RIPK1 inhibitor is useful in treating a RIPK1-related disease or disorder.
